(19) Europäisches Patentamt
European Patent Office
Office européen des brevets

(11) EP 4 364 570 A1

(12) EUROPEAN PATENT APPLICATION
published in accordance with Art. 153(4) EPC

(43) Date of publication:
**08.05.2024 Bulletin 2024/19**

(21) Application number: **22833162.5**

(22) Date of filing: **28.06.2022**

(51) International Patent Classification (IPC):
***A01N 43/836*** (2006.01) ***A01M 1/20*** (2006.01)
***A01P 7/02*** (2006.01) ***A01P 7/04*** (2006.01)
***C07D 401/04*** (2006.01) ***C07D 413/04*** (2006.01)

(52) Cooperative Patent Classification (CPC):
**A01M 1/20; A01N 43/82; A01P 7/02; A01P 7/04; C07D 401/04; C07D 413/04**

(86) International application number:
**PCT/JP2022/025760**

(87) International publication number:
**WO 2023/277015 (05.01.2023 Gazette 2023/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.06.2021 JP 2021107328**

(71) Applicant: **Sumitomo Chemical Company Limited**
**Tokyo 103-6020 (JP)**

(72) Inventors:
- **SHIODA, Takayuki**
  **Takarazuka-shi, Hyogo 665-8555 (JP)**
- **TASHIRO, Masayuki**
  **Takarazuka-shi, Hyogo 665-8555 (JP)**
- **MINEGISHI, Hidemitsu**
  **Takarazuka-shi, Hyogo 665-8555 (JP)**
- **TAMASHIMA, Hiroto**
  **Takarazuka-shi, Hyogo 665-8555 (JP)**
- **SAITO, Yasumasa**
  **Tokyo 103-6020 (JP)**

(74) Representative: **Vossius & Partner**
**Patentanwälte Rechtsanwälte mbB**
**Siebertstrasse 3**
**81675 München (DE)**

(54) **HETEROCYCLIC COMPOUND AND RESISTANT HARMFUL ARTHROPOD-CONTROLLING METHOD FOR COMPOSITION CONTAINING SAME**

(57) The present invention provides a compound that has an excellent efficacy for controlling resistant harmful arthropods, a composition comprising the compound, and a method using the compound or the composition. A compound represented by formula (1) [wherein $R^1$ represents a C1-C6 chain hydrocarbon group, etc., $R^3$ and $R^7$ each represents a hydrogen atom, etc., $R^4$ and $R^6$ each represents a hydrogen atom, etc., $R^5$ represent a hydrogen atom, etc., Q represents a group represented by Q1, etc. (where # represents a binding site to a pyridine ring, and • represents a binding site to a benzene ring), $A^1$ represents a nitrogen atom, etc., $A^2$ represents a nitrogen atom, etc., and $A^3$ represents a nitrogen atom, etc.]
or its N-oxide, or salts thereof has an excellent efficacy on controlling resistant harmful arthropods.

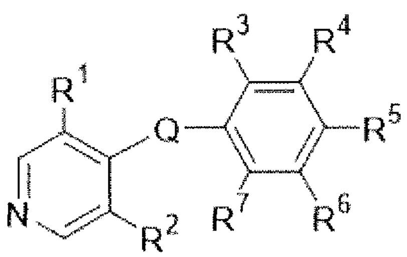

(1)

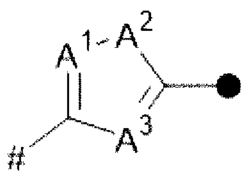

Q1

EP 4 364 570 A1

## Description

TECHNICAL FIELD

This application claims priority to and the benefit of Japanese Patent Application No. 2021-107328 filed June 29, 2021, the entire contents of which are incorporated herein by reference.

The present invention relates to a method for controlling resistant harmful arthropods using a heterocyclic compound and a composition comprising the same. The present invention relates to also a heterocyclic compound and a composition for controlling resistant harmful arthropods comprising the same.

BACKGROUND ART

To date, in order to control harmful arthropods, some compounds have been studied. For example, a certain class of compound has been known to have an effect on controlling pests (see Patent Document 1).

CITATION LIST

PATENT DOCUMENT

Patent Document 1: JP 2002-114783 A

SUMMARY OF THE INVENTION

(PROBLEMS TO BE SOLVED BY INVENTION)

An object of the present invention is to provide a method for controlling resistant harmful arthropods using a heterocyclic compound or a composition comprising the same. An object of the present invention is also to provide a heterocyclic compound having an excellent efficacy for controlling resistant harmful arthropods.

(MEANS TO SOLVE PROBLEMS)

The present inventors have intensively studied to find a method showing an excellent efficacy for controlling resistant harmful arthropods, and as a result, found that a compound represented by the below-mentioned formula (I) as well as a composition comprising the same compound have an excellent efficacy for controlling resistant harmful arthropods.

That is, the present invention includes the followings.

[1] A method for controlling a resistant harmful arthropod which comprises applying an effective amount of a compound represented by formula (I):

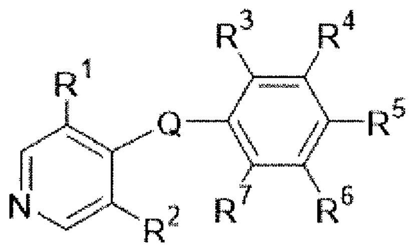

(I)

[wherein

$R^1$ represents a C1-C6 chain hydrocarbon group, a C1-C6 alkoxy group {the C1-C6 chain hydrocarbon group and the C1-C6 alkoxy group may be optionally substituted with one or more substituents selected from Group A}, a C3-C7 cycloalkyl group which may be optionally substituted with one or more substituents selected from Group B, a C3-C7 cycloalkenyl group, a phenyl group, a three- to seven- membered nonaromatic heterocyclic group, a five- or six- membered aromatic heterocyclic group {the C3-C7 cycloalkenyl group, the phenyl group, the three- to seven- membered nonaromatic heterocyclic group, and the five- or six- membered aromatic heterocyclic group may be optionally substituted with one or more substituents selected from Group C}, a nitro

group, $NR^8R^9$, $S(0)_mR^{10}$, or a halogen atom,
m is 0, 1 or 2,
$R^2$ represents a C1-C6 chain hydrocarbon group, a C1-C6 alkoxy group {the C1-C6 chain hydrocarbon group and the C1-C6 alkoxy group may be optionally substituted with one or more substituents selected from Group A}, a C3-C7 cycloalkyl group which may be optionally substituted with one or more substituents selected from Group B, a C3-C7 cycloalkenyl group, a phenyl group, a three- to seven- membered nonaromatic heterocyclic group, a five- or six- membered aromatic heterocyclic group {the C3-C7 cycloalkenyl group, the phenyl group, the three- to seven- membered nonaromatic heterocyclic group, and the five- or six- membered aromatic heterocyclic group may be optionally substituted with one or more substituents selected from Group C}, a nitro group, $NR^{8a}R^{9a}$, $S(O)_nR^{10a}$, a halogen atom, or a hydrogen atom,
n is 0, 1 or 2,
$R^3$ and $R^7$ are identical or different from each other and each represents a C1-C6 chain hydrocarbon group which may be optionally substituted with one or more halogen atoms, a halogen atom, or a hydrogen atom,
$R^4$ and $R^6$ are identical or different from each other and each represents a C1-C6 chain hydrocarbon group, a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group, a C3-C6 cycloalkyl group, a phenyl group, a five- or six-membered aromatic heterocyclic group {the C1-C6 chain hydrocarbon group, the C1-C6 alkoxy group, the C3-C6 alkenyloxy group, the C3-C6 alkynyloxy group, the C1-C6 alkylsulfanyl group, the C1-C6 alkylsulfinyl group, the C1-C6 alkylsulfonyl group, the C3-C6 cycloalkyl group, the phenyl group, and the five- or six-membered aromatic heterocyclic group may be optionally substituted with one or more halogen atoms}, a cyano group, a nitro group, a halogen atom, or a hydrogen atom,
$R^5$ represents a C1-C6 chain hydrocarbon group, a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C3-C6 alkenyloxy group, the C3-C6 alkynyloxy group, the C1-C6 alkylsulfanyl group, the C1-C6 alkylsulfinyl group, and the C1-C6 alkylsulfonyl group may be optionally substituted with one or more halogen atoms}, a nitro group, a halogen atom, or a hydrogen atom,
$R^8$ and $R^{8a}$ are identical or different from each other and each represents a C1-C6 chain hydrocarbon group which may be optionally substituted with one or more halogen atoms, or a hydrogen atom,
$R^9$ and $R^{9a}$ are identical or different from each other and each represents a C1-C6 chain hydrocarbon group, a C3-C7 cycloalkyl group, a C3-C7 cycloalkenyl group {the C1-C6 chain hydrocarbon group, the C3-C7 cycloalkyl group and the C3-C7 cycloalkenyl group may be optionally substituted with one or more substituents selected from Group C}, or a hydrogen atom,
$R^{10}$ and $R^{10a}$ are identical or different from each other and each represents a C1-C6 chain hydrocarbon group which may be optionally substituted with one or more halogen atoms,
Q represents a group represented by Q1, a group represented by Q2, a group represented by Q3, or a group represented by Q4 (where # represents a binding site to a pyridine ring, and • represents a binding site to a benzene ring),

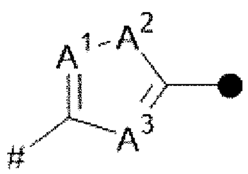
Q1

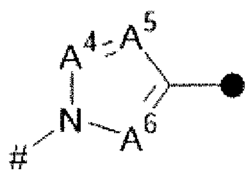
Q2

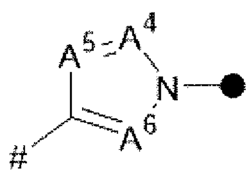
Q3

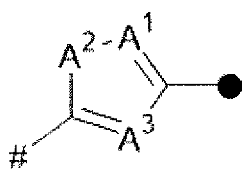
Q4

$A^1$ represents a nitrogen atom, or $CR^{11}$,
$A^2$ represents an oxygen atom, a sulfur atom, or $NR^{12}$,
$A^3$ represents a nitrogen atom, or $CR^{13}$,
$A^4$ represents a nitrogen atom, or $CR^{14}$,
$A^5$ represents a nitrogen atom, or $CR^{15}$,
$A^6$ represents a nitrogen atom, or $CR^{16}$,
$R^{11}$, $R^{13}$, $R^{14}$, $R^{15}$, and $R^{16}$ are identical or different from each other and each represents a C1-C6 chain hydrocarbon group which may be optionally substituted with one or more halogen atoms, a halogen atom, or a hydrogen atom,
$R^{12}$ represents a C1-C6 chain hydrocarbon group which may be optionally substituted with one or more halogen atoms, or a hydrogen atom.

Group A is a group consisting of a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group, a C3-C6 cycloalkyl group {the C1-C6 alkoxy group, the C3-C6 alkenyloxy group, the C3-C6 alkynyloxy group, the C1-C6 alkylsulfanyl group, the C1-C6 alkylsulfinyl group, the C1-C6 alkylsulfonyl group, and the C3-C6 cycloalkyl group may be optionally substituted with one or more halogen atoms), a cyano group, and a halogen atom.

Group B is a group consisting of a C1-C6 chain hydrocarbon group, a C1-C6 alkoxy group, a C3-C6 alkenyloxy group, a C3-C6 alkynyloxy group {the C1-C6 chain hydrocarbon group, the C1-C6 alkoxy group, the C3-C6 alkenyloxy group, and the C3-C6 alkynyloxy group may be optionally substituted with one or more halogen atoms), a hydroxy group, a cyano group, a nitro group, and a halogen atom.

Group C is a group consisting of a C1-C6 alkyl group which may be optionally substituted with one or more halogen atoms, a cyano group, and a halogen atom.]

(hereinafter, the compound represented by formula (I) is referred to as "Present compound N"), or its N-oxide or a salt thereof (hereinafter, the compound represented by formula (I), or its N-oxide or a salt thereof are referred to as "Present compound")

to the resistant harmful arthropod or a habitat of the resistant harmful arthropod.

[2] The method according to [1] wherein in the compound represented by formula (I), or its N-oxide or a salt thereof,

Q represents a group represented by formula Q1 or a group represented by formula Q4,

$A^1$ and $A^3$ represents a nitrogen atom,

$A^2$ represents an oxygen atom or $NR^{12}$,

$R^1$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_mR^{10}$, or a halogen atom,

$R^2$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_nR^{10a}$, a halogen atom, or a hydrogen atom.

[3] The method according to [1] or [2] wherein in the compound represented by formula (I), or its N-oxide or a salt thereof,

Q represents a group represented by formula Q1.

[4] The method according to [1] wherein in the compound represented by formula (1), or its N-oxide or a salt thereof,

Q represents a group represented by formula Q2 or a group represented by formula Q3,

$A^4$ represents $CR^{14}$,

$A^5$ represents a nitrogen atom or $CR^{15}$,

$A^6$ represents a nitrogen atom,

$R^1$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_mR^{10}$, or a halogen atom,

$R^2$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_nR^{10a}$, a halogen atom, or a hydrogen atom.

[5] The method according to [1] or [4] wherein in the compound represented by formula (I), or its N-oxide or a salt thereof,

Q represents a group represented by formula Q2.

[6] A compound represented by formula (II):

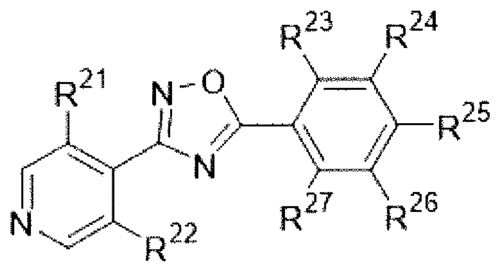

( II )

[wherein

$R^{21}$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, $S(O)_pR^{28}$ or a halogen atom,
$R^{22}$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, $S(O)_qR^{29}$, a halogen atom, or a hydrogen atom,
$R^{23}$, $R^{25}$ and $R^{27}$ are identical or different from each other and each represents a fluorine atom or a hydrogen atom,
$R^{24}$ represents a C1-C6 alkoxy group which is substituted with one or more halogen atoms, or a fluorine atom,
$R^{26}$ represents a halogen atom, or a hydrogen atom,
$R^{28}$ and $R^{29}$ are identical to or different from each other and each represents a C1-C6 chain hydrocarbon group which may be optionally substituted with one or more halogen atoms, and
p and q each independently is 0, 1 or 2]

or its N-oxide or a salt thereof (hereinafter, a compound represented by formula (II), its N-oxide or a salt thereof referred to as "Compound AA of the present invention").
[7] The compound according to [6] wherein

$R^{21}$ represents a C1-C6 chain hydrocarbon group which may be optionally substituted with one or more halogen atoms, or a halogen atom,
$R^{22}$ represents a C1-C6 chain hydrocarbon group which may be optionally substituted with one or more halogen atoms, a halogen atom, or a hydrogen atom,
$R^{23}$, $R^{25}$ and $R^{27}$ are identical or different from each other and each represents a fluorine atom or a hydrogen atom,
$R^{24}$ represents a C1-C6 alkoxy group which is substituted with one or more halogen atoms, or a fluorine atom, and
$R^{26}$ represents a halogen atom, or a hydrogen atom,

or its N-oxide, or a salt thereof
(hereinafter, the compound according to [7], its N-oxide or a salt thereof is referred to as "Compound A of the present invention").

[8] A compound represented by formula (II-a):

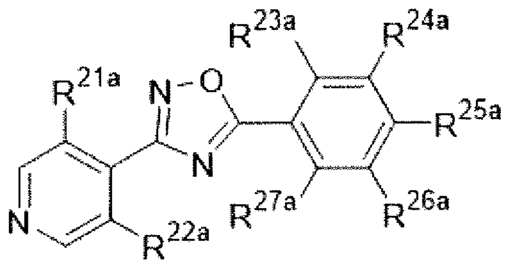
( II-a )

[wherein

$R^{21a}$ represents a C1-C6 alkoxy group, a C3-C7 cycloalkyl group {the C1-C6 alkoxy group, and the C3-C7 cycloalkyl group may be optionally substituted with one or more halogen atoms}, or $S(O)_rR^{28a}$,
$R^{22a}$ represents a C1-C6 alkoxy group, a C3-C7 cycloalkyl group {the C1-C6 alkoxy group, and the C3-C7 cycloalkyl group may be optionally substituted with one or more halogen atoms}, $S(O)_sR^{29a}$ or a halogen atom,
$R^{23a}$, $R^{25a}$ and $R^{27a}$ are identical or different from each other and each represents a halogen atom or a hydrogen atom,
$R^{24a}$ represents a C1-C6 chain hydrocarbon group which may be optionally substituted with one or more halogen atoms, a C1-C6 alkoxy group which is substituted with one or more halogen atoms, or a halogen atom,
$R^{26a}$ represents a C1-C6 chain hydrocarbon group which may be optionally substituted with one or more halogen atoms, a halogen atom, or a hydrogen atom,
$R^{28a}$ and $R^{29a}$ are identical or different from each other and each represents a C1-C6 chain hydrocarbon group

which may be optionally substituted with one or more halogen atoms, and
r and s each independently represents 0, 1 or 2], its N-oxide or a salt thereof

(hereinafter, the compound represented by formula (II-a), its N-oxide or a salt thereof are referred to as "Compound A2 of the present invention").

[9] A compound represented by formula (III):

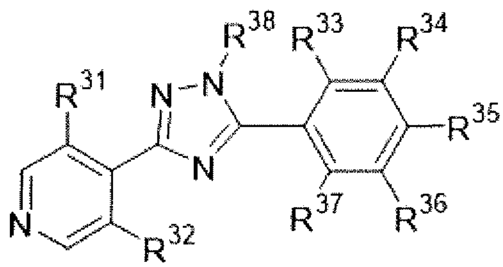

( III )

[wherein

a combination of $R^{31}$, $R^{32}$, $R^{33}$, $R^{34}$, $R^{35}$, $R^{36}$, and $R^{37}$ represents

a combination wherein
$R^{31}$ and $R^{32}$ represent a chlorine atom, and $R^{33}$, $R^{35}$ and $R^{37}$ represent a hydrogen atom,
$R^{34}$ represents a C1-C6 alkoxy group which is substituted with one or more halogen atoms, and
$R^{36}$ represents a halogen atom; or,
a combination wherein
$R^{31}$ represents a C1-C6 chain hydrocarbon group which may be optionally substituted with one or more halogen atoms or a bromine atom,
$R^{32}$ represents a C1-C6 chain hydrocarbon group which may be optionally substituted with one or more halogen atoms, a bromine atom, or a hydrogen atom,
$R^{33}$, $R^{35}$ and $R^{37}$ are identical or different from each other and each represents a fluorine atom or a hydrogen atom,
$R^{34}$ represents a C1-C6 chain hydrocarbon group which may be optionally substituted with one or more halogen atoms, a C1-C6 alkoxy group which may be optionally substituted with one or more halogen atoms, or a halogen atom, and
$R^{36}$ represents a halogen atom or a hydrogen atom, and

$R^{38}$ represents a C1-C6 chain hydrocarbon group which may be optionally substituted with one or more halogen atoms, or a hydrogen atom],

or its N-oxide, or a salt thereof
(hereinafter, the compound represented by formula (III), its N-oxide or a salt thereof is referred to as "Compound B of the present invention").

[10] A compound represented by formula (IV):

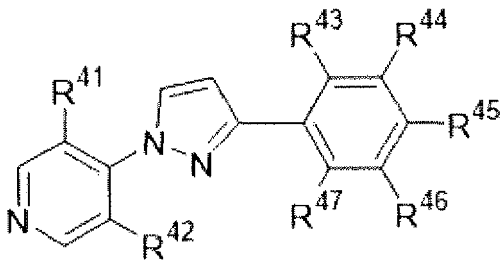

( IV )

[wherein

$R^{41}$ represents a C1-C6 chain hydrocarbon group which may be optionally substituted with one or more halogen atoms, or a halogen atom,
$R^{12}$ represents a C1-C6 chain hydrocarbon group which may be optionally substituted with one or more halogen atoms, a halogen atom, or a hydrogen atom,
$R^{43}$, $R^{45}$ and $R^{47}$ are identical or different from each other and each represents a fluorine atom or a hydrogen atom,
$R^{44}$ represents a C1-C6 chain hydrocarbon group which may be optionally substituted with one or more halogen atoms, a C1-C6 alkoxy group which may be optionally substituted with one or more halogen atoms, a halogen atom, or a hydrogen atom, and
$R^{46}$ represents a halogen atom or a hydrogen atom]

, its N-oxide or a salt thereof
(hereinafter, a compound represented by formula (IV), its N-oxide or a salt thereof is referred to as "Compound C of the present invention").

[11] A composition comprising the compound according to any one of [6] to [10], its N-oxide or a salt thereof, and an inert carrier.
[12] A composition which comprises one or more ingredients selected from the group consisting of Group (a), Group (b), Group (c) and Group (d), as well as the compound according to any one of [6] to [10], or its N-oxide or a salt thereof:

Group (a): a group consisting of an insecticidal ingredient, a miticidal ingredient, and a nematicidal ingredient;
Group (b): a fungicidal ingredient;
Group (c): a plant growth modulating ingredient; and
Group (d): a repellent ingredient.

[13] A method for controlling a resistant harmful arthropod which comprises applying an effective amount of the compound according to any one of [6] to [10], its N-oxide or a salt thereof, or an effective amount of the composition according to [12] to the resistant harmful arthropod or a habitat where a resistant harmful arthropod lives.
[14] The method according to any one of [1] to [5] or [13] wherein the resistant harmful arthropod is the order of Hemiptera pest.
[15] The method according to any one [1] to [5] or [13] wherein the resistant harmful arthropod is selected from an Aphididae family, a Delphacidae family, or an Aleyrodidae family.
[16] A seed or vegetative reproduction organ carrying an effective amount of the compound according to any one of [6] to [10], its N-oxide or a salt thereof, or an effective amount of the composition according to [12].

[EFFECT OF INVENTION]

The present invention can control resistant harmful arthropods.

MODE FOR CARRYING OUT THE INVENTION

The substituents as used herein are explained as follows.
The term "halogen atom" represents a fluorine atom, a chlorine atom, a bromine atom, or an iodine atom.
When a substituent has two or more halogen atoms or substituents, the halogen atoms or the substituents may be identical to or different from each other.
The expression "CX-CY" as used herein represents that the number of carbon atoms is from X to Y. For example, the expression "C1-C6" represents that the number of carbon atoms is from 1 to 6.
The term "chain hydrocarbon group" represents an alkyl group, an alkenyl group, or an alkynyl group.
Examples of the "alkyl group" include methyl group, ethyl group, propyl group, isopropyl group, 1,1-dimethylpropyl group, 1,2-dimethylpropyl group, 1-ethylpropyl group, butyl group, sec-butyl group, tert-butyl group, pentyl group, and hexyl group.
Examples of the "alkenyl group" include vinyl group, 1-propenyl group, 2-propenyl group, 1-methyl-1-propenyl group, 1-methyl-2-propenyl group, 1,2-dimethy-l-propenyl group, 1-ethyl-2-propenyl group, 3-butenyl group, 4-pentenyl group, and 5-hexenyl group.
Examples of the "alkynyl group" include ethynyl group, 1-propynyl group, 2-propynyl group, 1-methyl-2-propynyl group, 1,1-dimethyl-2-propynyl group, 1-ethyl-2-propynyl group, 2-butynyl group, 4-pentynyl group, and 5-hexynyl group.
Examples of the "alkoxy group" include methoxy group, ethoxy group, propoxy group, isopropoxy group, butoxy group, tert-butoxy group, pentyloxy group, and hexyloxy group.

Examples of the "alkenyloxy group" include 2-propenyloxy group, 2-butenyloxy group, and 5-hexenyloxy group.
Examples of the "alkynyloxy group" include 2-propynyloxy group, 2-butynyloxy group, and 5-hexynyloxy group.
Examples of the "alkylsulfanyl group" include methylsulfanyl group, ethylsulfanyl group, isopropylsulfanyl group, and hexylsulfanyl group.
Examples of the "alkylsulfinyl group" include methylsulfinyl group, ethylsulfinyl group, isopropylsulfinyl group, and hexylsulfinyl group.
Examples of the "alkylsulfonyl group" include methylsulfonyl group, ethylsulfonyl group, isopropylsulfonyl group, and hexylsulfonyl group.
Examples of the "cycloalkyl group" include cyclopropyl group, cyclobutyl group, cyclopentyl group, cyclohexyl group, cycloheptyl group.
Examples of the "cycloalkenyl group" include cyclopropenyl group, cyclobutenyl group, cyclopentenyl group, cyclohexenyl group, and cycloheptenyl group.
Examples of the "three- to seven- membered nonaromatic heterocyclic group" include aziridinyl group, oxiranyl group, thiranyl group, azetidinyl group, oxetanyl group, thietanyl group, pyrrolidinyl group, tetrahydrofuranyl group, tetrahydrothienyl group, piperidyl group, pyranyl group, dihydropyranyl group, tetrahydropyranyl group, tetrahydrothiopyranyl group, azepanyl group, oxepanyl group, thiepanyl group, pyrazolynyl group, pyrazolidinyl group, imidazolinyl group, imidazolidinyl group, oxazolinyl group, thiazolinyl group, oxazolidinyl group, thiazolidinyl group, isoxazolinyl group, isoxazolidinyl group, isothiazolynyl group, isothiazolidinyl group, dioxolyl group, dioxalanyl group, dioxanyl group, morpholinyl group, thiomorpholinyl group, and piperazinyl group.
Examples of a five- or six- membered aromatic heterocyclic group include pyrrolyl group, furyl group, thienyl group, pyrazolyl group, imidazolyl group, triazolyl group, tetrazolyl group, oxazolyl group, isoxazolyl group, thiazolyl group, isothiazolyl group, oxadiazolyl group, thiadiazolyl group, pyridyl group, pyridazinyl group, pyrimidinyl group, pyrazinyl group, triazinyl group and tetrazinyl group.
Examples of the N-oxide compound of the compound represented by formula (I) include the compound represented by the following formula.

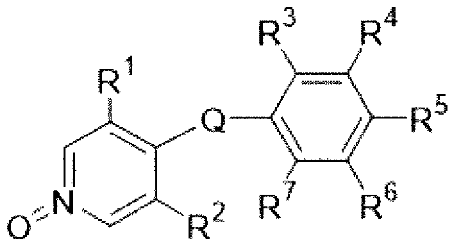

[wherein the symbols are the same as defined above]
The compound AA of the present invention, the compound A of the present invention, the compound A2 of the present invention, the compound B of the present invention, and the compound C of the present invention are collectively referred to as "Compound of the present invention".
The present compound (where the compound of the present invention is included) may be existed as one or more stereoisomers. Examples of the stereoisomer include enantiomer, diastereoisomer, and geometric isomer. Each stereoisomer, and stereoisomer mixture(s) in an arbitrary ratio thereof are included in the present compound.
The compound represented by formula (I) or its N-oxide may form acid addition salts. Examples of the acid to form the acid addition salt include inorganic acids such as hydrogen chloride, phosphoric acid, and sulfuric acid; and organic acids such as acetic acid, trifluoroacetic acid, benzoic acid, and p-toluenesulfonic acid. The acid addition salt may be obtained by mixing the compound represented by formula (I) or its N-oxide with an acid.
Embodiments of the present compound N include the following compounds.
[Embodiment 1] A present compound N wherein $R^1$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_mR^{10}$, or a halogen atom, and $R^2$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_nR^{10a}$, a halogen atom, or a hydrogen atom.
[Embodiment 2] A present compound N wherein $R^1$ represents a halogen atom, and $R^2$ represents a halogen atom or a hydrogen atom.
[Embodiment 3] A present compound N wherein $R^1$ and $R^2$ represent a chlorine atom,

[Embodiment 4] A present compound N wherein Q represents a group represented by formula Q1, or a group represented by formula Q4.

[Embodiment 5] A present compound N wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 6] A present compound N wherein Q represents a group represented by formula Q1, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 7] A present compound N wherein Q represents a group represented by formula Q1, $A^1$ and $A^3$ represent a nitrogen atom, and $A^2$ represents an oxygen atom.

[Embodiment 8] A present compound N wherein Q represents a group represented by formula Q1, $A^1$ and $A^3$ represent a nitrogen atom, $A^2$ represents $NR^{12}$, and $R^{12}$ represents a C1-C6 chain hydrocarbon group which may be optionally substituted with one or more halogen atoms.

[Embodiment 9] The compound according to Embodiment 1 wherein Q represents a group represented by formula Q1, or a group represented by formula Q4.

[Embodiment 10] The compound according to Embodiment 2 wherein Q represents a group represented by formula Q1, or a group represented by formula Q4.

[Embodiment 11] The compound according to Embodiment 3 wherein Q represents a group represented by formula Q1, or a group represented by formula Q4.

[Embodiment 12] The compound according to Embodiment 1 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 13] The compound according to Embodiment 2 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 14] The compound according to Embodiment 3 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 15] The compound according to Embodiment 1 wherein Q represents a group represented by formula Q1, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 16] The compound according to Embodiment 2 wherein Q represents a group represented by formula Q1, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 17] The compound according to Embodiment 3 wherein Q represents a group represented by formula Q1, $A^1$ and $A^3$ represent a nitrogen atom, $A^2$ represents an oxygen atom or $NR^{12}$, and $R^{12}$ represents a C1-C6 chain hydrocarbon group which may be optionally substituted with one or more halogen atoms.

[Embodiment 18] The compound according to Embodiment 1 wherein Q represents a group represented by formula Q1, $A^1$ and $A^3$ represent a nitrogen atom, and $A^2$ represents an oxygen atom.

[Embodiment 19] The compound according to Embodiment 2 wherein Q represents a group represented by formula Q1, $A^1$ and $A^3$ represent a nitrogen atom, and $A^2$ represents an oxygen atom.

[Embodiment 20] The compound according to Embodiment 3 wherein Q represents a group represented by formula Q1, $A^1$ and $A^3$ represent a nitrogen atom, and $A^2$ represents an oxygen atom.

[Embodiment 21] The compound according to Embodiment 1 wherein Q represents a group represented by formula Q1, $A^1$ and $A^3$ represent a nitrogen atom, $A^2$ represents $NR^{12}$, and $R^{12}$ represents a C1-C6 chain hydrocarbon group which may be optionally substituted with one or more halogen atoms.

[Embodiment 22] The compound according to Embodiment 2 wherein Q represents a group represented by formula Q1, $A^1$ and $A^3$ represent a nitrogen atom, $A^2$ represents $NR^{12}$, and $R^{12}$ represents a C1-C6 chain hydrocarbon group which may be optionally substituted with one or more halogen atoms.

[Embodiment 23] The compound according to Embodiment 3 wherein Q represents a group represented by formula Q1, $A^1$ and $A^3$ represent a nitrogen atom, $A^2$ represents $NR^{12}$, and $R^{12}$ represents a C1-C6 chain hydrocarbon group which may be optionally substituted with one or more halogen atoms.

[Embodiment 24] The compound according to any one of Embodiment 1 to Embodiment 23 or the present compound N wherein $R^3$ and $R^7$ are identical or different from each other and each represents a halogen atom or a hydrogen atom.

[Embodiment 25] The compound according to any one of Embodiment 1 to Embodiment 23 or the present compound N wherein $R^4$ and $R^6$ are identical or different from each other and each represents a C1-C6 chain hydrocarbon group, a C1-C6 alkoxy group, a C3-C6 cycloalkyl group {the C1-C6 chain hydrocarbon group, the C1-C6 alkoxy group, and the C3-C6 cycloalkyl group may be optionally substituted with one or more halogen atoms}, a cyano group, a nitro group, a halogen atom, or a hydrogen atom, and $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a nitro group, a halogen atom, or a hydrogen atom.

[Embodiment 26] The compound according to any one of Embodiment 1 to Embodiment 23 or the present compound N wherein $R^3$ and $R^7$ are identical or different from each other and each represents a halogen atom or a hydrogen atom, $R^4$ and $R^6$ are identical or different from each other and each represents a C1-C6 chain hydrocarbon group, a C1-C6 alkoxy group, a C3-C6 cycloalkyl group {the C1-C6 chain hydrocarbon group, the C1-C6 alkoxy group, and the C3-C6 cycloalkyl group may be optionally substituted with one or more halogen atoms}, a cyano group, a nitro group, a halogen atom, or a hydrogen atom, and $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a nitro group, a halogen atom, or a hydrogen atom.

[Embodiment 27] The compound according to any one of Embodiment 1 to Embodiment 23 or the present compound N wherein $R^3$ and $R^7$ represent a hydrogen atom, $R^4$ and $R^6$ are identical or different from each other and each represents a C1-C6 chain hydrocarbon group, a C1-C6 alkoxy group, a C3-C6 cycloalkyl group {the C1-C6 chain hydrocarbon group, the C1-C6 alkoxy group, and the C3-C6 cycloalkyl group may be optionally substituted with one or more halogen atoms}, a cyano group, a nitro group, a halogen atom, or a hydrogen atom, and $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a nitro group, a halogen atom, or a hydrogen atom.

[Embodiment 28] The compound according to any one of Embodiment 1 to Embodiment 23 or the present compound N wherein $R^4$, $R^5$ and $R^6$ are identical or different from each other and each represents a C1-C6 chain hydrocarbon group, a C1-C6 alkoxy group {the C1-C6 chain hydrocarbon group and the C1-C6 alkoxy group may be optionally substituted with one or more halogen atoms}, a halogen atom, or a hydrogen atom.

[Embodiment 29] The compound according to any one of Embodiment 1 to Embodiment 23 or the present compound N wherein $R^3$ and $R^7$ are identical or different from each other and each represents a halogen atom or a hydrogen atom, $R^4$, $R^5$ and $R^6$ are identical or different from each other and each represents a C1-C6 chain hydrocarbon group, a C1-C6 alkoxy group {the C1-C6 chain hydrocarbon group and the C1-C6 alkoxy group may be optionally substituted with one or more halogen atoms}, a halogen atom, or a hydrogen atom.

[Embodiment 30] The compound according to any one of Embodiment 1 to Embodiment 23 or the present compound N wherein $R^3$ and $R^7$ represent a hydrogen atom, $R^4$, $R^5$ and $R^6$ are identical or different from each other and each represents a C1-C6 chain hydrocarbon group, a C1-C6 alkoxy group {the C1-C6 chain hydrocarbon group and the C1-C6 alkoxy group may be optionally substituted with one or more halogen atoms}, a halogen atom, or a hydrogen atom.

[Embodiment 31] The compound according to any one of Embodiment 1 to Embodiment 23 or the present compound N wherein $R^4$ and $R^6$ are identical or different from each other and each represents a C1-C6 chain hydrocarbon group which may be optionally substituted with one or more halogen atoms, or a hydrogen atom, and $R^5$ represents a halogen atom or a hydrogen atom.

[Embodiment 32] The compound according to any one of Embodiment 1 to Embodiment 23 or the present compound N wherein $R^3$ and $R^7$ are identical or different from each other and each represents a halogen atom or a hydrogen atom, $R^4$ and $R^6$ are identical or different from each other and each represents a C1-C6 chain hydrocarbon group which may be optionally substituted with one or more halogen atoms, or a hydrogen atom, and $R^5$ represents a halogen atom or a hydrogen atom.

[Embodiment 33] The compound according to any one of Embodiment 1 to Embodiment 23 or the present compound N wherein $R^3$ and $R^7$ represent a hydrogen atom, $R^4$ and $R^6$ are identical or different from each other and each represents a C1-C6 chain hydrocarbon group which may be optionally substituted with one or more halogen atoms, or a hydrogen atom, and $R^5$ represents a halogen atom or a hydrogen atom.

[Embodiment 34] The compound according to any one of Embodiment 1 to Embodiment 23 or the present compound N wherein $R^4$ and $R^6$ are identical or different from each other and each represents a C1-C6 alkoxy group which may be optionally substituted with one or more halogen atoms, and $R^5$ represents a halogen atom or a hydrogen atom.

[Embodiment 35] The compound according to any one of Embodiment 1 to Embodiment 23 or the present compound N wherein $R^3$ and $R^7$ are identical or different from each other and each represents a halogen atom or a hydrogen atom, $R^4$ and $R^6$ are identical or different from each other and each represents a C1-C6 alkoxy group which may be optionally substituted with one or more halogen atoms, and $R^5$ represents a halogen atom or a hydrogen atom.

[Embodiment 36] The compound according to any one of Embodiment 1 to Embodiment 23 or the present compound

N wherein $R^3$ and $R^7$ represent a hydrogen atom, $R^4$ and $R^6$ are identical or different from each other and each represents a C1-C6 alkoxy group which may be optionally substituted with one or more halogen atoms, and $R^5$ represents a halogen atom or a hydrogen atom.

[Embodiment 37] The compound according to any one of Embodiment 1 to Embodiment 23 or the present compound N wherein $R^4$, $R^5$ and $R^6$ are identical or different from each other and each represents a halogen atom or a hydrogen atom.

[Embodiment 38] The compound according to any one of Embodiment 1 to Embodiment 23 or the present compound N wherein $R^3$ and $R^7$ are identical or different from each other and each represents a halogen atom or a hydrogen atom, and $R^4$, $R^5$ and $R^6$ are identical or different from each other and each represents a halogen atom or a hydrogen atom.

[Embodiment 39] The compound according to any one of Embodiment 1 to Embodiment 23 or the present compound N wherein $R^3$ and $R^7$ represent a hydrogen atom, and $R^4$, $R^5$ and $R^6$ are identical or different from each other and each represents a halogen atom or a hydrogen atom.

[Embodiment N1] The present compound N wherein $R^1$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, $S(O)_mR^{10}$, or a halogen atom, and $R^2$ represents a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, $S(O)_nR^{10a}$, a halogen atom, or a hydrogen atom.

[Embodiment N2] The present compound N wherein $R^1$ represents a C1-C4 alkoxy group, a cyclopropyl group {the C1-C4 alkoxy group, and the cyclopropyl group may be optionally substituted with one or more halogen atoms}, $S(O)_mR^{10}$, or a halogen atom, $R^2$ represents a halogen atom or a hydrogen atom, and $R^{10}$ represents a C1-C4 chain hydrocarbon group which may be optionally substituted with one or more halogen atoms.

[Embodiment N3] The present compound N wherein $R^1$ represents a C1-C4 alkoxy group, a C1-C4 alkylsulfanyl group {the C1-C4 alkoxy group, and the C1-C4 alkylsulfanyl group may be optionally substituted with one or more halogen atoms}, or a halogen atom, and $R^2$ represents a halogen atom or a hydrogen atom.

[Embodiment N4] The present compound N wherein $R^1$ represents a C1-C4 alkoxy group, a C1-C4 alkylsulfanyl group {the C1-C4 alkoxy group, and the C1-C4 alkylsulfanyl group may be optionally substituted with one or more halogen atoms}, or a halogen atom, and $R^2$ represents a chlorine atom or a hydrogen atom.

(Embodiment N5] The present compound N wherein $R^1$ represents a C1-C4 alkoxy group, a C1-C4 alkylsulfanyl group, or a chlorine atom, and $R^2$ represents a chlorine atom or a hydrogen atom.

[Embodiment N6] The present compound N wherein $R^1$ represents a C1-C4 alkoxy group, a C1-C4 alkylsulfanyl group, or a chlorine atom, and $R^2$ represents a chlorine atom.

[Embodiment N7} The present compound N wherein $R^1$ represents a C1-C4 alkoxy group or a chlorine atom, and $R^2$ represents a chlorine atom.

[Embodiment N8] The present compound N wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms.

[Embodiment N9] The present compound N wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms.

[Embodiment N10] The compound according to Embodiment N1 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N11] The compound according to Embodiment N2 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N12] The compound according to Embodiment N3 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N13] The compound according to Embodiment N4 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N14] The compound according to Embodiment N5 wherein Q represents a group represented by

formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N15] The compound according to Embodiment N6 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N16] The compound according to Embodiment N7 wherein Q represents a group represented by formula Q1, or a group represented by formula Q4, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N17] The compound according to Embodiment N1 wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N18] The compound according to Embodiment N2 wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N19] The compound according to Embodiment N3 wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N20] The compound according to Embodiment N4 wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N21] The compound according to Embodiment N5 wherein Q represents a group represented by formula Q2, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N22] The compound according to Embodiment N6 wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N23] The compound according to Embodiment N7 wherein Q represents a group represented by formula Q1, $A^1$ and $A^3$ represent a nitrogen atom, $A^2$ represents an oxygen atom or $NR^{12}$, and $R^{12}$ represents a methyl group which may be optionally substituted with one or more halogen atoms, or a hydrogen atom.

[Embodiment N24] The compound according to Embodiment N1 wherein Q represents a group represented by formula Q1, $A^1$ and $A^3$ represent a nitrogen atom, $A^2$ represents an oxygen atom.

[Embodiment N25] The compound according to Embodiment N2 wherein Q represents a group represented by formula Q1, $A^1$ and $A^3$ represent a nitrogen atom, $A^2$ represents an oxygen atom.

[Embodiment N26] The compound according to Embodiment N3 wherein Q represents a group represented by formula Q1, $A^1$ and $A^3$ represent a nitrogen atom, $A^2$ represents an oxygen atom.

[Embodiment N27] The compound according to Embodiment N4 wherein Q represents a group represented by formula Q1, $A^1$ and $A^3$ represent a nitrogen atom, $A^2$ represents an oxygen atom.

[Embodiment N28] The compound according to Embodiment N5 wherein Q represents a group represented by formula Q1, $A^1$ and $A^3$ represent a nitrogen atom, $A^2$ represents an oxygen atom.

[Embodiment N29] The compound according to Embodiment N6 wherein Q represents a group represented by formula Q1, $A^1$ and $A^3$ represent a nitrogen atom, $A^2$ represents an oxygen atom.

[Embodiment N30] The compound according to Embodiment N7 wherein Q represents a group represented by formula Q1, $A^1$ and $A^3$ represent a nitrogen atom, $A^2$ represents an oxygen atom.

[Embodiment N31] The compound according to any one of Embodiment 1 to Embodiment 23, Embodiment N1 to Embodiment N30, or the present compound N wherein $R^3$ and $R^7$ are identical or different from each other and each represents a methyl group, a halogen atom, or a hydrogen atom.

[Embodiment N32] The compound according to any one of Embodiment 1 to Embodiment 23, Embodiment N1 to Embodiment N30, or the present compound N wherein $R^3$ represents a methyl group, a chlorine atom, or a hydrogen atom, and $R^7$ represents a hydrogen atom.

[Embodiment N33] The compound according to any one of Embodiment 1 to Embodiment 23, Embodiment N1 to Embodiment N30, or the present compound N wherein $R^3$ and $R^7$ represents a hydrogen atom.

[Embodiment N34] The compound according to any one of Embodiment 1 to Embodiment 23, Embodiment N1 to Embodiment N30, or the present compound N wherein $R^4$ and $R^6$ are identical or different from each other and each represents a C1-C6 chain hydrocarbon group, a C1-C6 alkoxy group, a C1-C6 alkylsulfanyl group {the C1-C6 chain hydrocarbon group, the C1-C6 alkoxy group, and the C1-C6 alkylsulfanyl group may be optionally substituted

with one or more halogen atoms}, a cyano group, a nitro group, a halogen atom, or a hydrogen atom.

[Embodiment N35] The compound according to any one of Embodiment 1 to Embodiment 23, Embodiment N1 to Embodiment N30, or the present compound N wherein $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a nitro group, a halogen atom, or a hydrogen atom.

[Embodiment N36] The compound according to any one of Embodiment 1 to Embodiment 23, Embodiment N1 to Embodiment N30, or the present compound N wherein $R^4$ and $R^6$ are identical or different from each other and each represents a C1-C6 chain hydrocarbon group, a C1-C6 alkoxy group, a C1-C6 alkylsulfanyl group {the C1-C6 chain hydrocarbon group, the C1-C6 alkoxy group, and the C1-C6 alkylsulfanyl group may be optionally substituted with one or more halogen atoms}, a cyano group, a nitro group, a halogen atom, or a hydrogen atom, and $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a halogen atom, or a hydrogen atom.

[Embodiment N37] The compound according to any one of Embodiment 1 to Embodiment 23, Embodiment N1 to Embodiment N30, or the present compound N wherein $R^4$ and $R^6$ are identical or different from each other and each represents a C1-C4 alkyl group, a C1-C4 alkoxy group, a C1-C4 alkylsulfanyl group {the C1-C4 alkyl group, the C1-C4 alkoxy group, and the C1-C4 alkylsulfanyl group may be optionally substituted with one or more halogen atoms}, a cyano group, a nitro group, a halogen atom, or a hydrogen atom, and $R^5$ represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom.

[Embodiment N38] The compound according to any one of Embodiment 1 to Embodiment 23, Embodiment N1 to Embodiment N30, or the present compound N wherein $R^3$ represents a methyl group, a chlorine atom, or a hydrogen atom, $R^4$ and $R^6$ are identical or different from each other and each represents a C1-C6 chain hydrocarbon group, a C1-C6 alkoxy group, a C1-C6 alkylsulfanyl group {the C1-C6 chain hydrocarbon group, the C1-C6 alkoxy group, and the C1-C6 alkylsulfanyl group may be optionally substituted with one or more halogen atoms), a cyano group, a nitro group, a halogen atom, or a hydrogen atom, and $R^7$ represents a hydrogen atom.

[Embodiment N39] The compound according to any one of Embodiment 1 to Embodiment 23, Embodiment N1 to Embodiment N30, or the present compound N wherein $R^3$ represents a methyl group, a chlorine atom, or a hydrogen atom, $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a nitro group, a halogen atom, or a hydrogen atom, and $R^7$ represents a hydrogen atom.

[Embodiment N40] The compound according to any one of Embodiment 1 to Embodiment 23, Embodiment N1 to Embodiment N30, or the present compound N wherein $R^3$ represents a methyl group, a chlorine atom, or a hydrogen atom, $R^4$ and $R^6$ are identical or different from each other and each represents a C1-C6 chain hydrocarbon group, a C1-C6 alkoxy group, a C1-C6 alkylsulfanyl group {the C1-C6 chain hydrocarbon group, the C1-C6 alkoxy group, and the C1-C6 alkylsulfanyl group may be optionally substituted with one or more halogen atoms}, a cyano group, a nitro group, a halogen atom, or a hydrogen atom, $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a halogen atom, or a hydrogen atom, and $R^7$ represents a hydrogen atom.

[Embodiment N41] The compound according to any one of Embodiment 1 to Embodiment 23, Embodiment N1 to Embodiment N30, or the present compound N wherein $R^3$ represents a methyl group, a chlorine atom, or a hydrogen atom, $R^4$ and $R^6$ are identical or different from each other and each represents a C1-C4 alkyl group, a C1-C4 alkoxy group, a C1-C4 alkylsulfanyl group {the C1-C4 alkyl group, the C1-C4 alkoxy group, and the C1-C4 alkylsulfanyl group may be optionally substituted with one or more halogen atoms}, a cyano group, a nitro group, a halogen atom, or a hydrogen atom, $R^5$ represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom, and $R^7$ represents a hydrogen atom.

[Embodiment N42] The compound according to any one of Embodiment 1 to Embodiment 23, Embodiment N1 to Embodiment N30, or the present compound N wherein $R^3$ and $R^7$ represent a hydrogen atom, $R^4$ and $R^6$ are identical or different from each other and each represents a C1-C6 chain hydrocarbon group, a C1-C6 alkoxy group, a C1-C6 alkylsulfanyl group {the C1-C6 chain hydrocarbon group, the C1-C6 alkoxy group, and the C1-C6 alkylsulfanyl group may be optionally substituted with one or more halogen atoms}, a cyano group, a nitro group, a halogen atom, or a hydrogen atom.

[Embodiment N43] The compound according to any one of Embodiment 1 to Embodiment 23, Embodiment N1 to Embodiment N30, or the present compound N wherein $R^3$ and $R^7$ represent a hydrogen atom, $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a nitro group, a halogen atom, or a hydrogen atom.

[Embodiment N44] The compound according to any one of Embodiment 1 to Embodiment 23, Embodiment N1 to Embodiment N30, or the present compound N wherein $R^3$ and $R^7$ represent a hydrogen atom, $R^4$ and $R^6$ are identical or different from each other and each represents a C1-C6 chain hydrocarbon group, a C1-C6 alkoxy group, a C1-C6 alkylsulfanyl group {the C1-C6 chain hydrocarbon group, the C1-C6 alkoxy group, and the C1-C6 alkylsulfanyl group may be optionally substituted with one or more halogen atoms}, a cyano group, a nitro group, a halogen atom, or a hydrogen atom, and $R^5$ represents a C1-C6 chain hydrocarbon group, a C1-C6 alkoxy group {the C1-C6 chain hydrocarbon group and the C1-C6 alkoxy group may be optionally substituted with one or more halogen atoms}, a halogen atom, or a hydrogen atom.

[Embodiment N45] The compound according to any one of Embodiment 1 to Embodiment 23, Embodiment N1 to Embodiment N30, or the present compound N wherein $R^3$ and $R^7$ represent a hydrogen atom, $R^4$ and $R^6$ are identical or different from each other and each represents a C1-C4 alkyl group, a C1-C4 alkoxy group, a C1-C4 alkylsulfanyl group {the C1-C4 alkyl group, the C1-C4 alkoxy group, and the C1-C4 alkylsulfanyl group may be optionally substituted with one or more halogen atoms}, a cyano group, a nitro group, a halogen atom, or a hydrogen atom, and $R^5$ represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom.

[Embodiment N46] The compound according to any one of Embodiment 1 to Embodiment 23, Embodiment N1 to Embodiment N30, or the present compound N wherein $R^4$, $R^5$ and $R^6$ are identical or different from each other and each represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom.

[Embodiment N47] The compound according to any one of Embodiment 1 to Embodiment 23, Embodiment N1 to Embodiment N30, or the present compound N wherein $R^3$ represents a methyl group, a chlorine atom, or a hydrogen atom, $R^4$, $R^5$ and $R^6$ are identical or different from each other and each represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom, and $R^7$ represents a hydrogen atom.

[Embodiment N48] The compound according to any one of Embodiment 1 to Embodiment 23, Embodiment N1 to Embodiment N30, or the present compound N wherein $R^3$ and $R^7$ represent a hydrogen atom, $R^4$, $R^5$ and $R^6$ are identical or different from each other and each represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom.

[Embodiment N49] The compound according to any one of Embodiment 1 to Embodiment 23, Embodiment N1 to Embodiment N30, or the present compound N wherein $R^3$ and $R^7$ represent a hydrogen atom, $R^4$, $R^5$ and $R^6$ are identical or different from each other and each represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom.

[Embodiment N50] The compound according to any one of Embodiment 1 to Embodiment 23, Embodiment N1 to Embodiment N30, or the present compound N wherein $R^3$ and $R^7$ represent a hydrogen atom, $R^4$ and $R^5$ are identical or different from each other and each represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom, and $R^6$ represents a hydrogen atom.

[Embodiment N51] The compound according to any one of Embodiment 1 to Embodiment 23, Embodiment N1 to Embodiment N30, or the present compound N wherein $R^3$ and $R^7$ represent a hydrogen atom, $R^4$ represents a C1-C4 alkyl group, a C1-C4 alkoxy group {the C1-C4 alkyl group and the C1-C4 alkoxy group may be optionally substituted with one or more halogen atoms}, a halogen atom, or a hydrogen atom, $R^5$ represents a halogen atom or a hydrogen atom, and $R^6$ represents a hydrogen atom.

Examples of Embodiment of the compound AA of the present invention include the following compounds.

[Embodiment AA1] A compound AA of the present invention wherein $R^{21}$ represents a C1-C6 chain hydrocarbon group, a C1-C6 alkoxy group, $S(O)_nR^{28}$, or a halogen atom, $R^{22}$ represents a chlorine atom or a hydrogen atom, and $R^{28}$ represents a C1-C6 chain hydrocarbon group.

[Embodiment AA2] A compound AA of the present invention wherein $R^{21}$ represents a C1-C4 alkyl group, a C1-C4 alkoxy group, a C1-C4 alkylsulfanyl group, or a chlorine atom, and $R^{22}$ represents a chlorine atom or a hydrogen atom.

[Embodiment AA3] A compound AA of the present invention wherein $R^{23}$ and $R^{27}$ represent a hydrogen atom.

[Embodiment AA4] The compound according to Embodiment AA1 wherein $R^{23}$ and $R^{27}$ represent a hydrogen atom.

[Embodiment AA5] The compound according to Embodiment AA2 wherein $R^{23}$ and $R^{27}$ represent a hydrogen atom.

[Embodiment AA6] The compound according to any one of Embodiment AA1 to Embodiment AA5 or the compound AA of the present invention wherein $R^{24}$ represents a C1-C4 alkoxy group which is substituted with one or more halogen atoms, or a fluorine atom, and $R^{25}$ represents a fluorine atom or a hydrogen atom.

[Embodiment AA7] The compound according to any one of Embodiment AA1 to Embodiment AA5 or the compound AA of the present invention wherein $R^{24}$ represents a C1-C4 alkoxy group which is substituted with one or more fluorine atoms, or a fluorine atom, and $R^{25}$ represents a fluorine atom or a hydrogen atom.

[Embodiment AA8] The compound according to any one of Embodiment AA1 to Embodiment AA5 or the compound AA of the present invention wherein $R^{24}$ represents a C1-C2 alkoxy group which is substituted with one or more fluorine atoms, or a fluorine atom, and $R^{25}$ represents a fluorine atom or a hydrogen atom.

[Embodiment AA9] The compound according to any one of Embodiment AA1 to Embodiment AA5 or the compound AA of the present invention wherein $R^{24}$ represents a methoxy group which is substituted with one or more fluorine atoms, or a fluorine atom, and $R^{25}$ represents a fluorine atom or a hydrogen atom.

[Embodiment AA10] The compound according to any one of Embodiment AA1 to Embodiment AA5 or the compound AA of the present invention wherein $R^{24}$ represents a methoxy group which is substituted with one or more fluorine atoms, or a fluorine atom, and $R^{25}$ represents a hydrogen atom.

Examples of the compound A2 of the present invention include the following compounds.

[Embodiment A2-1] A compound A2 of the present invention wherein $R^{21a}$ represents a C1-C4 alkoxy group, or a C1-C4 alkylsulfanyl group, $R^{22a}$ represents a chlorine atom, $R^{23a}$ and $R^{27a}$ represent a hydrogen atom, $R^{21a}$ represents a C1-C4 alkyl group, a C1-C4 alkoxy group {the C1-C4 alkyl group and the C1-C4 alkoxy group may be optionally substituted with one or more halogen atoms}, or a halogen atom, and $R^{25a}$ represents a fluorine atom or a hydrogen atom.

Examples of the compound B of the present invention include the following compounds.

[Embodiment B1] A compound B of the present invention wherein $R^{31}$ and $R^{32}$ represent a chlorine atom, $R^{33}$, $R^{35}$ and $R^{37}$ represent a hydrogen atom, $R^{34}$ represents a C1-C4 alkoxy group which may be optionally substituted with one or more halogen atoms, $R^{36}$ represents a halogen atom, and $R^{38}$ represents a methyl group or a hydrogen atom.

[Embodiment B2] A compound B of the present invention wherein $R^{31}$ and $R^{32}$ represent a chlorine atom, $R^{33}$, $R^{35}$ and $R^{37}$ represent a hydrogen atom, $R^{34}$ represents a C1-C4 alkoxy group is substituted with one or more fluorine atoms, $R^{36}$ represents a fluorine atom, and $R^{38}$ represents a methyl group or a hydrogen atom.

Examples of the compound C of the present invention include the following compounds.

[Embodiment C1] A compound C of the present invention wherein $R^{41}$ and $R^{42}$ represent a chlorine atom, and $R^{43}$ and $R^{47}$ represent a hydrogen atom.

[Embodiment C2] The compound according to Embodiment C1 wherein $R^{44}$ represents a halogen atom or a hydrogen atom, $R^{45}$ represents a fluorine atom or a hydrogen atom, $R^{46}$ represents a halogen atom or a hydrogen atom, and $R^{43}$ and $R^{47}$ represent a hydrogen atom.

Next, a process for preparing the present compound (including the compound of the present invention) is described.

Process 1

A compound represented by formula (1-1) (hereinafter, referred to as "compound (1-1)") can be prepared by reacting a compound represented by formula (M-1) (hereinafter, referred to as "compound (M-1)") with a compound represented by formula (M-2) (hereinafter, referred to as "compound (M-2)") in the presence of a base and a catalyst.

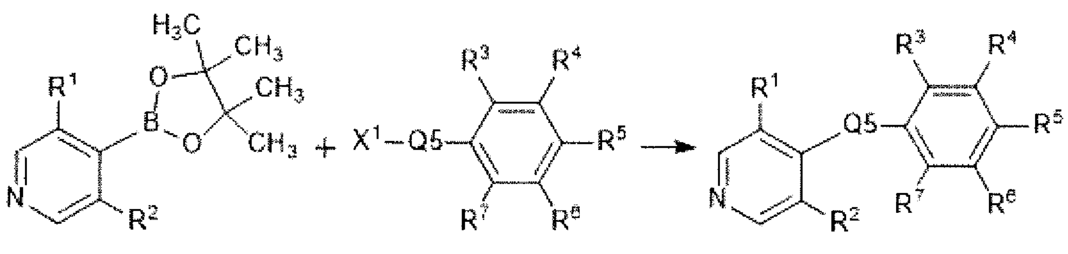

( M-1 ) ( M-2 ) ( I-1 )

[wherein Q5 represents a group represented by formula Q1, a group represented by formula Q3, or a group represented by formula Q4, $X^1$ represents a chlorine atom, a bromine atom, or an iodine atom, and the other symbols are the same as defined above]

The reaction is usually conducted in a solvent or in the absence of a solvent. Examples of the solvent include ethers (such as tetrahydrofuran (hereinafter, referred to as "THF"), 1,4-dioxane, 1,2-dimethoxyethane (hereinafter, referred to as "DME"), methyl tert-butyl ether (hereinafter, referred to as "MTBE"), diethyl ether) (hereinafter, collectively referred to as "ethers"); halogenated hydrocarbons (such as dichloromethane, chloroform) (hereinafter, collectively referred to as "halogenated hydrocarbons"); hydrocarbons (such as hexane, toluene, xylene) (hereinafter, collectively referred to as "hydrocarbons"); apromatic polar solvents (such as N,N-dimethylformamide (hereinafter, referred to as "DMF"), N-methylpyrrolidone, dimethylsulfoxide (hereinafter, referred to as "DMSO") (hereinafter, collectively referred to as "apromatic polar solvents"); nitriles (such as acetonitrile) hereinafter, collectively referred to as "nitriles"); water and mixtures of these solvents.

Examples of the bases to be used in the reaction include organic bases (such as triethylamine, diisopropyl ethyl amine, pyridine, and 4-(dimethylamino)pyridine)) (hereinafter, collectively referred to as "organic bases"); alkali metal carbonates (such as sodium carbonate, and potassium carbonate) (hereinafter, collectively referred to as "alkali metal carbonates"); alkali metal hydroxides (such as sodium hydroxide, and potassium hydroxide) (hereinafter, collectively referred to as "alkali metal hydroxides"); alkali metal fluorides (such as sodium fluoride, potassium fluoride, cesium fluoride); and alkali metal phosphates (such as tripotassium phosphate) (hereinafter, collectively referred to as "alkali metal phosphates").

Examples of the catalysts to be used in the reaction include palladium (II) acetate, dichlorobis(triphenylphosphine)palladium (II), bis(diphenylphosphane ferrocenyl)palladium (II) dichloride).

In the reaction, the compound (M-2) is usually used within a range of 1 to 10 molar ratio (s), the base is usually used within a range of 1 to 10 molar ratio(s), and the catalyst is usually used within a range of 0.0001 to 1 molar ratio(s), as opposed to 1 mole of the compound (M-1).

A ligand may be used in the reaction as needed. Examples of the ligand include triphenylphosphine, 4,5-bis(diphenylphosphino)-9,9-dimethyl xanthene (hereinafter, referred to as "Xantphos"), 2,2'-bis(diphenylphosphino)-1,1'-binaphthyl, 1,1'-bis(diphenylphosphino)ferrocene, 2-dicylohexylphoshino-2',4',6'-triisopropylbiphenyl, 2-dicyclohexylphoshino-2',6'-dimethoxybiphenyl, 1,2-bis(diphenylphoshino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylenediamine.

When a ligand is used in the reaction, the ligand is usually used within a range of 0.01 to 0.5 molar ratios, as opposed to 1 mole of the compound (M-1).

The reaction temperature of the reaction is usually within a range of 0 to 150°C. The reaction period is usually within a range of 0.1 to 24 hours.

When the reaction is completed, water is added to the reaction mixtures, and the reaction mixtures are extracted with organic solvent(s), and the organic layers are worked up (for example, drying and concentration) to obtain the compound (1-1).

The Compound (M-2) is a commercially available compound, or can be prepared according to a known method.

Process 2

A compound represented by formula (1-2) (hereinafter, referred to as "compound (1-2)") can be prepared by reacting a compound represented by formula (M-3) (hereinafter, referred to as "compound (M-3)") with a compound represented by formula (M-4) (hereinafter, referred to as "compound (M-4)") in the presence of a base.

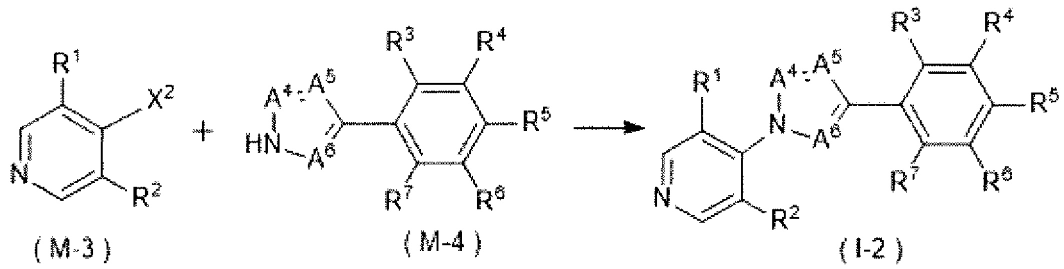

(M-3) (M-4) (I-2)

[wherein $X^2$ represents a fluorine atom or a chlorine atom, and the other symbols are the same as defined above]

The reaction is conducted in a solvent. Examples of the solvent include ethers, halogenated hydrocarbons, hydrocarbons, apromatic polar solvents, nitriles, and mixtures of these solvents.

Examples of the bases to be used in the reaction include organic bases, alkali metal carbonates, alkali metal hydroxides, and alkali metal phosphates.

In the reaction, the compound (M-4) is usually used within a range of 1 to 10 molar ratio (s), and the base is

used within a range of 1 to 10 molar ratio(s), as opposed to 1 mole of the compound (M-4).

A metallic catalyst may be used in the reaction as needed. Examples of the metallic catalyst include copper catalysts (such as copper (I) iodide, copper (I) bromide, copper (I) chloride, copper (I) oxide, copper (I) trifluoromethane sulfonate benzene complex, tetrakis(acetonitrile)copper (I) hexafluorophosphate, and copper (I) 2-thiophenecarboxylate); nickel catalysts (such as bis(cyclooctadiene)nickel (0), nickel (II) chloride); palladium catalysts (such as palladium (II) acetate, tetrakis(triphenylphosphine) palladium (0), tris(dibenzylideneacetone)dipalladium (II)).

When the metallic catalyst is used in the reaction, the metallic catalyst is usually used within a range of 0.01 to 0.5 molar ratios as opposed to 1 mole of the compound (M-3).

A ligand may be used in the reaction as needed. Examples of the ligand include triphenylphosphine, 4,5-bis(diphenylphosphino)-9,9-dimethyl xanthene (hereinafter, referred to as "Xantphos"), 2,2'-bis(diphenylphosphino)-1,1'-binaphthyl, 1,1'-bis(diphenylphosphino)ferrocene, 2-dicylohexylphoshino-2',4',6'-triisopropylbiphenyl, 2-dicyclohexylphoshino-2',6'-dimethoxybiphenyl, 1,2-bis(diphenylphoshino)ethane, 2,2'-bipyridine, 2-aminoethanol, 8-hydroxyquinoline, 1,10-phenanthroline, trans-1,2-cyclohexanediamine, trans-N,N'-dimethylcyclohexane-1,2-diamine, and N,N'-dimethylenediamine.

When a ligand is used in the reaction, the ligand is usually used within a range of 0.01 to 0.5 molar ratios, as opposed to 1 mole of the compound (M-3).

The reaction temperature of the reaction is usually within a range of 0 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (I-2).

The compound (M-3) or the compound (M-4) is a commercially available compound, or can be prepared according to a known method.

Process 3

A compound represented by formula (1-3) (hereinafter, referred to as "compound (1-3)") can be prepared by reacting a compound represented by formula (M-5) (hereinafter, referred to as "compound (M-5) ") with a compounds represented by formula (M-6) (hereinafter, referred to as "compound (M-6)") in the presence of a base.

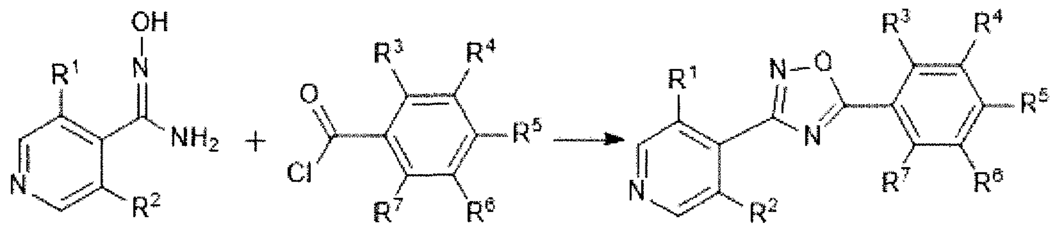

( M-5 ) ( M-6 ) ( I-3 )

[wherein the symbols are the same as defined above]

The reaction is usually conducted in a solvent. Examples of the solvents include ethers, halogenated hydrocarbons, hydrocarbons, apromatic polar solvents, nitriles and mixtures of these solvents.

Examples of the bases to be used in the reaction include organic bases, alkali metal carbonates, alkali metal hydroxides, and alkali metal phosphates.

In the reaction, the compound (M-6) is usually conducted within a range of 1 to 10 molar ratio (s) as opposed to 1 mole of the compound (M-5).

The reaction temperature is usually within a range of 0 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (M-3).

The compound (R-1) is a commercially available compound or can be prepared according to known method.

Process 4

A compound represented by formula (1-4) (hereinafter, referred to as "compound (1-4)") can be prepared by reacting a compound represented by formula (M-7) (hereinafter, referred to as "compound (M-7)") in the presence of an

acid.

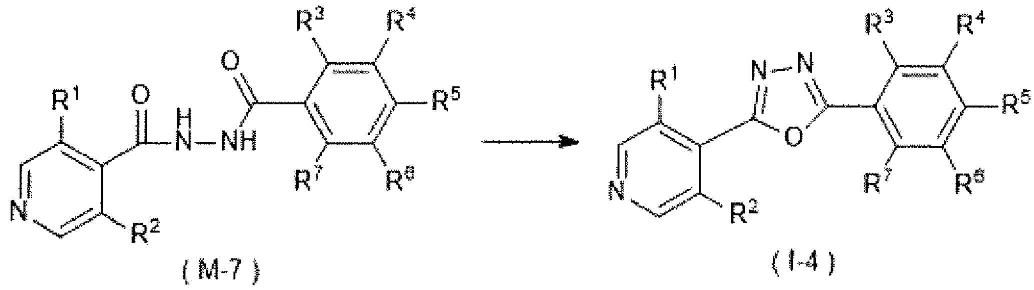

( M-7 ) ( I-4 )

[wherein the symbols are the same as defined above]

The reaction is usually conducted in a solvent. Examples of the solvent include ethers, halogenated hydrocarbons, hydrocarbons, apromatic polar solvents, nitriles, and mixtures of these solvents.

Examples of the acid include p-toluenesulfonyl chloride.

In the reaction, the acid is usually within a range of 0.1 to 10 molar ratios, as opposed to 1 mole of the compound (M-7) .

The reaction temperature is usually within a range of 0 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (I-4).

Process 5

A compound represented by formula (1-5) (hereinafter, referred to as "compound (1-5)") can be prepared by reacting a compound represented by formula (M-8) (hereinafter, referred to as "compound (M-8)") with a compound represented by formula (M-9) (hereinafter, referred to as "compound (M-9)") in the presence of a base.

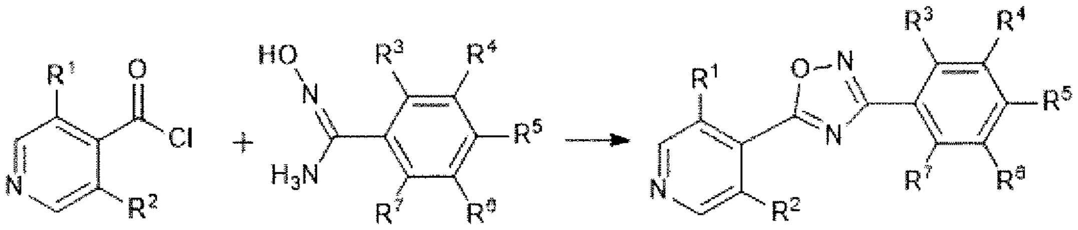

( M-8 ) ( M-9 ) ( I-5 )

[wherein the symbols are the same as defined above]

The reaction may be conducted by using the compound (M-9) in place of the compound (M-5) and using the compound (M-8) in place of the compound (M-5) according to the process 3.

The compound (M-8) is a commercially available compound or can be prepared according to known method.

Process 6

A compound represented by formula (1-6) (hereinafter, referred to as "compound (1-6)") can be prepared by reacting a compound represented by formula (M-10) (hereinafter, referred to as "compound (M-10)") with hydrazine.

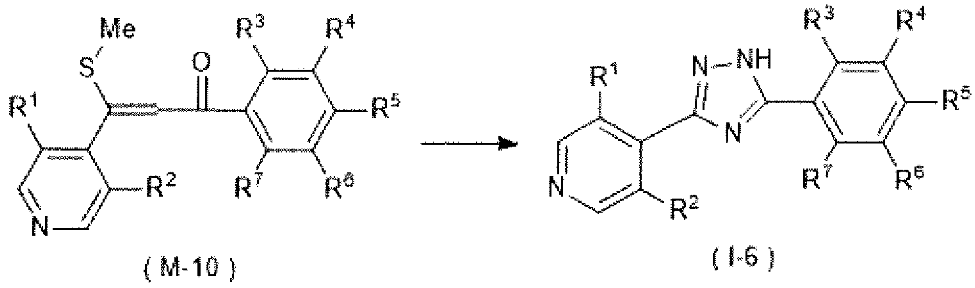

( M-10 ) ( I-6 )

[wherein the symbols are the same as defined above]

The reaction is usually conducted in a solvent. Examples of the solvent include ethers, halogenated hydrocarbons, hydrocarbons, apromatic polar solvents, nitriles and mixtures of these solvents.

Examples of the hydrazine include hydrazine monohydrate or hydrazine acid addition salts (such as hydrazine hydrochloride, or hydrazine sulfate).

In the reaction, the hydrazine is usually used within a range of 0.5 to 10 molar ratios, as opposed to 1 mole of the compound (M-10).

The reaction temperature is usually within a range of 0 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (1-6).

The compound (M-10) can be prepared according to the method described in WO 2012/098416 A1.

Process 7

A compound represented by formula (1-7) (hereinafter, referred to as "compound (1-7)") can be prepared by reacting the compound (1-6) with a compound represented by formula (M-11) (hereinafter, referred to as "compound (M-11)") in the presence of a base.

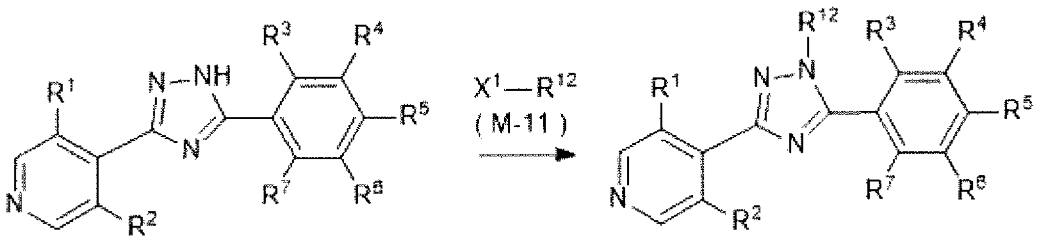


( I-6 ) ( I-7)

[wherein the symbols are the same as defined above]

The reaction is usually conducted in a solvent. Examples of the solvent include ethers, halogenated hydrocarbons, hydrocarbons, apromatic polar solvents, nitriles and mixtures of these solvents.

Examples of the bases to be used in the reaction include organic bases, alkali metal carbonates, alkali metal hydroxides, and alkali metal phosphates.

In the reaction, the compound (M-11) is usually used within a range of 5 to 10 molar ratios, and the base is usually used within a range of 0.5 to 10 molar ratios, as opposed to 1 mole of the compound (1-6).

The reaction temperature is usually within a range of 0 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (1-7).

The compound (M-11) is a commercially available compound, or can be prepared according known method.

Process 8

A N-oxide of the compound represented by formula (I) can be prepared by reacting the compound represented by formula (I) with an oxidizing agent. The reaction can be conducted according to the method described in U.S. publication No. 2018/0009778 or WO 2016/121970.

Reference Process 1

The compound (M-5) can be prepared by reacting a compound represented by formula (M-5S) (hereinafter, referred to as "compound (M-5S)") with hydroxylamine.

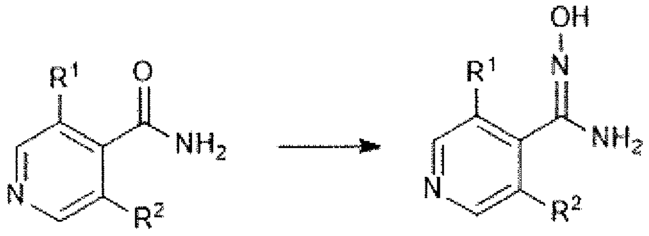

( M-5S ) ( M-5 )

[wherein the symbols are the same as defined above]

The reaction is usually conducted in a solvent. Examples of the solvent include alcohols (such as methanol, ethanol) (hereinafter, collectively referred to as "alcohols"); ethers; halogenated hydrocarbons; hydrocarbons; apromatic polar solvents; nitriles and mixtures of these solvents.

Examples of the hydroxylamines include hydroxylamine acid addition salts (such as hydroxylamine hydrochlorides, and hydroxylamine sulfate).

In the reaction, the hydroxylamine is usually used within a range of 0.5 to 10 molar ratios as opposed to 1 mole of the compound (M-5S).

An acid may be used in the reaction as needed. Examples of the acid include inorganic acids (such as hydrochloride, phosphoric acid, sulfuric acid); and organic acids (such as acetic acid, trifluoroacetic acid, benzoic acid, p-toluene sulfonic acid). When an acid is used in the reaction, the acid is usually used within a range of 0.01 to 0.5 molar ratios as opposed to 1 mole of the compound (M-5S).

The reaction temperature is usually within a range of 0 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (M-5).

The compound (M-5S) is a commercially available compound, or can be prepared according to known method.

Reference Process 2

The compound (M-9) can be prepared by reacting a compound represented by formula (M-9S) (hereinafter, referred to as "compound (M-9S)" with hydroxyl amine.

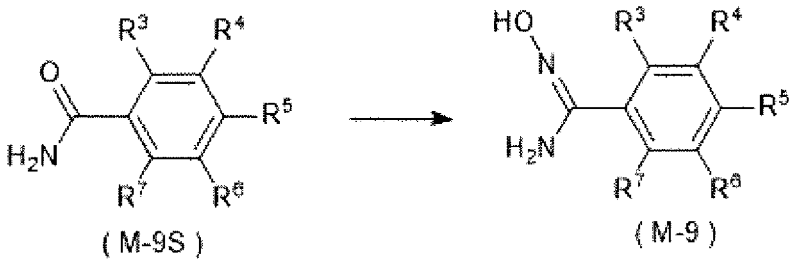

( M-9S ) ( M-9 )

[wherein the symbols are the same as defined above]

The reaction can be conducted by using the compound (M-9S) in place of the compound (M-5S) according to

the Reference process 1.

The compound (M-9S) is a commercially available compound, or can be prepared according to known method.

Reference Process 3

The compound (M-7) can be prepared by reacting the compound (M-8) with a compound represented by formula (M-7S) (hereinafter, referred to as "compound (M-7S)") in the presence of a base.

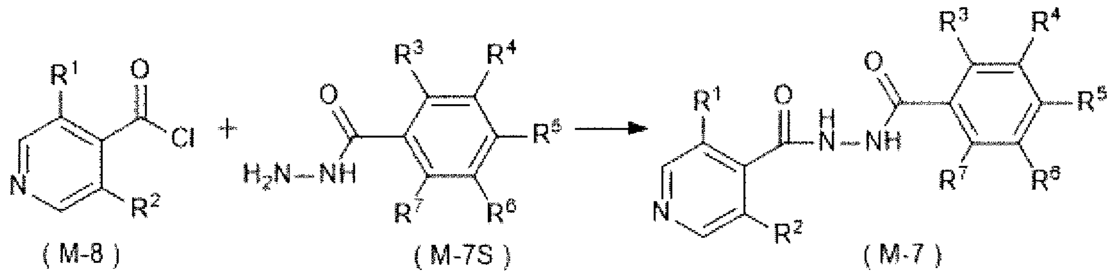

The reaction is conducted in a solvent or in the absence of a solvent. Examples of the solvent include ethers, halogenated hydrocarbons, hydrocarbons, nitriles, nitrogen-containing aromatic compounds, apromatic polar solvents, and mixtures of these solvents.

Examples of the bases include organic bases; alkali metal carbonates; and alkali metal hydrides (such as sodium hydride, potassium hydride) (hereinafter, collectively referred to as "alkali metal hydrides"). In the reaction, the compound (M-7S) is usually used within a range of 0.5 to 1.5 molar ratios, and the base is usually used within a range of 1 to 3 molar ratios, as opposed to 1 mole of the compound (M-8).

The reaction temperature of the reaction is usually within a range of -20 to 150°C. The reaction period is usually within a range of 0.5 to 24 hours.

When the reaction is completed, water is added to the reaction mixtures, and the reaction mixtures are extracted with organic solvent(s), and the organic layers are worked up (for example, drying and concentration) to obtain the compound (M-7).

The compound (M-7S) is a commercially available compound, or can be prepared according to known method.

The compound of the present invention can be mixed or combined with one or more ingredients selected from the group consisting of the following Group (a), Group (b), Group (c), and Group (d) (hereinafter, referred to as "present ingredient").

The mixing or combining represents that the compound of the present invention and the present ingredient are used concurrently, separately, or at an interval.

When the compound of the present invention and the present ingredient are concurrently used, the compound of the present invention and the present ingredient may be incorporated as a separate formulation or one formulation.

One aspect of the present invention relates to a composition comprising one or more ingredients selected from the group consisting of the Group (a), the Group (b), the Group (c), and the Group (d) (that is, the present ingredient), and the compound of the present invention (hereinafter, referred to as "Composition A").

The Group (a) is a group consisting of acetylcholinesterase inhibitors (for example, carbamate insecticides and organophosphate insecticides), GABA-gated chloride ion channel blockers, (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mites growth regulators, microbial disruptors of insect midgut membranes, mitochondrial ATP synthase inhibitors, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, Inhibitors of mitochondrial electron transport chain complex I, II, III, and IV, voltage-dependent sodium channel blockers, Inhibitors of acetyl CoA carboxylase, ryanodine receptor modulators (for example, diamide insecticides), chordotonal organ modulators, microbial fungicides, and other insecticidal ingredients, other miticidal ingredients and other nematicidal ingredients. These agents are described in the classification based on the IRAC mode of action.

The Group (b) is a group consisting of nucleic acid synthesis inhibitors (for example, phenylamide fungicides and acylamino acid fungicides), cytostatic and cytoskeletal inhibitors (for example, MBC fungicides), respiration inhibitors (for example, QoI fungicides and QiI fungicides), amino-acid synthesis and protein synthesis inhibitors (for example, anilinopyridine fungicides), signal-transduction inhibitors, lipid synthesis and membrane synthesis inhibitors, sterol biosynthesis inhibitors (for example, DMI fungicides such as triazoles), cell wall synthesis inhibitors, melanin synthesis inhibitors, plant defense inducer, multisite fungicides, microbial fungicides, and other fungicidal ingredients. These agents are described in the classification based on the FRAC mode of action.

The Group (c) represents a group of plant growth modulating ingredients (including mycorrhizal fungus and rhizobia).

The Group (d) represents a group of repellent ingredients.

Examples of combinations of the present ingredient and the compound of the present invention are recited as follows. For example, the "alanycarb + SX" indicates a combination of alanycarb and SX.

The abbreviation "SX" means to any one of the compounds of the present invention selected from the compound classes SX1 to SX1397. Also any of the present ingredients as described below are a known ingredient, and can be obtained from a commercially available drug or can be prepared according to a known method. When the present ingredient represents a microorganism, the present ingredient can be obtained from a microorganism depositary authority. The number in parentheses represents CAS RN (registered trademark).

A combination of the present ingredient in the above-mentioned Group (a) and the compound of the present invention:

abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, Celastrus angulatus (bark of Celastrus angulatus) + SX, bendiocarb + SX, benfluthrin + SX, benfuracarb + SX, bensultap + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cycloniliprole + SX, cyclobutrifluram) + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC (2-methyl-4,6-dinitrophenol) + SX, doramectin + SX, dried leaves of Dryopteris filix-mas + SX, emamectin-benzoate + SX, empenthrin + SX, endosulfan + SX, EPN (O-ethyl O-(4-nitrophenyl)phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX , etoxazole + SX, extract of Artemisia absinthium + SX, extract of Azadirachta indica + SX, extract of Cassia nigricans + SX, extract of clitoria ternatea + SX, extract of Symphytum officinale + SX, extracts or simulated blend of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-Q- (methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl. + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl

+ SX, potassium cyanide + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, 2-({2-fluoro-4-methyl-5-[(2,2,2-trifluoroethyl)sulfinyl]phenyl}imino)-3-(2,2,2-trifluoroethyl)-1,3-thiazolidin-4-one (1445683-71-5) + SX, (2Z)-2-({2-fluoro-4-methyl-5-[(R)-(2,2,2-trifluoroethyl)sulfinyl]phenyl}imino)-3-(2,2,2-trifluoroethyl)-1,3-thiazolidin-4-one (2377084-09-6) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, BT crop protein Cry1Ab + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus BV-0001 strain + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua mNPV + SX, Spodoptera littoralis mNPV + SX, Spodoptera litura NPV + SX, Arthrobotrys dactyloides + SX, Bacillus firmus strain GB-126 + SX, Bacillus firmus strain 1-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR3 71 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis vaR7 216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain

GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

A combination of the present ingredient in the above-mentioned Group (b) and the compound of the present invention:

acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper (II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper (II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin +SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder

+ SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (3949178-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R,2S,5S)-5-(4-chlorobenzyl)-2-(chloromethyl)-2-methyl-1-(1H-1,2,4-triazol-1-ylmethyl)cyclopentan-1-ol (1801930-06-2) + SX, (1S,2R,5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R,2S,5S)-2-(chloromethyl)-5-(4-fluorobenzyl)-2-methyl-1-(1H-1,2,4-triazol-1-ylmethyl)cyclopentan-1-ol (1801930-08-4) + SX, (1S,2,R5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboxamide (2132414-04-9) + SX, N-(1-benzyl-3,3,3-trifluoro-1-methylpropyl)-8-fluoroquinoline-3-carboxamide (2132414-00-5) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl)methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenylJmethyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenylJmethyl)urea + SX, N-methyl-N'-({ 4-[5-(trifluoromethyl)-1,2,4-oxadiazol-3-yl]phenyl}methyl)urea +

SX, 1-({4-[5-(trifluoromethyl)-1,2,4-oxadiazol-3-yl]phenyl}methyl)pyrrolidin-2-one + SX, 1-(14-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-l-({4-[5-(trifluoromethyl)-1,2,4-oxadiazol-3-yl]phenyl}methyl}piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-ζtrifluoromethyl)-1,2,4-oxadiazol-3-yl]phenyl)methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({ [1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl) (2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5, 6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, Agrobacterium radiobactor strain K1026 + SX, Agrobacterium radiobactor strain K84 + SX, Bacillus amyloliquefaciens strain PTA-4838 (Aveo(Trademark)) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MB1600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24

+ SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G4 1 + SX, Trichoderma virens strain GL-21 + SX, Trichoderma viride + SX, Variovorax paradoxus strain CGF4526 + SX, Harpin protein + SX.

A combination of the present ingredient in the above-mentioned Group (c) and the compound of the present invention:

1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyric acid + SX, methyl 5-(trifluoromethyl)benzo[b:thiophene-2-carboxylate + SX, 3-[(6-chloro-4-phenylquinazolin-2-yl)amino]propane-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

A combination of the present ingredient in the above-mentioned Group (d) and the compound of the present invention:

anthraquinone + SX, deet + SX, icaridin + SX.

Examples of the ratio of the compound of the present invention to the present ingredient include, but are not

particularly limited to 1000 : 1 to 1 : 1000, 500 : 1 to 1 : 500, 100 : 1 to 1 : 100, 50 : 1, 20 : 1, 10 : 1, 9 : 1, 8 : 1, 7 : 1, 6 . 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, and 1 : 50 in the weight ratio (the compound of the present invention: the present ingredient).

The present compound or the compound of the present invention has control effect on harmful arthropods having a reduced agent-sensitivity (which is also called "showing a resistance") to a certain insecticide, a certain mitecide, a certain molluscicide, or a certain nematicide (herein, collectively referred to as "Resistant harmful arthropod"), harmful mollusks, and harmful nematodes. Examples of the insecticide, the mitecide, the molluscicide and the nematodes include the following agents belonging to the Group a1 to Group a26.

Group a1: Acetylcholinesterase inhibitors

Group 1A carbamate compound and Group 1B organophosphate compound based on IRAC mode of action classification, which is a group consisting of alanycarb, aldicarb, bendiocarb, benfuracarb, butocarboxim, butoxycarboxim, carbaryl: NAC, carbofuran, carbosulfan, ethiofencarb, fenobucarb: BPMC, formetanate, furathiocarb, isoprocarb: MIPC, methiocarb, methomyl, methoxychlor, metolcarb, oxamyl, pirimicarb, promecarb, propoxur: PHC, thiodicarb, thiofanox, triazamate, trimethacarb, xylylcarb, acephate, azamethiphos, azinphos-ethyl, azinphos-methyl, cadusafos, chlorethoxyfos, chlorfenvinphos, chlormephos, chlorpyrifos, chlorpyrifos-methyl, coumaphos, cyanophos: CYAP, demeton, demeton-S-methyl, diazinon, dichlorvos: DDVP, dicrotophos, dimethoate, dimethylvinphos, disulfoton, EPN, ethion, ethoprophos, famphur, fenamiphos, fenchlorphos, fenitrothion: MEP, fenthion: MPP, fosthiazate, heptenophos, imicyafos, isofenphos, isopropyl-O-(methoxyaminothiophosphoryl)salicylate, isoxathion, malathion, mecarbam, methamidophos, methidathion: DMTP, mevinphos, monocrotophos, naled: BRP, omethoate, oxydemeton-methyl, parathion, parathion-methyl, phenthoate: PAP, phorate, phosalone, phosmet: PMP, phosphamidon, phoxim, pirimiphos-methyl, profenofos, propetamphos, prothiofos, pyraclofos, pyrazophos, pyridaphenthion, quinalphos, sulfotep, sulprofos, tebupirimfos, temephos, terbufos, tetrachlorvinphos, thiometon, toxaphene, triazophos, trichlorfon: DEP and vamidothion.

Group a2: GABA-gated chloride ion channel blockers

Group 2A cyclodiene organochlorine compound and Group 2B phenylpyrazole compound based on IRAC mode of action classification, which is a group consisting of chlordane, endosulfan, dieldrin, ethiprole, fipronil, flufiprole, and pyriprole.

Group a3: Sodium channel modulators

Group 3A pyrethroid compound and Group 3B compound based on IRAC mode of action classification, which is a group consisting of acrinathrin, allethrin, bifenthrin, kappa-bifenthrin, bioallethrin, bioresmethrin, cycloprothrin, cyfluthrin, beta-cyfluthrin, cyhalothrin, gamma-cyhalothrin, lambda-cyhalothrin, cypermethrin, alpha-cypermethrin, beta-cypermethrin, theta-cypermethrin, zeta-cypermethrin, sigma-cypermethrin, cyphenothrin, deltamethrin, empenthrin, esfenvalerate, etofenprox, fenpropathrin, fenvalerate, flucythrinate, flumethrin, fluvalinate, tau-fluvalinate, halfenprox, heptafluthrin, imiprothrin, kadethrin, meperfluthrin, momfluorothrin, permethrin, phenothrin, prallethrin, pyrethrins, resmethrin, silafluofen, tefluthrin, kappa- tefluthrin, tetramethrin, tetramethylfluthrin, tralomethrin, transfluthrin, benfluthrin, flufenoprox, flumethrin, furamethrin, metofluthrin, profluthrin, dimefluthrin, and DDT.

Group a4: Nicotinic acetylcholine receptor competitive modulators

Group 4A neonicotinoid compound, 4B Nicotine compound, 4C sulfoximine compound, 4D butenolide compound, and 4E mesoionic compound, based on IRAC mode of action classification, which is a group consisting of acetamiprid, clothianidin, dinotefuran, imidacloprid, nitenpyram, thiacloprid, thiamethoxam, flupyradifurone, sulfoxaflor, cycloxaprid, triflumezopyrim, dicloromezotiaz and flupyrimin.

Group a5: Nicotinic acetylcholine receptor allosteric modulators

Group 5 Spinosyn compound based on IRAC mode of action classification, which is a group consisting of spinosad and pinetoram.

Group a6: Glutamate-gated chloride channel allosteric modulators

Group 6 abamectin compound and milbemycin compound, based on IRAC mode of action classification, which is a group consisting of abamectin, emamectin-benzoate, lepimectin, milbemectin, dimadectin, doramectin, eprinomectin,

ivermectin, latidectin, selamectin, milbemycin D, moxidectin and nemadectin.

Group a7: Juvenile hormone mimics

Group 7A juvenile hormone analogues, Group 7B fenoxycarb, and Group 7C pyriproxyfen, which is a group consisting of hydroprene, kinoprene, methoprene, fenoxycarb and pyriproxyfen.

Group a8: Chordotonal organ TRPV channel modulators

Group 9 pyridine azomethine derivatives based on IRAC mode of action classification, which is a group consisting of Pymetrozine and Pyrifluquinazon.

Group a9: Microbial disruptors of insect midgut membranes

Group 11A Bacillus thuringiensis, Group 11B Bacillus sphaericus, based on IRAC mode of action classification, which is a group consisting ofBacillus sphaericus, Bacillus thuringiensis strain BD#32, Bacillus thuringiensis strain AQ52, Bacillus thuringiensis subsp. aizawai strain ABTS-1857, Bacillus thuringiensis subsp. kurstaki strain HD-1, Bacillus thuringiensis subsp. kurstaki strain BMP123, Bacillus thuringiensis subsp. Tenebriosis strain NB176, Bacillus thuringiensis var. israelensis, Bacillus thuringiensis var.aegypti, Bacillus thuringiensis var. colmeri, Bacillus thuringiensis var. darmstadiensis, Bacillus thuringiensis var. dendrolimus, Bacillus thuringiensis var. galleriae, Bacillus thuringiensis var. japonensis, Bacillus thuringiensis subsp. morrisoni, Bacillus thuringiensis var. san diego, Bacillus thuringiensis subsp. Thuringiensis strain MPPL002, Bacillus thuringiensis var. 7216, Bacillus thuringiensis var. T36, delta-endotoxins imparting a tolerance to Lepidoptera insects (such as Cry1A, Cry1Ab, altered CrylAb (partially detected Cry1Ab), Cry1Ac, Cry1Ab-Ac (hybrid protein by fusing Cry1Ab and Cry1Ac), Cry1C, Cry1F, Cry1Fa2 (modified cry1F), moCrylF (modified Cry1F), Cry1A. 105 (hybrid protein by fusing Cry1Ab, Cry1Ac, and Cry1F), Cry2Ab2, Cry2Ae, Cry9C, Vip3A, Vip3Aa20), delta-endotoxins imparting a tolerance to Coleoptera insects (such as Cry3A, mCry3A (modified Cry3A), Cry3Bb1, Cry34Ab1, Cry35Ab1), and delta-endotoxins having insecticidal activity to Culicidae family (such as Cry1A, Cry4A, Cry4B, Cry11A).

Group a10: Uncouplers of oxidative phosphorylation via disruption of the proton gradient

Group 13 compound based on IRAC mode of action classification, which is a group consisting of chlorfenapyr and sulfluramid.

Group a11: Nicotinic acetylcholine receptor channel blockers

Group 14 nereistoxin analogues based on IRAC mode of action classification, which is a group consisting of bensultap, cartap, cartap hydrochloride, thiocyclam, thiosultap-disodium, and thiosultap-monosodium.

Group a12: Inhibitors of chitin biosynthesis

Group 15 benzoylurea compound and Group 16 buprofezin based on IRAC mode of action classification, which is a group consisting of bistrifluron, chlorfluazuron, diflubenzuron, flucycloxuron, flufenoxuron, hexaflumuron, lufenuron, novaluron, noviflumuron, teflubenzuron, triflumuron, and buprofezin.

Group a13: Moulting disruptors

Group 17 compound based on IRAC mode of action classification, which is a group consisting of cyromazine.

Group a14: Moulting formone (Ecdysone) receptor agonists,

Group 18 diacylhydrazine compound based on IRAC mode of action classification, which is a group consisting of chromafenozide, halofenozide, methoxyfenozide, and tebufenozide.

Group a15: Voltage-dependent sodium channel blockers

Group 22A oxadiazines and Group 22B semicarbazones based on IRAC mode of action classification, which is a group consisting of indoxacarb and metaflumizone.

Group a16: Inhibitors of acetyl CoA carboxylase

Group 23 tetronic acid and tetramic acid derivatives based on IRAC mode of action classification, which is a group consisting of spirodiclofen, spiromesifen and spirotetramat.

Group a17: Ryanodine receptor modulators

Group 28 diamide compound based on IRAC mode of action classification, which is a group consisting of chlorantraniliprole, cyantraniliprole, cycloniliprole, flubendiamide, tetraniliprole, cyhalodiamide and tetrachlorantraniliprole

Group a18: Chordotonal organ modulators

Group 29 compound based on IRAC mode of action classification, which is a group consisting of Flonicamid.

Group a19: Mites growth regulators

Group 10 compound based on IRAC mode of action classification, which is a group consisting of etoxozole, clofentezine, and hexythiazox.

Group a20: Octopamine receptor agonists

Group 19 compound based on IRAC mode of action classification, which is a group consisting of amitraz.

Group a21: Inhibitors of mitochondrial electron transport chain complex IV

Group 24 compound based on IRAC mode of action classification, which is a group consisting of phosphine.

Group a22: Isoxazoline compound which is a group consisting of afoxolaner, fluralaner, sarolaner, and otilaner.

Group a23: Mitochondrial ATP synthase inhibitors

Group 12 compound based on IRAC mode of action classification, which is a group consisting of diafenthiuron.

Group a24: Inhibitors of mitochondrial electron transport chain complex III

Group 20 compound based on IRAC mode of action classification, which is a group consisting of acequinocyl and bifenazate.

Group a25: Inhibitors of mitochondrial electron transport chain complex I (MET I)

Group 21 compound based on IRAC mode of action classification, which is a group consisting of pyridaben, and fenpyroximate.

Group a26: Compound selected from a group consisting of azadiractin, and pyridalyl.

Examples of the causes of reduction in sensitivity include [1] an amino acid substitution in target protein, [2] a decrease of target protein, [3] a metabolism enhancement, [4] a reduction in a skin permeability, and [5] an enhancement of excretory function with membrane transporters. In [1], the target protein may contain one or a plural of amino acid substitutions. [3], the term of "metabolism enhancement" means an increased activity of metabolic enzyme (which is also caled "detoxification-decomposition enzyme") (such as cytochrome P450, carboxylesterase and glutathion-S-transferase). Also the causes of the reduction in sensitivity may include any one of or a plural of the groups selected from [1] the amino acid substitution in target protein, [2] the decrease in target protein, [3] the metabolism enhancement, [4] the reduction in a skin permeability, and [5] the enhancement of excretory function with membrane transporters.

Examples of harmful arthropods (such as harmful insects and harmful mites) having reduced sensitivity, harmful mollusks having reduced sensitivity, and harmful nematodes having reduced sensitivity include the followings.

The order of Hemiptera which has a reduced sensitivity to one or more compounds selected from Group a2;

The order of Hemiptera which has a reduced sensitivity to one or more compounds selected from Group a4;
The order of Hemiptera which has a reduced sensitivity to one or more compounds selected from Group a8;
The order of Hemiptera which has a reduced sensitivity to one or more compounds selected from Group a12;
The order of Hemiptera which has a reduced sensitivity to one or more compounds selected from Group a2 and one or more compounds selected from Group a4;
The order of Hemiptera which has a reduced sensitivity to one or more compounds selected from Group a2 and one or more compounds selected from Group a8;
The order of Hemiptera which has a reduced sensitivity to one or more compounds selected from Group a2 and buprofezin belonging to Group a12;
The order of Hemiptera which has a reduced sensitivity to one or more compounds selected from Group a4 and one or more compounds selected from Group a8;
The order of Hemiptera which has a reduced sensitivity to one or more compounds selected from Group a4 and buprofezin belonging to Group a12;
The order of Hemiptera which has a reduced sensitivity to one or more compounds selected from Group a8 and buprofezin belonging to Group a12;
The order of Hemiptera which has a reduced sensitivity to one or more compounds selected from Group a2, one or more compounds selected from Group a4, and one or more compounds selected from Group a8;
The order of Hemiptera which has a reduced sensitivity to one or more compounds selected from Group a2, one or more compounds selected from Group a4, and buprofezin belonging to Group a12;
The order of Hemiptera which has a reduced sensitivity to one or more compounds selected from Group a2, one or more compounds selected from Group a8, and buprofezin belonging to Group a12;
The order of Hemiptera which has a reduced sensitivity to one or more compounds selected from Group a4, one or more compounds selected from Group a8, and buprofezin belonging to Group a12;
The order of Hemiptera which has a reduced sensitivity to one or more compounds selected from Group a2, one or more compounds selected from Group a4, and one or more compounds selected from Group a8, and buprofezin belonging to Group a12;
The family Delphacidae which has a reduced sensitivity to one or more compounds selected from Group a2;
The family Delphacidae which has a reduced sensitivity to one or more compounds selected from Group a4;
The family Delphacidae which has a reduced sensitivity to one or more compounds selected from Group a8;
The family Delphacidae which has a reduced sensitivity to one or more compounds selected from Group a12;
The family Delphacidae which has a reduced sensitivity to one or more compounds selected from Group a2 and one or more compounds selected from Group a4;
The family Delphacidae which has a reduced sensitivity to one or more compounds selected from Group a2 and one or more compounds selected from Group a8;
The family Delphacidae which has a reduced sensitivity to one or more compounds selected from Group a2 and buprofezin belonging to Group a12;
The family Delphacidae which has a reduced sensitivity to one or more compounds selected from Group a4, and pymetrozine belonging to Group a8;
The family Delphacidae which has a reduced sensitivity to one or more compounds selected from Group a4, and buprofezin belonging to Group a12;
The family Delphacidae which has a reduced sensitivity to one or more compounds selected from Group a2, one or more compounds selected from Group a4, and pymetrozine belonging to Group a8;
The family Delphacidae which has a reduced sensitivity to one or more compounds selected from Group a2, one or more compounds selected from Group a4, and buprofezin belonging to Group a12;
The family Delphacidae which has a reduced sensitivity to one or more compounds selected from Group a2, pymetrozine belonging to Group a8, and buprofezin belonging to Group a12;
The family Delphacidae which has a reduced sensitivity to one or more compounds selected from Group a4, pymetrozine belonging to Group a8, and buprofezin belonging to Group a12;
The family Delphacidae which has a reduced sensitivity to one or more compounds selected from Group a2, one or more compounds selected from Group a4, pymetrozine belonging to Group a8, and buprofezin belonging to Group a12;
Nilaparvata lugens, Sogatella furcifera, or Laodelphax striatellus each which has a reduced sensitivity to one or more compounds selected from Group a2;
Nilaparvata lugens, Sogatella furcifera, or Laodelphax striatellus each which has a reduced sensitivity to one or more compounds selected from Group a4;
Nilaparvata lugens, Sogatella furcifera, or Laodelphax striatellus each which has a reduced sensitivity to one or more compounds selected from Group a8;
Nilaparvata lugens, Sogatella furcifera, or Laodelphax striatellus each which has a reduced sensitivity to one or

more compounds selected from Group a12;

Nilaparvata lugens, Sogatella furcifera, or Laodelphax striatellus each which has a reduced sensitivity to one or more compounds selected from Group a2 and one or more compounds selected from Group a4;

Nilaparvata lugens, Sogatella furcifera, or Laodelphax striatellus each which has a reduced sensitivity to one or more compounds selected from Group a2 and pymetrozine belonging to Group a8;

Nilaparvata lugens, Sogatella furcifera, or Laodelphax striatellus each which has a reduced sensitivity to one or more compounds selected from Group a2 and buprofezin belonging to Group a12;

Nilaparvata lugens, Sogatella furcifera, or Laodelphax striatellus each which has a reduced sensitivity to one or more compounds selected from Group a4 and pymetrozine belonging to Group a8;

Nilaparvata lugens, Sogatella furcifera, or Laodelphax striatellus each which has a reduced sensitivity to one or more compounds selected from Group a4 and buprofezin belonging to Group a12;

Nilaparvata lugens, Sogatella furcifera, or Laodelphax striatellus each which has a reduced sensitivity to one or more compounds selected from Group a2, one or more compounds selected from Group a4 and pymetrozine belonging to Group a8;

Nilaparvata lugens, Sogatella furcifera, or Laodelphax striatellus each which has a reduced sensitivity to one or more compounds selected from Group a2, one or more compounds selected from Group a4 and buprofezin belonging to Group a12;

Nilaparvata lugens, Sogatella furcifera, or Laodelphax striatellus each which has a reduced sensitivity to one or more compounds selected from Group a2, pymetrozine belonging to Group a8 and buprofezin belonging to Group a12;

Nilaparvata lugens, Sogatella furcifera, or Laodelphax striatellus each which has a reduced sensitivity to one or more compounds selected from Group a4, pymetrozine belonging to Group a8 and buprofezin belonging to Group a12;

Nilaparvata lugens, Sogatella furcifera, or Laodelphax striatellus each which has a reduced sensitivity to one or more compounds selected from Group a2, one or more compounds selected from Group a4, pymetrozine belonging to Group a8 and buprofezin belonging to Group a12;

The family of Aphididae which has a reduced sensitivity to one or more compounds selected from Group a3;

The family of Aphididae which has a reduced sensitivity to one or more compounds selected from Group a8;

The family of Aphididae which has a reduced sensitivity to one or more compounds selected from Group a3 and one or more compounds selected from Group a4;

The family of Aphididae which has a reduced sensitivity to one or more compounds selected from Group a3 and one or more compounds selected from Group a8;

The family of Aphididae which has a reduced sensitivity to one or more compounds selected from Group a4 and one or more compounds selected from Group a8;

The family of Aphididae which has a reduced sensitivity to one or more compounds selected from Group a3, one or more compounds selected from Group a4, and one or more compounds selected from Group a8;

Myzus persicae which has a reduced sensitivity to one or more compounds selected from Group a1;

Aphis gossypii or Myzus persicae each which has a reduced sensitivity to one or more compounds selected from Group a3;

Aphis gossypii or Myzus persicae each which has a reduced sensitivity to one or more compounds selected from Group a4;

Aphis gossypii or Myzus persicae each which has a reduced sensitivity to one or more compounds selected from Group a8;

Aphis gossypii or Myzus persicae each which has a reduced sensitivity to one or more compounds selected from Group a3 and one or more compounds selected from Group a4;

Aphis gossypii or Myzus persicae each which has a reduced sensitivity to one or more compounds selected from Group a3 and one or more compounds selected from Group a8;

Aphis gossypii or Myzus persicae each which has a reduced sensitivity to one or more compounds selected from Group a4 and one or more compounds selected from Group a8;

Aphis gossypii or Myzus persicae each which has a reduced sensitivity to one or more compounds selected from Group a3, one or more compounds selected from Group a4 and one or more compounds selected from Group a8;

Bemisia tabaci which has a reduced sensitivity to one or more compounds selected from Group a3;

Bemisia tabaci which has a reduced sensitivity to one or more compounds selected from Group a4;

Myzus persicae which has a reduced sensitivity to one or more compounds selected from Group a1;

Aphis gossypii which has a reduced sensitivity to carbamate compounds selected from Group a1 and/or organophosphate compounds selected from Group a1;

Nilaparvata lugens which has a reduced sensitivity to malathion, chlorpyrifos, diazinon, fenobucarb, carbofuran, ethiprole, fipronil, flufiprole, etofenprox, dinotefuran, imidacloprid, nitenpyram, sulfoxaflor, thiacloprid, thiamethoxam,

pymetrozine, and / or buprofezin;
Sogatella furcifera which has a reduced sensitivity to isoprocarb, chlorpyrifos, imidacloprid, and /or buprofezin;
Amrasca biguttula biguttula which has a reduced sensitivity to imidacloprid, thiamethoxam, acetamiprid, thiacloprid, clothianidin, and / or dinotefuran;
Amrasca biguttula biguttula which has a reduced sensitivity to pirimicarb, and / or organophosphate compounds selected from Group a1;
Myzus persicae which has a reduced sensitivity to triazamate, pirimicarb, endosulfan, deltamethrin, lambda-cyhalothrin, DDT, and / or imidacloprid;
Nasonovia ribisnigri which has a reduced sensitivity to pirimicarb, methomyl, acephate, dieldrin, and / or endosulfan;
Sitobion avenae which has a reduced sensitivity to lambda-cyhalothrin;
Trialeurodes vaporariorum which has a reduced sensitivity to one or more compounds selected from Group a4, bifenthrin and / or spiromesifen;
Bemisia tabaci which has a reduced sensitivity to pirimiphos-methyl, profenofos, endosulfan, fipronil, lambda-cyhalothrin, bifenthrin, alpha-cypermethrin, cartap, buprofezin, spiromesifen, chlorantraniliprole, diafenthiuron, and / or azadirachti;
Diaphorina citri which has a reduced sensitivity to chlorpyrifos, bifenthrin, cypermethrin, acetamiprid, imidacloprid, thiamethoxam, nitenpyram, and / or chlorfenapyr;
Euschistus heros which has a reduced sensitivity to monocrotophos, methamidophos, acephate, endosulfan, beta-cyfluthrin, and / or imidacloprid;
The order of Lepidoptera which has a reduced sensitivity to one or more compounds selected from Group a14;
The order of Lepidoptera which has a reduced sensitivity to one or more compounds selected from Group a15;
The order of Lepidoptera which has a reduced sensitivity to one or more compounds selected from Group a17;
The order of Lepidoptera which has a reduced sensitivity to one or more compounds selected from Group a14, and one or more compounds selected from Group a17;
Plutella xylostella which has a reduced sensitivity to one or more compounds selected from Group a15;
Plutella xylostella which has a reduced sensitivity to one or more compounds selected from Group a17;
Cnaphalocrocis medinalis which has a reduced sensitivity to one or more compounds selected from Group a14;
Cnaphalocrocis medinalis which has a reduced sensitivity to one or more compounds selected from Group a17;
Cnaphalocrocis medinalis which has a reduced sensitivity to one or more compounds selected from Group a14, and one or more compounds selected from Group a1.7;
Heliothis virescens which has a reduced sensitivity to one or more compounds selected from Group a3;
Helicoverpa armigera which has a reduced sensitivity to one or more compounds selected from Group a9;
Helicoverpa zea which has a reduced sensitivity to one or more compounds selected from Group a9;
Homona magnanima which has a reduced sensitivity to one or more compounds selected from Group a14;
Homona magnanima which has a reduced sensitivity to one or more compounds selected from Group a17;
Adoxophyes honmai which has a reduced sensitivity to one or more compounds selected from Group a14;
Adoxophyes honmai which has a reduced sensitivity to one or more compounds selected from Group a17;
Adoxophyes honmai which has a reduced sensitivity to one or more compounds selected from Group a14 and more compounds selected from Group a17;
Plutella xylostella which has a reduced sensitivity to one or more compounds selected from Group a3;
Tuta absoluta which has a reduced sensitivity to one or more compounds selected from Group a17;
Chilo suppressalis which has a reduced sensitivity to endosulfan, fipronil, and / or thiosultap-monosodium;
Ostrinia nubilalis which has a reduced sensitivity to deltamethrin, and / or lambda-cyhalothrin;
Diatraea saccharalis which has a reduced sensitivity to Cry1A, and / or CrylAc each which is a delta-endotoxin;
Leucinodes orbonalis which has a reduced sensitivity to profenofos, carbaryl, chlorpyrifos, endosulfan, deltamethrin, and / or fenvalerat;
Spodoptera litura which has a reduced sensitivity to profenofos, chlorpyrifos, quinalphos, phoxim, triazophos, methomyl, thiodicarb, endosulfan, fipronil, cypermethrin, deltamethrin, beta-cyfluthrin, spinosad, abamectin, emamectin-benzoate, lufenuron, diflubenzuron, methoxyfenozide, and / or indoxacar;
Spodoptera exigua which has a reduced sensitivity to chlorpyrifos, deltamethrin, cypermethrin, spinosad, abamectin, emamectin-benzoate, lufenuron, indoxacarb, and / or methoxyfenozide;
Mamestra brassicae which has a reduced sensitivity to Cry1Ab, CrylAc, Cry2Ab2, Cry2Ae, and / or CrylF each which is a delta-endotoxin;
Heliothis virescens which has a reduced sensitivity to sulprofos, profenofos, monocrotophos, parathion-methyl, thiodicarb, methomyl, toxaphene, endosulfan, cypermethrin, deltamethrin, DDT, and / or spinosad;
Helicoverpa armigera which has a reduced sensitivity to monocrotophos, chlorpyrifos, profenofos, methomyl, thiodicarb, toxaphene, endosulfan, fenvalerate, deltamethrin, cypermethrin, DDT, indoxacarb, and /or chlorantraniliprole;

Helicoverpa zea which has a reduced sensitivity to parathion-methyl, cypermethrin, endosulfan, and / or DDT;
Cydia pomonella which has a reduced sensitivity to lambda-cyhalothrin, thiacloprid, and / or methoxyfenozide;
Lobesia botrana which has a reduced sensitivity to deltamethrin, and / or indoxacarb;
Plutella xylostella which has a reduced sensitivity to methomyl, dieldrin, permethrin, fipronil, spinosad, abamectin, emamectin-benzoate, CrylC (which is a delta-endotoxin), lufenuron, indoxacarb, chlorantraniliprole, flubendiamide, and / or diafenthiuron;
The order of Thysanoptera which has a reduced sensitivity to one or more compounds selected from Group a2;
The order of Thysanoptera which has a reduced sensitivity to one or more compounds selected from Group a3;
The order of Thysanoptera which has a reduced sensitivity to one or more compounds selected from Group a4;
The order of Thysanoptera which has a reduced sensitivity to one or more compounds selected from Group a9;
The order of Thysanoptera which has a reduced sensitivity to one or more compounds selected from Group a17;
Thrips tabaci which has a reduced sensitivity to one or more compounds selected from Group a3;
Thrips tabaci which has a reduced sensitivity to profenofos, chlorpyrifos, deltamethrin and / or lambda-cyhalothrin;
Frankliniella occidentalis which has a reduced sensitivity to methiocarb, methomyl, bendiocarb, formetanate, carbofuran, malathion, chlorpyrifos, diazinon, methamidophos, dimethoate, dichlorvos, acephate, methidathion, toxaphene, endosulfan, fipronil, acrinathrin, alpha-cypermethrin, bifenthrin, deltamethrin, esfenvalerate, permethrin, taufluvalinate), cypermethrin, fenvalerate, cyfluthrin, imidacloprid, spinosad, spinetoram, abamectin, pyriproxyfen, and / or cyantraniliprole;
The order of Diptera which has a reduced sensitivity to one or more compounds selected from Group a2;
The order of Diptera which has a reduced sensitivity to one or more compounds selected from Group a3;
The order of Diptera which has a reduced sensitivity to one or more compounds selected from Group a4;
The order of Diptera which has a reduced sensitivity to one or more compounds selected from Group a9;
The order of Diptera which has a reduced sensitivity to one or more compounds selected from Group a17;
Liriomyza sativae which has a reduced sensitivity to permethrin, and / or fenvalerate;
Liriomyza trifolii which has a reduced sensitivity to methamidophos, parathion-methyl, pyrazophos, demeton, chlorpyrifos, triazophos, permethrin, cypermethrin, DDT, spinosad, abamectin, and / or cyromazine;
The order of Coleoptera which has a reduced sensitivity to one or more compounds selected from Group a2;
The order of Coleoptera which has a reduced sensitivity to one or more compounds selected from Group a3;
The order of Coleoptera which has a reduced sensitivity to one or more compounds selected from Group a4;
The order of Coleoptera which has a reduced sensitivity to one or more compounds selected from Group a9;
The order of Coleoptera which has a reduced sensitivity to one or more compounds selected from Group a17;
The order of Coleoptera which has a reduced sensitivity to one or more compounds selected from Group a2 and one or more compounds selected from Group a4;
The family of Chrysomelidae which has a reduced sensitivity to one or more compounds selected from Group a2;
The family of Chrysomelidae which has a reduced sensitivity to one or more compounds selected from Group a3;
The family of Chrysomelidae which has a reduced sensitivity to one or more compounds selected from Group a4;
The family of Chrysomelidae which has a reduced sensitivity to one or more compounds selected from Group a9;
The family of Chrysomelidae which has a reduced sensitivity to one or more compounds selected from Group a17;
The family of Chrysomelidae which has a reduced sensitivity to one or more compounds selected from Group a2 and one or more compounds selected from Group a4;
Oulema oryzae which has a reduced sensitivity to one or more compounds selected from Group a2;
Oulema oryzae which has a reduced sensitivity to one or more compounds selected from Group a4;
Oulema oryzae which has a reduced sensitivity to one or more compounds selected from Group a2 and one or more compounds selected from Group a4;
The genus of Diabrotica spp. which has a reduced sensitivity to one or more compounds selected from Group a2;
The genus of Diabrotica spp. which has a reduced sensitivity to one or more compounds selected from Group a3;
The genus of Diabrotica spp. which has a reduced sensitivity to one or more compounds selected from Group a4;
The genus of Diabrotica spp. which has a reduced sensitivity to one or more compounds selected from Group a9;
The genus of Diabrotica spp. which has a reduced sensitivity to one or more compounds selected from Group a17;
Diabrotica virgifera virgifera which has a reduced sensitivity to one or more compounds selected from Group a2;
Diabrotica virgifera virgifera which has a reduced sensitivity to one or more compounds selected from Group a3;
Diabrotica virgifera virgifera which has a reduced sensitivity to one or more compounds selected from Group a4;
Diabrotica virgifera virgifera which has a reduced sensitivity to one or more compounds selected from Group a9;
Diabrotica virgifera virgifera which has a reduced sensitivity to one or more compounds selected from Group a17;
Diabrotica undecimpunctata howardi which has a reduced sensitivity to one or more compounds selected from Group a2;
Diabrotica undecimpunctata howardi which has a reduced sensitivity to one or more compounds selected from Group a3;

Diabrotica undecimpunctata howardi which has a reduced sensitivity to one or more compounds selected from Group a4;
Diabrotica undecimpunctata howardi which has a reduced sensitivity to one or more compounds selected from Group a9;
Diabrotica undecimpunctata howardi which has a reduced sensitivity to one or more compounds selected from Group a17;
Diabrotica barberi which has a reduced sensitivity to one or more compounds selected from Group a3;
Diabrotica barberi which has a reduced sensitivity to one or more compounds selected from Group a4;
Diabrotica barberi which has a reduced sensitivity to one or more compounds selected from Group a9;
Diabrotica barberi which has a reduced sensitivity to one or more compounds selected from Group a17;
Diabrotica speciosa which has a reduced sensitivity to one or more compounds selected from Group a2;
Diabrotica speciosa which has a reduced sensitivity to one or more compounds selected from Group a3;
Diabrotica speciosa which has a reduced sensitivity to one or more compounds selected from Group a4;
Diabrotica speciosa which has a reduced sensitivity to one or more compounds selected from Group a9;
Diabrotica speciosa which has a reduced sensitivity to one or more compounds selected from Group a17;
Leptinotarsa decemlineata which has a reduced sensitivity to one or more compounds selected from Group a2;
Leptinotarsa decemlineata which has a reduced sensitivity to one or more compounds selected from Group a3;
Leptinotarsa decemlineata which has a reduced sensitivity to one or more compounds selected from Group a4;
Leptinotarsa decemlineata which has a reduced sensitivity to one or more compounds selected from Group a11;
Leptinotarsa decemlineata which has a reduced sensitivity to one or more compounds selected from Group a17;
Phyllotreta striolata which has a reduced sensitivity to one or more compounds selected from Group a3;
Phyllotreta striolata which has a reduced sensitivity to one or more compounds selected from Group a4;
Phyllotreta striolata which has a reduced sensitivity to one or more compounds selected from Group a17;
Phyllotreta cruciferae which has a reduced sensitivity to one or more compounds selected from Group a3;
Phyllotreta cruciferae which has a reduced sensitivity to one or more compounds selected from Group a4;
Phyllotreta cruciferae which has a reduced sensitivity to one or more compounds selected from Group a17;
Phyllotreta pusilla which has a reduced sensitivity to one or more compounds selected from Group a3;
Phyllotreta pusilla which has a reduced sensitivity to one or more compounds selected from Group a4;
Phyllotreta pusilla which has a reduced sensitivity to one or more compounds selected from Group a17;
Psylliodes chrysocephala which has a reduced sensitivity to one or more compounds selected from Group a3;
Psylliodes chrysocephala which has a reduced sensitivity to one or more compounds selected from Group a4;
Psylliodes chrysocephala which has a reduced sensitivity to one or more compounds selected from Group a17;
Psylliodes punctulata which has a reduced sensitivity to one or more compounds selected from Group a3;
Psylliodes punctulata which has a reduced sensitivity to one or more compounds selected from Group a4;
Psylliodes punctulata which has a reduced sensitivity to one or more compounds selected from Group a17;
The family of Curculionidae which has a reduced sensitivity to one or more compounds selected from Group a2;
The family of Curculionidae which has a reduced sensitivity to one or more compounds selected from Group a4;
The family of Curculionidae which has a reduced sensitivity to one or more compounds selected from Group a2 and one or more compounds selected from Group a4;
Lissorhoptrus oryzophilus which has a reduced sensitivity to one or more compounds selected from Group a2;
Lissorhoptrus oryzophilus which has a reduced sensitivity to one or more compounds selected from Group a4;
Lissorhoptrus oryzophilus which has a reduced sensitivity to one or more compounds selected from Group a2 and one or more compounds selected from Group a4;
The family of Elateridae which has a reduced sensitivity to one or more compounds selected from Group a2;
The family of Elateridae which has a reduced sensitivity to one or more compounds selected from Group a3;
The family of Elateridae which has a reduced sensitivity to one or more compounds selected from Group a4;
Melanotus okinawensis which has a reduced sensitivity to one or more compounds selected from Group a2;
Melanotus okinawensis which has a reduced sensitivity to one or more compounds selected from Group a3;
Melanotus okinawensis which has a reduced sensitivity to one or more compounds selected from Group a4;
Agriotes fuscicollis which has a reduced sensitivity to one or more compounds selected from Group a2;
Agriotes fuscicollis which has a reduced sensitivity to one or more compounds selected from Group a3;
Agriotes fuscicollis which has a reduced sensitivity to one or more compounds selected from Group a4;
Melanotus legatus which has a reduced sensitivity to one or more compounds selected from Group a2;
Melanotus legatus which has a reduced sensitivity to one or more compounds selected from Group a3;
Melanotus legatus which has a reduced sensitivity to one or more compounds selected from Group a4;
The Genus of Anchastus spp. which has a reduced sensitivity to one or more compounds selected from Group a2;
The Genus of Anchastus spp. which has a reduced sensitivity to one or more compounds selected from Group a3;
The Genus of Anchastus spp. which has a reduced sensitivity to one or more compounds selected from Group a4;

The genus of Conoderus spp. which has a reduced sensitivity to one or more compounds selected from Group a2;
The genus of Conoderus spp. which has a reduced sensitivity to one or more compounds selected from Group a3;
The genus of Conoderus spp. which has a reduced sensitivity to one or more compounds selected from Group a4;
The genus of Ctenicera spp. which has a reduced sensitivity to one or more compounds selected from Group a2;
The genus of Ctenicera spp. which has a reduced sensitivity to one or more compounds selected from Group a3;
The genus of Ctenicera spp. which has a reduced sensitivity to one or more compounds selected from Group a4;
The genus of Limonius spp. which has a reduced sensitivity to one or more compounds selected from Group a2;
The genus of Limonius spp. which has a reduced sensitivity to one or more compounds selected from Group a3;
The genus of Limonius spp. which has a reduced sensitivity to one or more compounds selected from Group a4;
The genus of Aeolus spp. which has a reduced sensitivity to one or more compounds selected from Group a2;
The genus of Aeolus spp. which has a reduced sensitivity to one or more compounds selected from Group a3;
The genus of Aeolus spp. which has a reduced sensitivity to one or more compounds selected from Group a4;
Psylliodes chrysocephala which has a reduced sensitivity to lambda-cyhalothrin;
Leptinotarsa decemlineata which has a reduced sensitivity to carbosulfan, carbofuran, phosalone, chlorpyrifos, azinphos-methyl, cyhalothrin, lambda-cyhalothrin, deltamethrin, permethrin, imidacloprid, dinotefuran, clothianidin, thiacloprid, thiamethoxam, and / or nitenpyram;
Brassicogethes aeneus which has a reduced sensitivity to phosalone, lambda-cyhalothrin, cypermethrin, alpha-cypermethrin, zeta-cypermethrin, bifenthrin, beta-cyfluthrin, deltamethrin, esfenvalerate, tau-fluvalinate, and / or acetamiprid.
The order of Acari which has a reduced sensitivity to one or more compounds selected from Group a2;
The order of Acari which has a reduced sensitivity to one or more compounds selected from Group a3;
The order of Acari which has a reduced sensitivity to one or more compounds selected from Group a4;
The order of Acari which has a reduced sensitivity to one or more compounds selected from Group a9;
The order of Acari which has a reduced sensitivity to one or more compounds selected from Group a17;
Tetranychus urticae which has a reduced sensitivity to one or more compounds selected from Group a19, bifenthrin, abamectin, milbemectin, acequinocyl and / or bifenazate;
Panonychus ulmi which has a reduced sensitivity to fenpropathrin, spirodiclofen, clofentezine, hexythiazox, pyridaben, and / or fenpyroximate.
Cimex lectularius which has a reduced sensitivity to one or more compounds selected from Group a1;
Cimex lectularius which has a reduced sensitivity to one or more compounds selected from Group a2;
Cimex lectularius which has a reduced sensitivity to one or more compounds selected from Group a3;
Cimex lectularius which has a reduced sensitivity to one or more compounds selected from Group a4;
Cimex lectularius which has a reduced sensitivity to one or more compounds selected from Group a5;
Cimex lectularius which has a reduced sensitivity to one or more compounds selected from Group a10;
Cimex lectularius which has a reduced sensitivity to bendiocarb, diazinon, dichlorvos, fenitrothion, fenobucarb, malathion, propoxur, dieldrin, fipronil, allethrin, bifenthrin, cyfluthrin, beta-cyfluthrin, lambda-cyhalothrin, cypermethrin, alpha-cypermethrin, deltamethrin, esfenvalerate, etofenprox, permethrin, phenothrin, pyrethrins, tetramethrin, DDT, acetamiprid, dinotefuran, imidacloprid, thiamethoxam, spinosad, and / or chlorfenapyr;
Cimex hemipterus which has a reduced sensitivity to one or more compounds selected from Group a1;
Cimex hemipterus which has a reduced sensitivity to one or more compounds selected from Group a2;
Cimex hemipterus which has a reduced sensitivity to one or more compounds selected from Group a3;
Cimex hemipterus which has a reduced sensitivity to one or more compounds selected from Group a4;
Cimex hemipterus which has a reduced sensitivity to one or more compounds selected from Group a10;
Cimex hemipterus which has a reduced sensitivity to bendiocarb, diazinon, fenitrothion, fenobucarb, malathion, propoxur, dieldrin, fipronil, allethrin, bifenthrin, cyfluthrin, lambda-cyhalothrin, cypermethrin, alpha-cypermethrin, deltamethrin, esfenvalerate, etofenprox, permethrin, DDT, imidacloprid, and / or chlorfenapyr.
Musca domestica which has a reduced sensitivity to one or more compounds selected from Group a1;
Musca domestica which has a reduced sensitivity to one or more compounds selected from Group a2;
Musca domestica which has a reduced sensitivity to one or more compounds selected from Group a3;
Musca domestica which has a reduced sensitivity to one or more compounds selected from Group a4;
Musca domestica which has a reduced sensitivity to one or more compounds selected from Group a5;
Musca domestica which has a reduced sensitivity to one or more compounds selected from Group a6;
Musca domestica which has a reduced sensitivity to one or more compounds selected from Group a7;
Musca domestica which has a reduced sensitivity to one or more compounds selected from Group a11;
Musca domestica which has a reduced sensitivity to one or more compounds selected from Group a12;
Musca domestica which has a reduced sensitivity to one or more compounds selected from Group a13;
Musca domestica which has a reduced sensitivity to one or more compounds selected from Group a14;
Musca domestica which has a reduced sensitivity to one or more compounds selected from Group a15;

Musca domestica which has a reduced sensitivity to azamethiphos, azinphos-methyl, chlorpyrifos, chlorpyrifos-methyl, diazinon, dichlorvos, dimethoate, fenchlorphos, fenitrothion, methomyl, methoxychlor, naled, parathion-methyl, phoxim, profenofos, propoxur, tetrachlorvinphos, triazophos, trichlorfon, dieldrin, endosulfan, fipronil, bifenthrin, cyfluthrin, beta-cyfluthrin, lambda-cyhalothrin, cypermethrin, beta-cypermethrin, cyphenothrin, deltamethrin, esfenvalerate, phenothrin, pyrethrins, resmethrin, DDT, acetamiprid, dinotefuran, imidacloprid, nitenpyram, thiamethoxam, spinosad, abamectin, emamectin-benzoate, methoprene, pyriproxyfen, cartap, diflubenzuron, lufenuron, triflumuron, cyromazine, methoxyfenozide, and / or indoxacarb;
Simulium damnosum which has a reduced sensitivity to one or more compounds selected from Group a1;
Simulium damnosum which has a reduced sensitivity to one or more compounds selected from Group a3;
Culex pipiens pallens which has a reduced sensitivity to one or more compounds selected from Group a2;
Culex pipiens pallens which has a reduced sensitivity to one or more compounds selected from Group a3;
Culex pipiens pallens which has a reduced sensitivity to bifenthrin, beta-cyfluthrin, lambda-cyhalothrin, cypermethrin, deltamethrin, permethrin, and / or resmethrin.
Culex pipiens f. molestus which has a reduced sensitivity to one or more compounds selected from Group a3;
Culex pipiens f. molestus hich has reduced sensitivity to deltamethrin, etofenprox, permethrin, and / or phenothrin;
Culex quinquefasciatus which has a reduced sensitivity to one or more compounds selected from Group a1;
Culex quinquefasciatus which has a reduced sensitivity to one or more compounds selected from Group a3;
Culex quinquefasciatus which has a reduced sensitivity to temephos, bifenthrin, beta-cyfluthrin, lambda-cyhalothrin, cypermethrin, deltamethrin, permethrin, and / or resmethrin;
Aedes aegypti which has a reduced sensitivity to one or more compounds selected from Group a1;
Aedes aegypti which has a reduced sensitivity to one or more compounds selected from Group a3;
Aedes aegypti which has a reduced sensitivity to one or more compounds selected from Group a7;
Aedes aegypti which has a reduced sensitivity to one or more compounds selected from Group a9;
Aedes aegypti which has a reduced sensitivity to chlorpyrifos, dichlorvos, fenitrothion, fenthion, malathion, pirimiphos-methyl, propoxur, temephos, allethrin, bifenthrin, cyfluthrin, lambda-cyhalothrin, cypermethrin, alpha-cypermethrin, deltamethrin, etofenprox, permethrin, phenothrin, DDT, and / or methoprene;
Aedes aegypti which has a reduced sensitivity to Cry4A, or Cry4B each which is a delta-endotoxin.
Anopheles gambiae which has a reduced sensitivity to one or more compounds selected from Group a1;
Anopheles gambiae which has a reduced sensitivity to one or more compounds selected from Group a2;
Anopheles gambiae which has a reduced sensitivity to one or more compounds selected from Group a3;
Anopheles gambiae which has a reduced sensitivity to bendiocarb, fenitrothion, pirimiphos-methyl, dieldrin, lambda-cyhalothrin, deltamethrin, permethrin, and / or DDT;
Anopheles coluzzii which has a reduced sensitivity to one or more compounds selected from Group a1;
Anopheles coluzzii which has a reduced sensitivity to one or more compounds selected from Group a2;
Anopheles coluzzii which has a reduced sensitivity to one or more compounds selected from Group a3;
Anopheles coluzzii which has a reduced sensitivity to bendiocarb, fenitrothion, dieldrin, lambda-cyhalothrin, deltamethrin, permethrin, and / or DDT;
Anopheles arabiensis which has a reduced sensitivity to one or more compounds selected from Group a1;
Anopheles arabiensis which has a reduced sensitivity to one or more compounds selected from Group a2;
Anopheles arabiensis which has a reduced sensitivity to one or more compounds selected from Group a3;
Anopheles arabiensis which has a reduced sensitivity to bendiocarb, malathion, dieldrin, lambda-cyhalothrin, deltamethrin, permethrin, and / or DDT;
Anopheles funestus which has a reduced sensitivity to one or more compounds selected from Group a2;
Anopheles funestus which has a reduced sensitivity to one or more compounds selected from Group a3;
Anopheles funestus which has a reduced sensitivity to dieldrin, lambda-cyhalothrin, deltamethrin, permethrin, and / or DDT;
Tribolium castaneum which has a reduced sensitivity to one or more compounds selected from Group a1;
Tribolium castaneum which has a reduced sensitivity to one or more compounds selected from Group a3;
Tribolium castaneum which has a reduced sensitivity to one or more compounds selected from Group a5;
Tribolium castaneum which has a reduced sensitivity to one or more compounds selected from Group a21;
Tribolium castaneum which has a reduced sensitivity to carbaryl, chlorpyrifos, chlorpyrifos-methyl, cyanophos, diazinon, dichlorvos, fenitrothion, malathion, phoxim, pirimiphos-methyl, promecarb, propoxur, temephos, tetrachlorvinphos, bioresmethrin, cyfluthrin, cyhalothrin, cypermethrin, deltamethrin, permethrin, phenothrin, pyrethrins, DDT, spinosad, and / or phosphine;
Blattella germanica which has a reduced sensitivity to one or more compounds selected from Group a1;
Blattella germanica which has a reduced sensitivity to one or more compounds selected from Group a2;
Blattella germanica which has a reduced sensitivity to one or more compounds selected from Group a3;
Blattella germanica which has a reduced sensitivity to one or more compounds selected from Group a4;

Blattella germanica which has a reduced sensitivity to one or more compounds selected from Group a6;
Blattella germanica which has a reduced sensitivity to chlorpyrifos, malathion, propoxur, fipronil, beta-cyfluthrin, cypermethrin, deltamethrin, permethrin, pyrethrins, DDT, imidacloprid, and / or abamectin;
Ctenocephalides felis which has a reduced sensitivity to one or more compounds selected from Group a1;
Ctenocephalides felis which has a reduced sensitivity to one or more compounds selected from Group a2;
Ctenocephalides felis which has a reduced sensitivity to one or more compounds selected from Group a3;
Ctenocephalides felis which has a reduced sensitivity to one or more compounds selected from Group a4;
Ctenocephalides felis which has a reduced sensitivity to one or more compounds selected from Group a5;
Ctenocephalides felis which has a reduced sensitivity to bendiocarb, carbaryl, chlorfenvinphos, chlorpyrifos, diazinon, fenthion, isofenphos, propetamphos, propoxur, malathion, fipronil, cyfluthrin, cypermethrin, fluvalinate, permethrin, pyrethrins, imidacloprid, and / or spinosad;
Dermacentor variabilis which has a reduced sensitivity to one or more compounds selected from Group a3;
Dermacentor variabilis which has a reduced sensitivity to DDT;
Rhipicephalus microplus which has a reduced sensitivity to one or more compounds selected from Group a1;
Rhipicephalus microplus which has a reduced sensitivity to one or more compounds selected from Group a2;
Rhipicephalus microplus which has a reduced sensitivity to one or more compounds selected from Group a3;
Rhipicephalus microplus which has a reduced sensitivity to one or more compounds selected from Group a6;
Rhipicephalus microplus which has a reduced sensitivity to one or more compounds selected from Group a20;
Rhipicephalus microplus which has a reduced sensitivity to carbaryl, chlorpyrifos, coumaphos, fipronil, pyriprole, cypermethrin, deltamethrin, flumethrin, permethrin, ivermectin, and / or amitraz;
Rhipicephalus sanguineus which has a reduced sensitivity to one or more compounds selected from Group a1;
Rhipicephalus sanguineus which has a reduced sensitivity to one or more compounds selected from Group a3;
Rhipicephalus sanguineus which has a reduced sensitivity to one or more compounds selected from Group a20;
Rhipicephalus sanguineus which has a reduced sensitivity to permethrin, and / or amitraz;

Examples of harmful arthropods (such as harmful insects and harmful mites) having reduced sensitivity, harmful mollusks having reduced sensitivity, and harmful nematodes having reduced sensitivity wherein the reduced sensitivity is caused by one or more amino acid substitutions in target protein include the followings.

Aphis gossypii which has an amino acid substitution with R81T in β subunit of nicotinic acetylcholine receptor;
Myzus persicae which has an amino acid substitution with R81T in β subunit of nicotinic acetylcholine receptor;
Plutella xylostella which has an amino acid substitution with G4946E in ryanodine receptor;
Plutella xylostella which has an amino acid substitution with F1845Y or V1848I in ryanodine receptor;
Sogatella furcifera which has an amino acid substitution with A2'N in GABA-gated chloride ion channel;
Aphis gossypii which has amino acid substitution(s) with S431F, and / or A302S in acetylcholinesterase;
Bemisia tabaci which has amino acid substitution(s) with L925I, and / or T929V in voltage-dependent sodium channel;
Myzus persicae which has an amino acid substitution with S431F in acetylcholinesterase;
Myzus persicae which has amino acid substitution(s) with L1014F, M918T, and / or TL932F in voltage-dependent sodium channel;
Myzus persicae which has amino acid substitution(s) with A302S, and / or A302G in GABA-gated chloride ion channel;
Frankliniella occidentalis which has amino acid substitution(s) with L1014F, and / or M918T in voltage-dependent sodium channel;
Frankliniella occidentalis which has amino acid substitution(s) with G275E in α6 subunit of nicotinic acetylcholine receptor;
Helicoverpa zea which has amino acid substitution(s) with L1029H, V421M, V421A, V421G and / or I951V in α6 subunit of voltage-dependent sodium channel;
Heliothis virescens which has amino acid substitution(s) with L1029H, D1561V, E1565G, and / or V421M in voltage-dependent sodium channel;
Leptinotarsa decemlineata which has amino acid substitution(s) with R30K, S291G, and / or I392T in acetylcholinesterase;
Leptinotarsa decemlineata which has an amino acid substitution with L1014F in voltage-dependent sodium channel;
Sogatella furcifera which has an amino acid substitution with S298P in acetylcholinesterase type 1;
Tetranychus urticae which has an amino acid substitution with G323D in GluCllsubunit of glutamate receptor;
Tetranychus urticae which has an amino acid substitution with G326E in GluCl3subunit of glutamate receptor;
Thrips tabaci which has amino acid substitution(s) with M918T, M918L, T929I, V1010A, and / or L1014F in voltage-dependent sodium channel;
Trialeurodes vaporariorum which has amino acid substitution(s) with L925I, and /or T929I in voltage-dependent sodium channel;

Tuta absoluta which has amino acid substitution(s) with G4903V, G4903E, I4746T, and / or I4746M in ryanodine receptor;
Nilaparvata lugens which has an amino acid substitution with A301S in GABA-gated chloride ion channel;
Nilaparvata lugens which has an amino acid substitution with Y151S in α1 and α3 subunits of nicotinic acetylcholine receptor;
Ostrinia nubilalis which has an amino acid substitution with L1014F in voltage-dependent sodium channel;
Panonychus ulmi which has an amino acid substitution with F1538I in voltage-dependent sodium channel;
Psylliodes chrysocephala which has an amino acid substitution with L1014F in voltage-dependent sodium channel;
Sitobion avenae which has an amino acid substitution with L1014F in voltage-dependent sodium channel;
Plutella xylostella which has amino acid substitution(s) with A282S, A282G, and / or A302S in GABA-gated chloride ion channel;
Plutella xylostella which has amino acid substitution(s) with M918I, T929I, and /or L1014F in voltage-dependent sodium channel.
Cimex lectularius which has amino acid substitution(s) with V419L, L925I, and /or I936F in α subunit of voltage-dependent sodium channel;
Musca domestica which has amino acid substitution(s) with V180L, V260L, G262A, G262V, G342A, G342V, G365A, F327Y, F407Y, and / or G445A in acetylcholinesterase;
Musca domestica which has amino acid substitution(s) with M918T, L1014F and / or L1014H in α subunit of voltage-dependent sodium channel;
Musca domestica which has an amino acid substitution with A302S in GABA-gated chloride ion channel;
Culex pipiens pallens which has amino acid substitution(s) with L1014F, and / or L1014S in voltage-dependent sodium channel;
Culex pipiens pallens which has an amino acid substitution with A302S in GABA-gated chloride ion channel;
Culex pipiens f. molestus which has amino acid substitution(s) with L1014F, and / or L1014S in voltage-dependent sodium channel;
Culex quinquefasciatus which has amino acid substitution(s) with L1014F, and / or L1014S in voltage-dependent sodium channel;
Culex quinquefasciatus which has an amino acid substitution with G119S in acetylcholinesterase;
Aedes aegypti which has amino acid substitution(s) with V410L, G923V, L982W, S989P, I1011M, I1011V, V1016I, V1016G, T1520I, F1534C, and / or D1763Y in voltage-dependent sodium channel;
Anopheles gambiae which has amino acid substitution(s) with L1014S, and / or N1575Y in voltage-dependent sodium channel;
Anopheles gambiae which has an amino acid substitution with G119S in acetylcholinesterase;
Anopheles coluzzii which has amino acid substitution(s) with L1014F, and / or N1575Y in voltage-dependent sodium channel;
Anopheles arabiensis which has an amino acid substitution with L1014F in voltage-dependent sodium channel;
Anopheles funestus which has amino acid substitution(s) with A296S, and / or V327I in GABA-gated chloride ion channel;
Tribolium castaneum which has an amino acid substitution with A302S in GABA-gated chloride ion channel;
Blattella germanica which has amino acid substitution(s) with D58G, E434K, C764R, L933F, and /or P1880L in voltage-dependent sodium channel;
Blattella germanica which has an amino acid substitution with A302S in GABA-gated chloride ion channel;
Ctenocephalides felis which has amino acid substitution(s) with T929V, and / or L1014F in voltage-dependent sodium channel;
Ctenocephalides felis which has an amino acid substitution with A302 in GABA-gated chloride ion channel;
Rhipicephalus microplus which has amino acid substitution(s) with C190A, and / or F1550I in voltage-dependent sodium channel.

Examples of harmful arthropods (such as harmful insects and harmful mites) having reduced sensitivity, harmful mollusks having reduced sensitivity, and harmful nematodes having reduced sensitivity wherein the reduced sensitivity is caused by a decrease of target protein include the followings.

Musca domestica in which an expression level of α2 subunit in nicotinic acetylcholine receptor.

Examples of the causes of the increased activity of metabolic enzyme include an overexpression (which is also called "quantitative variation") of a metabolic enzyme gene, and an increased affinity (which is also called "qualitative variation") with a drug due to amino acid substitution(s) in metabolic enzyme. One or a plural of the cause (s) of the increased activity of these metabolic enzyme may be included.

Examples of harmful arthropods (such as harmful insects and harmful mites) having increased activity of metabolic enzyme, harmful mollusks having increased activity of metabolic enzyme and harmful nematodes having increased

activity of metabolic enzyme wherein the reduced sensitivity is caused by an overexpression of metabolic enzyme include the followings.

Nilaparvata lugens in which CYP6ER1 (one kind of cytochrome P450 gene) is overexpressed;
Amrasca biguttula biguttula in which a glutathione-S-transferase gene is overexpressed;
Aphis gossypii in which a cytochrome P450 monooxygenase gene is overexpressed;
Bemisia tabaci in which CYP6CM1 (one kind of cytochrome P450-dependent monooxygenase gene) is overexpressed:

Bemisia tabaci in which cytochrome P450-dependent monoxygenase gene is overexpressed;
Chilo suppressalis in which carboxylesterase gene, and /or microsomal-O-demethylase gene is / are overexpressed;
Cydia pomonella in which glutathione-S-transferase gene and /or cytochrome P450 monooxygenase gene is overexpressed;
Diatraea saccharalis in which an expression level of three kinds of aminopeptidase N genes (DsAPN1, DsAPN2, and DsAPN3) and / or DsCAD1 (one kind of cadherin gene) is / are decreased;
Liriomyza trifolii in which cytochrome P450 gene is overexpressed;
Brassicogethes aeneus in which cytochrome P450 gene is overexpressed;
Myzus persicae in which CYP6CY3 (one kind of cytochrome P450 gene) is overexpressed;
Myzus persicae in which a gene of E4 as one kind of carboxylesterases and / or a FE4 gene as one kind of carboxylesterases is / are overexpressed;
Nasonovia ribisnigri in which esterase gene is overexpressed;
Nasonovia ribisnigri in which a glutathione S-transferases gene is overexpressed;
Euschistus heros in which esterase gene is overexpressed;
Euschistus heros in which EST-2 gene as one kind of cholinesterase, and / or EST-4 gene as one kind of cholinesterase is / are overexpressed;
Frankliniella occidentalis in which esterase gene is overexpressed;
Frankliniella occidentalis in which cytochrome P450 monooxygenase gene is overexpressed;
Frankliniella occidentalis in which glutathione S-transferase gene is overexpressed;
Helicoverpa armigera in which CYP337B3 (one kind of cytochrome P450 gene) is overexpressed;
Helicoverpa armigera in which cytochrome P450 monooxygenases gene is overexpressed;
Helicoverpa armigera in which glutathione S-transferases gene is overexpressed;
Helicoverpa armigera in which esterase gene is overexpressed;
Heliothis virescens in which glutathione-S-transferase gene is overexpressed;
Heliothis virescens in which esterase gene is overexpressed;
Heliothis virescens in which cytochrome P450 gene is overexpressed;
Leptinotarsa decemlineata in which glutathione-S-transferasegene is overexpresse;
Leptinotarsa decemlineata in which esterase gene is overexpressed;
Leptinotarsa decemlineata in which cytochrome P450 gene is overexpressed;
Tetranychus urticae in which TuGSTd14 (one kind of glutathione-S-transferase gene) is overexpresse;
Trialeurodes vaporariorum in which CYP6CM1 (one kind of cytochrome P450 gene) is overexpressed;
Tribolium castaneum in which cytochrome P450 gene is overexpressed;
Nilaparvata lugens in which expression level of CYP6AY1 (one kind of cytochrome P450 gene) is decreased;
Nilaparvata lugens in which carboxylesterases gene is overexpressed;
Nilaparvata lugens in which Nl-EST1 gene as one kind of carboxylesterases is overexpressed;
Nilaparvata lugens in which CYP6ER1, CYP6AY1, and /or CYP6FU1 (one kind of cytochrome P450 gene) is / are overexpressed;
Ostrinia nubilalis in which cytochrome P450 monooxygenase gene is overexpressed;
Ostrinia nubilalis in which glutathione-S-transferase gene is overexpressed;
Panonychus ulmi in which cytochrome P450-dependent monoxygenase gene is overexpressed;
Psylliodes chrysocephala in which cytochrome P450 gene is overexpressed;
Cimex lectularius in which CYP397A1, CYP398A1, CYP6DN1, CYP4CM1, and / or CYP400A1 (each is one kind of cytochrome P450 gene) is / are overexpressed;
Cimex lectularius in which CE3959, and / or CE21331 (each is one kind of carboxylesterases gene) is /are overexpressed;
Cimex lectularius in which S1 (one kind of glutathione-S-transferase gene) is overexpressed;
Musca domestica in which CYP6A1, CYP6A5v1, CYP6A5v2, CYP6A36, CYP6A40, CYP6D1, CYP6D1v1, CYP6D3, CYP6D8, CYP6G2, and / or CYP6G4 (each is one kind of cytochrome P450 gene) is /are overexpressed;
Culex quinquefasciatus in which CYP9M10, CYP6AA7 and / or CYP4H34 (each is one kind of cytochrome P450 gene) is overexpressed;
Aedes aegypti in which esterase gene is overexpressed;
Aedes aegypti in which glutathione-S-transferase gene is overexpressed;
Aedes aegypti in which CYP4D24, CYP6BB2, CYP6F3, CYP6M11, CYP6N12, and / or CYP9M6 (each is one kind of cytochrome P450 gene) is overexpressed;
Aedes aegypti in which carboxylesterase gene is overexpressed;
Anopheles gambiae in which CYP4P16, CYP4P17, CYP6P3, CYP6Z1, CYP12F1, and / or CYP325A3 (each is one kind of cytochrome P450 gene) is overexpressed;
Anopheles gambiae in which GSTE2 (one kind of glutathione-S-transferase gene) is overexpressed;
Anopheles gambiae in which PX13A, and / or PX13B (each is one kind of peroxidase gene) is overexpressed;
Anopheles gambiae in which esterase gene is overexpressed;
Anopheles arabiensis in which CYP6P4 (one kind of cytochrome P450 gene) is overexpressed;
Anopheles arabiensis in which carboxylesterase gene is overexpressed;
Anopheles arabiensis in which beta-esterase gene is overexpressed;
Anopheles funestus in which CYP6P4, and / or CYP6P9 (each is one kind of cytochrome P450 gene) is overexpressed;
Tribolium castaneum in which CYP6BQ9 (one kind of cytochrome P450 gene) is overexpressed;
Tribolium castaneum in which glutathione-S-transferase gene is overexpressed;
Tribolium castaneum in which Epsilon (one kind of glutathione-S-transferase gene) is overexpressed;
Blattella germanica icin which CYP4G19 (one kind of cytochrome P450 gene) is overexpressed.

Examples of harmful arthropods having an increased activity of metabolic enzyme (such as harmful insects having an increased activity of metabolic enzyme, harmful mites having an increased activity of metabolic enzyme), harmful mollusks having an increased activity of metabolic enzyme and harmful nematodes having an increased activity of metabolic enzyme wherein the increased activity of metabolic enzyme is caused by increased affinity with a drug due to amino acid substitution(s) in metabolic enzyme include the followings.

Nilaparvata lugens which has CYP6ER1vA (one kind of mutated CYP6ER1) in which amino acid substitutions with T318S, A375Δ and A376G are contained;
Nilaparvata lugens which has CYP6ER1vB (one kind of mutated CYP6ER1) in which amino acid substitutions with T318S and A377Δ are contained;
Musca domestica which has carboxyesterase in which amino acid substitutions with W251L, and / or W251S are contained;
Tribolium castaneum which has dihydrolipoamide dehydrogenase in which amino acid substitution(s) with P45S, and / or G131.

Examples of harmful arthropods (such as harmful insects, harmful mites) having a plural of causes of increased activity of metabolic enzyme, harmful mollusks having a plural of causes of increased activity of metabolic enzyme, and harmful nematodes having a plural of causes of increased activity of metabolic enzyme include Nilaparvata lugens in which in addition to having an overexpression of CYP6ER1, have a reduced sensitivity to imidacloprid due to containing CYP6ER1vA and CYP6ER1vB include Nilaparvata lugens.

Examples of harmful arthropods (such as harmful insects, harmful mites) having reduced sensitivity, harmful mollusks having reduced sensitivity, and harmful nematodes having reduced sensitivity wherein the reduced sensitivity is caused by a reduction in a skin permeability include the followings.

Helicoverpa armigera having reduced sensitivity to Pyrethroids (such as cypermethrin, esfenvalerate) due to a reduction in a skin permeability in cuticula of a drug;

Cimex lectularius having reduced sensitivity to Pyrethroids (such as beta-cyfluthrin) due to an overexpression of C2, C10, and / or C13 (each is one kind of presumptive epidermal protein gene).

Examples of harmful arthropods (such as harmful insects, harmful mites) having reduced sensitivity, harmful mollusks having reduced sensitivity, and harmful nematodes having reduced sensitivity wherein the reduced sensitivity is caused by an enhanced excretory function with a membrane transporter include the followings.

Cimex lectularius in which Abc8, Abc9, Abc10 and / or Abc11 (each is a gene of ABC transporters) is / are overexpressed.

Examples of pests belonging to the family of Aphididae include Aphis gossypii, Myzus persicae, and Nasonovia ribisnigri.

Examples of the pests belonging to the family Delphacidae include Nilaparvata lugens, Sogatella furcifera, and

Laodelphax striatellus.

Examples of the pests belonging to the family of Aleyrodidae include Bemisia tabaci, and Trialeurodes vaporariorum.

The method for controlling harmful arthropods of the present invention is conducted by applying an effective amount of the Present compound, the compound of the present invention or the Composition A to a harmful pest directly and/or a habitat where the harmful pest lives (for example, plant, soil, an interior of a house, and animal). Examples of a method for controlling harmful arthropods of the present invention include foliar application, soil application, root application, shower application, smoking application, water-surface application, and seed application.

The Present compound or the Composition A is usually used by mixing it with inert carrier (s) such as solid carrier(s), liquid carrier(s), and gaseous carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, a tablet, an aerosol formulation, a resin formulation, or the like. In addition to these formulations, the Present compound or the Composition A may be used by formulating it into a dosage form described in Manual on development and use of FAO and WHO Specifications for pesticides, FAO Plant Production and Protection Papers-271-276, prepared by the FAO/WHO Joint Meeting on Pesticide Specifications, 2016, ISSN:0259-2517.

These formulations usually comprise 0.0001 to 99% by weight ratio of the Present compound, the compound of the present invention, or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the gaseous carrier include fluorocarbon, butane gas, LPG (liquefied petroleum gas), dimethyl ether, nitrogen, and carbon dioxide.

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates). The specific examples of the surfactant include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol Aβ (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANGLE (registered trademark).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

As used herein, examples of the plant include whole plant, stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Examples of a method for controlling harmful arthropods by applying an effective amount of the Present compound, the compound of the present invention, or the Composition A to soils include a method of applying an effective

amount of the Present compound or the Composition A to soils before planting plants or after planting plants, a method of applying an effective amount of the Present compound or the Composition A to a root part of a crop to be protected from damage such as ingestion by harmful arthropods, and a method of controlling harmful arthropods that ingest a plant by permeating and transferring an effective amount of the Present compound or the Composition A from a root into the interior of the plant body. Specifically, examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sawing?, broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the Present compound, the compound of the present invention, or the Composition A to seeds or vegetative reproductive organs, and specific examples thereof include spraying treatment in which a suspension of the Present compound, the compound of the present invention, or the Composition A is sprayed onto seed surface or the vegetative reproductive organ surface in the form of mist; smearing treatment in which the Present compound, the compound of the present invention, or the Composition A is coated a surface of seeds or the vegetative reproductive organ; a soaking treatment in which the seeds are soaked into the solution of the Present compound, the compound of the present invention, or the Composition A for a certain time; and a method for coating the seeds or the vegetative reproductive organ with a carrier containing the Present compound, the compound of the present invention, or the Composition A (film coating treatment, pellet coating treatment). Examples of the above-described vegetative reproductive organ include particularly seed potato.

When the Composition A is applied to seeds or vegetative reproductive organs, the Composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for exmaple, a method in which the formulations comprising as an active component, the Present compound or the compound of the present invention, either only are applied, and seeds or vegetative reproductive organs are air dried, followed by applying the formulations comprising the Present ingredient: and a method in which the formulations comprising as an active component the present compound, or the compound of the present invention, and the Present ingredients are applied, and seeds or vegetative reproductive organs are air dried, followed by applying the formulations comprising the Present ingredients other than the already-applied Present ingredients, are included.

As used herein, seeds or vegetative reproductive organs carrying the present compound, or the compound of the present invention, or the Composition A means seeds or vegetative reproductive organs in the state where the present compound, or the compound of the present invention, or the Composition A is adhered to a surface of the seeds or the vegetative reproductive organ. The above-described seeds or vegetative reproductive organs carrying the present compound, or the compound of the present invention, or the Composition A may be adhered by any other materials that are different from the present compound, or the compound of the present invention, or the Composition A before or after being adhered the present compound, or the compound of the present invention, or the Composition A to the seeds or vegetative reproductive organs.

Also, when the Composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound, or the compound of the present

invention, or the Composition A can be obtained, for example, by applying the formulations comprising the present compound, or the compound of the present invention, or the Composition A by the above-described application method to seeds to seeds or vegetative reproductive organs.

When the Present compound, or the compound of the present invention, or the Composition A is applied for controlling harmful arthropods in agricultural fields, the application dose thereof is usually within a range of 1 to 10,000 g of the Present compound or the compound of the present invention per 10,000 $m^2$. In the case of being applied to seeds or vegetative reproductive organs, the dose of application dose thereof is usually within a range of 0.001 to 100 g of the Present compound or the compound of the present invention per 1 Kg of seeds. When the Present compound, or the compound of the present invention or the Composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

Also, the resin preparation which is processed into a sheet or a string may be applied by winding a plant with a sheet or a string of the resin preparation, putting a string of the resin preparation around a crop so that the plant is surrounded by the string, or laying a sheet of the resin preparation on the soil surface near the root of a plant.

When the Present compound, the compound of the present invention or the Composition A is used to control harmful arthropods that live inside a house, the application dose as an amount of the Present compound or the compound of the present invention is usually within a range from 0.01 to 1,000 mg per 1 $m^2$ of an area to be treated, in the case of using it on a planar area. In the case of using it spatially, the application dose as an amount of the Present compound or the compound of the present invention is usually within a range from 0.01 to 500 mg per 1 $m^3$ of the space to be treated. When the Present compound, the compound of the present invention or the Composition A is formulated into emulsifiable concentrates, wettable powders, flowables or the others, such formulations are usually applied after diluting it with water in such a way that a concentration of the active ingredient is within a range from 0.1 to 10,000 ppm. In the case of being formulated into oil solutions, aerosols, smoking agents, poison baits and the others, such formulations are used as itself without diluting it.

When the Present compound, the compound of the present invention or the composition A is used for controlling external parasites of livestock such as cows, horses, pigs, sheep, goats and chickens and small animals such as dogs, cats, rats and mice, the composition of the present invention may be applied to the animals by a known method in the veterinary field. Specifically, when systemic control is intended, the composition of the present invention is administered to the animals as a tablet, a mixture with feed or a suppository, or by injection (including intramuscular, subcutaneous, intravenous and intraperitoneal injections). On the other hand, when non-systemic control is intended, the composition of the present invention is applied to the animals by means of spraying of the oil solution or aqueous solution, pour-on or spot-on treatment, or washing of the animal with a shampoo formulation, or by putting a collar or ear tag made of the resin formulations to the animal. In the case of being administered to an animal body, the dose of the Present compound, the compound of the present invention, or the composition A is usually within a range from 0.1 to 1,000 mg per 1 kg of an animal body weight.

Also, the composition of the Present compound or the Composition A may be used as an agent for controlling pests in agricultural lands such as paddy fields, fields, turfs, and orchards. Examples of the plants include the followings. corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit),

nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

EXAMPLES

Hereinafter, the present invention is explained in more detail by Preparation Examples, Formulation Examples, and Test Examples, however, the present invention should not be limited to these Examples.

As used herein, Me represents methyl group, Et represents ethyl group, Pr represents propyl group, i-Pr represents isopropyl group, t-Bu represents tert-butyl group, c-Pr represents cyclopropyl group, c-Bu represents cyclobutyl group, c-Pen represents cyclopentyl group, c-Hex represents cyclohexyl group, $CF_3$ represents trifluoromethyl group, $OCF_3$represents trifluoromethoxy group, OEt represents ethoxy group, OPr represents propoxy group, O(i-Pr) represents isopropoxy group, Ph represents phenyl group, Py2 represents 2-pyridyl group, Py3 represents 3-pyridyl group, and Py4 represents 4-pyridyl group, and Bn represents benzyl group. When c-Pr, c-Bu, c-Pen, c-Hex, Ph, Py2, Py3, and Py4 has any substituent(s), the substituent(s) is described together with a substitution position before the symbol. For example, 1-CN-c-Pr represents 1-cyanocyclopropyl group, 3,4-$F_2$-Ph represents 3,4-difluorophenyl group, 4-$CF_3$-Py2 represents 4-(trifluoromethyl)-2-pyridyl group, and 5-$OCH_2CF_2CF_3$-Py2 represents 5-(2,2,3,3,3-pentafluoropropoxy)-2-pyridyl group.

Firstly, Preparation examples of the present compound (including the compound of the present invention) are described.

When a physical property of a compound is measured by a liquid chromatography / mass spectrometry analysis (hereinafter, referred to as "LCMS"), a measured molecular ion value $[M+H]^+$ or $[M-H]^-$ and a retention time (hereinafter, referred to as "RT") is described. The measured condition for liquid chromatography (hereinafter, referred to as "LC") is described below.

[LC condition]

Column: L-column2 ODS, inner diameter 4.6 mm, length 30 mm, particle size 3 μm
(Chemicals Evaluation and Research Institute, Japan)
UV measurement wavelength: 254 nm
Mobile phase: A solution: 0.1 % aqueous formic acid solution, B solution: 0.1 % formic acid in acetonitrile
Flow Rate: 2.0 mL/min
Pump: LC-20AD (manufacturer SHIMADZU) 2 umps (high pressure gradient)
Gradient condition: The following concentration gradient shown in [Table LC1] is liquid-transferred.

[Table LC1]

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

[MS condition]
Detector: LCMS-2020 (manufacturer SHIMADZU)
Ionization method: DUIS

Preparation Example 1

To a mixture of 3-chloro-N'-hydroxypyridine (which was prepared according to the method described in JP 2002-114783 A) 0.20 g and DMF 5 mL was added benzoyl chloride 0.18 g under ice-cooling, followed by adding triethylamine 0.13 g dropwise in ice-cooling, and the mixture was stirred at 140°C for 6 hours. After the mixture was cooled to room temperature, aqueous saturated sodium hydrocarbonate solution was added, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane = 30 : 70) to obtain the Present compound represented by the following formula 0.31 g.

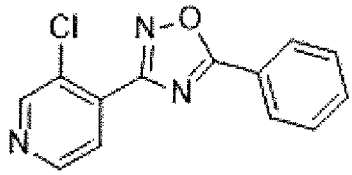

Present compound 1: $^1$H-NMR ($CDCl_3$) δ: 8.81 (1H, s), 8.67 (1H, d), 8.22 (2H, d), 8.01 (1H, d), 7.65-7.55 (3H, m).

Preparation Example 1-1

The compounds which were prepared according to the Preparation Example 1 and their physical property value are shown below.

A compound represented by formula (B-1):

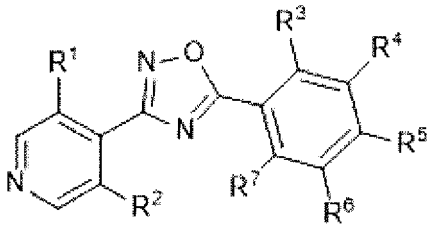 (B-1)

wherein a combination of $R^1$, $R^2$, $R^3$, $R^4$, $R^5$, $R^6$ and $R^7$ represents any combinations described in [Table B-1] and [Table B-2].

[Table B-1]

| Present compound | $R^1$ | $R^2$ | $R^3$ | $R^4$ | $R^5$ | $R^6$ | $R^7$ |
|---|---|---|---|---|---|---|---|
| 2 | Cl | H | H | H | F | H | H |
| 3 | Cl | H | $CH_3$ | H | F | H | H |
| 4 | Cl | Cl | H | H | H | H | H |
| 5 | Cl | Cl | Cl | H | H | H | H |
| 6 | Cl | Cl | H | Cl | H | H | H |
| 7 | Cl | Cl | H | H | Cl | H | H |
| 8 | Cl | Cl | $CH_3$ | H | H | H | H |
| 9 | Cl | Cl | H | $CH_3$ | H | H | H |
| 10 | Cl | Cl | H | H | $CH_3$ | H | H |
| 11 | Cl | Cl | H | H | F | H | H |
| 12 | Cl | Cl | Cl | H | Cl | H | H |

(continued)

| Present compound | $R^1$ | $R^2$ | $R^3$ | $R^4$ | $R^5$ | $R^6$ | $R^7$ |
|---|---|---|---|---|---|---|---|
| 13 | Cl | Cl | $CH_3$ | H | Cl | H | H |
| 14 | Cl | Cl | H | Cl | Cl | H | H |
| 15 | Cl | Cl | H | Cl | H | Cl | H |
| 16 | Cl | Cl | H | CN | H | H | H |
| 17 | Cl | Cl | H | $NO_2$ | H | H | H |
| 18 | Cl | Cl | H | $OCH_3$ | H | H | H |
| 21 | Cl | Cl | H | $CF_3$ | H | H | H |
| 37 | Cl | H | H | $SCF_3$ | H | H | H |
| 38 | Cl | H | H | Cl | H | H | H |
| 40 | Cl | Cl | H | Br | H | H | H |
| 41 | Cl | Cl | H | I | H | H | H |
| 42 | Cl | Cl | H | t-Bu | H | H | H |
| 44 | Cl | Cl | H | $CH_3$ | H | $CH_3$ | H |
| 45 | Cl | Cl | H | Cl | F | H | H |
| 47 | Cl | Cl | H | Br | F | H | H |
| 49 | Br | Br | H | $CF_3$ | H | H | H |

[Table B-2]

| Compound of the present invention | $R^1$ | $R^2$ | $R^3$ | $R^4$ | $R^5$ | $R^6$ | $R^7$ |
|---|---|---|---|---|---|---|---|
| 19 | Cl | Cl | H | F | F | H | H |
| 20 | Cl | Cl | H | F | H | F | H |
| 22 | Cl | Cl | H | $OCF_3$ | H | H | H |
| 23 | Cl | Cl | H | $OCF_3$ | H | F | H |
| 36 | Cl | H | H | $OCF_3$ | H | H | H |
| 39 | Cl | H | H | F | H | Cl | H |
| 43 | Cl | Cl | H | $OCHF_2$ | H | H | H |
| 46 | Cl | Cl | H | F | H | Cl | H |
| 48 | Cl | Cl | H | F | H | Br | H |
| 56 | Cl | Cl | H | F | H | H | H |

Present compound 2: $^1$H-NMR ($CDCl_3$) δ: 8.82 (1H, s), 8.67 (1H, d), 8.28-8.22 (2H, m), 7.99 (1H, d), 7.30-7.24 (2H, m).

Present compound 3: $^1$H-NMR ($CDCl_3$) δ: 8.83 (1H, s), 8.68 (1H, d), 8.24-8.18 (1H, m), 8.01 (1H, d), 7.13-7.07 (2H, m), 2.80 (3H, s).

Present compound 4: $^1$H-NMR ($CDCl_3$) 5: 8.67 (2H, s), 8.21 (2H, dd), 7.64-7.54 (3H, m).

Present compound 5: $^1$H-NMR ($CDCl_3$) δ: 8.60 (2H, s), 8.10 (1H, dd), 7.55-7.35 (3H, m).

Present compound 6: $^1$H-NMR ($CDCl_3$) δ: 8.69 (2H, s), 8.21 (1H, s), 8.11 (1H, d), 7.62 (1H, d), 7.53 (1H, t)

Present compound 7: $^1$H-NMR ($CDCl_3$) δ: 8.68 (2H, s), 8.16 (2H, d), 7.57 (2H, d) .

Present compound 8: $^1$H-NMR ($CDCl_3$) δ: 8.59 (2H, s), 8.09 (1H, d), 7.42 (1H, t), 7.29 (2H, d), 2.68 (3H, s) .

Present compound 9: $^1$H-NMR ($CDCl_3$) δ: 8.68 (2H, s), 8.04 (1H, br s), 8.04-8.00 (1H, m), 7.46 (2H, d), 2.47 (3H, s).

Present compound 10: $^1$H-NMR ($CDCl_3$) δ: 8.68 (2H, s), 8.10 (2H, d), 7.38 (2H, d), 2.47 (3H, s).

Present compound 11: $^1$H-NMR ($CDCl_3$) δ: 8.68 (2H, s), 8.26-8.23 (2H, m), 7.31-7.24 (2H, m).

Present compound 12: $^1$H-NMR ($CDCl_3$) δ: 8.60 (2H, s), 8.08 (1H, d), 7.57 (1H, s), 7.39 (1H, d).

Present compound 13: $^1$H-NMR ($CDCl_3$) δ: 8.69 (2H, s), 8.14 (1H, d), 7.40 (1H, s), 7.38 (1H, d), 2.75 (3H, s).

Present compound 14: $^1$H-NMR ($CDCl_3$) δ: 8.69 (2H, s), 8.32 (1H, s), 8.05 (1H, d), 7.68 (1H, d).

Present compound 15: $^1$H-NMR ($CDCl_3$) δ: 8.69 (2H, s), 8.11 (2H, s), 7.64 (1H, s).

Present compound 16: $^1$H-NMR ($CDCl_3$) δ: 8.70 (2H, s), 8.52 (1H, br s), 8.45 (1H, dd), 7.94 (1H, dd), 7.75 (1H, t).

Present compound 17: $^1$H-NMR ($CDCl_3$) δ: 9.08 (1H, br s), 8.70 (2H, s), 8.55 (2H, dt), 7.85 (1H, dt).

Present compound 18: $^1$H-NMR ($CDCl_3$) δ: 8.59 (2H, s), 7.72 (1H, dd), 7.61 (1H, t), 7.38 (1H, t), 7.08 (1H, dd), 3.81 (3H, s) .

Compound of the present invention 19: $^1$H-NMR ($CDCl_3$) δ: 8.69 (2H, s), 8.09-8.01 (2H, m), 7.45-7.36 (1H, m).

Compound of the present invention 20: $^1$H-NMR ($CDCl_3$) δ: 8.72 (2H, s), 7.79-7.78 (2H, m), 7.15-7.13 (1H, m).

Present compound 21: $^1$H-NMR ($CDCl_3$) δ: 8.70 (2H, s), 8.51 (1H, br s), 8.42 (1H, d), 7.92 (1H, d), 7.76 (1H, t).

Compound of the present invention 22: $^1$H-NMR ($CDCl_3$) δ: 8.69 (2H, s), 8.17 (1H, d), 8.08 (1H, s), 7.66-7.64 (1H, m), 7.51 (1H, d).

Compound of the present invention 23: $^1$H-NMR ($CDCl_3$) δ: 8.96 (2H, s), 8.17-8.15 (1H, m), 8.04 (1H, s), 7.93 (1H, d).

Compound of the present invention 36: $^1$H-NMR ($CDCl_3$) δ: 8.84 (1H, s), 8.70 (1H, d), 8.19 (1H, d), 8.10 (1H, s), 8.01 (1H, d), 7.66 (1H, t), 7.52 (1H, d).

Present compound 37: $^1$H-NMR ($CDCl_3$) δ: 8.84 (1H, s), 8.70 (1H, d), 8.54 (1H, s), 8.36 (1H, d), 8.02 (1H, d), 7.95 (1H, d), 7.69 (1H, t).

Present compound 38: $^1$H-NMR ($CDCl_3$) δ: 8.85 (1H, s), 8.71 (1H, d), 8.25-8.23 (1H, m), 8.13 (1H, d), 8.04-8.01 (1H, m), 7.66-7.62 (1H, m), 7.57-7.52 (1H, m).

Compound of the present invention 39; $^1$H-NMR ($CDCl_3$) δ: 8.85 (1H, s), 8.71 (1H, d), 8.06-8.05 (1H, m), 8.00 (1H, d), 7.87-7.83 (1H, m), 7.41-7.38 (1H, m).

Present compound 40: $^1$H-NMR ($CDCl_3$) δ: 8.70 (2H, s), 8.40-8.39 (1H, m), 8.17 (1H, d), 7.80 (1H, d), 7.48 (1H, t).

Present compound 41: $^1$H-NMR ($CDCl_3$) δ: 8.70 (2H, s), 8.60-8.58 (1H, m), 8.22-8.18 (1H, m), 8.01-7.98 (1H, m), 7.34 (1H, t).

Present compound 42: $^1$H-NMR ($CDCl_3$) δ: 8.68 (2H, s), 8.24 (1H, s), 8.04 (1H, d), 7.69 (1H, d), 7.52 (1H, t), 1.40 (9H, s).

Compound of the present invention 43: $^1$H-NMR ($CDCl_3$) δ: 8.70 (2H, s), 8.10 (1H, d), 8.00-7.98 (1H, m), 7.61 (1H, t), 7.46-7.43 (1H, m), 6.63 (1H, t).

Present compound 44: $^1$H-NMR ($CDCl_3$) δ: 8.68 (2H, s), 7.85 (2H, s), 7.29 (1H, s), 2.43 (6H, s) .

Present compound 45: $^1$H-NMR ($CDCl_3$) δ: 8.69 (2H, s), 8.34-8.30 (1H, m), 8.16-8.11 (1H, m), 7.37 (1H, t).

Compound of the present invention 46: $^1$H-NMR ($CDCl_3$) δ: 8.70 (2H, s), 8.06-8.04 (1H, m), 7.84 (1H, d), 7.40 (1H, d).

Present compound 47: $^1$H-NMR ($CDCl_3$) δ: 8.69 (2H, s), 8.49-8.46 (1H, m), 8.20-8.16 (1H, m), 7.34 (1H, t).

Compound of the present invention 48: $^1$H-NMR ($CDCl_3$) δ: 8.70 (2H, s), 8.20-8.19 (1H, m), 7.90-7.86 (1H, m), 7.57-7.53 (1H, m) .

Present compound 49: $^1$H-NMR ($CDCl_3$) δ: 8.84 (2H, s), 8.52 (1H, s), 8.43 (1H, d), 7.93 (1H, d), 7.76 (1H, t).

Compound of the present invention 56: $^1$H-NMR ($CDCl_3$) δ: 8.70 (2H, s), 8.06-8.02 (1H, m), 7.96-7.91 (1H, m), 7.61-7.55 (1H, m), 7.40-7.34 (1H, m).

Preparation Example 2

To a mixture of 3,5-dichloro-N-hydroxyisonicotine imidoyl chloride 0.40 g, and tetrahydrofuran 20 mL was added 4-ethenyl-1,2-dfluorobenzene 20 mL under ice-cooling, followed by adding triethyl amine 0.55 mL dropwise under ice-cooling, and the mixture was stirred at room temperature for 20 hours. Water was added to the resulting mixture, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane = 25 : 75) to obtain the Present compound 24 represented by the following formula 0.30 g.

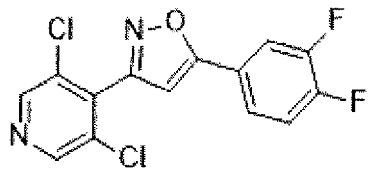

Present compound 24: $^1$H-NMR ($CDCl_3$) δ: 8.66 (2H, s), 7.73-7.57 (2H, m), 7.37-7.28 (1H, m), 6.65 (1H, s).

Preparation Example 3

To a mixture of 3,5-dichloropyridine-4-carboxylic acid 0.73 g and toluene 10 mL was added thionyl chloride 0.33 mL at room temperature, and the mixture was stirred at reflux for 3 hours. After the mixture was cooled to room temperature, toluene was evaporated, and DMF 5 mL and 3,4-difluorobenzohydrazide 0.72 g were added to the resulting

residue, and the mixture was stirred at reflux for 8 hours. Aqueous saturated sodium hydrocarbonate solution 50 mL was added to the resulting mixture and the mixture was extracted with ethyl acetate. The resulting organic layer was dried over anhydrous magnesium sulfate, and concentrated under reduced pressure. The resulting residue was subjected to a silica gel column chromatography (ethyl acetate : hexane = 1 : 1) to obtain 3,5-dichloro-N'-(3,4-difluorobenzoyl)pyridine-4-carbohydrazide 0.21 g.

To a mixture of the obtained 3,5-dichloro-N'-(3,4-difluorobenzoyl)pyridine-4-carbohydrazide 0.21 g and acetonitrile 5 mL were added p-toluenesulfonyl chloride 0.17 g and triethylamine 0.35 mL successively at room temperature, and the mixture was stirred at room temperature for 3 hours. An aqueous saturated sodium hydrocarbonate solution was added to the resulting mixture, and the mixture was extracted with ethyl acetate. The resulting organic layer was dried over anhydrous magnesium sulfate, and concentrated under reduced pressure. The resulting residue was subjected to a silica gel column chromatography (ethyl acetate : hexane = 1 : 10) to obtain the Present compound 25 represented by the following formula 0.11 g.

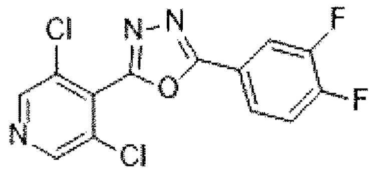

Present compound 25: $^1$H-NMR ($CDCl_3$) $\delta$: 8.77 (2H, s), 8.04-7.95 (2H, m), 7.44-7.38 (1H, m).

Preparation Example 4

To a mixture of 3-methoxypyridine-4-carboxylic acid 0.53 g and toluene 15 mL were added thionyl chloride 0.3 mL and DMF 0.1 mL at room temperature, and the mixture was stirred at reflux for 3 hours. After the mixture was cooled to room temperature, toluene was evaporated, and THF 5 mL was added to the resulting residue. To the resulting mixture were added a mixture of 3,4-difluorobenzene carboxamide amide 0.4 g and THF 10 mL, and diazabicycloundecene 0.86 mL successively at room temperature, and the mixture was stirred at room temperature for 3 hours. An aqueous saturated sodium hydrocarbonate solution was added to the resulting mixture, and the mixture was extracted with ethyl acetate. The resulting organic layer was dried over anhydrous magnesium sulfate, and concentrated under reduced pressure. The resulting residue was subjected to a silica gel column chromatography (ethyl acetate - hexane = 1 : 10) to obtain the Present compound 26 represented by the following formula 0.24 g.

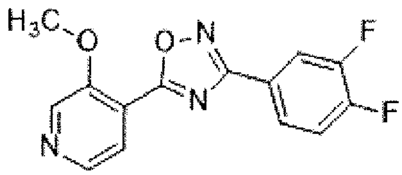

Present compound 26: $^1$H-NMR ($CDCl_3$) $\delta$: 8.65 (1H, s), 8.51 (1H, d), 8.08-7.99 (3H, m), 7.39-7.30 (1H, m), 4.18 (3H, s).

Preparation Example 5

A mixture of 3-(3,4-difluorophenyl)-1H-pyrazole (which was prepared according to the method described in WO 2020/043866) 900 mg, 3,4,5-trichloropyridine 910 mg, potassium carbonate 1.0 g, and DMF 10 mL was stirred at 50°C for 8 hours. Water was added to the resulting mixture, and the mixture was extracted with ethyl acetate. The resulting organic layer was dried anhydrous sodium sulfate, and concentrated under reduced pressure. The resulting residue was subjected to a silica gel column chromatography (ethyl acetate : hexane = 30 : 70) to obtain the Compound of the present invention 27 represented by the following formula 1.0 g.

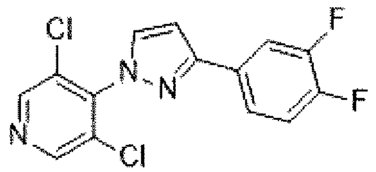

Compound of the present invention 27: $^1$H-NMR ($CDCl_3$) $\delta$: 8.70 (2H, s), 7.71-7.69 (2H, m), 7.59-7.58 (1H, m), 7.23-7.20 (1H, m), 6.80 (1H, d).

Preparation Example 6

A mixture of 3,5-dichloropyridine-4-carbohydrazide (which was prepared according to the method described in WO 2012/098416) 2.2 g, 4-chloro-benzonitrile 1.38 g, potassium carbonate 1.38 g, and 1-butanol 20 mL was stirred at 140°C for 6 hours. Water was added to the resulting mixture, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane = 30 : 70) to obtain the Present compound 28 represented by the following formula 390 mg.

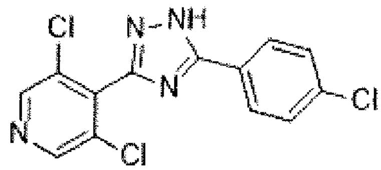

Present compound 28: LCMS: 325 $[M+H]^+$, RT = 1.94 min.

Preparation Example 6-1

The compounds which were prepared according to the Preparation Example 6 and their physical property value are shown below.

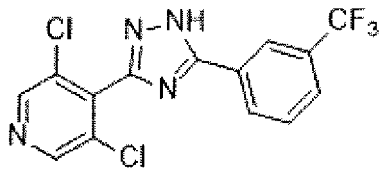

Present compound 33: $^1$H-NMR ($CDCl_3$) $\delta$: 8.68 (2H, s), 8.38 (1H, s), 8.28 (1H, d), 7.76 (1H, d), 7.68-7.63 (1H, m).

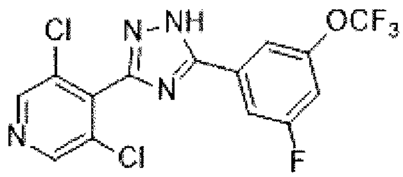

Present compound 34: $^1$H-NMR ($CDCl_3$) $\delta$: 8.68 (2H, s), 7.83-7.79 (2H, m), 7.10-7.05 (1H, m).

Preparation Example 7

A mixture of the Present compound 28 390 mg, methyl iodide 94 $\mu$L, cesium carbonate 492 mg, and DMF 5 mL was stirred at room temperature for 6 hours. Water was added to the resulting mixture, and the mixture was extracted with ethyl acetate. The resulting organic layer was dried over anhydrous sodium sulfate, and concentrated under reduced pressure. The resulting residue was subjected to a silica gel column chromatography (ethyl acetate : hexane = 30 : 70) to obtain the Present compound 29 represented by the following formula 50 mg.

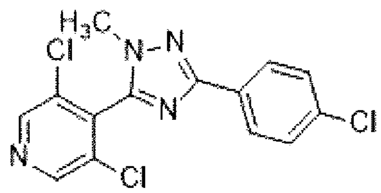

Present compound 29: 8.62 (2H, s), 7.73 (2H, d), 7.53 (2H, d), 4.12 (3H, s).

Preparation Example 8

The compounds which were prepared according to the Preparation Example 7 and their physical property value are shown below.

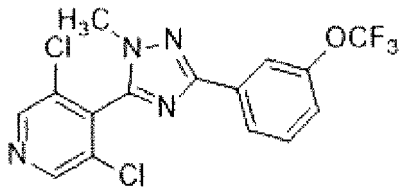

Present compound 30: $^1$H-NMR ($CDCl_3$) δ: 8.63 (2H, s), 7.74 (1H, d), 7.65-7.60 (2H, m), 7.41 (1H, d), 4.15 (3H, s).

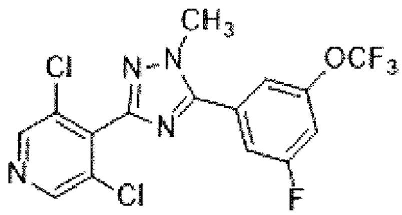

Compound of the present invention 31: $^1$H-NMR ($CDCl_3$) δ: 8.72 (2H, s), 7.85-7.80 (2H, m), 7.04-7.00 (1H, m), 3.83 (3H, s).

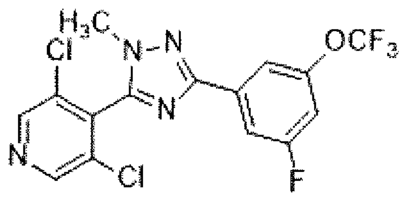

Present compound 32: $^1$H-NMR ($CDCl_3$) δ: 8.63 (2H, s), 8.05-8.03 (1H, m), 7.86 (1H, m), 7.73(1H, s), 4.16 (3H, s).

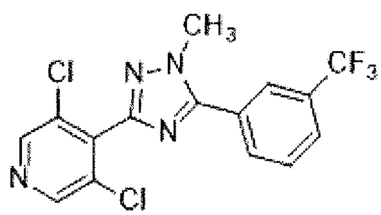

Present compound 35: $^1$H-NMR ($CDCl_3$) δ: 8.72 (2H, s), 8.43 (1H, s), 8.33 (1H, d), 7.70-7.67 (1H, m), 7.61-7.57 (1H, m), 3.83 (3H, s).

Preparation Example 9

A mixture of the Present compound 49 160 mg, methylboronic acid 65.8 mg, [1,1'-bis(diphenylphoshino)ferrocene]dichloropalladium (II) 52.7 mg, cesium carbonate 469 mg and DMF 2 mL was stirred at 80°C under nitrogen atmosphere for 4 hours. Water was added to the resulting mixture, and the mixture was extracted with ethyl acetate. The resulting organic layer was dried anhydrous sodium sulfate, and concentrated under reduced pressure. The resulting residue was subjected to a silica gel column chromatography to obtain the Present compound 50 represented by the following formula 30 mg.

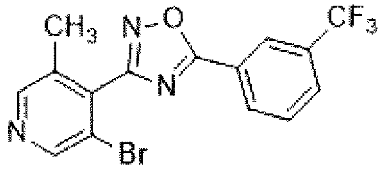

Present compound 50: $^1$H-NMR ($CDCl_3$) $\delta$: 8.76 (1H, s), 8.54 (1H, s), 8.51 (1H, s), 8.42 (1H, d), 7.93-7.91 (1H, m), 7.78-7.73 (1H, m), 2.32 (3H, s).

Preparation Example 10

The compounds which were prepared according to the Preparation Example 9 and their physical property value are shown below.

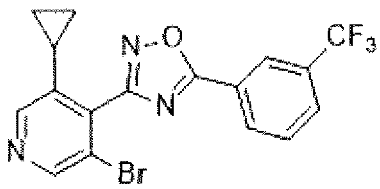

Compound of the present invention 51: $^1$H-NMR ($CDCl_3$) $\delta$: 8.72 (1H, s), 8.51 (1H, s), 8.43 (1H, d), 8.33 (1H, s), 7.93-7.90 (1H, m), 7.75 (1H, t), 1.87-1.79 (1H, m), 0.95-0.90 (2H, m), 0.82-0.78 (2H, m).

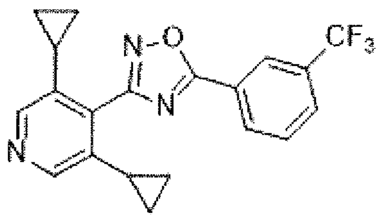

Compound of the present invention 52: $^1$H-NMR ($CDCl_3$) $\delta$: 8.52 (1H, s), 8.43 (1H, d), 8.25 (2H, s), 7.92-7.90 (1H, m), 7.75 (1H, t), 1.88-1.81 (2H, m), 0.90-0.83 (4H, m), 0.77-0.73 (4H, m).

Preparation Example 11

To a mixture of sodium hydride (in oil, 60 %) 0.2 g, the Compound of the present invention 21 180 mg and THF 2 mL was added methanol 1 mL, and the mixture was stirred at 80°C for 4 hours. Water was added to the resulting mixture, and the mixture was extracted with ethyl acetate. The resulting organic layer was dried anhydrous sodium sulfate, and concentrated under reduced pressure. The resulting residue was subjected to a silica gel column chromatography to obtain the Compound of the present invention 53 represented by the following formula 90 mg.

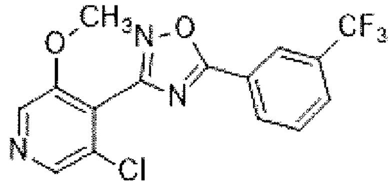

Compound of the present invention 53: $^1$H-NMR ($CDCl_3$) δ: 8.50 (1H, s), 8.45 (1H, s), 8.41 (1H, d), 8.38 (1H, s), 7.90 (1H, d), 7.73 (1H, t), 3.96 (3H, s).

Preparation Example 11-1

The compounds which were prepared according to the Preparation Example 11 and their physical property value are shown below.

A compound represented by formula (C-1):

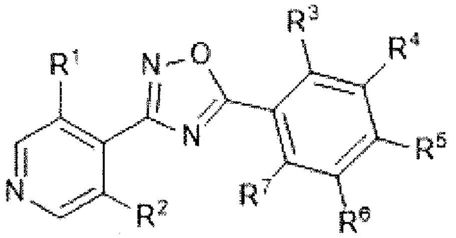

(C-1)

wherein a combination of $R^1$, $R^2$, $R^3$, $R^4$, $R^5$, $R^6$ and $R^7$ represents any combinations described in [Table C-1] and [Table C-2].

[Table C-1]

| Present compound | $R^1$ | $R^2$ | $R^3$ | $R^4$ | $R^5$ | $R^6$ | $R^7$ |
|---|---|---|---|---|---|---|---|
| 67 | OEt | Cl | H | Br | OEt | H | H |
| 68 | O(i-Pr) | Cl | H | O(i-Pr) | H | F | H |

[Table C-2]

| Compound of the present invention | $R^1$ | $R^2$ | $R^3$ | $R^4$ | $R^5$ | $R^6$ | $R^7$ |
|---|---|---|---|---|---|---|---|
| 54 | OEt | Cl | H | $CF_3$ | H | H | H |
| 55 | O(i-Pr) | Cl | H | $CF_3$ | H | H | H |
| 57 | $OCH_3$ | Cl | H | F | H | H | H |
| 58 | OEt | Cl | H | F | H | H | H |
| 59 | $SCH_3$ | Cl | H | F | H | H | H |
| 60 | $OCH_3$ | Cl | H | Cl | H | H | H |
| 61 | O(i-Pr) | Cl | H | Cl | H | H | H |
| 62 | $OCH_3$ | Cl | H | $CH_3$ | H | H | H |
| 63 | OEt | Cl | H | $CH_3$ | H | H | H |
| 64 | $OCH_3$ | Cl | H | $CH_3$ | H | $CH_3$ | H |
| 65 | OEt | Cl | H | $OCF_3$ | H | H | H |
| 66 | OEt | Cl | H | Br | F | H | H |
| 69 | $SCH_3$ | Cl | H | $CF_3$ | H | H | H |
| 70 | $SCH_3$ | Cl | H | Cl | H | H | H |
| 71 | $SCH_3$ | $SCH_3$ | H | Cl | H | H | H |

Compound of the present invention 54: $^1$H-NMR ($CDCl_3$) δ: 8.50 (1H, s), 8.41 (2H, d), 8.36 (1H, s), 7.90 (1H,

d), 7.73 (1H, t), 4.22 (2H, q), 1.36 (3H, t).

Compound of the present invention 55: $^1$H-NMR ($CDCl_3$) δ: 8.50 (1H, s), 8.43-8.32 (3H, m), 7.89 (1H, d), 7.73 (1H, t), 4.74-4.68 (1H, m), 1.32 (6H, d).

Compound of the present invention 57: $^1$H-NMR ($CDCl_3$) δ: 8.45 (1H, s), 8.38 (1H, s), 8.04-8.01 (1H, m), 7.95-7.91 (1H, m), 7.60-7.53 (1H, m), 7.38-7.33 (1H, m), 3.96 (3H, s).

Compound of the present invention 58: $^1$H-NMR ($CDCl_3$) δ: 8.42 (1H, s), 8.35 (1H, s), 8.04-8.01 (1H, m), 7.94-7.90 (1H, m), 7.60-7.53 (1H, m), 7.38-7.32 (1H, m), 4.22 (2H, q), 1.36 (3H, t).

Compound of the present invention 59: $^1$H-NMR ($CDCl_3$) δ: 8.57 (1H, s), 8.53 (1H, s), 8.05-8.01 (1H, m), 7.95-7.91 (1H, m), 7.60-7.54 (1H, m), 7.38-7.33 (1H, m), 2.55 (3H, s).

Compound of the present invention 60: $^1$H-NMR ($CDCl_3$) δ: 8.45 (1H, s), 8.38 (1H, s), 8.24-8.22 (1H, m), 8.13-8.09 (1H, m), 7.64-7.60 (1H, m), 7.52 (1H, t), 3.95 (3H, s).

Compound of the present invention 61: $^1$H-NMR ($CDCl_3$) δ: 8.39 (1H, s), 8.37 (1H, s), 8.24-8.21 (1H, m), 8.13-8.10 (1H, m), 7.64-7.60 (1H, m), 7.53 (1H, t), 4.73-4.67 (1H, m), 1.33 (6H, d).

Compound of the present invention 62: $^1$H-NMR ($CDCl_3$) δ: 8.44 (1H, s), 8.36 (1H, s), 8.05 (1H, s), 8.03-8.00 (1H, m), 7.46-7.44 (2H, m), 3.95 (3H, s), 2.46 (3H, s).

Compound of the present invention 63: $^1$H-NMR ($CDCl_3$) δ: 8.41 (1H, s), 8.34 (1H, s), 8.05 (1H, s), 8.03-8.01 (1H, m), 7.46-7.44 (2H, m), 4.21 (2H, q), 2.46 (3H, s), 1.35 (3H, t).

Compound of the present invention 64: $^1$H-NMR ($CDCl_3$) δ: 8.43 (1H, s), 8.35 (1H, s), 7.84-7.82 (2H, m), 7.26-7.24 (1H, m), 3.93 (3H, s), 2.41 (6H, s).

Compound of the present invention 65: $^1$H-NMR ($CDCl_3$) δ: 8.42 (1H, s), 8.35 (1H, s), 8.19-8.16 (1H, m), 8.08 (1H, s), 7.63 (1H, t), 7.52-7.48 (1H, m), 4.22 (2H, q), 1.36 (3H, t).

Compound of the present invention 66: $^1$H-NMR ($CDCl_3$) δ: 8.69-8.66 (2H, m), 8.42-8.40 (1H, m), 8.14-8.09 (1H, m), 7.04-7.00 (1H, m), 4.22 (2H, q), 1.54 (3H, t).

Present compound 67: $^1$H-NMR ($CDCl_3$) δ: 8.42-8.41 (1H, m), 8.40 (1H, s), 8.33 (1H, s), 8.13-8.09 (1H, m), 7.01 (1H, d), 4.26-4.17 (4H, m), 1.53 (3H, t), 1.35 (3H, t).

Present compound 68: $^1$H-NMR ($CDCl_3$) δ: 8.39 (1H, s), 8.36 (1H, s), 7.53-7.51 (1H, m), 7.50-7.46 (1H, m), 6.87-6.83 (1H, m), 4.73-4.61 (2H, m), 1.39 (6H, d), 1.33 (6H, d).

Compound of the present invention 69: $^1$H-NMR ($CDCl_3$) δ: 8.58 (1H, s), 8.54 (1H, s), 8.52-8.50 (1H, m), 8.44-8.41 (1H, m), 7.93-7.90 (1H, m), 7.77-7.72 (1H, m), 2.56 (3H, s).

Compound of the present invention 70: $^1$H-NMR ($CDCl_3$) δ: 8.57 (1H, s), 8.53 (1H, s), 8.25-8.23 (1H, m), 8.14-8.10 (1H, m), 7.64-7.61 (1H, m), 7.53 (1H, t), 2.55 (3H, s).

Compound of the present invention 71: $^1$H-NMR ($CDCl_3$) δ: 8.45 (2H, s), 8.23 (1H, s), 8.15-8.09 (1H, m), 7.65-7.58 (1H, m), 7.56-7.49 (1H, m), 2.52 (6H, s).

Compound of the present invention 77: $^1$H-NMR ($CDCl_3$) δ: 8.56 (1H, s), 8.53 (1H, s), 7.78-7.74 (2H, m), 7.13-7.07 (1H, m), 2.54 (3H, s).

Compound of the present invention 78: $^1$H-NMR ($CDCl_3$) δ: 8.55 (1H, s), 8.50 (1H, s), 7.84 (2H, s), 7.26 (1H, s), 2.53 (3H, s), 2.41 (6H, s).

Preparation Example 12

A mixture of the Compound of the present invention 69 160 mg, chloroform 2 mL, and meta-chloroperoxybenzoic acid 212 mg was stirred at room temperature for 2 hours. The resulting mixture was washed with aqueous sodium thiosulfate solution and aqueous sodium hydrogen carbonate solution successively. The resulting organic layer was dried anhydrous sodium sulfate, and concentrated under reduced pressure. The resulting residue was subjected to a silica gel column chromatography to obtain the Compound of the present invention 72 represented by the following formula 150 mg.

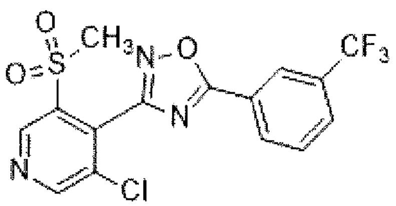

Compound of the present invention 72: $^1$H-NMR ($CDCl_3$) δ: 9.30 (1H, s), 9.07 (1H, s), 8.48 (1H, s), 8.41 (1H, d), 7.92 (1H, d), 7.75 (1H, t), 3.36 (3H, s).

Preparation Example 13

A mixture of the Present compound 21 360 mg, chloroform 4 mL, and meta-chloroperoxybenzoic acid 247 mg was stirred at 70°C for 2 hours. The resulting mixture was washed with aqueous sodium thiosulfate solution, and aqueous sodium hydrogen carbonate solution successively. The resulting organic layer was dried over anhydrous sodium sulfate, and concentrated under reduced pressure. The resulting residue was subjected to a silica gel column chromatography to obtain the Present compound 73 represented by the following formula 250 mg.

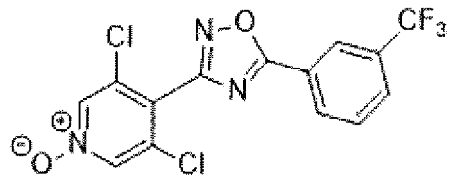

Present compound 73: $^1$H-NMR ($CDCl_3$) δ: 8.49 (1H, s), 8.41 (1H, d), 8.32 (2H, s), 7.93 (1H, d), 7.75 (1H, t).

Preparation Example 14

A mixture of the Compound of the present invention 69 50 mg, chloroform 2 mL, and meta-chloroperoxybenzoic acid 33 mg was stirred at 0°C for 1 hours. The resulting mixture was washed with aqueous sodium thiosulfate solution and aqueous sodium hydrogen carbonate solution successively. The resulting organic layer was dried anhydrous sodium sulfate, and concentrated under reduced pressure. The resulting residue was subjected to a silica gel column chromatography to obtain the Compound of the present invention 74 represented by the following formula 40 mg.

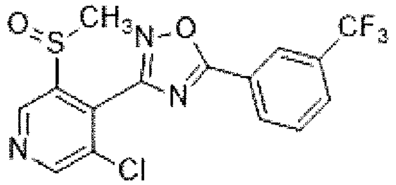

Compound of the present invention 74: $^1$H-NMR ($CDCl_3$) δ: 9.32 (1H, s), 8.96 (1H, s), 8.47 (1H, s), 8.41 (1H, d), 7.95 (1H, d), 7.78 (1H, t), 3.07 (3H, s).

Preparation Example 15

The compounds which were prepared according to the Preparation Example 14 and their physical property value are shown below.

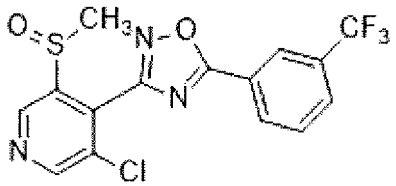

Compound of the present invention 75: $^1$H-NMR ($CDCl_3$) δ: 9.32 (1H, s), 8.96 (1H, s), 8.03-8.00 (1H, m), 7.92-7.88 (1H, m), 7.64-7.58 (1H, m), 7.43-7.38 (1H, m), 3.08 (3H, s).

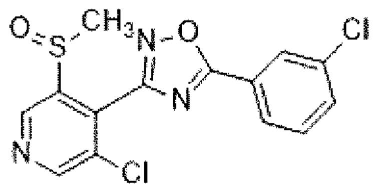

Compound of the present invention 76: $^1$H-NMR ($CDCl_3$) δ: 9.32 (1H, s), 8.96 (1H, s), 8.20-8.18 (1H, m),

8.12-8.08 (1H, m), 7.68-7.65 (1H, m), 7.58-7.54 (1H, m), 3.07 (3H, s).

Next, examples of the Present compounds and the Compounds of the present invention, which are prepared according to any one of the Preparation Examples described in Working Examples and the Processes described herein are indicated below.

A compound represented by formula (L-1):

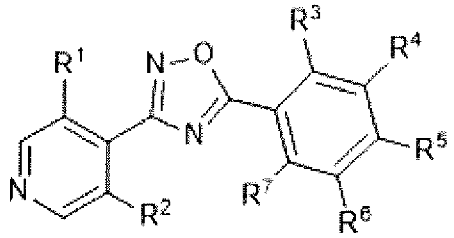

(L-1)

(hereinafter, referred to as "Compound (L-l)")
wherein $R^{1b}$ represents a fluorine atom, $R^2$ represents a hydrogen atom, $R^4$, $R^5$, $R^6$, and $R^7$ represent a hydrogen atom, and $R^3$ represents any substituents described in [Table L1] (hereinafter, referred to as "Compound class SX1").

[Table L1]

| |
|---|
| $CF_3$ |
| $CHF_2$ |
| $CH_2CF_3$ |
| $CF_2CF_3$ |
| $CH_2CF_2CF_3$ |
| $CF_2CF_2CF_3$ |
| $CF_2CF_2CF_2CF_3$ |
| $CF_2CF_2CF_2CF_2CF_3$ |
| $CH_3$ |
| $CH_2CH_3$ |
| $CH(CH_3)_2$ |
| $CH_2CH_2CH_3$ |
| $CH_2CH_2CH_2CH_3$ |
| $C(CH_3)_3$ |
| $CH=CH_2$ |
| 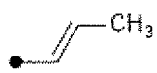 |
| 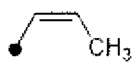 |
| $CCH_3=CH_2$ |
| F |
| Cl |
| Br |

(continued)

| |
|---|
| I |

[Table L2]

| |
|---|
| $OCH_3$ |
| $OCH_2CH_3$ |
| $OCH(CH_3)_2$ |
| $OCH_2CH_2CH_3$ |
| $OCH_2CH(CH_3)_2$ |
| $OCF_3$ |
| $OCHF_2$ |
| $OCH_2CF_3$ |
| $OCH_2CHF_2$ |
| $OCF_2CF_3$ |
| $OCH(CH_3)CF_3$ |
| $OCH_2CF_2CHF_2$ |
| $OCH_2CF_2CF_3$ |
| $OCF_2CF_2CF_3$ |
| $SCH_3$ |
| $SCH_2CH_3$ |
| $SCH(CH_3)_2$ |
| $SCF_3$ |
| $SCH_2CF_3$ |
| $SCF_2CF_3$ |
| $S(O)CH_3$ |
| $S(O)CH_2CH_3$ |
| $S(O)CH(CH_3)_2$ |
| $S(O)CF_3$ |
| $S(O)CH_2CF_3$ |
| $S(O)CF_2CF_3$ |
| $S(O)_2CH_3$ |
| $S(O)_2CH_2CH_2$ |
| $S(O)_2CH(CH_3)_2$ |
| $S(O)_2CF_3$ |
| $S(O)_2CH_2CF_3$ |
| $S(O)_2CF_2CF_3$ |

[Table L3]

| |
|---|
| Ph |
| 2-F-Ph |
| 3-F-Ph |
| 4-F-Ph |
| 2-Cl-Ph |
| 3-Cl-Ph |
| 4-Cl-Ph |
| 2-Br-Ph |
| 3-Br-Ph |
| 4-Br-Ph |
| 2,6-$F_2$-Ph |
| 3,5-$F_2$-Ph |
| 2,4-$F_2$-Ph |
| 2,4,6-$F_3$-Ph |
| 2,6-$Cl_2$-Ph |
| 3,5-$Cl_2$-Ph |
| 2,4-$Cl_2$-Ph |
| Py2 |
| Py3 |
| Py4 |
| c-Pr |
| CN |

A present compound (L-1) wherein $R^1$ and $R^2$ represent a fluorine atom, $R^4$, $R^5$, $R^6$ and $R^7$ represent a hydrogen atom, and $R^3$ represents any one substituent indicated in [Table Z1] (hereinafter, referred to as "Compound class SX2").

A present compound (L-1) wherein $R^1$ represents a chlorine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX3").

A present compound (L-1) wherein $R^1$ and $R^2$ represent a chlorine atom, $R^4$, $R^5$, $R^6$ and $R^7$ represent a hydrogen atom, and $R^3$ represents any one substituent indicated in [Table L1] (hereinafter, referred to as "Compound class SX4").

A present compound (L-1) wherein $R^1$ represents a bromine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX5").

A present compound (L-1) wherein $R^1$ and $R^2$ represent a bromine atom, $R^4$, $R^5$, $R^6$ and $R^7$ represent a hydrogen atom, and $R^3$ represents any one substituent indicated in [Table L1] (hereinafter, referred to as "Compound class SX6").

A present compound (L-1) wherein $R^1$ represents an iodine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX7").

A present compound (L-1) wherein $R^1$ and $R^2$ represent an iodine atom, $R^4$, $R^5$, $R^6$ and $R^7$ represent a hydrogen atom, and $R^3$ represents any one substituent indicated in [Table L1] (hereinafter, referred to as "Compound class SX8").

A present compound (L-1) wherein $R^1$ represents a methyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX9").

A present compound (L-1) wherein $R^1$ and $R^2$ represent a methyl group, $R^4$, $R^5$, $R^6$ and $R^7$ represent a hydrogen

atom, and $R^3$ represents any one substituent indicated in [Table L1] (hereinafter, referred to as "Compound class SX10").

A present compound (L-1) wherein $R^1$ represents an ethyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX11").

A present compound (L-1) wherein $R^1$ and $R^2$ represent an ethyl group, $R^4$, $R^5$, $R^6$ and $R^7$ represent a hydrogen atom, and $R^3$ represents any one substituent indicated in [Table L1] (hereinafter, referred to as "Compound class SX12").

A present compound (L-1) wherein $R^1$ represents a propyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX13").

A present compound (L-1) wherein $R^1$ and $R^2$ represent a propyl group, $R^4$, $R^5$, $R^6$ and $R^7$ represent a hydrogen atom, and $R^3$ represents any one substituent indicated in [Table L1] (hereinafter, referred to as "Compound class SX14").

A present compound (L-1) wherein $R^1$ represents an isopropyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX15").

A present compound (L-1) wherein $R^1$ and $R^2$ represent an isopropyl group, $R^4$, $R^5$, $R^6$ and $R^7$ represent a hydrogen atom, and $R^3$ represents any one substituent indicated in [Table L1] (hereinafter, referred to as "Compound class SX16").

A present compound (L-1) wherein $R^1$ represents a cyclopropyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX17").

A present compound (L-1) wherein $R^1$ and $R^2$ represent a cyclopropyl group, $R^4$, $R^5$, $R^6$ and $R^7$ represent a hydrogen atom, and $R^3$ represents any one substituent indicated in [Table L1] (hereinafter, referred to as "Compound class SX18").

A present compound (L-1) wherein $R^1$ represents $CF_3$, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX19").

A present compound (L-1) wherein $R^1$ and $R^2$ represent $CF_3$, $R^4$, $R^5$, $R^6$ and $R^7$ represent a hydrogen atom, and $R^3$ represents any one substituent indicated in [Table LI] (hereinafter, referred to as "Compound class SX20").

A present compound (L-1) wherein $R^1$ represents a chlorine atom, $R^2$ represents a bromine atom, $R^4$, $R^5$, $R^6$ and $R^7$ represent a hydrogen atom, and $R^3$ represents any one substituent indicated in [Table L1] (hereinafter, referred to as "Compound class SX21").

A present compound (L-1) wherein $R^1$ represents a chlorine atom, $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX22").

A present compound (L-1) wherein $R^1$ represents a chlorine atom, $R^2$ represents a methyl group, $R^4$, $R^5$, $R^6$ and $R^7$ represent a hydrogen atom, and $R^3$ represents any one substituent indicated in [Table L1] (hereinafter, referred to as "Compound class SX23").

A present compound (L-1) wherein $R^1$ represents a chlorine atom, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX24").

A present compound (L-1) wherein $R^1$ represents a chlorine atom, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX25").

A present compound (L-1) wherein $R^1$ represents a chlorine atom, $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX26").

A present compound (L-1) wherein $R^1$ represents a chlorine atom, $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX27").

A present compound (L-1) wherein $R^1$ represents a chlorine atom, $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX28").

A present compound (L-1) wherein $R^1$ represents a bromine atom, $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX29").

A present compound (L-1) wherein $R^1$ represents a bromine atom, $R^2$ represents a methyl group, $R^4$, $R^5$, $R^6$ and $R^7$ represent a hydrogen atom, and $R^3$ represents any one substituent indicated in [Table L1] (hereinafter, referred to as "Compound class SX30").

A present compound (L-1) wherein $R^1$ represents a bromine atom, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX31").

A present compound (L-1) wherein $R^1$ represents a bromine atom, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX32").

A present compound (L-1) wherein $R^1$ represents a bromine atom, $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX33").

A present compound (L-1) wherein $R^1$ represents a bromine atom, $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX34").

A present compound (L-1) wherein $R^1$ represents a bromine atom, $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX35").

A present compound (L-1) wherein $R^1$ represents a methyl group, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX36").

A present compound (1-1) wherein $R^1$ represents a methyl group, $R^2$ represents a propyl group, $R^4$, $R^5$, $R^6$ and $R^7$ represent a hydrogen atom, and $R^3$ represents any one substituent indicated in [Table 11) (hereinafter, referred to as "Compound class SX37").

A present compound (L-1) wherein $R^1$ represents an ethyl group, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX38").

A present compound (L-1) wherein $R^1$ represents a fluorine atom, $R^2$ represents a hydrogen atom, $R^3$, $R^5$, $R^6$ and $R^7$ represent a hydrogen atom, and $R^4$ represents any one substituent indicated in [Table 11] to [Table L3] (hereinafter, referred to as "Compound class SX39").

A present compound (L-1) wherein $R^1$ and $R^2$ represent a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX40").

A present compound (L-1) wherein $R^1$ represents a chlorine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX41").

A present compound (L-1) wherein $R^1$ and $R^2$ represent a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX42").

A present compound (L-1) wherein $R^1$ represents a bromine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX43").

A present compound (L-1) wherein $R^1$ and $R^2$ represent a bromine atom, $R^3$, $R^5$, $R^6$ and $R^7$ represent a hydrogen atom, and $R^4$ represents any one substituent indicated in [Table L1] to [Table L3] (hereinafter, referred to as "Compound class SX44") .

A present compound (L-1) wherein $R^1$ represents an iodine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX45").

A present compound (L-1) wherein $R^1$ and $R^2$ represent an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX46").

A present compound (L-1) wherein $R^1$ represents a methyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX47").

A present compound (L-1) wherein $R^3$ and $R^2$ represent a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX48").

A present compound (L-1) wherein $R^1$ represents an ethyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX49").

A present compound (L-1) wherein $R^1$ and $R^2$ represent an ethyl group, $R^3$, $R^5$, $R^6$ and $R^7$ represent a hydrogen

atom, and $R^4$ represents any one substituent indicated in [Table L1] to [Table L3] (hereinafter, referred to as "Compound class SX50").

A present compound (L-1) wherein $R^1$ represents a propyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX51").

A present compound (L-1) wherein $R^1$ and $R^2$ represent a propyl group, $R^3$, $R^5$, $R^6$ and $R^7$ represent a hydrogen atom, and $R^4$ represents any one substituent indicated in [Table L1) to [Table L3] (hereinafter, referred to as "Compound class SX52").

A present compound (L-1) wherein $R^1$ represents an isopropyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX53").

A present compound (L-1) wherein $R^1$ and $R^2$ represent an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX54").

A present compound (L-1) wherein $R^1$ represents a cyclopropyl group, $R^2$ represents a hydrogen atom, $R^3$, $R^5$, $R^6$ and $R^7$ represent a hydrogen atom, and $R^4$ represents any one substituent indicated in [Table L1) to [Table L3] (hereinafter, referred to as "Compound class SX55").

A present compound (L-1) wherein $R^1$ and $R^2$ represent a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX56").

A present compound (L-1) wherein $R^1$ represents $CF_3$, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX57").

A present compound (L-1) wherein $R^1$ and $R^2$ represent $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX58").

A present compound (L-1) wherein $R^1$ represents a chlorine atom, $R^2$ represents a bromine atom, $R^3$, $R^5$, $R^6$ and $R^7$ represent a hydrogen atom, and $R^4$ represents any one substituent indicated in [Table L1] to [Table L3] (hereinafter, referred to as "Compound class SX59").

A present compound (L-1) wherein $R^1$ represents a chlorine atom, $R^2$ represents an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX60").

A present compound (L-1) wherein $R^1$ represents a chlorine atom, $R^2$ represents a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SK62").

A present compound (L-1) wherein $R^1$ represents a chlorine atom, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SK62").

A present compound (L-1) wherein $R^1$ represents a chlorine atom, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX63").

A present compound (L-1) wherein $R^1$ represents a chlorine atom, $R^2$ represents an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX64").

A present compound (L-1) wherein $R^1$ represents a chlorine atom, $R^2$ represents a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX65").

A present compound (L-1) wherein $R^1$ represents a chlorine atom, $R^2$ represents $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX66").

A present compound (L-1) wherein $R^1$ represents a bromine atom, $R^2$ represents an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX67").

A present compound (L-1) wherein $R^1$ represents a bromine atom, $R^2$ represents a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX68").

A present compound (L-1) wherein $R^1$ represents a bromine atom, $R^2$ represents an ethyl group, $R^3$, $R^5$, $R^6$ and $R^7$ represent a hydrogen atom, and $R^4$ represents any one substituent indicated in [Table L1] to [Table L3] (hereinafter,

referred to as "Compound class SX69").

A present compound (L-1) wherein $R^1$ represents a bromine atom, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX70").

A present compound (L-1) wherein $R^1$ represents a bromine atom, $R^2$ represents an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX71").

A present compound (L-1) wherein $R^1$ represents a bromine atom, $R^2$ represents a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX72").

A present compound (L-1) wherein $R^1$ represents a bromine atom, $R^2$ represents $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX73").

A present compound (L-1) wherein $R^1$ represents a methyl group, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX74").

A present compound (L-1) wherein $R^1$ represents a methyl group, $R^2$ represents a propyl group, $R^3$, $R^3$, $R^6$ and $R^7$ represent a hydrogen atom, and $R^4$ represents any one substituent indicated in [Table L1] to [Table L3] (hereinafter, referred to as "Compound class SX75").

A present compound (L-1) wherein $R^1$ represents an ethyl group, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX76").

A present compound (L-1) wherein $R^1$ represents a fluorine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX77").

A present compound (L-1) wherein $R^1$ and $R^2$ represent a fluorine atom, $R^3$, $R^4$, $R^6$ and $R^7$ represent a hydrogen atom, and $R^5$ represents any one substituent indicated in [Table L1] to [Table L2] (hereinafter, referred to as "Compound class SX78").

A present compound (L-1) wherein $R^1$ represents a chlorine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX79").

A present compound (L-1) wherein $R^1$ and $R^2$ represent a chlorine atom, $R^3$, $R^4$, $R^6$ and $R^7$ represent a hydrogen atom, and $R^5$ represents any one substituent indicated in [Table L1] to [Table L2] (hereinafter, referred to as "Compound class SX80").

A present compound (L-1) wherein $R^1$ represents a bromine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX81").

A present compound (L-1) wherein $R^1$ and $R^2$ represent a bromine atom, $R^3$, $R^4$, $R^6$ and $R^7$ represent a hydrogen atom, and $R^5$ represents any one substituent indicated in [Table L1] to [Table L2] (hereinafter, referred to as "Compound class SX82").

A present compound (L-1) wherein $R^1$ represents an iodine atom, $R^2$ represents a hydrogen atom, $R^3$, $R^4$, $R^6$ and $R^7$ represent a hydrogen atom, and $R^5$ represents any one substituent indicated in [Table Z1] to [Table L2] (hereinafter, referred to as "Compound class SX83").

A present compound (L-1) wherein $R^1$ and $R^2$ represent an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX84").

A present compound (L-1) wherein $R^1$ represents a methyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX85").

A present compound (L-1) wherein $R^1$ and $R^2$ represent a methyl group, $R^3$, $R^4$, $R^6$ and $R^7$ represent a hydrogen atom, and $R^6$ represents any one substituent indicated in [Table L1] to [Table L2] (hereinafter, referred to as "Compound class SX86").

A present compound (L-1) wherein $R^1$ represents an ethyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX87").

A present compound (L-1) wherein $R^1$ and $R^2$ represent an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX88").

A present compound (L-1) wherein $R^1$ represents a propyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX89").

A present compound (L-1) wherein $R^1$ and $R^2$ represent a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX90").

A present compound (L-1) wherein $R^1$ represents an isopropyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX91").

A present compound (L-1) wherein $R^1$ and $R^2$ represent a an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX92").

A present compound (L-1) wherein $R^1$ represents a cyclopropyl group, $R^2$ represents a hydrogen atom, $R^3$, $R^4$, $R^5$ and $R^7$ represent a hydrogen atom, and $R^5$ represents any one substituent indicated in [Table L1] to [Table L2] (hereinafter, referred to as "Compound class SX93").

A present compound (L-1) wherein $R^1$ and $R^2$ represent a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX94").

A present compound (L-1) wherein $R^1$ represents $CF_3$, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX95").

A present compound (L-1) wherein $R^1$ and $R^2$ represent $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX96").

A present compound (L-1) wherein $R^1$ represents a chlorine atom, $R^2$ represents a bromine atom, $R^3$, $R^4$, $R^6$ and $R^7$ represent a hydrogen atom, and $R^5$ represents any one substituent indicated in [Table L1] to [Table L2] (hereinafter, referred to as "Compound class SX97").

A present compound (L-1) wherein $R^1$ represents a chlorine atom, $R^2$ represents an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX98").

A present compound (L-1) wherein $R^1$ represents a chlorine atom, $R^2$ represents a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX99").

A present compound (L-1) wherein $R^1$ represents a chlorine atom, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX100").

A present compound (L-1) wherein $R^1$ represents a chlorine atom, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX101").

A present compound (Z-1) wherein $R^1$ represents a chlorine atom, $R^2$ represents an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX102").

A present compound (L-1) wherein $R^1$ represents a chlorine atom, $R^2$ represents a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX103").

A present compound (L-1) wherein $R^1$ represents a chlorine atom, $R^2$ represents $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX104").

A present compound (L-1) wherein $R^1$ represents a bromine atom, $R^2$ represents an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX105").

A present compound (L-1) wherein $R^1$ represents a bromine atom, $R^2$ represents a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX106").

A present compound (L-1) wherein $R^1$ represents a bromine atom, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX107").

A present compound (L-1) wherein $R^1$ represents a bromine atom, $R^2$ represents a propyl group, $R^3$, $R^4$, $R^6$

and $R^7$ represent a hydrogen atom, and $R^5$ represents any one substituent indicated in [Table L1] to [Table L2] (hereinafter, referred to as "Compound class SX108").

A present compound (L-1) wherein $R^1$ represents a bromine atom, $R^2$ represents an isopropyl group, $R^3$, $R^4$, $R^5$ and $R^7$ represent a hydrogen atom, and $R^5$ represents any one substituent indicated in [Table L1] to [Table L2] (hereinafter, referred to as "Compound class SX109").

A present compound (L-1) wherein $R^1$ represents a bromine atom, $R^2$ represents a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX110").

A present compound (L-1) wherein $R^1$ represents a bromine atom, $R^2$ represents $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX111").

A present compound (L-1) wherein $R^1$ represents a methyl group, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX112").

A present compound (L-1) wherein $R^1$ represents a methyl group, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX113").

A present compound (L-1) wherein $R^1$ represents an ethyl group, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX114").

A present compound (L-1) wherein $R^1$ represents a fluorine atom, $R^2$ represents a hydrogen atom, $R^3$ represents a fluorine atom, $R^4$, $R^6$ and $R^7$ represent a hydrogen atom, and $R^5$ represents any one substituent indicated in [Table L1] to [Table L2] (hereinafter, referred to as "Compound class SX115").

A present compound (L-1) wherein $R^1$ represents a fluorine atom, $R^2$ represents a hydrogen atom, $R^3$ represents a chlorine atom, $R^4$, $R^6$ and $R^7$ represent a hydrogen atom, and $R^5$ represents any one substituent indicated in [Table L1] to [Table L2] (hereinafter, referred to as "Compound class SX116") .

A present compound (L-1) wherein $R^1$ represents a fluorine atom, $R^2$ represents a hydrogen atom, $R^3$ represents a bromine atom, $R^4$, $R^6$ and $R^7$ represent a hydrogen atom, and $R^5$ represents any one substituent indicated in [Table L1] to [Table L2] (hereinafter, referred to as "Compound class SX117").

A present compound (L-1) wherein $R^1$ represents a fluorine atom, $R^2$ represents a hydrogen atom, $R^3$ represents an iodine atom, $R^4$, $R^6$ and $R^7$ represent a hydrogen atom, and $R^5$ represents any one substituent indicated in [Table L1] to [Table L2] (hereinafter, referred to as "Compound class SX118").

A present compound (L-1) wherein $R^1$ represents a fluorine atom, $R^2$ represents a hydrogen atom, $R^3$ represents a methyl group, $R^4$, $R^6$ and $R^7$ represent a hydrogen atom, and $R^5$ represents any one substituent indicated in [Table L1] to [Table L2] (hereinafter, referred to as "Compound class SX119").

A present compound (L-1) wherein $R^1$ represents a fluorine atom, $R^2$ represents a hydrogen atom, $R^3$ represents a methoxy group, $R^4$, $R^6$ and $R^7$ represent a hydrogen atom, and $R^5$ represents any one substituent indicated in [Table L1] to [Table L2] (hereinafter, referred to as "Compound class SX120").

A present compound (L-1) wherein $R^1$ represents a fluorine atom, $R^2$ represents a hydrogen atom, $R^3$ represents $CF_3$, $R^4$, $R^6$ and $R^7$ represent a hydrogen atom, and $R^5$ represents any one substituent indicated in [Table L1] to [Table L2] (hereinafter, referred to as "Compound class SX121").

A present compound (L-1) wherein $R^1$ represents a fluorine atom, $R^2$ represents a hydrogen atom, $R^3$ represents $OCF_3$, $R^4$, $R^6$ and $R^7$ represent a hydrogen atom, and $R^5$ represents any one substituent indicated in [Table L1] to [Table L2] (hereinafter, referred to as "Compound class SX122") .

A present compound (L-1) wherein $R^1$ represents a fluorine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX123").

A present compound (L-1) wherein $R^1$ represents a fluorine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX124").

A present compound (L-1) wherein $R^1$ represents a fluorine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX125").

A present compound (L-1) wherein $R^1$ represents a fluorine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX126").

A present compound (L-1) wherein $R^1$ represents a fluorine atom, $R^2$ represents a hydrogen atom, $R^5$ represents a methyl group, $R^3$, $R^6$ and $R^7$ represent a hydrogen atom, and $R^4$ represents any one substituent indicated in [Table

L1] to [Table L3] (hereinafter, referred to as "Compound class SX127").

A present compound (L-1) wherein $R^1$ represents a fluorine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX128").

A present compound (L-1) wherein $R^1$ represents a fluorine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX129").

A present compound (L-1) wherein $R^1$ represents a fluorine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX130").

A present compound (L-1) wherein $R^1$ represents a fluorine atom, $R^2$ represents a hydrogen atom, $R^4$ represents a fluorine atom, $R^3$, $R^5$ and $R^7$ represent a hydrogen atom, and $R^6$ represents any one substituent indicated in [Table L1] to [Table L3] (hereinafter, referred to as "Compound class SX131").

A present compound (L-1) wherein $R^1$ represents a fluorine atom, $R^2$ represents a hydrogen atom, $R^4$ represents a chlorine atom, $R^3$, $R^5$ and $R^7$ represent a hydrogen atom, and $R^6$ represents any one substituent indicated in [Table L1] to [Table L3] (hereinafter, referred to as "Compound class SX132").

A present compound (L-1) wherein $R^1$ represents a fluorine atom, $R^2$ represents a hydrogen atom, $R^4$ represents a bromine atom, $R^3$, $R^5$ and $R^7$ represent a hydrogen atom, and $R^6$ represents any one substituent indicated in [Table L1] to [Table L3] (hereinafter, referred to as "Compound class SX133").

A present compound (L-1) wherein $R^1$ represents a fluorine atom, $R^2$ represents a hydrogen atom, $R^4$ represents an iodine atom, $R^3$, $R^5$ and $R^7$ represent a hydrogen atom, and $R^6$ represents any one substituent indicated in [Table L1] to [Table L3] (hereinafter, referred to as "Compound class SX134").

A present compound (L-1) wherein $R^1$ represents a fluorine atom, $R^2$ represents a hydrogen atom, $R^4$ represents a methyl group, $R^3$, $R^5$ and $R^7$ represent a hydrogen atom, and $R^6$ represents any one substituent indicated in [Table L1] to [Table L3] (hereinafter, referred to as "Compound class SX135").

A present compound (L-1) wherein $R^1$ represents a fluorine atom, $R^2$ represents a hydrogen atom, $R^4$ represents a methoxy group, $R^3$, $R^5$ and $R^7$ represent a hydrogen atom, and $R^6$ represents any one substituent indicated in [Table L1] to [Table L3] (hereinafter, referred to as "Compound class SX136").

A present compound (L-1) wherein $R^1$ represents a fluorine atom, $R^2$ represents a hydrogen atom, $R^4$ represents $CF_3$, $R^3$, $R^5$ and $R^7$ represent a hydrogen atom, and $R^6$ represents any one substituent indicated in [Table L1] to [Table L3] (hereinafter, referred to as "Compound class SX137").

A present compound (L-1) wherein $R^1$ represents a fluorine atom, $R^2$ represents a hydrogen atom, $R^4$ represents $OCF_3$, $R^3$, $R^5$ and $R^7$ represent a hydrogen atom, and $R^6$ represents any one substituent indicated in [Table L1] to [Table L3] (hereinafter, referred to as "Compound class SX138").

A present compound (L-1) wherein $R^1$ represents a chlorine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX139").

A present compound (L-1) wherein $R^1$ represents a chlorine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX140").

A present compound (L-1) wherein $R^1$ represents a chlorine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX141").

A present compound (L-1) wherein $R^1$ represents a chlorine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX192").

A present compound (L-1) wherein $R^1$ represents a chlorine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX143").

A present compound (L-1) wherein $R^1$ represents a chlorine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX144").

A present compound (L-1) wherein $R^1$ represents a chlorine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX145").

A present compound (L-1) wherein $R^1$ represents a chlorine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX146").

A present compound (L-1) wherein $R^1$ represents a chlorine atom, $R^2$ represents a hydrogen atom, $R^4$ represents a fluorine atom, $R^3$, $R^5$ and $R^7$ represent a hydrogen atom, and $R^6$ represents any one substituent indicated in [Table L1] to [Table L3] (hereinafter, referred to as "Compound class SX147").

A present compound (L-1) wherein $R^1$ represents a chlorine atom, $R^2$ represents a hydrogen atom, $R^4$ represents a chlorine atom, $R^3$, $R^5$ and $R^7$ represent a hydrogen atom, and $R^6$ represents any one substituent indicated in [Table L1] to [Table L3] (hereinafter, referred to as "Compound class SX148").

A present compound (L-1) wherein $R^1$ represents a chlorine atom, $R^2$ represents a hydrogen atom, $R^4$ represents a bromine atom, $R^3$, $R^5$ and $R^7$ represent a hydrogen atom, and $R^6$ represents any one substituent indicated in [Table L1] to [Table L3] (hereinafter, referred to as "Compound class SX149").

A present compound (L-1) wherein $R^1$ represents a chlorine atom, $R^2$ represents a hydrogen atom, $R^4$ represents an iodine atom, $R^3$, $R^5$ and $R^7$ represent a hydrogen atom, and $R^6$ represents any one substituent indicated in [Table L1] to [Table L3] (hereinafter, referred to as "Compound class SX150").

A present compound (L-1) wherein $R^1$ represents a chlorine atom, $R^2$ represents a hydrogen atom, $R^4$ represents a methyl group, $R^3$, $R^5$ and $R^7$ represent a hydrogen atom, and $R^6$ represents any one substituent indicated in [Table L1] to [Table L3] (hereinafter, referred to as "Compound class SX151").

A present compound (L-1) wherein $R^1$ represents a chlorine atom, $R^2$ represents a hydrogen atom, $R^4$ represents a methoxy group, $R^3$, $R^5$ and $R^7$ represent a hydrogen atom, and $R^6$ represents any one substituent indicated in [Table L1] to [Table L3] (hereinafter, referred to as "Compound class SX152") .

A present compound (L-1) wherein $R^1$ represents a chlorine atom, $R^2$ represents a hydrogen atom, $R^4$ represents $CF_3$, $R^3$, $R^5$ and $R^7$ represent a hydrogen atom, and $R^6$ represents any one substituent indicated in [Table L1] to [Table L3] (hereinafter, referred to as "Compound class SX153").

A present compound (L-1) wherein $R^1$ represents a chlorine atom, $R^2$ represents a hydrogen atom, $R^4$ represents $OCF_3$, $R^3$, $R^5$ and $R^7$ represent a hydrogen atom, and $R^6$ represents any one substituent indicated in [Table L1] to [Table L3] (hereinafter, referred to as "Compound class SX154") .

A present compound (L-1) wherein $R^1$ represents a bromine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX155") .

A present compound (L-1) wherein $R^1$ represents a bromine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX156") .

A present compound (L-1) wherein $R^1$ represents a bromine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX157").

A present compound (L-1) wherein $R^1$ represents a bromine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX158") .

A present compound (L-1) wherein $R^1$ represents a bromine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX159") .

A present compound (L-1) wherein $R^1$ represents a bromine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX160") .

A present compound (L-1) wherein $R^1$ represents a bromine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX161").

A present compound (L-1) wherein $R^1$ represents a bromine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX162").

A present compound (L-1) wherein $R^1$ represents a bromine atom, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX163").

A present compound (L-1) wherein $R^1$ represents a bromine atom, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX164").

A present compound (L-1) wherein $R^1$ represents a bromine atom, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX165") .

A present compound (L-1) wherein $R^1$ represents a bromine atom, $R^2$ represents a hydrogen atom, $R^6$ represents

an iodine atom, $R^3$, $R^5$ and $R^7$ represent a hydrogen atom, and $R^4$ represents any one substituent indicated in [Table L1] to [Table L3] (hereinafter, referred to as "Compound class SX166").

A present compound (L-1) wherein $R^1$ represents a bromine atom, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX167") .

A present compound (L-1) wherein $R^1$ represents a bromine atom, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX168") .

A present compound (L-1) wherein $R^1$ represents a bromine atom, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX169").

A present compound (L-1) wherein $R^1$ represents a bromine atom, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX170").

A present compound (L-1) wherein $R^1$ represents an iodine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX171").

A present compound (L-1) wherein $R^1$ represents an iodine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX172").

A present compound (L-1) wherein $R^1$ represents an iodine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX173") .

A present compound (L-1) wherein $R^1$ represents an iodine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX174") .

A present compound (L-1) wherein $R^1$ represents an iodine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX175") .

A present compound (L-1) wherein $R^1$ represents an iodine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX176").

A present compound (L-1) wherein $R^1$ represents an iodine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX177").

A present compound (L-1) wherein $R^1$ represents an iodine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX178").

A present compound (L-1) wherein $R^1$ represents an iodine atom, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX179").

A present compound (L-1) wherein $R^1$ represents an iodine atom, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX180").

A present compound (L-1) wherein $R^1$ represents an iodine atom, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX181").

A present compound (L-1) wherein $R^1$ represents an iodine atom, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX182").

A present compound (L-1) wherein $R^1$ represents an iodine atom, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX183") .

A present compound (L-1) wherein $R^1$ represents an iodine atom, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX184") .

A present compound (L-1) wherein $R^1$ represents an iodine atom, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table

L3] (hereinafter, referred to as "Compound class SX185").

A present compound (L-1) wherein $R^1$ represents an iodine atom, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX186").

A present compound (L-1) wherein $R^1$ represents a methyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX187").

A present compound (L-1) wherein $R^1$ represents a methyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX188").

A present compound (L-1) wherein $R^1$ represents a methyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX189").

A present compound (L-1) wherein $R^1$ represents a methyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX190").

A present compound (L-1) wherein $R^1$ represents a methyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX191").

A present compound (L-1) wherein $R^1$ represents a methyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX192").

A present compound (L-1) wherein $R^1$ represents a methyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX193").

A present compound (L-1) wherein $R^1$ represents a methyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX194").

A present compound (L-1) wherein $R^1$ represents a methyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX195").

A present compound (L-1) wherein $R^1$ represents a methyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX196").

A present compound (L-1) wherein $R^1$ represents a methyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX197[11]).

A present compound (L-1) wherein $R^1$ represents a methyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX198").

A present compound (L-1) wherein $R^1$ represents a methyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX199").

A present compound (Z-1) wherein $R^1$ represents a methyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX200") .

A present compound (L-1) wherein $R^1$ represents a methyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX201").

A present compound (L-1) wherein $R^1$ represents a methyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX202").

A present compound (L-1) wherein $R^1$ represents an ethyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX203") .

A present compound (L-1) wherein $R^1$ represents an ethyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX204") .

A present compound (L-1) wherein $R^1$ represents an ethyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX205") .

A present compound (L-1) wherein $R^1$ represents an ethyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX206").

A present compound (L-1) wherein $R^1$ represents an ethyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX207") .

A present compound (L-1) wherein $R^1$ represents an ethyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX208") .

A present compound (L-1) wherein $R^1$ represents an ethyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX209").

A present compound (L-1) wherein $R^1$ represents an ethyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX210").

A present compound (L-1) wherein $R^1$ represents an ethyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX211").

A present compound (L-1) wherein $R^1$ represents an ethyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX212").

A present compound (L-1) wherein $R^1$ represents an ethyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX213").

A present compound (L-1) wherein $R^1$ represents an ethyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX214").

A present compound (L-1) wherein $R^1$ represents an ethyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX215").

A present compound (L-1) wherein $R^1$ represents an ethyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX216").

A present compound (L-1) wherein $R^1$ represents an ethyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX217").

A present compound (L-1) wherein $R^1$ represents an ethyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX218").

A present compound (L-1) wherein $R^1$ represents $CF_3$, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX219").

A present compound (L-1) wherein $R^1$ represents $CF_3$, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX220").

A present compound (Z-1) wherein $R^1$ represents $CF_3$, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX221").

A present compound (Z-1) wherein $R^1$ represents $CF_3$, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX222").

A present compound (L-1) wherein $R^1$ represents $CF_3$, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX223").

A present compound (L-1) wherein $R^1$ represents $CF_3$, $R^2$ represents a hydrogen atom, $R^5$ represents a methoxy

group, $R^3$, $R^6$ and $R^7$ represent a hydrogen atom, and $R^4$ represents any one substituent indicated in [Table L1] to [Table L3] (hereinafter, referred to as "Compound class SX229").

A present compound (L-1) wherein $R^1$ represents $CF_3$, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX225").

A present compound (L-1) wherein $R^1$ represents $CF_3$, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX226").

A present compound (L-1) wherein $R^1$ represents $CF_3$, $R^2$ represents a hydrogen atom, $R^6$ represents a fluorine atom, R3, $R^5$ and $R^7$ represent a hydrogen atom, and $R^4$ represents any one substituent indicated in [Table L1] to [Table L3] (hereinafter, referred to as "Compound class SX227").

A present compound (L-1) wherein $R^1$ represents $CF_3$, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX228").

A present compound (L-1) wherein $R^1$ represents $CF_3$, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX229").

A present compound (L-1) wherein $R^1$ represents $CF_3$, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX230").

A present compound (L-1) wherein $R^1$ represents $CF_3$, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX231").

A present compound (L-1) wherein $R^1$ represents $CF_3$, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX232").

A present compound (L-1) wherein $R^1$ represents $CF_3$, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX233").

A present compound (L-1) wherein $R^1$ represents $CF_3$, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX234").

A present compound (L-1) wherein $R^1$ represents a cyclopropyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX235").

A present compound (L-1) wherein $R^1$ represents a cyclopropyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX236").

A present compound (L-1) wherein $R^1$ represents a cyclopropyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX237").

A present compound (L-1) wherein $R^1$ represents a cyclopropyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX238").

A present compound (L-1) wherein $R^1$ represents a cyclopropyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX239").

A present compound (L-1) wherein $R^1$ represents a cyclopropyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX240").

A present compound (L-1) wherein $R^1$ represents a cyclopropyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX241").

A present compound (L-1) wherein $R^1$ represents a cyclopropyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX242").

A present compound (L-1) wherein $R^1$ represents a cyclopropyl group, $R^2$ represents a hydrogen atom, $R^6$ represents a fluorine atom, $R^3$, $R^5$ and $R^7$ represent a hydrogen atom, and $R^4$ represents any one substituent indicated

in [Table L1] to [Table L3] (hereinafter, referred to as "Compound class SX243").

A present compound (L-1) wherein $R^1$ represents a cyclopropyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX244").

A present compound (L-1) wherein $R^1$ represents a cyclopropyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX245[11]).

A present compound (L-1) wherein $R^1$ represents a cyclopropyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX246").

A present compound (L-1) wherein $R^1$ represents a cyclopropyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX247").

A present compound (L-1) wherein $R^1$ represents a cyclopropyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX248").

A present compound (L-1) wherein $R^1$ represents a cyclopropyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX249").

A present compound (L-1) wherein $R^1$ represents a cyclopropyl group, $R^2$ represents a hydrogen atom, $R^6$ represents $OCF_3$, $R^3$, $R^5$ and $R^7$ represent a hydrogen atom, and $R^4$ represents any one substituent indicated in [Table v1] to [Table L3] (hereinafter, referred to as "Compound class SX250").

A present compound (L-1) wherein $R^1$ represents a methylsulfanyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX251").

A present compound (L-1) wherein $R^1$ represents a methylsulfanyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX252").

A present compound (L-1) wherein $R^1$ represents a methylsulfanyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX253").

A present compound (L-1) wherein $R^1$ represents a methylsulfanyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX254").

A present compound (L-1) wherein $R^1$ represents a methylsulfanyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX255").

A present compound (L-1) wherein $R^1$ represents a methylsulfanyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX256").

A present compound (L-1) wherein $R^1$ represents a methylsulfanyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX257").

A present compound (L-1) wherein $R^1$ represents a methylsulfanyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX258").

A present compound (L-1) wherein $R^1$ represents a methylsulfanyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX259").

A present compound (L-1) wherein $R^1$ represents a methylsulfanyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX260").

A present compound (L-1) wherein $R^1$ represents a methylsulfanyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX261").

A present compound (Z-1) wherein $R^1$ represents a methylsulfanyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX262").

A present compound (L-1) wherein $R^1$ represents a methylsulfanyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX263").

A present compound (L-1) wherein $R^1$ represents a methylsulfanyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX264").

A present compound (L-1) wherein $R^1$ represents a methylsulfanyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX265").

A present compound (L-1) wherein $R^1$ represents a methylsulfanyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX266").

A present compound (L-1) wherein $R^1$ represents a methylsulfanyl group, $R^2$ represents a hydrogen atom, $R^7$ represents C-F, $R^3$, $R^5$ and $R^6$ represent a hydrogen atom, and $R^4$ represents any one substituent indicated in [Table L1] to [Table L3] (hereinafter, referred to as "Compound class SX267").

A present compound (L-1) wherein $R^1$ represents a methylsulfanyl group, $R^2$ represents a hydrogen atom, $R^7$ represents C-Cl, $R^3$, $R^5$ and $R^6$ represent a hydrogen atom, and $R^4$ represents any one substituent indicated in [Table L1] to [Table L3] (hereinafter, referred to as "Compound class SX268").

A present compound (L-1) wherein $R^1$ represents a methylsulfanyl group, $R^2$ represents a hydrogen atom, $R^7$ represents C-Br, $R^3$, $R^5$ and $R^6$ represent a hydrogen atom, and $R^4$ represents any one substituent indicated in [Table L1] to [Table L3] (hereinafter, referred to as "Compound class SX269").

A present compound (Z-1) wherein $R^1$ represents a methylsulfanyl group, $R^2$ represents a hydrogen atom, $R^7$ represents C-I, $R^3$, $R^5$ and $R^6$ represent a hydrogen atom, and $R^4$ represents any one substituent indicated in [Table L1] to [Table L3] (hereinafter, referred to as "Compound class SX270").

A present compound (L-1) wherein $R^1$ represents a methylsulfanyl group, $R^2$ represents a hydrogen atom, $R^7$ represents C-Me, $R^3$, $R^5$ and $R^6$ represent a hydrogen atom, and $R^4$ represents any one substituent indicated in [Table L1] to [Table L3] (hereinafter, referred to as "Compound class SX271").

A present compound (L-1) wherein $R^1$ represents a methylsulfanyl group, $R^2$ represents a hydrogen atom, $R^7$ represents C-OMe, $R^3$, $R^5$ and $R^6$ represent a hydrogen atom, and $R^4$ represents any one substituent indicated in [Table L1] to [Table L3] (hereinafter, referred to as "Compound class SX272").

A present compound (L-1) wherein $R^1$ represents a methylsulfanyl group, $R^2$ represents a hydrogen atom, $R^7$ represents C-$CF_3$, $R^3$, $R^5$ and $R^6$ represent a hydrogen atom, and $R^4$ represents any one substituent indicated in [Table L1] to [Table L3] (hereinafter, referred to as "Compound class SX273").

A present compound (L-1) wherein $R^1$ represents a methylsulfanyl group, $R^2$ represents a hydrogen atom, $R^7$ represents C-$OCF_3$, $R^3$, $R^5$ and $R^6$ represent a hydrogen atom, and $R^4$ represents any one substituent indicated in [Table L1] to [Table L3] (hereinafter, referred to as "Compound class SX274").

A present compound (L-1) wherein $R^1$ represents a methylsulfanyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX275").

A present compound (L-1) wherein $R^1$ represents a methylsulfanyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX276").

A present compound (L-1) wherein $R^1$ represents a methylsulfanyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX277").

A present compound (L-1) wherein $R^1$ represents a methylsulfanyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX278").

A present compound (L-1) wherein $R^1$ represents a methylsulfanyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX279").

A present compound (L-1) wherein $R^1$ represents a methylsulfanyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX280").

A present compound (L-1) wherein $R^1$ represents a methylsulfanyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX281").

A present compound (L-1) wherein $R^1$ represents a methylsulfanyl group, $R^2$ represents a hydrogen atom, $R^5$

represents $OCF_3$, $R^3$, $R^6$ and $R^7$ represent a hydrogen atom, and $R^4$ represents any one substituent indicated in [Table L1] to [Table L3] (hereinafter, referred to as "Compound class SX282").

A present compound (L-1) wherein $R^1$ represents a methylsulfanyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX283").

A present compound (L-1) wherein $R^1$ represents a methylsulfanyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX284").

A present compound (L-1) wherein $R^1$ represents a methylsulfanyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX285").

A present compound (L-1) wherein $R^1$ represents a methylsulfanyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX286").

A present compound (L-1) wherein $R^1$ represents a methylsulfanyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX287").

A present compound (L-1) wherein $R^1$ represents a methylsulfanyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX288").

A present compound (L-1) wherein $R^1$ represents a methylsulfanyl group, $R^2$ represents a hydrogen atom, $R^6$ represents $CF_3$, $R^3$, $R^5$ and $R^7$ represent a hydrogen atom, and $R^4$ represents any one substituent indicated in [Table L2] to [Table L3] (hereinafter, referred to as "Compound class SX289").

A present compound (L-1) wherein $R^1$ represents a methylsulfanyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX290").

A present compound (L-1) wherein $R^1$ and $R^2$ represent a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX291").

A present compound (L-1) wherein $R^1$ and $R^2$ represent a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX292").

A present compound (L-1) wherein $R^1$ and $R^2$ represent a fluorine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX293").

A present compound (L-1) wherein $R^1$ and $R^2$ represent a fluorine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX294").

A present compound (L-1) wherein $R^1$ and $R^2$ represent a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX295").

A present compound (L-1) wherein $R^1$ and $R^2$ represent a fluorine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX296").

A present compound (L-1) wherein $R^1$ and $R^2$ represent a fluorine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX297").

A present compound (L-1) wherein $R^1$ and $R^2$ represent a fluorine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX298").

A present compound (L-1) wherein $R^1$ and $R^2$ represent a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX299").

A present compound (L-1) wherein $R^1$ and $R^2$ represent a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX300").

A present compound (L-1) wherein $R^1$ and $R^2$ represent a fluorine atom, $R^6$ represents a bromine atom, $R^3$, $R^5$ and $R^7$ represent a hydrogen atom, and $R^4$ represents any one substituent indicated in [Table L1] to [Table L3]

(hereinafter, referred to as "Compound class SX301").

A present compound (L-1) wherein $R^1$ and $R^2$ represent a fluorine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX302").

A present compound (L-1) wherein $R^1$ and $R^2$ represent a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX303").

A present compound (L-1) wherein $R^1$ and $R^2$ represent a fluorine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX304").

A present compound (L-1) wherein $R^1$ and $R^2$ represent a fluorine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX305").

A present compound (L-1) wherein $R^1$ and $R^2$ represent a fluorine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX306").

A present compound (L-1) wherein $R^1$ and $R^2$ represent a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX307").

A present compound (L-1) wherein $R^1$ and $R^2$ represent a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX308").

A present compound (1-1) wherein $R^1$ and $R^2$ represent a chlorine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX309").

A present compound (L-1) wherein $R^1$ and $R^2$ represent a chlorine atom, $R^5$ represents an iodine atom, $R^3$, $R^6$ and $R^7$ represent a hydrogen atom, and $R^4$ represents any one substituent indicated in [Table 11] to [Table L3] (hereinafter, referred to as "Compound class SX310").

A present compound (L-1) wherein $R^1$ and $R^2$ represent a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX311").

A present compound (L-1) wherein $R^1$ and $R^2$ represent a chlorine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX312").

A present compound (L-1) wherein $R^1$ and $R^2$ represent a chlorine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX313").

A present compound (Z-1) wherein $R^1$ and $R^2$ represent a chlorine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX314").

A present compound (L-1) wherein $R^1$ and $R^2$ represent a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX315").

A present compound (L-1) wherein $R^1$ and $R^2$ represent a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX316").

A present compound (L-1) wherein $R^1$ and $R^2$ represent a chlorine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX317").

A present compound (L-1) wherein $R^1$ and $R^2$ represent a chlorine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX318").

A present compound (L-1) wherein $R^1$ and $R^2$ represent a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX319").

A present compound (Z-1) wherein $R^1$ and $R^2$ represent a chlorine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX320").

A present compound (L-1) wherein $R^1$ and $R^2$ represent a chlorine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX321").

A present compound (L-1) wherein $R^1$ and $R^2$ represent a chlorine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX322").

A present compound (L-1) wherein $R^1$ and $R^2$ represent a bromine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX323").

A present compound (L-1) wherein $R^1$ and $R^2$ represent a bromine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX324").

A present compound (L-1) wherein $R^1$ and $R^2$ represent a bromine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX325").

A present compound (L-1) wherein $R^1$ and $R^2$ represent a bromine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX326").

A present compound (L-1) wherein $R^1$ and $R^2$ represent a bromine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX327").

A present compound (L-1) wherein $R^1$ and $R^2$ represent a bromine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX328").

A present compound (L-1) wherein $R^1$ and $R^2$ represent a bromine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX329").

A present compound (L-1) wherein $R^1$ and $R^2$ represent a bromine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX330").

A present compound (L-1) wherein $R^1$ and $R^2$ represent a bromine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX331").

A present compound (L-1) wherein $R^1$ and $R^2$ represent a bromine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX332").

A present compound (L-1) wherein $R^1$ and $R^2$ represent a bromine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX333").

A present compound (L-1) wherein $R^1$ and $R^2$ represent a bromine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX334").

A present compound (L-1) wherein $R^1$ and $R^2$ represent a bromine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX335").

A present compound (L-1) wherein $R^1$ and $R^2$ represent a bromine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX336").

A present compound (L-1) wherein $R^1$ and $R^2$ represent a bromine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX337").

A present compound (L-1) wherein $R^1$ and $R^2$ represent a bromine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX338").

A present compound (L-1) wherein $R^1$ and $R^2$ represent an iodine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX339").

A present compound (L-1) wherein $R^1$ and $R^2$ represent an iodine atom, $R^5$ represents a chlorine atom, $R^3$,

$R^6$ and $R^7$ represent a hydrogen atom, and $R^4$ represents any one substituent indicated in [Table L1] to [Table L3] (hereinafter, referred to as "Compound class SX340").

A present compound (L-1) wherein $R^1$ and $R^2$ represent an iodine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX341").

A present compound (L-1) wherein $R^1$ and $R^2$ represent an iodine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX342").

A present compound (L-1) wherein $R^1$ and $R^2$ represent an iodine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX343").

A present compound (L-1) wherein $R^1$ and $R^2$ represent an iodine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX344").

A present compound (Z-1) wherein $R^1$ and $R^2$ represent an iodine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX345").

A present compound (L-1) wherein $R^1$ and $R^2$ represent an iodine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX346").

A present compound (L-1) wherein $R^1$ and $R^2$ represent an iodine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX347").

A present compound (L-1) wherein $R^1$ and $R^2$ represent an iodine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX348").

A present compound (L-1) wherein $R^1$ and $R^2$ represent an iodine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX349").

A present compound (L-1) wherein $R^1$ and $R^2$ represent an iodine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX350").

A present compound (L-1) wherein $R^1$ and $R^2$ represent an iodine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX351").

A present compound (L-1) wherein $R^1$ and $R^2$ represent an iodine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX352").

A present compound (L-1) wherein $R^1$ and $R^2$ represent an iodine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX353").

A present compound (L-1) wherein $R^1$ and $R^2$ represent an iodine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX354").

A present compound (L-1) wherein $R^1$ and $R^2$ represent a methyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX355").

A present compound (L-1) wherein $R^1$ and $R^2$ represent a methyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX356").

A present compound (L-1) wherein $R^1$ and $R^2$ represent a methyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX357").

A present compound (L-1) wherein $R^1$ and $R^2$ represent a methyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX358").

A present compound (L-1) wherein $R^1$ and $R^2$ represent a methyl group, $R^5$ represents a methyl group, $R^3$, $R^6$ and $R^7$ represent a hydrogen atom, and $R^4$ represents any one substituent indicated in [Table L1] to [Table L3] (hereinafter,

referred to as "Compound class SX359").

A present compound (L-1) wherein $R^1$ and $R^2$ represent a methyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX360").

A present compound (L-1) wherein $R^1$ and $R^2$ represent a methyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX361").

A present compound (L-1) wherein $R^1$ and $R^2$ represent a methyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX362").

A present compound (L-1) wherein $R^1$ and $R^2$ represent a methyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX363").

A present compound (L-1) wherein $R^1$ and $R^2$ represent a methyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX364").

A present compound (L-1) wherein $R^1$ and $R^2$ represent a methyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX365").

A present compound (L-1) wherein $R^1$ and $R^2$ represent a methyl group, $R^6$ represents a bromine atom, $R^3$, $R^5$ and $R^7$ represent an iodine atom, and $R^4$ represents any one substituent indicated in [Table L1] to [Table L3] (hereinafter, referred to as "Compound class SX366").

A present compound (L-1) wherein $R^1$ and $R^2$ represent a methyl group, $R^6$ represents a methyl group, $R^3$, $R^5$ and $R^7$ represent an iodine atom, and $R^4$ represents any one substituent indicated in [Table L1] to [Table L3] (hereinafter, referred to as "Compound class SX367").

A present compound (L-1) wherein $R^1$ and $R^2$ represent a methyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX368").

A present compound (L-1) wherein $R^1$ and $R^2$ represent a methyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX369").

A present compound (Z-1) wherein $R^1$ and $R^2$ represent a methyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX370").

A present compound (L-1) wherein $R^1$ and $R^2$ represent an ethyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX371").

A present compound (L-1) wherein $R^1$ and $R^2$ represent an ethyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX372").

A present compound (L-1) wherein $R^1$ and $R^2$ represent an ethyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX373").

A present compound (L-1) wherein $R^1$ and $R^2$ represent an ethyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX374").

A present compound (L-1) wherein $R^1$ and $R^2$ represent an ethyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX375").

A present compound (L-1) wherein $R^1$ and $R^2$ represent an ethyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX376").

A present compound (L-1) wherein $R^1$ and $R^2$ represent an ethyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX377").

A present compound (L-1) wherein $R^1$ and $R^2$ represent an ethyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX378").

A present compound (L-1) wherein $R^1$ and $R^2$ represent an ethyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX379").

A present compound (Z-1) wherein $R^1$ and $R^2$ represent an ethyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX380").

A present compound (L-1) wherein $R^1$ and $R^2$ represent an ethyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX381").

A present compound (L-1) wherein $R^1$ and $R^2$ represent an ethyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX382").

A present compound (L-1) wherein $R^1$ and $R^2$ represent an ethyl group, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX383").

A present compound (1-1) wherein $R^1$ and $R^2$ represent an ethyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX384").

A present compound (L-1) wherein $R^1$ and $R^2$ represent an ethyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX385").

A present compound (L-1) wherein $R^1$ and $R^2$ represent an ethyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX386").

A present compound (L-1) wherein $R^1$ and $R^2$ represent $CF_3$, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX387").

A present compound (L-1) wherein $R^1$ and $R^2$ represent $CF_3$, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX388").

A present compound (Z-1) wherein $R^1$ and $R^2$ represent $CF_3$, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX389").

A present compound (L-1) wherein $R^1$ and $R^2$ represent $CF_3$, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX390").

A present compound (L-1) wherein $R^1$ and $R^2$ represent $CF_3$, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX391").

A present compound (L-1) wherein $R^1$ and $R^2$ represent $CF_3$, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX392").

A present compound (L-1) wherein $R^1$ and $R^2$ represent $CF_3$, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX393").

A present compound (L-1) wherein $R^1$ and $R^2$ represent $CF_3$, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX394").

A present compound (L-1) wherein $R^1$ and $R^2$ represent $CF_3$, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX395").

A present compound (L-1) wherein $R^1$ and $R^2$ represent $CF_3$, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX396").

A present compound (L-1) wherein $R^1$ and $R^2$ represent $CF_3$, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX397").

A present compound (L-1) wherein $R^1$ and $R^2$ represent $CF_3$, $R^6$ represents an iodine atom, $R^3$, $R^5$ and $R^7$

represent a hydrogen atom, and $R^4$ represents any one substituent indicated in [Table L1] to [Table L3] (hereinafter, referred to as "Compound class SX398").

A present compound (L-1) wherein $R^1$ and $R^2$ represent $CF_3$, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX399").

A present compound (L-1) wherein $R^1$ and $R^2$ represent $CF_3$, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX400").

A present compound (L-1) wherein $R^1$ and $R^2$ represent $CF_3$, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX401").

A present compound (L-1) wherein $R^1$ and $R^2$ represent $CF_3$, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX402").

A present compound (L-1) wherein $R^1$ and $R^2$ represent a cyclopropyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX403").

A present compound (L-1) wherein $R^1$ and $R^2$ represent a cyclopropyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX404").

A present compound (L-1) wherein $R^1$ and $R^2$ represent a cyclopropyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX405").

A present compound (L-1) wherein $R^1$ and $R^2$ represent a cyclopropyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX406").

A present compound (L-1) wherein $R^1$ and $R^2$ represent a cyclopropyl group, $R^5$ represents a methyl group, $R^3$, $R^6$ and $R^7$ represent a hydrogen atom, and $R^4$ represents any one substituent indicated in [Table Z1] to [Table L3] (hereinafter, referred to as "Compound class SX407").

A present compound (L-1) wherein $R^1$ and $R^2$ represent a cyclopropyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX408").

A present compound (L-1) wherein $R^1$ and $R^2$ represent a cyclopropyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX409").

A present compound (L-1) wherein $R^2$ and $R^2$ represent a cyclopropyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX410").

A present compound (L-1) wherein $R^1$ and $R^2$ represent a cyclopropyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX411").

A present compound (L-1) wherein $R^1$ and $R^2$ represent a cyclopropyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX412").

A present compound (L-1) wherein $R^1$ and $R^2$ represent a cyclopropyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX413").

A present compound (L-1) wherein $R^1$ and $R^2$ represent a cyclopropyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX414").

A present compound (L-1) wherein $R^1$ and $R^2$ represent a cyclopropyl group, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX415").

A present compound (L-1) wherein $R^1$ and $R^2$ represent a cyclopropyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX416").

A present compound (L-1) wherein $R^1$ and $R^2$ represent a cyclopropyl group, $R^6$ represents $CF_3$, $R^3$, $R^5$ and $R^7$ represent a hydrogen atom, and $R^4$ represents any one substituent indicated in [Table L1] to [Table L3] (hereinafter,

referred to as "Compound class SX417").

A present compound (L-1) wherein $R^1$ and $R^2$ represent a cyclopropyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX418").

A present compound (L-1) wherein $R^1$ represents a chlorine atom, $R^2$ represents any one group selected from a hydrogen atom, a fluorine atom, a chlorine atom, a bromine atom, an iodine atom, a methyl group, an ethyl group, a propyl group, an isopropyl group, a cyclopropyl group, or $CF_3$, and $R^1$, $R^2$, $R^3$, $R^4$, $R^5$ represent a hydrogen atom (hereinafter, referred to as "Compound class SX419").

A compound represented by formula (L-2):

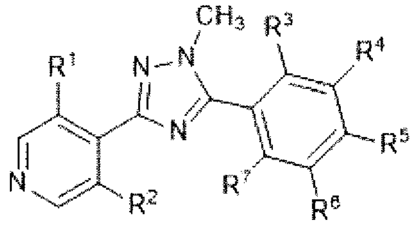

(L-2)

(hereinafter, referred to as "Compound (L-2)")
wherein $R^1$ represents a fluorine atom, $R^2$ represents a hydrogen atom, and $R^4$, $R^5$, $R^6$, and $R^7$ represent a hydrogen atom, and $R^3$ represents any one substituent indicated in [Table L1] (hereinafter, referred to as "Compound class SX420").

A present compound (L-2) wherein $R^1$ and $R^2$ represent a fluorine atom, $R^4$, $R^5$, $R^6$ and $R^7$ represent a hydrogen atom, and $R^3$ represents any one substituent indicated in [Table L1] (hereinafter, referred to as "Compound class SX421").

A present compound (L-2) wherein $R^1$ represents a chlorine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX422").

A present compound (L-2) wherein $R^1$ and $R^2$ represent a chlorine atom, $R^4$, $R^5$, $R^6$ and $R^7$ represent a hydrogen atom, and $R^3$ represents any one substituent indicated in [Table L1] (hereinafter, referred to as "Compound class SX423").

A present compound (L-2) wherein $R^1$ represents a bromine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX424").

A present compound (L-2) wherein $R^1$ and $R^2$ represents a bromine atom, $R^4$, $R^5$, $R^6$ and $R^7$ represent a hydrogen atom, and $R^3$ represents any one substituent indicated in [Table L1] (hereinafter, referred to as "Compound class SX425").

A present compound (L-2) wherein $R^1$ represents an iodine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX426").

A present compound (L-2) wherein $R^1$ and $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX427").

A present compound (L-2) wherein $R^1$ represents a methyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX428").

A present compound (L-2) wherein $R^1$ and $R^2$ represents a methyl group, $R^4$, $R^5$, $R^6$ and $R^7$ represent a hydrogen atom, and $R^3$ represents any one substituent indicated in [Table L1] (hereinafter, referred to as "Compound class SX429").

A present compound (L-2) wherein $R^1$ represents an ethyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX430").

A present compound (L-2) wherein $R^1$ and $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX431").

A present compound (L-2) wherein $R^1$ represents a propyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX432").

A present compound (L-2) wherein $R^1$ and $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX433").

A present compound (L-2) wherein $R^1$ represents an isopropyl group, $R^2$ represents a hydrogen atom, $R^4$, $R^5$,

$R^6$ and $R^7$ represent a hydrogen atom, and $R^3$ represents any one substituent indicated in [Table L1] (hereinafter, referred to as "Compound class SX434").

A present compound (L-2) wherein $R^1$ and $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX435").

A present compound (L-2) wherein $R^1$ represents a cyclopropyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX436").

A present compound (L-2) wherein $R^1$ and $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX437").

A present compound (L-2) wherein $R^1$ represents $CF_3$, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX438").

A present compound (L-2) wherein $R^1$ and $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX439").

A present compound (L-2) wherein $R^1$ represents a chlorine atom, $R^2$ represents a bromine atom, $R^4$, $R^5$, $R^6$ and $R^7$ represent a hydrogen atom, and $R^3$ represents any one substituent indicated in [Table L1] (hereinafter, referred to as "Compound class SX440").

A present compound (L-2) wherein $R^1$ represents a chlorine atom, $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX441").

A present compound (L-2) wherein $R^1$ represents a chlorine atom, $R^2$ represents a methyl group m, $R^4$, $R^5$, $R^6$ and $R^7$ represent a hydrogen atom, and $R^3$ represents any one substituent indicated in [Table L1] (hereinafter, referred to as "Compound class SX442").

A present compound (L-2) wherein $R^1$ represents a chlorine atom, $R^2$ represents an ethyl group m, $R^4$, $R^5$, $R^6$ and $R^7$ represent a hydrogen atom, and $R^3$ represents any one substituent indicated in [Table L1] (hereinafter, referred to as "Compound class SX443").

A present compound (L-2) wherein $R^1$ represents a chlorine atom, $R^2$ represents a propyl group m, $R^4$, $R^5$, $R^6$ and $R^7$ represent a hydrogen atom, and $R^3$ represents any one substituent indicated in [Table L1] (hereinafter, referred to as "Compound class SX444").

A present compound (L-2) wherein $R^1$ represents a chlorine atom, $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX445").

A present compound (L-2) wherein $R^1$ represents a chlorine atom, $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX446").

A present compound (L-2) wherein $R^1$ represents a chlorine atom, $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX447").

A present compound (L-2) wherein $R^1$ represents a bromine atom, $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX448").

A present compound (L-2) wherein $R^1$ represents a bromine atom, $R^2$ represents a methyl group, $R^4$, $R^5$, $R^6$ and $R^7$ represent a hydrogen atom, and $R^3$ represents any one substituent indicated in [Table L1] (hereinafter, referred to as "Compound class SX449").

A present compound (L-2) wherein $R^1$ represents a bromine atom, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX450").

A present compound (L-2) wherein $R^1$ represents a bromine atom, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX451").

A present compound (L-2) wherein $R^1$ represents a bromine atom, $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX452").

A present compound (L-2) wherein $R^1$ represents a bromine atom, $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX453").

A present compound (L-2) wherein $R^1$ represents a bromine atom, $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX454").

A present compound (L-2) wherein $R^1$ represents a methyl group, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX455").

A present compound (L-2) wherein $R^1$ represents a methyl group, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX456").

A present compound (L-2) wherein $R^1$ represents an ethyl group, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX457").

A present compound (L-2) wherein $R^1$ represents a fluorine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX458").

A present compound (L-2) wherein $R^1$ and $R^2$ represent a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX459").

A present compound (L-2) wherein $R^1$ represents a chlorine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX460").

A present compound (L-2) wherein $R^1$ and $R^2$ represent a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX461").

A present compound (L-2) wherein $R^1$ represents a bromine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX462").

A present compound (L-2) wherein $R^1$ and $R^2$ represent a bromine atom, $R^3$, $R^5$, $R^6$ and $R^7$ represent a hydrogen atom, and $R^4$ represents any one substituent indicated in [Table L1] to [Table L3] (hereinafter, referred to as "Compound class SX463").

A present compound (L-2) wherein $R^1$ represents an iodine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX464").

A present compound (L-2) wherein $R^1$ and $R^2$ represent an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX465").

A present compound (L-2) wherein $R^1$ represents a methyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX466").

A present compound (L-2) wherein $R^1$ and $R^2$ represent a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX467").

A present compound (L-2) wherein $R^1$ represents an ethyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX468").

A present compound (L-2) wherein $R^1$ and $R^2$ represent an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX469").

A present compound (L-2) wherein $R^1$ represents a propyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX470").

A present compound (L-2) wherein $R^1$ and $R^2$ represent a propyl group, $R^3$, $R^5$, $R^6$ and $R^7$ represent a hydrogen atom, and $R^4$ represents any one substituent indicated in [Table L1] (hereinafter, referred to as "Compound class SX471").

A present compound (L-2) wherein $R^1$ represents an isopropyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX472").

A present compound (L-2) wherein $R^1$ and $R^2$ represent an isopropyl group, $R^3$, $R^5$, $R^6$ and $R^7$ represent a hydrogen atom, and $R^4$ represents any one substituent indicated in [Table L1] to [Table L3] (hereinafter, referred to as

"Compound class SX473").

A present compound (L-2) wherein $R^1$ represents a cyclopropyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX474").

A present compound (L-2) wherein $R^1$ and $R^2$ represent a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX475").

A present compound (L-2) wherein $R^1$ represents $CF_3$, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX476").

A present compound (L-2) wherein $R^1$ and $R^2$ represent $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX477").

A present compound (L-2) wherein $R^1$ represents a chlorine atom, $R^2$ represents a bromine atom, $R^3$, $R^5$, $R^6$ and $R^7$ represent a hydrogen atom, and $R^4$ represents any one substituent indicated in [Table L1] to [Table L3] (hereinafter, referred to as "Compound class SX478").

A present compound (L-2) wherein $R^1$ represents a chlorine atom, $R^2$ represents an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX479").

A present compound (L-2) wherein $R^1$ represents a chlorine atom, $R^2$ represents a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX480").

A present compound (L-2) wherein $R^1$ represents a chlorine atom, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX481").

A present compound (L-2) wherein $R^1$ represents a chlorine atom, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX482").

A present compound (L-2) wherein $R^1$ represents a chlorine atom, $R^2$ represents an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX483").

A present compound (L-2) wherein $R^1$ represents a chlorine atom, $R^2$ represents a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX484").

A present compound (L-2) wherein $R^1$ represents a chlorine atom, $R^2$ represents $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX485").

A present compound (L-2) wherein $R^1$ represents a bromine atom, $R^2$ represents an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX486").

A present compound (L-2) wherein $R^1$ represents a bromine atom, $R^2$ represents a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX487").

A present compound (L-2) wherein $R^1$ represents a bromine atom, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX488").

A present compound (L-2) wherein $R^1$ represents a bromine atom, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX489").

A present compound (L-2) wherein $R^1$ represents a bromine atom, $R^2$ represents an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX490").

A present compound (L-2) wherein $R^1$ represents a bromine atom, $R^2$ represents a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX491").

A present compound (L-2) wherein $R^1$ represents a bromine atom, $R^2$ represents $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX492").

A present compound (L-2) wherein $R^1$ represents a methyl group, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX493").

A present compound (L-2) wherein $R^1$ represents a methyl group, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX494").

A present compound (L-2) wherein $R^1$ represents an ethyl group, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX495").

A present compound (L-2) wherein $R^1$ represents a fluorine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX496").

A present compound (L-2) wherein $R^1$ and $R^2$ represent a fluorine atom, $R^3$, $R^4$, $R^6$ and $R^7$ represent a hydrogen atom, and $R^5$ represents any one substituent indicated in [Table L1] to [Table L2] (hereinafter, referred to as "Compound class SX497").

A present compound (L-2) wherein $R^1$ represents a chlorine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX498").

A present compound (L-2) wherein $R^1$ and $R^2$ represent a chlorine atom, $R^3$, $R^4$, $R^6$ and $R^7$ represent a hydrogen atom, and $R^5$ represents any one substituent indicated in [Table L1] to [Table L2] (hereinafter, referred to as "Compound class SX499").

A present compound (L-2) wherein $R^1$ represents a bromine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX500").

A present compound (L-2) wherein $R^1$ and $R^2$ represent a bromine atom, $R^3$, $R^4$, $R^6$ and $R^7$ represent a hydrogen atom, and $R^5$ represents any one substituent indicated in [Table L1] to [Table L2] (hereinafter, referred to as "Compound class SX501").

A present compound (L-2) wherein $R^1$ represents an iodine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX502").

A present compound (L-2) wherein $R^1$ and $R^2$ represent an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX503").

A present compound (L-2) wherein $R^1$ represents a methyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX504").

A present compound (L-2) wherein $R^1$ and $R^2$ represent a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX505").

A present compound (L-2) wherein $R^1$ represents an ethyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX506").

A present compound (L-2) wherein $R^1$ and $R^2$ represent an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX507").

A present compound (L-2) wherein $R^1$ represents a propyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX508").

A present compound (L-2) wherein $R^1$ and $R^2$ represent a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX509").

A present compound (L-2) wherein $R^1$ represents an isopropyl group, $R^2$ represents a hydrogen atom, $R^3$, $R^4$, $R^5$ and $R^7$ represent a hydrogen atom, and $R^5$ represents any one substituent indicated in [Table L1] to [Table L2] (hereinafter, referred to as "Compound class SX510").

A present compound (L-2) wherein $R^1$ and $R^2$ represent an isopropyl group, $R^3$, $R^4$, $R^6$ and $R^7$ represent a hydrogen atom, and $R^3$ represents any one substituent indicated in [Table L1] to [Table L2] (hereinafter, referred to as "Compound class SX511").

A present compound (L-2) wherein $R^1$ represents a cyclopropyl group, $R^2$ represents a hydrogen atom, $R^3$,

$R^4$, $R^6$ and $R^7$ represent a hydrogen atom, and $R^5$ represents any one substituent indicated in [Table 11] to [Table L2] (hereinafter, referred to as "Compound class SX512").

A present compound (L-2) wherein $R^1$ and $R^2$ represent a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX513").

A present compound (L-2) wherein $R^1$ represents $CF_3$, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX514").

A present compound (L-2) wherein $R^1$ and $R^2$ represent $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX515").

A present compound (L-2) wherein $R^1$ represents a chlorine atom, $R^2$ represents a bromine atom, $R^3$, $R^4$, $R^6$ and $R^7$ represent a hydrogen atom, and $R^5$ represents any one substituent indicated in [Table L1] to [Table L2] (hereinafter, referred to as "Compound class SX516").

A present compound (L-2) wherein $R^3$ represents a chlorine atom, $R^2$ represents an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX517").

A present compound (L-2) wherein $R^1$ represents a chlorine atom, $R^2$ represents a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX518").

A present compound (L-2) wherein $R^1$ represents a chlorine atom, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX519").

A present compound (L-2) wherein $R^1$ represents a chlorine atom, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX520").

A present compound (L-2) wherein $R^1$ represents a chlorine atom, $R^2$ represents an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX521").

A present compound (L-2) wherein $R^1$ represents a chlorine atom, $R^2$ represents a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX522") .

A present compound (L-2) wherein $R^1$ represents a chlorine atom, $R^2$ represents $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX523").

A present compound (L-2) wherein $R^1$ represents a bromine atom, $R^2$ represents an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX524").

A present compound (L-2) wherein $R^1$ represents a bromine atom, $R^2$ represents a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX525").

A present compound (L-2) wherein $R^1$ represents a bromine atom, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX526").

A present compound (L-2) wherein $R^1$ represents a bromine atom, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX527").

A present compound (L-2) wherein $R^1$ represents a bromine atom, $R^2$ represents an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX528").

A present compound (L-2) wherein $R^1$ represents a bromine atom, $R^2$ represents a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX529").

A present compound (L-2) wherein $R^1$ represents a bromine atom, $R^2$ represents $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX530").

A present compound (L-2) wherein $R^1$ represents a methyl group, $R^2$ represents an ethyl group, $R^3$, $R^4$, $R^6$ and $R^7$ represent a hydrogen atom, and $R^5$ represents any one substituent indicated in [Table L1] to [Table L2] (hereinafter,

referred to as "Compound class SX531").

A present compound (L-2) wherein $R^1$ represents a methyl group, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX532").

A present compound (L-2) wherein $R^1$ represents an ethyl group, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX533").

A present compound (L-2) wherein $R^1$ represents a fluorine atom, $R^2$ represents a hydrogen atom, $R^3$ represents a fluorine atom, $R^4$, $R^6$ and $R^7$ represent a hydrogen atom, and $R^5$ represents any one substituent indicated in [Table L1] to [Table L2] (hereinafter, referred to as "Compound class SX534").

A present compound (L-2) wherein $R^1$ represents a fluorine atom, $R^2$ represents a hydrogen atom, $R^3$ represents a chlorine atom, $R^4$, $R^6$ and $R^7$ represent a hydrogen atom, and $R^5$ represents any one substituent indicated in [Table L1] to [Table L2] (hereinafter, referred to as "Compound class SX535").

A present compound (L-2) wherein $R^1$ represents a fluorine atom, $R^2$ represents a hydrogen atom, $R^3$ represents a bromine atom, $R^4$, $R^6$ and $R^7$ represent a hydrogen atom, and $R^5$ represents any one substituent indicated in [Table L1] to [Table L2] (hereinafter, referred to as "Compound class SX536").

A present compound (L-2) wherein $R^1$ represents a fluorine atom, $R^2$ represents a hydrogen atom, $R^3$ represents an iodine atom, $R^4$, $R^6$ and $R^7$ represent a hydrogen atom, and $R^5$ represents any one substituent indicated in [Table L1] to [Table L2] (hereinafter, referred to as "Compound class SX537").

A present compound (L-2) wherein $R^1$ represents a fluorine atom, $R^2$ represents a hydrogen atom, $R^3$ represents a methyl group, $R^4$, $R^6$ and $R^7$ represent a hydrogen atom, and $R^5$ represents any one substituent indicated in [Table L1] to [Table L2] (hereinafter, referred to as "Compound class SX538").

A present compound (L-2) wherein $R^1$ represents a fluorine atom, $R^2$ represents a hydrogen atom, $R^3$ represents a methoxy group, $R^4$, $R^6$ and $R^7$ represent a hydrogen atom, and $R^5$ represents any one substituent indicated in [Table L1] to [Table L2] (hereinafter, referred to as "Compound class SX539").

A present compound (L-2) wherein $R^1$ represents a fluorine atom, $R^2$ represents a hydrogen atom, $R^3$ represents $CF_3$, $R^4$, $R^6$ and $R^7$ represent a hydrogen atom, and $R^5$ represents any one substituent indicated in [Table L1] to [Table L2] (hereinafter, referred to as "Compound class SX540").

A present compound (L-2) wherein $R^1$ represents a fluorine atom, $R^2$ represents a hydrogen atom, $R^3$ represents $OCF_3$, $R^4$, $R^6$ and $R^7$ represent a hydrogen atom, and $R^5$ represents any one substituent indicated in [Table L1] to [Table L2] (hereinafter, referred to as "Compound class SX541").

A present compound (L-2) wherein $R^1$ represents a fluorine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX542").

A present compound (L-2) wherein $R^1$ represents a fluorine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX543").

A present compound (L-2) wherein $R^1$ represents a fluorine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX544").

A present compound (L-2) wherein $R^1$ represents a fluorine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX545").

A present compound (L-2) wherein $R^1$ represents a fluorine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX546").

A present compound (L-2) wherein $R^1$ represents a fluorine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX547").

A present compound (L-2) wherein $R^1$ represents a fluorine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX548").

A present compound (L-2) wherein $R^1$ represents a fluorine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX549").

A present compound (L-2) wherein $R^1$ represents a fluorine atom, $R^2$ represents a hydrogen atom, $R^4$ represents a fluorine atom, $R^3$, $R^5$ and $R^7$ represent a hydrogen atom, and $R^6$ represents any one substituent indicated in [Table L1] to [Table L3] (hereinafter, referred to as "Compound class SX550") .

A present compound (L-2) wherein $R^1$ represents a fluorine atom, $R^2$ represents a hydrogen atom, $R^4$ represents a chlorine atom, $R^3$, $R^5$ and $R^7$ represent a hydrogen atom, and $R^6$ represents any one substituent indicated in [Table L1] to [Table L3] (hereinafter, referred to as "Compound class SX551") .

A present compound (L-2) wherein $R^1$ represents a fluorine atom, $R^2$ represents a hydrogen atom, $R^4$ represents a bromine atom, $R^3$, $R^5$ and $R^7$ represent a hydrogen atom, and $R^6$ represents any one substituent indicated in [Table L1] to [Table L3] (hereinafter, referred to as "Compound class SX552").

A present compound (L-2) wherein $R^1$ represents a fluorine atom, $R^2$ represents a hydrogen atom, $R^4$ represents an iodine atom, $R^3$, $R^5$ and $R^7$ represent a hydrogen atom, and $R^6$ represents any one substituent indicated in [Table L1] to [Table L3] (hereinafter, referred to as "Compound class SX553").

A present compound (L-2) wherein $R^1$ represents a fluorine atom, $R^2$ represents a hydrogen atom, $R^4$ represents a methyl group, $R^3$, $R^5$ and $R^7$ represent a hydrogen atom, and $R^6$ represents any one substituent indicated in [Table L1] to [Table L3] (hereinafter, referred to as "Compound class SX554").

A present compound (L-2) wherein $R^1$ represents a fluorine atom, $R^2$ represents a hydrogen atom, $R^4$ represents a methoxy group, $R^3$, $R^5$ and $R^7$ represent a hydrogen atom, and $R^6$ represents any one substituent indicated in [Table L1] to [Table L3] (hereinafter, referred to as "Compound class SX555").

A present compound (L-2) wherein $R^1$ represents a fluorine atom, $R^2$ represents a hydrogen atom, $R^4$ represents $CF_3$, $R^3$, $R^5$ and $R^7$ represent a hydrogen atom, and $R^6$ represents any one substituent indicated in [Table L1] to [Table L3] (hereinafter, referred to as "Compound class SX556").

A present compound (L-2) wherein $R^1$ represents a fluorine atom, $R^2$ represents a hydrogen atom, $R^4$ represents $OCF_3$, $R^3$, $R^5$ and $R^7$ represent a hydrogen atom, and $R^6$ represents any one substituent indicated in [Table L1] to [Table L3] (hereinafter, referred to as "Compound class SX557").

A present compound (L-2) wherein $R^1$ represents a chlorine atom, $R^2$ represents a hydrogen atom, $R^5$ represents a fluorine atom, $R^3$, $R^6$ and $R^7$ represent a hydrogen atom, and $R^4$ represents any one substituent indicated in [Table 11] to [Table L3] (hereinafter, referred to as "Compound class SX558").

A present compound (L-2) wherein $R^1$ represents a chlorine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX559").

A present compound (L-2) wherein $R^1$ represents a chlorine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX560") .

A present compound (L-2) wherein $R^1$ represents a chlorine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX561") .

A present compound (L-2) wherein $R^1$ represents a chlorine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX562").

A present compound (L-2) wherein $R^1$ represents a chlorine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX563").

A present compound (L-2) wherein $R^1$ represents a chlorine atom, $R^2$ represents a hydrogen atom, $R^5$ represents $CF_3$, $R^3$, $R^6$ and $R^7$ represent a hydrogen atom, and $R^4$ represents any one substituent indicated in [Table 11] to [Table L3] (hereinafter, referred to as "Compound class SX564").

A present compound (L-2) wherein $R^1$ represents a chlorine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX565").

A present compound (L-2) wherein $R^1$ represents a chlorine atom, $R^2$ represents a hydrogen atom, $R^4$ represents a fluorine atom, $R^3$, $R^6$ and $R^7$ represent a hydrogen atom, and $R^6$ represents any one substituent indicated in [Table L1] to [Table L3] (hereinafter, referred to as "Compound class SX566").

A present compound (L-2) wherein $R^1$ represents a chlorine atom, $R^2$ represents a hydrogen atom, $R^4$ represents a chlorine atom, $R^3$, $R^5$ and $R^7$ represent a hydrogen atom, and $R^6$ represents any one substituent indicated in [Table L1] to [Table L3] (hereinafter, referred to as "Compound class SX567").

A present compound (L-2) wherein $R^1$ represents a chlorine atom, $R^2$ represents a hydrogen atom, $R^4$ represents a bromine atom, $R^3$, $R^5$ and $R^7$ represent a hydrogen atom, and $R^6$ represents any one substituent indicated in [Table L1] to [Table L3] (hereinafter, referred to as "Compound class SX568").

A present compound (L-2) wherein $R^1$ represents a chlorine atom, $R^2$ represents a hydrogen atom, $R^4$ represents an iodine atom, $R^3$, $R^5$ and $R^7$ represent a hydrogen atom, and $R^6$ represents any one substituent indicated in [Table L1] to [Table L3] (hereinafter, referred to as "Compound class SX569").

A present compound (L-2) wherein $R^1$ represents a chlorine atom, $R^2$ represents a hydrogen atom, $R^4$ represents

a methyl group, $R^3$, $R^5$ and $R^7$ represent a hydrogen atom, and $R^6$ represents any one substituent indicated in [Table L1] to [Table L3] (hereinafter, referred to as "Compound class SX570").

A present compound (L-2) wherein $R^1$ represents a chlorine atom, $R^2$ represents a hydrogen atom, $R^4$ represents a methoxy group, $R^3$, $R^5$ and $R^7$ represent a hydrogen atom, and $R^6$ represents any one substituent indicated in [Table L1] to [Table L3] (hereinafter, referred to as "Compound class SX571").

A present compound (L-2) wherein $R^1$ represents a chlorine atom, $R^2$ represents a hydrogen atom, $R^4$ represents $CF_3$, $R^3$, $R^5$ and $R^7$ represent a hydrogen atom, and $R^6$ represents any one substituent indicated in [Table L1] to [Table L3] (hereinafter, referred to as "Compound class SX572").

A present compound (L-2) wherein $R^1$ represents a chlorine atom, $R^2$ represents a hydrogen atom, $R^4$ represents $OCF_3$, $R^3$, $R^5$ and $R^7$ represent a hydrogen atom, and $R^6$ represents any one substituent indicated in [Table L1] to [Table L3] (hereinafter, referred to as "Compound class SX573") .

A present compound (L-2) wherein $R^1$ represents a bromine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX574") .

A present compound (L-2) wherein $R^1$ represents a bromine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX575") .

A present compound (L-2) wherein $R^1$ represents a bromine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX576") .

A present compound (L-2) wherein $R^1$ represents a bromine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX577") .

A present compound (L-2) wherein $R^1$ represents a bromine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX578").

A present compound (L-2) wherein $R^1$ represents a bromine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX579").

A present compound (L-2) wherein $R^1$ represents a bromine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX580").

A present compound (L-2) wherein $R^4$ represents a bromine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX581").

A present compound (L-2) wherein $R^1$ represents a bromine atom, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX582").

A present compound (L-2) wherein $R^1$ represents a bromine atom, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX583") .

A present compound (L-2) wherein $R^1$ represents a bromine atom, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX584") .

A present compound (L-2) wherein $R^1$ represents a bromine atom, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX585") .

A present compound (L-2) wherein $R^1$ represents a bromine atom, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX586") .

A present compound (L-2) wherein $R^1$ represents a bromine atom, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX587") .

A present compound (L-2) wherein $R^1$ represents a bromine atom, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX588").

A present compound (L-2) wherein $R^1$ represents a bromine atom, $R^2$ represents a hydrogen atom, $R^5$ represents $OCF_3$, $R^3$, $R^5$ and $R^7$ represent a hydrogen atom, and $R^4$ represents any one substituent indicated in [Table L1] to [Table

L3] (hereinafter, referred to as "Compound class SX589").

A present compound (L-2) wherein $R^1$ represents an iodine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX590") .

A present compound (L-2) wherein $R^1$ represents an iodine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX591").

A present compound (L-2) wherein $R^1$ represents an iodine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX592") .

A present compound (L-2) wherein $R^1$ represents an iodine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX593") .

A present compound (L-2) wherein $R^1$ represents an iodine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX594") .

A present compound (L-2) wherein $R^1$ represents an iodine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX595") .

A present compound (L-2) wherein $R^1$ represents an iodine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX596").

A present compound (L-2) wherein $R^1$ represents an iodine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX597").

A present compound (L-2) wherein $R^1$ represents an iodine atom, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX598") .

A present compound (L-2) wherein $R^1$ represents an iodine atom, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX599") .

A present compound (L-2) wherein $R^1$ represents an iodine atom, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX600").

A present compound (L-2) wherein $R^1$ represents an iodine atom, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX601") .

A present compound (L-2) wherein $R^1$ represents an iodine atom, $R^2$ represents a hydrogen atom, $R^6$ represents a methyl group, $R^3$, $R^5$ and $R^7$ represent a hydrogen atom, and $R^4$ represents any one substituent indicated in [Table 11] to [Table L3] (hereinafter, referred to as "Compound class SX602").

A present compound (L-2) wherein $R^1$ represents an iodine atom, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX603") .

A present compound (L-2) wherein $R^1$ represents an iodine atom, $R^2$ represents a hydrogen atom, $R^6$ represents $CF_3$, $R^3$, $R^5$ and $R^7$ represent a hydrogen atom, and $R^4$ represents any one substituent indicated in [Table 11] to [Table L3] (hereinafter, referred to as "Compound class SX604").

A present compound (L-2) wherein $R^1$ represents an iodine atom, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX605").

A present compound (L-2) wherein $R^1$ represents a methyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX606").

A present compound (L-2) wherein $R^1$ represents a methyl group, $R^2$ represents a hydrogen atom, $R^5$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX607").

A present compound (L-2) wherein $R^1$ represents a methyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX608").

A present compound (L-2) wherein $R^1$ represents a methyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX609") .

A present compound (L-2) wherein $R^1$ represents a methyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX610") .

A present compound (L-2) wherein $R^1$ represents a methyl group, $R^2$ represents a hydrogen atom, $R^5$ represents a methoxy group, $R^3$, $R^6$ and $R^7$ represent a hydrogen atom, and $R^4$ represents any one substituent indicated in [Table 11] to [Table L3] (hereinafter, referred to as "Compound class SX611") .

A present compound (L-2) wherein $R^1$ represents a methyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX612").

A present compound (L-2) wherein $R^1$ represents a methyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX513").

A present compound (L-2) wherein $R^1$ represents a methyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX614") .

A present compound (L-2) wherein $R^1$ represents a methyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1) to [Table L3] (hereinafter, referred to as "Compound class SX615") .

A present compound (L-2) wherein $R^1$ represents a methyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX616") .

A present compound (L-2) wherein $R^1$ represents a methyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX617").

A present compound (L-2) wherein $R^1$ represents a methyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX618") .

A present compound (L-2) wherein $R^1$ represents a methyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX619") .

A present compound (L-2) wherein $R^1$ represents a methyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX620").

A present compound (L-2) wherein $R^1$ represents a methyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX621").

A present compound (L-2) wherein $R^1$ represents an ethyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX622").

A present compound (L-2) wherein $R^1$ represents an ethyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX623") .

A present compound (L-2) wherein $R^1$ represents an ethyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX624") .

A present compound (L-2) wherein $R^1$ represents an ethyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX625") .

A present compound (L-2) wherein $R^1$ represents an ethyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX626[11]) .

A present compound (L-2) wherein $R^1$ represents an ethyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX627") .

A present compound (L-2) wherein $R^1$ represents an ethyl group, $R^2$ represents a hydrogen atom, $R^5$ represents

$CF_3$, $R^3$, $R^6$ and $R^7$ represent a hydrogen atom, and $R^4$ represents any one substituent indicated in [Table L1] to [Table L3] (hereinafter, referred to as "Compound class SX628").

A present compound (L-2) wherein $R^1$ represents an ethyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX629").

A present compound (L-2) wherein $R^1$ represents an ethyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX630") .

A present compound (L-2) wherein $R^1$ represents an ethyl group, $R^2$ represents a hydrogen atom, $R^6$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX631") .

A present compound (L-2) wherein $R^1$ represents an ethyl group, $R^2$ represents a hydrogen atom, $R^6$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX632") .

A present compound (L-2) wherein $R^1$ represents an ethyl group, $R^2$ represents a hydrogen atom, $R^6$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX633") .

A present compound (L-2) wherein $R^1$ represents an ethyl group, $R^2$ represents a hydrogen atom, $R^6$ represents a methyl group, $R^3$, $R^6$ and $R^7$ represent a hydrogen atom, and $R^4$ represents any one substituent indicated in [Table L1] to [Table L3] (hereinafter, referred to as "Compound class SX634") .

A present compound (L-2) wherein $R^1$ represents an ethyl group, $R^2$ represents a hydrogen atom, $R^6$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX635").

A present compound (L-2) wherein $R^1$ represents an ethyl group, $R^2$ represents a hydrogen atom, $R^6$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX636").

A present compound (L-2) wherein $R^1$ represents an ethyl group, $R^2$ represents a hydrogen atom, $R^6$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX637").

A present compound (L-2) wherein $R^1$ represents $CF_3$, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX638").

A present compound (L-2) wherein $R^1$ represents $CF_3$, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX639").

A present compound (L-2) wherein $R^1$ represents $CF_3$, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX640").

A present compound (L-2) wherein $R^1$ represents $CF_3$, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX641").

A present compound (L-2) wherein $R^1$ represents $CF_3$, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX642").

A present compound (L-2) wherein $R^1$ represents $CF_3$, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX643").

A present compound (L-2) wherein $R^1$ "represents $CF_3$, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX644").

A present compound (L-2) wherein $R^1$ represents $CF_3$, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX645").

A present compound (L-2) wherein $R^1$ represents $CF_3$, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX646").

A present compound (L-2) wherein $R^1$ represents $CF_3$, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table

L3] (hereinafter, referred to as "Compound class SX647").

A present compound (L-2) wherein $R^1$ represents $CF_3$, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX648").

A present compound (L-2) wherein $R^1$ represents $CF_3$, $R^2$ represents a hydrogen atom, $R^6$ represents an iodine atom, $R^3$, $R^5$ and R' represent a hydrogen atom, and $R^4$ represents any one substituent indicated in [Table L1] to [Table L3] (hereinafter, referred to as "Compound class SX649").

A present compound (L-2) wherein $R^1$ represents $CF_3$, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX650").

A present compound (L-2) wherein $R^1$ represents $CF_3$, $R^2$ represents a hydrogen atom, $R^6$ represents a methoxy group, $R^3$, $R^5$ and $R^7$ represent a hydrogen atom, and $R^4$ represents any one substituent indicated in [Table Z1] to [Table L3] (hereinafter, referred to as "Compound class SX651").

A present compound (L-2) wherein $R^1$ represents $CF_3$, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX652").

A present compound (L-2) wherein $R^1$ represents $CF_3$, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX653").

A present compound (L-2) wherein $R^1$ represents a cyclopropyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX654").

A present compound (L-2) wherein $R^1$ represents a cyclopropyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX655").

A present compound (L-2) wherein $R^1$ represents a cyclopropyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX656").

A present compound (L-2) wherein $R^1$ represents a cyclopropyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX657").

A present compound (L-2) wherein $R^1$ represents a cyclopropyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX658").

A present compound (L-2) wherein $R^1$ represents a cyclopropyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX659").

A present compound (L-2) wherein $R^1$ represents a cyclopropyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX660") .

A present compound (L-2) wherein $R^1$ represents a cyclopropyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX661").

A present compound (L-2) wherein $R^1$ represents a cyclopropyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX662").

A present compound (L-2) wherein $R^1$ represents a cyclopropyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX663").

A present compound (L-2) wherein $R^1$ represents a cyclopropyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX564").

A present compound (L-2) wherein $R^1$ represents a cyclopropyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX665").

A present compound (L-2) wherein $R^1$ represents a cyclopropyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX666").

A present compound (L-2) wherein $R^1$ represents a cyclopropyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX667").

A present compound (L-2) wherein $R^1$ represents a cyclopropyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX668") .

A present compound (L-2) wherein $R^1$ represents a cyclopropyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX669") .

A present compound (L-2) wherein $R^1$ represents a methylsulfanyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX670").

A present compound (L-2) wherein $R^1$ represents a methylsulfanyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX671").

A present compound (L-2) wherein $R^1$ represents a methylsulfanyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX672").

A present compound (L-2) wherein $R^1$ represents a methylsulfanyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX673").

A present compound (L-2) wherein $R^1$ represents a methylsulfanyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX674").

A present compound (L-2) wherein $R^1$ represents a methylsulfanyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX675").

A present compound (L-2) wherein $R^1$ represents a methylsulfanyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX676") .

A present compound (L-2) wherein $R^1$ represents a methylsulfanyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX677").

A present compound (L-2) wherein $R^1$ represents a methylsulfanyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX678").

A present compound (L-2) wherein $R^1$ represents a methylsulfanyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX679").

A present compound (L-2) wherein $R^1$ represents a methylsulfanyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX680").

A present compound (L-2) wherein $R^1$ represents a methylsulfanyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX681").

A present compound (L-2) wherein $R^1$ represents a methylsulfanyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX682").

A present compound (L-2) wherein $R^1$ represents a methylsulfanyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX683").

A present compound (L-2) wherein $R^1$ represents a methylsulfanyl group, $R^2$ represents a hydrogen atom, $R^6$ represents $CF_3$, $R^3$, $R^5$ and $R^7$ represent a hydrogen atom, and $R^4$ represents any one substituent indicated in [Table 11] to [Table L3] (hereinafter, referred to as "Compound class SX684") .

A present compound (L-2) wherein $R^1$ represents a methylsulfanyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX685") .

A present compound (L-2) wherein $R^1$ represents a methylsulfanyl group, $R^2$ represents a hydrogen atom, $R^7$

represents C-F, $R^3$, $R^5$ and $R^6$ represent a hydrogen atom, and $R^4$ represents any one substituent indicated in [Table L1] to [Table L3] (hereinafter, referred to as "Compound class SX686").

A present compound (L-2) wherein $R^1$ represents a methylsulfanyl group, $R^2$ represents a hydrogen atom, $R^7$ represents C-Cl, $R^3$, $R^5$ and $R^6$ represent a hydrogen atom, and $R^4$ represents any one substituent indicated in [Table L1] to [Table L3] (hereinafter, referred to as "Compound class SX687").

A present compound (L-2) wherein $R^1$ represents a methylsulfanyl group, $R^2$ represents a hydrogen atom, $R^7$ represents C-Br, $R^3$, $R^5$ and $R^6$ represent a hydrogen atom, and $R^4$ represents any one substituent indicated in [Table L1] to [Table L3] (hereinafter, referred to as "Compound class SX688").

A present compound (L-2) wherein $R^1$ represents a methylsulfanyl group, $R^2$ represents a hydrogen atom, $R^7$ represents C-I, $R^3$, $R^5$ and $R^6$ represent a hydrogen atom, and $R^4$ represents any one substituent indicated in [Table L1] to [Table L3] (hereinafter, referred to as "Compound class SX689").

A present compound (L-2) wherein $R^1$ represents a methylsulfanyl group, $R^2$ represents a hydrogen atom, $R^7$ represents C-Me, $R^3$, $R^5$ and $R^6$ represent a hydrogen atom, and $R^4$ represents any one substituent indicated in [Table L1] to [Table L3] (hereinafter, referred to as "Compound class SX690").

A present compound (L-2) wherein $R^1$ represents a methylsulfanyl group, $R^2$ represents a hydrogen atom, $R^7$ represents C-OMe, $R^3$, $R^5$ and $R^6$ represent a hydrogen atom, and $R^4$ represents any one substituent indicated in [Table L1] to [Table L3] (hereinafter, referred to as "Compound class SX691").

A present compound (L-2) wherein $R^1$ represents a methylsulfanyl group, $R^2$ represents a hydrogen atom, $R^7$ represents C-$CF_3$, $R^3$, $R^5$ and $R^6$ represent a hydrogen atom, and $R^4$ represents any one substituent indicated in [Table L1] to [Table L3] (hereinafter, referred to as "Compound class SX692").

A present compound (L-2) wherein $R^1$ represents a methylsulfanyl group, $R^2$ represents a hydrogen atom, $R^7$ represents C-$OCF_3$, $R^3$, $R^5$ and $R^6$ represent a hydrogen atom, and $R^4$ represents any one substituent indicated in [Table L1] to [Table L3] (hereinafter, referred to as "Compound class SX693").

A present compound (L-2) wherein $R^1$ represents a methylsulfanyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX694").

A present compound (L-2) wherein $R^1$ represents a methylsulfanyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX695").

A present compound (L-2) wherein $R^1$ represents a methylsulfanyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX696").

A present compound (L-2) wherein $R^1$ represents a methylsulfanyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX697").

A present compound (L-2) wherein $R^1$ represents a methylsulfanyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX698").

A present compound (L-2) wherein $R^1$ represents a methylsulfanyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX699").

A present compound (L-2) wherein $R^1$ represents a methylsulfanyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX700").

A present compound (L-2) wherein $R^1$ represents a methylsulfanyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX701").

A present compound (L-2) wherein $R^1$ represents a methylsulfanyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX702").

A present compound (L-2) wherein $R^1$ represents a methylsulfanyl group, $R^2$ represents a hydrogen atom, $R^6$ represents a chlorine atom, $R^3$, $R^5$ and $R^7$ represent a hydrogen atom, and $R^4$ represents any one substituent indicated in [Table Z1] to [Table L3] (hereinafter, referred to as "Compound class SX703").

A present compound (L-2) wherein $R^1$ represents a methylsulfanyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX704").

A present compound (L-2) wherein $R^1$ represents a methylsulfanyl group, $R^2$ represents a hydrogen atom, $R^6$ represents an iodine atom, $R^3$, $R^5$ and $R^7$ represent a hydrogen atom, and $R^4$ represents any one substituent indicated

in [Table L1] to [Table L3] (hereinafter, referred to as "Compound class SX705").

A present compound (L-2) wherein $R^1$ represents a methylsulfanyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX706").

A present compound (L-2) wherein $R^1$ represents a methylsulfanyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX707").

A present compound (L-2) wherein $R^1$ represents a methylsulfanyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX708").

A present compound (L-2) wherein $R^1$ represents a methylsulfanyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX709").

A present compound (L-2) wherein $R^1$ and $R^2$ represents a fluorine atom, $R^5$ represents a fluorine atom, $R^3$, $R^6$ and $R^7$ represent a hydrogen atom, and $R^4$ represents any one substituent indicated in [Table Z1] to [Table L3] (hereinafter, referred to as "Compound class SX710").

A present compound (L-2) wherein $R^1$ and $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX711").

A present compound (L-2) wherein $R^1$ and $R^2$ represents a fluorine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX712").

A present compound (L-2) wherein $R^1$ and $R^2$ represents a fluorine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX713").

A present compound (L-2) wherein $R^1$ and $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX714").

A present compound (L-2) wherein $R^1$ and $R^2$ represents a fluorine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX715").

A present compound (L-2) wherein $R^1$ and $R^2$ represents a fluorine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX716").

A present compound (L-2) wherein $R^1$ and $R^2$ represents a fluorine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX717").

A present compound (L-2) wherein $R^1$ and $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX718").

A present compound (L-2) wherein $R^1$ and $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX719").

A present compound (L-2) wherein $R^1$ and $R^2$ represents a fluorine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX720").

A present compound (L-2) wherein $R^1$ and $R^2$ represents a fluorine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX721").

A present compound (L-2) wherein $R^1$ and $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX722").

A present compound (L-2) wherein $R^1$ and $R^2$ represents a fluorine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX723").

A present compound (L-2) wherein $R^1$ and $R^2$ represents a fluorine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX724").

A present compound (L-2) wherein $R^1$ and $R^2$ represents a fluorine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX725").

A present compound (L-2) wherein $R^1$ and $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX726").

A present compound (L-2) wherein $R^1$ and $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX727").

A present compound (L-2) wherein $R^1$ and $R^2$ represents a chlorine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX728").

A present compound (L-2) wherein $R^1$ and $R^2$ represents a chlorine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX729").

A present compound (L-2) wherein $R^1$ and $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX730").

A present compound (L-2) wherein $R^1$ and $R^2$ represents a chlorine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX731").

A present compound (L-2) wherein $R^1$ and $R^2$ represents a chlorine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX732").

A present compound (L-2) wherein $R^1$ and $R^2$ represents a chlorine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX733").

A present compound (L-2) wherein $R^1$ and $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX734").

A present compound (L-2) wherein $R^1$ and $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX735").

A present compound (L-2) wherein $R^1$ and $R^2$ represents a chlorine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX736").

A present compound (L-2) wherein $R^1$ and $R^2$ represents a chlorine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX737").

A present compound (L-2) wherein $R^1$ and $R^2$ represents a chlorine atom, $R^6$ represents a methyl group, $R^3$, $R^5$ and $R^7$ represent a hydrogen atom, and $R^4$ represents any one substituent indicated in [Table Z1] to [Table L3] (hereinafter, referred to as "Compound class SX738").

A present compound (L-2) wherein $R^1$ and $R^2$ represents a chlorine atom, $R^6$ represents a methoxy group, $R^3$, $R^5$ and $R^7$ represent a hydrogen atom, and $R^4$ represents any one substituent indicated in [Table Z1] to [Table L3] (hereinafter, referred to as "Compound class SX739").

A present compound (L-2) wherein $R^1$ and $R^2$ represents a chlorine atom, $R^6$ represents $CF_3$, $R^3$, $R^5$ and $R^7$ represent a hydrogen atom, and $R^4$ represents any one substituent indicated in [Table L1J to [Table L3] (hereinafter, referred to as "Compound class SX740").

A present compound (L-2) wherein $R^1$ and $R^2$ represents a chlorine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX741").

A present compound (L-2) wherein $R^1$ and $R^2$ represents a bromine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX742").

A present compound (L-2) wherein $R^1$ and $R^2$ represents a bromine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX743").

A present compound (L-2) wherein $R^1$ and $R^2$ represents a bromine atom, $R^5$ represents a bromine atom, $R^3$,

$R^6$ and $R^7$ represent a hydrogen atom, and $R^4$ represents any one substituent indicated in [Table L1] to [Table L3] (hereinafter, referred to as "Compound class SX744").

A present compound (L-2) wherein $R^1$ and $R^2$ represents a bromine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX745").

A present compound (L-2) wherein $R^1$ and $R^2$ represents a bromine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX746").

A present compound (L-2) wherein $R^1$ and $R^2$ represents a bromine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX747").

A present compound (L-2) wherein $R^1$ and $R^2$ represents a bromine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX748").

A present compound (L-2) wherein $R^1$ and $R^2$ represents a bromine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX749").

A present compound (L-2) wherein $R^1$ and $R^2$ represents a bromine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX750").

A present compound (L-2) wherein $R^1$ and $R^2$ represents a bromine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX751").

A present compound (L-2) wherein $R^1$ and $R^2$ represents a bromine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX752").

A present compound (L-2) wherein $R^1$ and $R^2$ represents a bromine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX753").

A present compound (L-2) wherein $R^1$ and $R^2$ represents a bromine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX754").

A present compound (L-2) wherein $R^1$ and $R^2$ represents a bromine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX755").

A present compound (L-2) wherein $R^1$ and $R^2$ represents a bromine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX756").

A present compound (L-2) wherein $R^1$ and $R^2$ represents a bromine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX757").

A present compound (L-2) wherein $R^1$ and $R^2$ represents an iodine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX758").

A present compound (L-2) wherein $R^1$ and $R^2$ represents an iodine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX759").

A present compound (L-2) wherein $R^1$ and $R^2$ represents an iodine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX760").

A present compound (L-2) wherein $R^1$ and $R^2$ represents an iodine atom, $R^5$ represents an iodine atom, $R^3$, $R^6$ and $R^7$ represent a hydrogen atom, and $R^4$ represents any one substituent indicated in [Table Z1] to [Table L3] (hereinafter, referred to as "Compound class SX761").

A present compound (L-2) wherein $R^1$ and $R^2$ represents an iodine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX762").

A present compound (L-2) wherein $R^1$ and $R^2$ represents an iodine atom, $R^5$ represents a methoxy group, $R^3$, $R^6$ and $R^7$ represent a hydrogen atom, and $R^4$ represents any one substituent indicated in [Table L1] to [Table L3]

(hereinafter, referred to as "Compound class SX763").

A present compound (L-2) wherein $R^1$ and $R^2$ represents an iodine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX764").

A present compound (L-2) wherein $R^1$ and $R^2$ represents an iodine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX765").

A present compound (L-2) wherein $R^1$ and $R^2$ represents an iodine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX766").

A present compound (L-2) wherein $R^1$ and $R^2$ represents an iodine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX767").

A present compound (L-2) wherein $R^1$ and $R^2$ represents an iodine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX768").

A present compound (L-2) wherein $R^1$ and $R^2$ represents an iodine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX769").

A present compound (L-2) wherein $R^1$ and $R^2$ represents an iodine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX770").

A present compound (L-2) wherein $R^1$ and $R^2$ represents an iodine atom, $R^6$ represents a methoxy group, $R^3$, $R^5$ and $R^7$ represent a hydrogen atom, and $R^4$ represents any one substituent indicated in [Table Z1] to [Table L3] (hereinafter, referred to as "Compound class SX771").

A present compound (L-2) wherein $R^1$ and $R^2$ represents an iodine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX772").

A present compound (L-2) wherein $R^1$ and $R^2$ represents an iodine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX773").

A present compound (L-2) wherein $R^1$ and $R^2$ represents a methyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX774").

A present compound (L-2) wherein $R^1$ and $R^2$ represents a methyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX775").

A present compound (L-2) wherein $R^1$ and $R^2$ represents a methyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX776").

A present compound (L-2) wherein $R^1$ and $R^2$ represents a methyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX777").

A present compound (L-2) wherein $R^1$ and $R^2$ represents a methyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX778").

A present compound (L-2) wherein $R^1$ and $R^2$ represents a methyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX779").

A present compound (L-2) wherein $R^1$ and $R^2$ represents a methyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX780").

A present compound (L-2) wherein $R^1$ and $R^2$ represents a methyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX781").

A present compound (L-2) wherein $R^1$ and $R^2$ represents a methyl group, $R^6$ represents a fluorine atom, $R^3$, $R^5$ and $R^7$ represent a hydrogen atom, and $R^4$ represents any one substituent indicated in [Table 11] to [Table L3] (hereinafter, referred to as "Compound class SX782$^{11}$).

A present compound (L-2) wherein $R^1$ and $R^2$ represents a methyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX783").

A present compound (L-2) wherein $R^1$ and $R^2$ represents a methyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX784").

A present compound (L-2) wherein $R^1$ and $R^2$ represents a methyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX785").

A present compound (L-2) wherein $R^1$ and $R^2$ represents a methyl group, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX786").

A present compound (L-2) wherein $R^1$ and $R^2$ represents a methyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX787").

A present compound (L-2) wherein $R^1$ and $R^2$ represents a methyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX788").

A present compound (L-2) wherein $R^1$ and $R^2$ represents a methyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX789").

A present compound (L-2) wherein $R^1$ and $R^2$ represents an ethyl group, $R^5$ represents a fluorine atom, $R^2$, $R^6$ and $R^7$ represent a hydrogen atom, and $R^4$ represents any one substituent indicated in [Table L1] to [Table L3] (hereinafter, referred to as "Compound class SX790").

A present compound (L-2) wherein $R^1$ and $R^2$ represents an ethyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX791").

A present compound (L-2) wherein $R^1$ and $R^2$ represents an ethyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX792").

A present compound (L-2) wherein $R^1$ and $R^2$ represents an ethyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX793").

A present compound (L-2) wherein $R^1$ and $R^2$ represents an ethyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX794").

A present compound (L-2) wherein $R^1$ and $R^2$ represents an ethyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX795").

A present compound (L-2) wherein $R^1$ and $R^2$ represents an ethyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX796").

A present compound (L-2) wherein $R^1$ and $R^2$ represents an ethyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX797").

A present compound (L-2) wherein $R^1$ and $R^2$ represents an ethyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX798").

A present compound (L-2) wherein $R^1$ and $R^2$ represents an ethyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX799").

A present compound (L-2) wherein $R^1$ and $R^2$ represents an ethyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX800").

A present compound (L-2) wherein $R^1$ and $R^2$ represents an ethyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX801").

A present compound (L-2) wherein $R^1$ and $R^2$ represents an ethyl group, $R^6$ represents a methyl group, $R^3$,

$R^5$ and $R^7$ represent a hydrogen atom, and $R^4$ represents any one substituent indicated in [Table L1] to [Table L3] (hereinafter, referred to as "Compound class SX802").

A present compound (L-2) wherein $R^1$ and $R^2$ represents an ethyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX803").

A present compound (L-2) wherein $R^1$ and $R^2$ represents an ethyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX804").

A present compound (L-2) wherein $R^1$ and $R^2$ represents an ethyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX805").

A present compound (L-2) wherein $R^1$ and $R^2$ represents $CF_3$, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX806").

A present compound (L-2) wherein $R^1$ and $R^2$ represents $CF_3$, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX807").

A present compound (L-2) wherein $R^1$ and $R^2$ represents $CF_3$, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX808").

A present compound (L-2) wherein $R^1$ and $R^2$ represents $CF_3$, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX809").

A present compound (L-2) wherein $R^1$ and $R^2$ represents $CF_3$, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX810").

A present compound (L-2) wherein $R^1$ and $R^2$ represents $CF_3$, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX811").

A present compound (L-2) wherein $R^1$ and $R^2$ represents $CF_3$, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX812").

A present compound (L-2) wherein $R^1$ and $R^2$ represents $CF_3$, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX813").

A present compound (L-2) wherein $R^1$ and $R^2$ represents $CF_3$, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX814").

A present compound (L-2) wherein $R^1$ and $R^2$ represents $CF_3$, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX815").

A present compound (L-2) wherein $R^1$ and $R^2$ represents $CF_3$, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX816").

A present compound (L-2) wherein $R^1$ and $R^2$ represents $CF_3$, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX817").

A present compound (L-2) wherein $R^1$ and $R^2$ represents $CF_3$, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX818").

A present compound (L-2) wherein $R^1$ and $R^2$ represents $CF_3$, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX819").

A present compound (L-2) wherein $R^1$ and $R^2$ represents $CF_3$, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX820").

A present compound (L-2) wherein $R^1$ and $R^2$ represents $CF_3$, $R^6$ represents $OCF_3$, $R^3$, $R^5$ and $R^7$ represent a hydrogen atom, and $R^4$ represents any one substituent indicated in [Table L1] to [Table L3] (hereinafter, referred to

as "Compound class SX821").

A present compound (L-2) wherein $R^1$ and $R^2$ represents a cyclopropyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX822").

A present compound (L-2) wherein $R^1$ and $R^2$ represents a cyclopropyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX823").

A present compound (L-2) wherein $R^1$ and $R^2$ represents a cyclopropyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX824").

A present compound (L-2) wherein $R^1$ and $R^2$ represents a cyclopropyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX825").

A present compound (L-2) wherein $R^1$ and $R^2$ represents a cyclopropyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX826").

A present compound (L-2) wherein $R^1$ and $R^2$ represents a cyclopropyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX827").

A present compound (L-2) wherein $R^1$ and $R^2$ represents a cyclopropyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX828").

A present compound (L-2) wherein $R^1$ and $R^2$ represents a cyclopropyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX829").

A present compound (L-2) wherein $R^1$ and $R^2$ represents a cyclopropyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX830").

A present compound (L-2) wherein $R^1$ and $R^2$ represents a cyclopropyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX831").

A present compound (L-2) wherein $R^1$ and $R^2$ represents a cyclopropyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX832").

A present compound (L-2) wherein $R^1$ and $R^2$ represents a cyclopropyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX833").

A present compound (L-2) wherein $R^1$ and $R^2$ represents a cyclopropyl group, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX834").

A present compound (L-2) wherein $R^1$ and $R^2$ represents a cyclopropyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX835").

A present compound (L-2) wherein $R^1$ and $R^2$ represents a cyclopropyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX836").

A present compound (L-2) wherein $R^1$ and $R^2$ represents a cyclopropyl group, $R^6$ represents $OCF_3$, $R^3$, $R^5$ and $R^7$ represent a hydrogen atom, and $R^4$ represents any one substituent indicated in [Table Z1] to [Table L3] (hereinafter, referred to as "Compound class SX837").

A present compound (L-2) wherein $R^1$ represents any one of group of a chlorine atom, $R^2$ represents any one group selected from a hydrogen atom, a fluorine atom, a chlorine atom, a bromine atom, an iodine atom, a methyl group, an ethyl group, a propyl group, an isopropyl group, a cyclopropyl group, or $CF_3$, and $R^3$, $R^4$, $R^5$, $R^6$, and $R^7$ represent a hydrogen atom (hereinafter, referred to as "Compound class SX838").

A compound represented by formula (L-3):

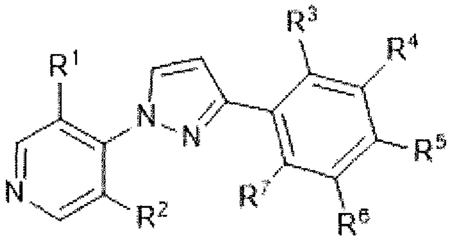

(L-3)

(hereinafter, referred to as "Compound (L-3)")
wherein $R^1$ represents a fluorine atom, $R^2$ represents a hydrogen atom, $R^4$, $R^5$, $R^6$ and $R^7$ represent a hydrogen atom, and $R^3$ represents any one substituent indicated in [Table 11] (hereinafter, referred to as "Compound class SX839").

A present compound (L-3) wherein $R^1$ and $R^2$ represent a fluorine atom, $R^4$, $R^5$, $R^6$ and $R^7$ represent a hydrogen atom, and $R^3$ represents any one substituent indicated in [Table L1] (hereinafter, referred to as "Compound class SX840").

A present compound (L-3) wherein $R^1$ represents a fluorine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX841").

A present compound (L-3) wherein $R^1$ and $R^2$ represent a chlorine atom, $R^4$, $R^5$, $R^6$ and $R^7$ represent a hydrogen atom, and $R^3$ represents any one substituent indicated in [Table L1] (hereinafter, referred to as "Compound class SX842").

A present compound (L-3) wherein $R^1$ represents a bromine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX843").

A present compound (L-3) wherein $R^1$ and $R^2$ represent a bromine atom, $R^4$, $R^5$, $R^6$ and $R^7$ represent a hydrogen atom, and $R^3$ represents any one substituent indicated in [Table L1] (hereinafter, referred to as "Compound class SX844").

A present compound (L-3) wherein $R^1$ represents an iodine atom, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX845").

A present compound (L-3) wherein $R^1$ and $R^2$ represent an iodine atom, $R^4$, $R^5$, $R^6$ and $R^7$ represent a hydrogen atom, and $R^3$ represents any one substituent indicated in [Table L1] (hereinafter, referred to as "Compound class SX846").

A present compound (L-3) wherein $R^1$ represents a methyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX847").

A present compound (L-3) wherein $R^1$ and $R^2$ represent a methyl group, $R^4$, $R^5$, $R^6$ and $R^7$ represent a hydrogen atom, and $R^3$ represents any one substituent indicated in [Table L1] (hereinafter, referred to as "Compound class SX848").

A present compound (L-3) wherein $R^1$ represents an ethyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX849").

A present compound (L-3) wherein $R^1$ and $R^2$ represent an ethyl group, $R^4$, $R^5$, $R^6$ and $R^7$ represent a hydrogen atom, and $R^3$ represents any one substituent indicated in [Table L1] (hereinafter, referred to as "Compound class SX850").

A present compound (L-3) wherein $R^1$ represents a propyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX851").

A present compound (L-3) wherein $R^1$ and $R^2$ represent a propyl group, $R^4$, $R^5$, $R^6$ and $R^7$ represent a hydrogen atom, and $R^3$ represents any one substituent indicated in [Table L1] (hereinafter, referred to as "Compound class SX852").

A present compound (L-3) wherein $R^1$ represents an isopropyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX853").

A present compound (L-3) wherein $R^1$ and $R^2$ represent an isopropyl group, $R^4$, $R^5$, $R^6$ and $R^7$ represent a hydrogen atom, and $R^3$ represents any one substituent indicated in [Table L1] (hereinafter, referred to as "Compound class SX854").

A present compound (L-3) wherein $R^1$ represents a cyclopropyl group, $R^2$ represents a hydrogen atom, $R^4$, $R^5$, $R^6$ and $R^7$ represent a hydrogen atom, and $R^3$ represents any one substituent indicated in [Table L1] (hereinafter, referred to as "Compound class SX855").

A present compound (L-3) wherein $R^1$ and $R^2$ represent a cyclopropyl group, $R^4$, $R^5$, $R^6$ and $R^7$ represent a hydrogen atom, and $R^3$ represents any one substituent indicated in [Table L1] (hereinafter, referred to as "Compound class SX856").

A present compound (L-3) wherein $R^1$ represents $CF_3$, $R^2$ represents a hydrogen atom, $R^4$, $R^5$, $R^6$ and $R^7$ represent a hydrogen atom, and $R^3$ represents any one substituent indicated in [Table L1] (hereinafter, referred to as

"Compound class SX857").

A present compound (L-3) wherein $R^1$ and $R^2$ represent $CF_3$, $R^4$, $R^5$, $R^6$ and $R^7$ represent a hydrogen atom, and $R^3$ represents any one substituent indicated in [Table L1] (hereinafter, referred to as "Compound class SX858").

A present compound (L-3) wherein $R^1$ represents a chlorine atom, $R^2$ represents a bromine atom, $R^4$, $R^5$, $R^6$ and $R^7$ represent a hydrogen atom, and $R^3$ represents any one substituent indicated in [Table L1] (hereinafter, referred to as "Compound class SX859").

A present compound (L-3) wherein $R^1$ represents a chlorine atom, $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX860").

A present compound (L-3) wherein $R^1$ represents a chlorine atom, $R^2$ represents a methyl group, $R^4$, $R^5$, $R^6$ and $R^7$ represent a hydrogen atom, and $R^3$ represents any one substituent indicated in [Table L1] (hereinafter, referred to as "Compound class SX861").

A present compound (L-3) wherein $R^1$ represents a chlorine atom, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX862").

A present compound (L-3) wherein $R^1$ represents a chlorine atom, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX863").

A present compound (L-3) wherein $R^1$ represents a chlorine atom, $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX864").

A present compound (L-3) wherein $R^1$ represents a chlorine atom, $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX865").

A present compound (L-3) wherein $R^1$ represents a chlorine atom, $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX866").

A present compound (L-3) wherein $R^1$ represents a bromine atom, $R^2$ represents an iodine atom, $R^4$, $R^5$, $R^6$ and $R^7$ represent a hydrogen atom, and $R^3$ represents any one substituent indicated in [Table L1] (hereinafter, referred to as "Compound class SX867").

A present compound (L-3) wherein $R^1$ represents a bromine atom, $R^2$ represents a methyl group, $R^4$, $R^5$, $R^6$ and $R^7$ represent a hydrogen atom, and $R^3$ represents any one substituent indicated in [Table L1] (hereinafter, referred to as "Compound class SX868").

A present compound (L-3) wherein $R^1$ represents a bromine atom, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX869").

A present compound (L-3) wherein $R^1$ represents a bromine atom, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX870").

A present compound (L-3) wherein $R^1$ represents a bromine atom, $R^2$ represents an isopropyl group, $R^4$, $R^5$, $R^6$ and $R^7$ represent a hydrogen atom, and $R^3$ represents any one substituent indicated in [Table L1] (hereinafter, referred to as "Compound class SX871").

A present compound (L-3) wherein $R^1$ represents a bromine atom, $R^2$ represents a cyclopropyl group, $R^4$, $R^5$, $R^6$ and $R^7$ represent a hydrogen atom, and $R^3$ represents any one substituent indicated in [Table L1] (hereinafter, referred to as "Compound class SX872").

A present compound (L-3) wherein $R^1$ represents a bromine atom, $R^2$ represents $CF_3$, $R^4$, $R^5$, $R^6$ and $R^7$ represent a hydrogen atom, and $R^3$ represents any one substituent indicated in [Table L1] (hereinafter, referred to as "Compound class SX873").

A present compound (L-3) wherein $R^1$ represents a methyl group, $R^2$ represents an ethyl group, $R^4$, $R^5$, $R^6$ and $R^7$ represent a hydrogen atom, and $R^3$ represents any one substituent indicated in [Table L1] (hereinafter, referred to as "Compound class SX874").

A present compound (L-3) wherein $R^1$ represents a methyl group, $R^2$ represents a propyl group, $R^4$, $R^5$, $R^6$ and $R^7$ represent a hydrogen atom, and $R^3$ represents any one substituent indicated in [Table 11] (hereinafter, referred to as "Compound class SX875").

A present compound (L-3) wherein $R^1$ represents an ethyl group, $R^2$ represents a propyl group, $R^4$, $R^5$, $R^6$ and $R^7$ represent a hydrogen atom, and $R^3$ represents any one substituent indicated in [Table L1] (hereinafter, referred to as "Compound class SX876").

A present compound (L-3) wherein $R^1$ represents a fluorine atom, $R^2$ represents a hydrogen atom, $R^3$, $R^5$, $R^6$

and $R^7$ represent a hydrogen atom, and $R^4$ represents any one substituent indicated in [Table L1] to [Table L3] (hereinafter, referred to as "Compound class SX877").

A present compound (L-3) wherein $R^1$ and $R^2$ represent a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX878").

A present compound (L-3) wherein $R^1$ represents a chlorine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX879").

A present compound (L-3) wherein $R^1$ and $R^2$ represent a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX880").

A present compound (L-3) wherein $R^1$ represents a bromine atom, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX881").

A present compound (L-3) wherein $R^1$ and $R^2$ represent a bromine atom, $R^3$, $R^5$, $R^6$ and $R^7$ represent a hydrogen atom, and $R^4$ represents any one substituent indicated in [Table L1] to [Table L3] (hereinafter, referred to as "Compound class SX882").

A present compound (L-3) wherein $R^1$ represents an iodine atom, $R^2$ represents a hydrogen atom, $R^3$, $R^5$, $R^6$ and $R^7$ represent a hydrogen atom, and $R^4$ represents any one substituent indicated in [Table Z1] to [Table L3] (hereinafter, referred to as "Compound class SX883").

A present compound (L-3) wherein $R^1$ and $R^2$ represent an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX884").

A present compound (L-3) wherein $R^1$ represents a methyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX885").

A present compound (L-3) wherein $R^1$ and $R^2$ represent a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX886").

A present compound (L-3) wherein $R^1$ represents an ethyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX887").

A present compound (L-3) wherein $R^1$ and $R^2$ represent an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX888").

A present compound (L-3) wherein $R^1$ represents a propyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX889").

A present compound (L-3) wherein $R^1$ and $R^2$ represent a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX890").

A present compound (L-3) wherein $R^1$ represents an isopropyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX891").

A present compound (L-3) wherein $R^1$ and $R^2$ represent an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX892").

A present compound (L-3) wherein $R^1$ represents a cyclopropyl group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX893").

A present compound (L-3) wherein $R^1$ and $R^2$ represent a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX894").

A present compound (L-3) wherein $R^1$ represents $CF_3$, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX895").

A present compound (L-3) wherein $R^1$ and $R^2$ represent $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class

SX896").

A present compound (L-3) wherein $R^1$ represents a chlorine atom, $R^2$ represents a bromine atom, $R^3$, $R^5$, $R^6$ and $R^7$ represent a hydrogen atom, and $R^4$ represents any one substituent indicated in [Table L1) to [Table L3] (hereinafter, referred to as "Compound class SX897").

A present compound (L-3) wherein $R^1$ represents a chlorine atom, $R^2$ represents an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX898").

A present compound (L-3) wherein $R^1$ represents a chlorine atom, $R^2$ represents a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX899").

A present compound (L-3) wherein $R^1$ represents a chlorine atom, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX900").

A present compound (L-3) wherein $R^1$ represents a chlorine atom, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX901").

A present compound (L-3) wherein $R^1$ represents a chlorine atom, $R^2$ represents an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX902").

A present compound (L-3) wherein $R^1$ represents a chlorine atom, $R^2$ represents a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX903").

A present compound (L-3) wherein $R^1$ represents a chlorine atom, $R^2$ represents $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX904").

A present compound (L-3) wherein $R^1$ represents a bromine atom, $R^2$ represents an iodine atom, $R^3$, $R^5$, $R^6$ and $R^7$ represent a hydrogen atom, and $R^4$ represents any one substituent indicated in [Table L1] to [Table L3] (hereinafter, referred to as "Compound class SX905").

A present compound (L-3) wherein $R^1$ represents a bromine atom, $R^2$ represents a methyl group, $R^3$, $R^5$, $R^6$ and $R^7$ represent a hydrogen atom, and $R^4$ represents any one substituent indicated in [Table L1] to [Table L3] (hereinafter, referred to as "Compound class SX906").

A present compound (L-3) wherein $R^1$ represents a bromine atom, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX907").

A present compound (L-3) wherein $R^1$ represents a bromine atom, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX908").

A present compound (L-3) wherein $R^1$ represents a bromine atom, $R^2$ represents an isopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX909").

A present compound (L-3) wherein $R^1$ represents a bromine atom, $R^2$ represents a cyclopropyl group, $R^3$, $R^5$, $R^6$ and $R^7$ represent a hydrogen atom, and $R^4$ represents any one substituent indicated in [Table L1] to [Table L3] (hereinafter, referred to as "Compound class SX910").

A present compound (L-3) wherein $R^1$ represents a bromine atom, $R^2$ represents $CF_3$, $R^3$, $R^5$, $R^6$ and $R^7$ represent a hydrogen atom, and $R^4$ represents any one substituent indicated in [Table L1] to [Table L3] (hereinafter, referred to as "Compound class SX911").

A present compound (L-3) wherein $R^1$ represents a methyl group, $R^2$ represents an ethyl group, $R^3$, $R^5$, $R^6$ and $R^7$ represent a hydrogen atom, and $R^4$ represents any one substituent indicated in [Table L1] to [Table L3] (hereinafter, referred to as "Compound class SX912").

A present compound (L-3) wherein $R^1$ represents a methyl group, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX913").

A present compound (L-3) wherein $R^1$ represents an ethyl group, $R^2$ represents a propyl group, $R^3$, $R^5$, $R^6$ and $R^7$ represent a hydrogen atom, and $R^4$ represents any one substituent indicated in [Table L1] to [Table L3] (hereinafter, referred to as "Compound class SX914").

A present compound (L-3) wherein $R^1$ represents a fluorine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX915").

A present compound (L-3) wherein $R^1$ and $R^2$ represent a fluorine atom, $R^3$, $R^4$, $R^6$ and $R^7$ represent a hydrogen atom, and $R^5$ represents any one substituent indicated in [Table L1] to [Table L2] (hereinafter, referred to as "Compound class SX916").

A present compound (L-3) wherein $R^1$ represents a chlorine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX917").

A present compound (L-3) wherein $R^1$ and $R^2$ represent a chlorine atom, $R^3$, $R^4$, $R^6$ and $R^7$ represent a hydrogen atom, and $R^5$ represents any one substituent indicated in [Table L1] to [Table L2] (hereinafter, referred to as "Compound class SX918").

A present compound (L-3) wherein $R^1$ represents a bromine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX919").

A present compound (L-3) wherein $R^1$ and $R^2$ represent a bromine atom, $R^3$, $R^4$, $R^6$ and $R^7$ represent a hydrogen atom, and $R^5$ represents any one substituent indicated in [Table L1] to [Table L2] (hereinafter, referred to as "Compound class SX920").

A present compound (L-3) wherein $R^1$ represents an iodine atom, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX921").

A present compound (L-3) wherein $R^1$ and $R^2$ represent an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX922").

A present compound (L-3) wherein $R^1$ represents a methyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX923").

A present compound (L-3) wherein $R^1$ and $R^2$ represent a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX924").

A present compound (L-3) wherein $R^1$ represents an ethyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX925").

A present compound (L-3) wherein $R^1$ and $R^2$ represent an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX926").

A present compound (L-3) wherein $R^1$ represents a propyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX927").

A present compound (L-3) wherein $R^1$ and $R^2$ represent a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX928").

A present compound (L-3) wherein $R^1$ represents an isopropyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX929").

A present compound (L-3) wherein $R^1$ and $R^2$ represent an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX930").

A present compound (L-3) wherein $R^1$ represents a cyclopropyl group, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX931").

A present compound (L-3) wherein $R^1$ and $R^2$ represent a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX932").

A present compound (L-3) wherein $R^1$ represents $CF_3$, $R^2$ represents a hydrogen atom, $R^3$, $R^4$, $R^6$ and $R^7$ represent a hydrogen atom, and $R^5$ represents any one substituent indicated in [Table L1] to [Table L2] (hereinafter, referred to as "Compound class SX933").

A present compound (L-3) wherein $R^1$ and $R^2$ represent $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX934").

A present compound (L-3) wherein $R^1$ represents a chlorine atom, $R^2$ represents a bromine atom, $R^3$, $R^4$, $R^6$

and $R^7$ represent a hydrogen atom, and $R^5$ represents any one substituent indicated in [Table L1] to [Table L2] (hereinafter, referred to as "Compound class SX935").

A present compound (L-3) wherein $R^1$ represents a chlorine atom, $R^2$ represents an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX936").

A present compound (L-3) wherein $R^1$ represents a chlorine atom, $R^2$ represents a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX937").

A present compound (L-3) wherein $R^1$ represents a chlorine atom, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX938").

A present compound (L-3) wherein $R^1$ represents a chlorine atom, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX939").

A present compound (L-3) wherein $R^1$ represents a chlorine atom, $R^2$ represents an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX940").

A present compound (L-3) wherein $R^1$ represents a chlorine atom, $R^2$ represents a cyclopropyl group, $R^3$, $R^4$, $R^6$ and $R^7$ represent a hydrogen atom, and $R^5$ represents any one substituent indicated in [Table L1) to [Table L2] (hereinafter, referred to as "Compound class SX941").

A present compound (L-3) wherein $R^1$ represents a chlorine atom, $R^2$ represents $CF_3$, $R^3$, $R^4$, $R^6$ and $R^7$ represent a hydrogen atom, and $R^5$ represents any one substituent indicated in [Table Z1] to [Table L2] (hereinafter, referred to as "Compound class SX942").

A present compound (L-3) wherein $R^1$ represents a bromine atom, $R^2$ represents an iodine atom, $R^3$, $R^4$, $R^6$ and $R^7$ represent a hydrogen atom, and $R^5$ represents any one substituent indicated in [Table L1] to [Table L2] (hereinafter, referred to as "Compound class SX943").

A present compound (L-3) wherein $R^1$ represents a bromine atom, $R^2$ represents a methyl group, $R^3$, $R^4$, $R^6$ and $R^7$ represent a hydrogen atom, and $R^5$ represents any one substituent indicated in [Table L1] to [Table L2] (hereinafter, referred to as "Compound class SX944").

A present compound (L-3) wherein $R^1$ represents a bromine atom, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX945").

A present compound (L-3) wherein $R^1$ represents a bromine atom, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX946").

A present compound (L-3) wherein $R^1$ represents a bromine atom, $R^2$ represents an isopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX947").

A present compound (L-3) wherein $R^1$ represents a bromine atom, $R^2$ represents a cyclopropyl group, $R^3$, $R^4$, $R^6$ and $R^7$ represent a hydrogen atom, and $R^5$ represents any one substituent indicated in [Table L1] to [Table L2] (hereinafter, referred to as "Compound class SX948").

A present compound (L-3) wherein $R^1$ represents a bromine atom, $R^2$ represents $CF_3$, $R^3$, $R^4$, $R^6$ and $R^7$ represent a hydrogen atom, and $R^5$ represents any one substituent indicated in [Table L1] to [Table L2] (hereinafter, referred to as "Compound class SX949").

A present compound (L-3) wherein $R^1$ represents a methyl group, $R^2$ represents an ethyl group, $R^3$, $R^4$, $R^6$ and $R^7$ represent a hydrogen atom, and $R^5$ represents any one substituent indicated in [Table L1] to [Table L2] (hereinafter, referred to as "Compound class SX950").

A present compound (L-3) wherein $R^1$ represents a methyl group, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX951").

A present compound (L-3) wherein $R^1$ represents an ethyl group, $R^2$ represents a propyl group, $R^3$, $R^4$, $R^6$ and $R^7$ represent a hydrogen atom, and $R^5$ represents any one substituent indicated in [Table L1] to [Table L2] (hereinafter, referred to as "Compound class SX952").

A present compound (L-3) wherein $R^1$ represents a fluorine atom, $R^2$ represents a hydrogen atom, $R^3$ represents a fluorine atom, $R^4$, $R^6$ and $R^7$ represent a hydrogen atom, and $R^5$ represents any one substituent indicated in [Table L1] to [Table L2] (hereinafter, referred to as "Compound class SX953") .

A present compound (L-3) wherein $R^1$ represents a fluorine atom, $R^2$ represents a hydrogen atom, $R^3$ represents a chlorine atom, $R^4$, $R^6$ and $R^7$ represent a hydrogen atom, and $R^5$ represents any one substituent indicated in [Table

L1] to [Table L2] (hereinafter, referred to as "Compound class SX954") .

A present compound (L-3) wherein $R^1$ represents a fluorine atom, $R^2$ represents a hydrogen atom, $R^3$ represents a bromine atom, $R^4$, $R^6$ and $R^7$ represent a hydrogen atom, and $R^5$ represents any one substituent indicated in [Table L1] to [Table L2] (hereinafter, referred to as "Compound class SX955).

A present compound (L-3) wherein $R^1$ represents a fluorine atom, $R^2$ represents a hydrogen atom, $R^3$ represents an iodine atom, $R^4$, $R^6$ and $R^7$ represent a hydrogen atom, and $R^5$ represents any one substituent indicated in [Table L1] to [Table L2] (hereinafter, referred to as "Compound class SX956).

A present compound (L-3) wherein $R^1$ represents a fluorine atom, $R^2$ represents a hydrogen atom, $R^3$ represents a methyl group, $R^4$, $R^6$ and $R^7$ represent a hydrogen atom, and $R^5$ represents any one substituent indicated in [Table Z1] to [Table L2] (hereinafter, referred to as "Compound class SX957).

A present compound (L-3) wherein $R^1$ represents a fluorine atom, $R^2$ represents a hydrogen atom, $R^3$ represents a methoxy group, $R^4$, $R^6$ and $R^7$ represent a hydrogen atom, and $R^5$ represents any one substituent indicated in [Table L1] to [Table L2] (hereinafter, referred to as "Compound class SX958).

A present compound (L-3) wherein $R^1$ represents a fluorine atom, $R^2$ represents a hydrogen atom, $R^3$ represents $CF_3$, $R^4$, $R^6$ and $R^7$ represent a hydrogen atom, and $R^5$ represents any one substituent indicated in [Table L1] to [Table L2] (hereinafter, referred to as "Compound class SX959).

A present compound (L-3) wherein $R^1$ represents a fluorine atom, $R^2$ represents a hydrogen atom, $R^3$ represents $OCF_3$, $R^4$, $R^6$ and $R^7$ represent a hydrogen atom, and $R^5$ represents any one substituent indicated in [Table L1] to [Table L2] (hereinafter, referred to as "Compound class SX960).

A present compound (L-3) wherein $R^1$ represents a fluorine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX961) .

A present compound (L-3) wherein $R^1$ represents a fluorine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX962).

A present compound (L-3) wherein $R^1$ represents a fluorine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX963).

A present compound (L-3) wherein $R^1$ represents a fluorine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX964).

A present compound (L-3) wherein $R^1$ represents a fluorine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX965).

A present compound (L-3) wherein $R^1$ represents a fluorine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX966).

A present compound (L-3) wherein $R^1$ represents a fluorine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX967).

A present compound (L-3) wherein $R^1$ represents a fluorine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX968).

A present compound (L-3) wherein $R^1$ represents a fluorine atom, $R^2$ represents a hydrogen atom, $R^4$ represents a fluorine atom, $R^3$, $R^5$ and $R^7$ represent a hydrogen atom, and $R^6$ represents any one substituent indicated in [Table L1] to [Table L3] (hereinafter, referred to as "Compound class SX969) .

A present compound (L-3) wherein $R^1$ represents a fluorine atom, $R^2$ represents a hydrogen atom, $R^4$ represents a chlorine atom, $R^3$, $R^5$ and $R^7$ represent a hydrogen atom, and $R^6$ represents any one substituent indicated in [Table L1] to [Table L3] (hereinafter, referred to as "Compound class SX970) .

A present compound (L-3) wherein $R^1$ represents a fluorine atom, $R^2$ represents a hydrogen atom, $R^4$ represents a bromine atom, $R^3$, $R^5$ and $R^7$ represent a hydrogen atom, and $R^6$ represents any one substituent indicated in [Table L1] to [Table L3] (hereinafter, referred to as "Compound class SX971) .

A present compound (L-3) wherein $R^1$ represents a fluorine atom, $R^2$ represents a hydrogen atom, $R^4$ represents an iodine atom, $R^3$, $R^5$ and $R^7$ represent a hydrogen atom, and $R^6$ represents any one substituent indicated in [Table L1] to [Table L3] (hereinafter, referred to as "Compound class SX972).

A present compound (L-3) wherein $R^1$ represents a fluorine atom, $R^2$ represents a hydrogen atom, $R^4$ represents a methyl group, $R^3$, $R^5$ and $R^7$ represent a hydrogen atom, and $R^6$ represents any one substituent indicated in [Table L1] to [Table L3] (hereinafter, referred to as "Compound class SX973).

A present compound (L-3) wherein $R^1$ represents a fluorine atom, $R^2$ represents a hydrogen atom, $R^4$ represents a methoxy group, $R^3$, $R^5$ and $R^7$ represent a hydrogen atom, and $R^6$ represents any one substituent indicated in [Table L1] to [Table L3] (hereinafter, referred to as "Compound class SX974).

A present compound (L-3) wherein $R^1$ represents a fluorine atom, $R^2$ represents a hydrogen atom, $R^4$ represents $CF_3$, $R^3$, $R^5$ and $R^7$ represent a hydrogen atom, and $R^6$ represents any one substituent indicated in [Table L1] to [Table L3] (hereinafter, referred to as "Compound class SX975).

A present compound (L-3) wherein $R^1$ represents a fluorine atom, $R^2$ represents a hydrogen atom, $R^4$ represents $OCF_3$, $R^3$, $R^5$ and $R^7$ represent a hydrogen atom, and $R^6$ represents any one substituent indicated in [Table L1] to [Table L3] (hereinafter, referred to as "Compound class SX976).

A present compound (L-3) wherein $R^1$ represents a chlorine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX977).

A present compound (L-3) wherein $R^1$ represents a chlorine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX978).

A present compound (L-3) wherein $R^1$ represents a chlorine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX979).

A present compound (L-3) wherein $R^1$ represents a chlorine atom, $R^2$ represents a hydrogen atom, $R^5$ represents an iodine atom, $R^3$, $R^6$ and $R^7$ represent a hydrogen atom, and $R^4$ represents any one substituent indicated in [Table 11] to [Table L3] (hereinafter, referred to as "Compound class SX980).

A present compound (L-3) wherein $R^1$ represents a chlorine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX981).

A present compound (L-3) wherein $R^1$ represents a chlorine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX982).

A present compound (L-3) wherein $R^1$ represents a chlorine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX983).

A present compound (L-3) wherein $R^1$ represents a chlorine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX984).

A present compound (L-3) wherein $R^1$ represents a chlorine atom, $R^2$ represents a hydrogen atom, $R^4$ represents a fluorine atom, $R^3$, $R^5$ and $R^7$ represent a hydrogen atom, and $R^6$ represents any one substituent indicated in [Table L1] to [Table L3] (hereinafter, referred to as "Compound class SX985).

A present compound (L-3) wherein $R^1$ represents a chlorine atom, $R^2$ represents a hydrogen atom, $R^4$ represents a chlorine atom, $R^3$, $R^5$ and $R^7$ represent a hydrogen atom, and $R^6$ represents any one substituent indicated in [Table L1] to [Table L3] (hereinafter, referred to as "Compound class SX985).

A present compound (L-3) wherein $R^1$ represents a chlorine atom, $R^2$ represents a hydrogen atom, $R^4$ represents a bromine atom, $R^3$, $R^5$ and $R^7$ represent a hydrogen atom, and $R^6$ represents any one substituent indicated in [Table L1] to [Table L3] (hereinafter, referred to as "Compound class SX987).

A present compound (L-3) wherein $R^1$ represents a chlorine atom, $R^2$ represents a hydrogen atom, $R^4$ represents an iodine atom, $R^3$, $R^5$ and $R^7$ represent a hydrogen atom, and $R^6$ represents any one substituent indicated in [Table L1] to [Table L3] (hereinafter, referred to as "Compound class SX988).

A present compound (L-3) wherein $R^1$ represents a chlorine atom, $R^2$ represents a hydrogen atom, $R^4$ represents a methyl group, $R^3$, $R^5$ and $R^7$ represent a hydrogen atom, and $R^6$ represents any one substituent indicated in [Table L1] to [Table L3] (hereinafter, referred to as "Compound class SX989).

A present compound (L-3) wherein $R^1$ represents a chlorine atom, $R^2$ represents a hydrogen atom, $R^4$ represents a methoxy group, $R^3$, $R^5$ and $R^7$ represent a hydrogen atom, and $R^6$ represents any one substituent indicated in [Table L1] to [Table L3] (hereinafter, referred to as "Compound class SX990).

A present compound (L-3) wherein $R^1$ represents a chlorine atom, $R^2$ represents a hydrogen atom, $R^4$ represents $CF_3$, $R^3$, $R^5$ and $R^7$ represent a hydrogen atom, and $R^6$ represents any one substituent indicated in [Table L1] to [Table L3] (hereinafter, referred to as "Compound class SX991).

A present compound (L-3) wherein $R^1$ represents a chlorine atom, $R^2$ represents a hydrogen atom, $R^4$ represents $OCF_3$, $R^3$, $R^5$ and $R^7$ represent a hydrogen atom, and $R^6$ represents any one substituent indicated in [Table L1] to [Table L3] (hereinafter, referred to as "Compound class SX992).

A present compound (L-3) wherein $R^1$ represents a bromine atom, $R^2$ represents a hydrogen atom, $R^5$ represents

a fluorine atom, $R^3$, $R^6$ and $R^7$ represent a hydrogen atom, and $R^4$ represents any one substituent indicated in [Table L1] to [Table L3] (hereinafter, referred to as "Compound class SX993) .

A present compound (L-3) wherein $R^1$ represents a bromine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX994) .

A present compound (L-3) wherein $R^1$ represents a bromine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX995) .

A present compound (L-3) wherein $R^1$ represents a bromine atom, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX996) .

A present compound (L-3) wherein $R^1$ represents a bromine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX997).

A present compound (L-3) wherein $R^1$ represents a bromine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX998).

A present compound (L-3) wherein $R^1$ represents a bromine atom, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX999).

A present compound (L-3) wherein $R^1$ represents a bromine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1000).

A present compound (L-3) wherein $R^1$ represents a bromine atom, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1001).

A present compound (L-3) wherein $R^1$ represents a bromine atom, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1002).

A present compound (L-3) wherein $R^1$ represents a bromine atom, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1003).

A present compound (L-3) wherein $R^1$ represents a bromine atom, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1004).

A present compound (L-3) wherein $R^1$ represents a bromine atom, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1005).

A present compound (L-3) wherein $R^1$ represents a bromine atom, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1006) .

A present compound (L-3) wherein $R^1$ represents a bromine atom, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1007).

A present compound (L-3) wherein $R^1$ represents a bromine atom, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1008).

A present compound (L-3) wherein $R^1$ represents an iodine atom, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1009).

A present compound (L-3) wherein $R^1$ represents an iodine atom, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1010).

A present compound (L-3) wherein $R^1$ represents an iodine atom, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1011).

A present compound (L-3) wherein $R^1$ represents an iodine atom, $R^2$ represents a hydrogen atom, $R^5$ represents an iodine atom, $R^3$, $R^6$ and $R^7$ represent a hydrogen atom, and $R^4$ represents any one substituent indicated in [Table

L1] to [Table L3] (hereinafter, referred to as "Compound class SX1012).

A present compound (L-3) wherein $R^1$ represents an iodine atom, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1013).

A present compound (L-3) wherein $R^1$ represents an iodine atom, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1014).

A present compound (L-3) wherein $R^1$ represents an iodine atom, $R^2$ represents a hydrogen atom, $R^3$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1015).

A present compound (L-3) wherein $R^1$ represents an iodine atom, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1016).

A present compound (L-3) wherein $R^1$ represents an iodine atom, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1017).

A present compound (L-3) wherein $R^1$ represents an iodine atom, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1018).

A present compound (L-3) wherein $R^1$ represents an iodine atom, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1019) .

A present compound (L-3) wherein $R^1$ represents an iodine atom, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1020) .

A present compound (L-3) wherein $R^1$ represents an iodine atom, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1021).

A present compound (L-3) wherein $R^1$ represents an iodine atom, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1022) .

A present compound (L-3) wherein $R^1$ represents an iodine atom, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1023).

A present compound (L-3) wherein $R^1$ represents an iodine atom, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1024).

A present compound (L-3) wherein $R^1$ represents a methyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1025).

A present compound (L-3) wherein $R^1$ represents a methyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1026).

A present compound (L-3) wherein $R^1$ represents a methyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1027).

A present compound (L-3) wherein $R^1$ represents a methyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1028) .

A present compound (L-3) wherein $R^1$ represents a methyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1029) .

A present compound (L-3) wherein $R^1$ represents a methyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1030) .

A present compound (L-3) wherein $R^1$ represents a methyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1031).

A present compound (L-3) wherein $R^1$ represents a methyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1032).

A present compound (L-3) wherein $R^1$ represents a methyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1033).

A present compound (L-3) wherein $R^1$ represents a methyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1034).

A present compound (L-3) wherein $R^1$ represents a methyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1035).

A present compound (L-3) wherein $R^1$ represents a methyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1036) .

A present compound (L-3) wherein $R^1$ represents a methyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1037).

A present compound (L-3) wherein $R^1$ represents a methyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1038).

A present compound (L-3) wherein $R^1$ represents a methyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1039).

A present compound (L-3) wherein $R^1$ represents a methyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1040).

A present compound (L-3) wherein $R^1$ represents an ethyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1041).

A present compound (L-3) wherein $R^1$ represents an ethyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1042).

A present compound (L-3) wherein $R^1$ represents an ethyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1043).

A present compound (L-3) wherein $R^1$ represents an ethyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1044).

A present compound (L-3) wherein $R^1$ represents an ethyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1045) .

A present compound (L-3) wherein $R^1$ represents an ethyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1046).

A present compound (L-3) wherein $R^1$ represents an ethyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1047).

A present compound (L-3) wherein $R^1$ represents an ethyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1) to [Table L3] (hereinafter, referred to as "Compound class SX1048).

A present compound (L-3) wherein $R^1$ represents an ethyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1049).

A present compound (L-3) wherein $R^1$ represents an ethyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1050).

A present compound (L-3) wherein $R^1$ represents an ethyl group, $R^2$ represents a hydrogen atom, $R^6$ represents

a bromine atom, $R^3$, $R^5$ and $R^7$ represent a hydrogen atom, and $R^4$ represents any one substituent indicated in [Table L1] to [Table L3] (hereinafter, referred to as "Compound class SX1051).

A present compound (L-3) wherein $R^1$ represents an ethyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1052).

A present compound (L-3) wherein $R^1$ represents an ethyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1053).

A present compound (L-3) wherein $R^1$ represents an ethyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1054) .

A present compound (L-3) wherein $R^1$ represents an ethyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1055).

A present compound (L-3) wherein $R^1$ represents an ethyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1056).

A present compound (L-3) wherein $R^1$ represents $CF_3$, $R^2$ represents a hydrogen atom, $R^5$ represents a fluorine atom, $R^3$, $R^6$ and $R^7$ represent a hydrogen atom, and $R^4$ represents any one substituent indicated in [Table L1J to [Table L3] (hereinafter, referred to as "Compound class SX1057).

A present compound (L-3) wherein $R^1$ represents $CF_3$, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1058).

A present compound (L-3) wherein $R^1$ represents $CF_3$, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1059).

A present compound (L-3) wherein $R^1$ represents $CF_3$, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1060).

A present compound (L-3) wherein $R^1$ represents $CF_3$, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1061).

A present compound (L-3) wherein $R^1$ represents $CF_3$, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1062).

A present compound (L-3) wherein $R^1$ represents $CF_3$, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1063).

A present compound (L-3) wherein $R^1$ represents $CF_3$, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1064).

A present compound (L-3) wherein $R^1$ represents $CF_3$, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1065).

A present compound (L-3) wherein $R^1$ represents $CF_3$, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1066).

A present compound (L-3) wherein $R^1$ represents $CF_3$, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1067).

A present compound (L-3) wherein $R^1$ represents $CF_3$, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1068).

A present compound (L-3) wherein $R^1$ represents $CF_3$, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1069).

A present compound (L-3) wherein $R^1$ represents $CF_3$, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table

L3] (hereinafter, referred to as "Compound class SX1070).

A present compound (L-3) wherein $R^1$ represents $CF_3$, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1071).

A present compound (L-3) wherein $R^1$ represents $CF_3$, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1072).

A present compound (L-3) wherein $R^1$ represents a cyclopropyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1073).

A present compound (L-3) wherein $R^1$ represents a cyclopropyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1074).

A present compound (L-3) wherein $R^1$ represents a cyclopropyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1075).

A present compound (L-3) wherein $R^1$ represents a cyclopropyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1076).

A present compound (L-3) wherein $R^1$ represents a cyclopropyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1077).

A present compound (L-3) wherein $R^1$ represents a cyclopropyl group, $R^2$ represents a hydrogen atom, $R^5$ represents a methoxy group, $R^3$, $R^6$ and $R^7$ represent a hydrogen atom, and $R^4$ represents any one substituent indicated in [Table Z1] to [Table L3] (hereinafter, referred to as "Compound class SX1078).

A present compound (L-3) wherein $R^1$ represents a cyclopropyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1079).

A present compound (L-3) wherein $R^1$ represents a cyclopropyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1080).

A present compound (L-3) wherein $R^1$ represents a cyclopropyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1081).

A present compound (L-3) wherein $R^1$ represents a cyclopropyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1082).

A present compound (L-3) wherein $R^1$ represents a cyclopropyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1083).

A present compound (L-3) wherein $R^1$ represents a cyclopropyl group, $R^2$ represents a hydrogen atom, $R^6$ represents an iodine atom, $R^3$, $R^5$ and $R^7$ represent a hydrogen atom, and $R^4$ represents any one substituent indicated in [Table L1J to [Table L3] (hereinafter, referred to as "Compound class SX1084).

A present compound (L-3) wherein $R^1$ represents a cyclopropyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1085).

A present compound (L-3) wherein $R^1$ represents a cyclopropyl group, $R^2$ represents a hydrogen atom, $R^5$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1086).

A present compound (L-3) wherein $R^1$ represents a cyclopropyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1087).

A present compound (L-3) wherein $R^1$ represents a cyclopropyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1088).

. A present compound (L-3) wherein $R^1$ represents a methylsulfanyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1089).

A present compound (L-3) wherein $R^1$ represents a methylsulfanyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1090).

A present compound (L-3) wherein $R^1$ represents a methylsulfanyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1091).

A present compound (L-3) wherein $R^1$ represents a methylsulfanyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1092).

A present compound (L-3) wherein $R^1$ represents a methylsulfanyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1093).

A present compound (L-3) wherein $R^1$ represents a methylsulfanyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1094).

A present compound (L-3) wherein $R^1$ represents a methylsulfanyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1095).

A present compound (L-3) wherein $R^1$ represents a methylsulfanyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1096).

A present compound (L-3) wherein $R^1$ represents a methylsulfanyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1097).

A present compound (L-3) wherein $R^1$ represents a methylsulfanyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1098).

A present compound (L-3) wherein $R^1$ represents a methylsulfanyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1099).

A present compound (L-3) wherein $R^1$ represents a methylsulfanyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1100).

A present compound (L-3) wherein $R^1$ represents a methylsulfanyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1101).

A present compound (L-3) wherein $R^1$ represents a methylsulfanyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1102).

A present compound (L-3) wherein $R^1$ represents a methylsulfanyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1103).

A present compound (L-3) wherein $R^1$ represents a methylsulfanyl group, $R^2$ represents a hydrogen atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1104).

A present compound (L-3) wherein $R^1$ represents a methylsulfanyl group, $R^2$ represents a hydrogen atom, $R^7$ represents C-F, $R^3$, $R^5$ and $R^6$ represent a hydrogen atom, and $R^4$ represents any one substituent indicated in [Table L1] to [Table L3] (hereinafter, referred to as "Compound class SX1105).

A present compound (L-3) wherein $R^1$ represents a methylsulfanyl group, $R^2$ represents a hydrogen atom, $R^7$ represents C-Cl, $R^3$, $R^5$ and $R^6$ represent a hydrogen atom, and $R^4$ represents any one substituent indicated in [Table L1] to [Table L3] (hereinafter, referred to as "Compound class SX1106) .

A present compound (L-3) wherein $R^1$ represents a methylsulfanyl group, $R^2$ represents a hydrogen atom, $R^7$ represents C-Br, $R^3$, $R^5$ and $R^6$ represent a hydrogen atom, and $R^4$ represents any one substituent indicated in [Table L1] to [Table L3] (hereinafter, referred to as "Compound class SX1107) .

A present compound (L-3) wherein $R^1$ represents a methylsulfanyl group, $R^2$ represents a hydrogen atom, $R^7$ represents C-I, $R^3$, $R^5$ and $R^6$ represent a hydrogen atom, and $R^4$ represents any one substituent indicated in [Table L1] to [Table L3] (hereinafter, referred to as "Compound class SX1108) .

A present compound (L-3) wherein $R^1$ represents a methylsulfanyl group, $R^2$ represents a hydrogen atom, $R^7$

represents C-Me, $R^3$, $R^5$ and $R^6$ represent a hydrogen atom, and $R^4$ represents any one substituent indicated in [Table L1] to [Table L3] (hereinafter, referred to as "Compound class SX1109) .

A present compound (L-3) wherein $R^1$ represents a methylsulfanyl group, $R^2$ represents a hydrogen atom, $R^7$ represents C-OMe, $R^3$, $R^5$ and $R^6$ represent a hydrogen atom, and $R^4$ represents any one substituent indicated in [Table L1] to [Table L3] (hereinafter, referred to as "Compound class SX1110).

A present compound (L-3) wherein $R^1$ represents a methylsulfanyl group, $R^2$ represents a hydrogen atom, $R^7$ represents C-$CF_3$, $R^3$, $R^5$ and $R^6$ represent a hydrogen atom, and $R^4$ represents any one substituent indicated in [Table L1] to [Table L3] (hereinafter, referred to as "Compound class SX1111).

A present compound (L-3) wherein $R^1$ represents a methylsulfanyl group, $R^2$ represents a hydrogen atom, $R^7$ represents C-$OCF_3$, $R^3$, $R^5$ and $R^6$ represent a hydrogen atom, and $R^4$ represents any one substituent indicated in [Table L1] to [Table L3] (hereinafter, referred to as "Compound class SX1112).

A present compound (L-3) wherein $R^1$ represents a methylsulfanyl group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1113).

A present compound (L-3) wherein $R^1$ represents a methylsulfanyl group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1114).

A present compound (L-3) wherein $R^1$ represents a methylsulfanyl group, $R^2$ represents a hydrogen atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1115).

A present compound (L-3) wherein $R^1$ represents a methylsulfanyl group, $R^2$ represents a hydrogen atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1116).

A present compound (L-3) wherein $R^1$ represents a methylsulfanyl group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1117).

A present compound (L-3) wherein $R^1$ represents a methylsulfanyl group, $R^2$ represents a hydrogen atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1118).

A present compound (L-3) wherein $R^1$ represents a methylsulfanyl group, $R^2$ represents a hydrogen atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1119).

A present compound (L-3) wherein $R^1$ represents a methylsulfanyl group, $R^2$ represents a hydrogen atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1120).

A present compound (L-3) wherein $R^1$ represents a methylsulfanyl group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1121).

A present compound (L-3) wherein $R^1$ represents a methylsulfanyl group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1122).

A present compound (L-3) wherein $R^1$ represents a methylsulfanyl group, $R^2$ represents a hydrogen atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1123).

A present compound (L-3) wherein $R^1$ represents a methylsulfanyl group, $R^2$ represents a hydrogen atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1124).

A present compound (L-3) wherein $R^1$ represents a methylsulfanyl group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1125).

A present compound (L-3) wherein $R^1$ represents a methylsulfanyl group, $R^2$ represents a hydrogen atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1126).

A present compound (L-3) wherein $R^1$ represents a methylsulfanyl group, $R^2$ represents a hydrogen atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1127).

A present compound (L-3) wherein $R^1$ represents a methylsulfanyl group, $R^2$ represents a hydrogen atom, $R^5$ represents $OCF_3$, $R^3$, $R^5$ and $R^7$ represent a hydrogen atom, and $R^4$ represents any one substituent indicated in [Table

L1] to [Table L3] (hereinafter, referred to as "Compound class SX1128).

A present compound (L-3) wherein $R^1$ and $R^2$ represent a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1129).

A present compound (L-3) wherein $R^1$ and $R^2$ represent a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1130).

A present compound (L-3) wherein $R^1$ and $R^2$ represent a fluorine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1131).

A present compound (L-3) wherein $R^1$ and $R^2$ represent a fluorine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1132).

A present compound (L-3) wherein $R^1$ and $R^2$ represent a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1133).

A present compound (L-3) wherein $R^1$ and $R^2$ represent a fluorine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1134).

A present compound (L-3) wherein $R^1$ and $R^2$ represent a fluorine atom, $R^5$ represents $CF_3$, $R^3$, $R^6$ and $R^7$ represent a hydrogen atom, and $R^4$ represents any one substituent indicated in [Table Z1] to [Table L3] (hereinafter, referred to as "Compound class SX1135).

A present compound (L-3) wherein $R^1$ and $R^2$ represent a fluorine atom, $R^5$ represents $OCF_3$, $R^3$, $R^6$ and $R^7$ represent a hydrogen atom, and $R^4$ represents any one substituent indicated in [Table Z1] to [Table L3] (hereinafter, referred to as "Compound class SX1136).

A present compound (L-3) wherein $R^1$ and $R^2$ represent a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1137).

A present compound (L-3) wherein $R^1$ and $R^2$ represent a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1138).

A present compound (L-3) wherein $R^1$ and $R^2$ represent a fluorine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1139).

A present compound (L-3) wherein $R^1$ and $R^2$ represent a fluorine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1140).

A present compound (L-3) wherein $R^1$ and $R^2$ represent a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1142).

A present compound (L-3) wherein $R^1$ and $R^2$ represent a fluorine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1142).

A present compound (L-3) wherein $R^1$ and $R^2$ represent a fluorine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1143).

A present compound (L-3) wherein $R^1$ and $R^2$ represent a fluorine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1144).

A present compound (L-3) wherein $R^1$ and $R^2$ represent a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1145).

A present compound (L-3) wherein $R^1$ and $R^2$ represent a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1146).

A present compound (L-3) wherein $R^1$ and $R^2$ represent a chlorine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1147).

A present compound (L-3) wherein $R^1$ and $R^2$ represent a chlorine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1148).

A present compound (L-3) wherein $R^1$ and $R^2$ represent a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1149).

A present compound (L-3) wherein $R^1$ and $R^2$ represent a chlorine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1150).

A present compound (L-3) wherein $R^1$ and $R^2$ represent a chlorine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1151).

A present compound (L-3) wherein $R^1$ and $R^2$ represent a chlorine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1152).

A present compound (L-3) wherein $R^1$ and $R^2$ represent a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1153).

A present compound (L-3) wherein $R^1$ and $R^2$ represent a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1154).

A present compound (L-3) wherein $R^1$ and $R^2$ represent a chlorine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1155).

A present compound (L-3) wherein $R^1$ and $R^2$ represent a chlorine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1156).

A present compound (L-3) wherein $R^1$ and $R^2$ represent a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1157).

A present compound (L-3) wherein $R^1$ and $R^2$ represent a chlorine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1159).

A present compound (L-3) wherein $R^1$ and $R^2$ represent a chlorine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1159).

A present compound (L-3) wherein $R^1$ and $R^2$ represent a chlorine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1160).

A present compound (L-3) wherein $R^1$ and $R^2$ represent a bromine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1161).

A present compound (L-3) wherein $R^1$ and $R^2$ represent a bromine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1162).

A present compound (L-3) wherein $R^1$ and $R^2$ represent a bromine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1163).

A present compound (L-3) wherein $R^1$ and $R^2$ represent a bromine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1164).

A present compound (L-3) wherein $R^1$ and $R^2$ represent a bromine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1165).

A present compound (L-3) wherein $R^1$ and $R^2$ represent a bromine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1166).

A present compound (L-3) wherein $R^1$ and $R^2$ represent a bromine atom, $R^5$ represents $CF_3$, $R^3$, $R^6$ and $R^7$

represent a hydrogen atom, and $R^4$ represents any one substituent indicated in [Table L1] to [Table L3] (hereinafter, referred to as "Compound class SX1167).

A present compound (L-3) wherein $R^1$ and $R^2$ represent a bromine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1168).

A present compound (L-3) wherein $R^1$ and $R^2$ represent a bromine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1169).

A present compound (L-3) wherein $R^1$ and $R^2$ represent a bromine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1170).

A present compound (L-3) wherein $R^1$ and $R^2$ represent a bromine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1171).

A present compound (L-3) wherein $R^1$ and $R^2$ represent a bromine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1172).

A present compound (L-3) wherein $R^1$ and $R^2$ represent a bromine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1173).

A present compound (L-3) wherein $R^3$- and $R^2$ represent a bromine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1174).

A present compound (L-3) wherein $R^1$ and $R^2$ represent a bromine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1175).

A present compound (L-3) wherein $R^1$ and $R^2$ represent a bromine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1176).

A present compound (L-3) wherein $R^1$ and $R^2$ represent an iodine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1177).

A present compound (L-3) wherein $R^1$ and $R^2$ represent an iodine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1178).

A present compound (L-3) wherein $R^1$ and $R^2$ represent an iodine atom, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1179).

A present compound (L-3) wherein $R^1$ and $R^2$ represent an iodine atom, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1180).

A present compound (L-3) wherein $R^1$ and $R^2$ represent an iodine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1181).

A present compound (L-3) wherein $R^1$ and $R^2$ represent an iodine atom, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1182).

A present compound (L-3) wherein $R^1$ and $R^2$ represent an iodine atom, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1183).

A present compound (L-3) wherein $R^1$ and $R^2$ represent an iodine atom, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1184).

A present compound (L-3) wherein $R^1$ and $R^2$ represent an iodine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1185).

A present compound (L-3) wherein $R^1$ and $R^2$ represent an iodine atom, $R^6$ represents a chlorine atom, $R^3$, $R^5$ and $R^7$ represent a hydrogen atom, and $R^4$ represents any one substituent indicated in [Table L1] to [Table L3]

(hereinafter, referred to as "Compound class SX1186).

A present compound (L-3) wherein $R^1$ and $R^2$ represent an iodine atom, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1187).

A present compound (L-3) wherein $R^1$ and $R^2$ represent an iodine atom, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1188).

A present compound (L-3) wherein $R^1$ and $R^2$ represent an iodine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1189).

A present compound (L-3) wherein $R^1$ and $R^2$ represent an iodine atom, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1190).

A present compound (L-3) wherein $R^1$ and $R^2$ represent an iodine atom, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1191).

A present compound (L-3) wherein $R^1$ and $R^2$ represent an iodine atom, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1192).

A present compound (L-3) wherein $R^1$ and $R^2$ represent a methyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1193).

A present compound (L-3) wherein $R^1$ and $R^2$ represent a methyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1194).

A present compound (L-3) wherein $R^1$ and $R^2$ represent a methyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1195).

A present compound (L-3) wherein $R^1$ and $R^2$ represent a methyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1196).

A present compound (L-3) wherein $R^1$ and $R^2$ represent a methyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1197).

A present compound (L-3) wherein $R^1$ and $R^2$ represent a methyl group, $R^5$ represents a methoxy group, $R^3$, $R^6$ and $R^{\eta}$ represent a hydrogen atom, and $R^4$ represents any one substituent indicated in [Table L1] to [Table L3] (hereinafter, referred to as "Compound class SX1198).

A present compound (L-3) wherein $R^1$ and $R^2$ represent a methyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1199).

A present compound (L-3) wherein $R^1$ and $R^2$ represent a methyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1200).

A present compound (L-3) wherein $R^1$ and $R^2$ represent a methyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1201).

A present compound (L-3) wherein $R^1$ and $R^2$ represent a methyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1202).

A present compound (L-3) wherein $R^1$ and $R^2$ represent a methyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1203).

A present compound (L-3) wherein $R^1$ and $R^2$ represent a methyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1204).

A present compound (L-3) wherein $R^1$ and $R^2$ represent a methyl group, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1205).

A present compound (L-3) wherein $R^1$ and $R^2$ represent a methyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1206).

A present compound (L-3) wherein $R^1$ and $R^2$ represent a methyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1207).

A present compound (L-3) wherein $R^1$ and $R^2$ represent a methyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1208).

A present compound (L-3) wherein $R^1$ and $R^2$ represent an ethyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1209).

A present compound (L-3) wherein $R^1$ and $R^2$ represent an ethyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1210).

A present compound (L-3) wherein $R^1$ and $R^2$ represent an ethyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1211).

A present compound (L-3) wherein $R^1$ and $R^2$ represent an ethyl group, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1212).

A present compound (L-3) wherein $R^1$ and $R^2$ represent an ethyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1213).

A present compound (L-3) wherein $R^1$ and $R^2$ represent an ethyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1214).

A present compound (L-3) wherein $R^1$ and $R^2$ represent an ethyl group, $R^5$ represents $CF_3$, $R^3$, $R^6$ and $R^7$ represent a hydrogen atom, and $R^4$ represents any one substituent indicated in [Table Z1] to [Table L3] (hereinafter, referred to as "Compound class SX1215).

A present compound (L-3) wherein $R^1$ and $R^2$ represent an ethyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1216).

A present compound (L-3) wherein $R^1$ and $R^2$ represent an ethyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1217).

A present compound (L-3) wherein $R^1$ and $R^2$ represent an ethyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1218).

A present compound (L-3) wherein $R^1$ and $R^2$ represent an ethyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1219).

A present compound (L-3) wherein $R^1$ and $R^2$ represent an ethyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1220).

A present compound (L-3) wherein $R^1$ and $R^2$ represent an ethyl group, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1221).

A present compound (L-3) wherein $R^1$ and $R^2$ represent an ethyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1222).

A present compound (L-3) wherein $R^1$ and $R^2$ represent an ethyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1223).

A present compound (L-3) wherein $R^1$ and $R^2$ represent an ethyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1224).

A present compound (L-3) wherein $R^1$ and $R^2$ represent $CF_3$, $R^5$ represents a fluorine atom, $R^3$, $R^6$ and $R^7$

represent a hydrogen atom, and $R^4$ represents any one substituent indicated in [Table L1] to [Table L3] (hereinafter, referred to as "Compound class SX1225).

A present compound (L-3) wherein $R^1$ and $R^2$ represent $CF_3$, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1226).

A present compound (L-3) wherein $R^1$ and $R^2$ represent $CF_3$, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1227).

A present compound (L-3) wherein $R^1$ and $R^2$ represent $CF_3$, $R^5$ represents an iodine atom, $R^3$, $R^6$ and $R^7$ represent a hydrogen atom, and $R^4$ represents any one substituent indicated in [Table L1] to [Table L3] (hereinafter, referred to as "Compound class SX1228).

A present compound (L-3) wherein $R^1$ and $R^2$ represent $CF_3$, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1229).

A present compound (L-3) wherein $R^1$ and $R^2$ represent $CF_3$, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1230).

A present compound (L-3) wherein $R^1$ and $R^2$ represent $CF_3$, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1231).

A present compound (L-3) wherein $R^1$ and $R^2$ represent $CF_3$, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1232).

A present compound (L-3) wherein $R^1$ and $R^2$ represent $CF_3$, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1233).

A present compound (L-3) wherein $R^1$ and $R^2$ represent $CF_3$, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1234).

A present compound (L-3) wherein $R^1$ and $R^2$ represent $CF_3$, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1235).

A present compound (L-3) wherein $R^1$ and $R^2$ represent $CF_3$, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1236).

A present compound (L-3) wherein $R^1$ and $R^2$ represent $CF_3$, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1237).

A present compound (L-3) wherein $R^1$ and $R^2$ represent $CF_3$, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1238).

A present compound (L-3) wherein $R^1$ and $R^2$ represent $CF_3$, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1239).

A present compound (L-3) wherein $R^1$ and $R^2$ represent $CF_3$, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1240).

A present compound (L-3) wherein $R^1$ and $R^2$ represent a cyclopropyl group, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1241).

A present compound (L-3) wherein $R^1$ and $R^2$ represent a cyclopropyl group, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1242).

A present compound (L-3) wherein $R^1$ and $R^2$ represent a cyclopropyl group, $R^5$ represents a bromine atom, $R^3$, $R^6$ and $R^7$ represent a hydrogen atom, and $R^4$ represents any one substituent indicated in [Table L1] to [Table L3] (hereinafter, referred to as "Compound class SX1243).

A present compound (L-3) wherein $R^1$ and $R^2$ represent a cyclopropyl group, $R^5$ represents an iodine atom, $R^3$, $R^6$ and $R^7$ represent a hydrogen atom, and $R^4$ represents any one substituent indicated in [Table L1] to [Table L3]

(hereinafter, referred to as "Compound class SX1244).

A present compound (L-3) wherein $R^1$ and $R^2$ represent a cyclopropyl group, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1245).

A present compound (L-3) wherein $R^1$ and $R^2$ represent a cyclopropyl group, $R^5$ represents a methoxy group, $R^3$, $R^6$ and $R^7$ represent a hydrogen atom, and $R^4$ represents any one substituent indicated in [Table L1] to [Table L3] (hereinafter, referred to as "Compound class SX1246).

A present compound (L-3) wherein $R^1$ and $R^2$ represent a cyclopropyl group, $R^5$ represents $CF_3$, $R^3$, $R^6$ and $R^7$ represent a hydrogen atom, and $R^4$ represents any one substituent indicated in [Table L1] to [Table L3] (hereinafter, referred to as "Compound class SX1247).

A present compound (L-3) wherein $R^1$ and $R^2$ represent a cyclopropyl group, $R^5$ represents $OCF_3$, $R^3$, $R^6$ and $R^7$ represent a hydrogen atom, and $R^4$ represents any one substituent indicated in [Table L1] to [Table L3] (hereinafter, referred to as "Compound class SX1248).

A present compound (L-3) wherein $R^1$ and $R^2$ represent a cyclopropyl group, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1249).

A present compound (L-3) wherein $R^1$ and $R^2$ represent a cyclopropyl group, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1250).

A present compound (L-3) wherein $R^1$ and $R^2$ represent a cyclopropyl group, $R^6$ represents a bromine atom, $R^3$, $R^5$ and $R^7$ represent a hydrogen atom, and $R^4$ represents any one substituent indicated in [Table L1] to [Table L3] (hereinafter, referred to as "Compound class SX1251).

A present compound (L-3) wherein $R^1$ and $R^2$ represent a cyclopropyl group, $R^6$ represents an iodine atom, $R^3$, $R^5$ and $R^7$ represent a hydrogen atom, and $R^4$ represents any one substituent indicated in [Table L1] to [Table L3] (hereinafter, referred to as "Compound class SX1252).

A present compound (L-3) wherein $R^1$ and $R^2$ represent a cyclopropyl group, $R^5$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1253).

A present compound (L-3) wherein $R^1$ and $R^2$ represent a cyclopropyl group, $R^6$ represents a methoxy group, $R^3$, $R^5$ and $R^7$ represent a hydrogen atom, and $R^4$ represents any one substituent indicated in [Table L1] to [Table L3] (hereinafter, referred to as "Compound class SX1254).

A present compound (L-3) wherein $R^1$ and $R^2$ represent a cyclopropyl group, $R^6$ represents $CF_3$, $R^3$, $R^5$ and $R^7$ represent a hydrogen atom, and $R^4$ represents any one substituent indicated in [Table L1] to [Table L3] (hereinafter, referred to as "Compound class SX1255).

A present compound (L-3) wherein $R^1$ and $R^2$ represent a cyclopropyl group, $R^6$ represents $OCF_3$, $R^3$, $R^5$ and $R^7$ represent a hydrogen atom, and $R^4$ represents any one substituent indicated in [Table L1] to [Table L3] (hereinafter, referred to as "Compound class SX1256).

A present compound (L-3) wherein $R^1$ represents a chlorine atom, $R^2$ represents a group selected from a hydrogen atom, a fluorine atom, a chlorine atom, a bromine atom, an iodine atom, a methyl group, an ethyl group, a propyl group, an isopropyl group, a cyclopropyl group, or $CF_3$, and $R^1$, $R^2$, $R^3$, $R^4$, and $R^5$ represent a hydrogen atom (hereinafter, referred to as "Compound class SX1257).

A present compound (L-1) wherein $R^1$ represents a chlorine atom, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1258).

A present compound (L-1) wherein $R^1$ represents a bromine atom, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1259).

A present compound (L-1) wherein $R^1$ represents a methoxy group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX1260).

A present compound (L-1) wherein $R^1$ represents a methoxy group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1261).

A present compound (L-1) wherein $R^1$ represents a methoxy group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1262).

A present compound (L-1) wherein $R^1$ represents an ethoxy group, $R^2$ represents a hydrogen atom, $R^3$, $R^5$, $R^6$ and $R^7$ represent a hydrogen atom, and $R^4$ represents any one substituent indicated in [Table Z1] to [Table L3]

(hereinafter, referred to as "Compound class SX1263).

A present compound (L-1) wherein $R^1$ represents an ethoxy group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1264).

A present compound (Z-1) wherein $R^1$ represents an ethoxy group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1265).

A present compound (L-1) wherein $R^1$ represents a propoxy group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX1266).

A present compound (L-1) wherein $R^1$ represents a propoxy group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1267).

A present compound (L-1) wherein $R^1$ represents a propoxy group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1268).

A present compound (L-1) wherein $R^1$ represents an isopropoxy group, $R^2$ represents a hydrogen atom, $R^3$, $R^5$, $R^6$ and $R^7$ represent a hydrogen atom, and $R^4$ represents any one substituent indicated in [Table L1] to [Table L3] (hereinafter, referred to as "Compound class SX1269).

A present compound (L-1) wherein $R^1$ represents an isopropoxy group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1270).

A present compound (L-1) wherein $R^1$ represents an isopropoxy group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1271).

A present compound (L-1) wherein $R^1$ represents a methylsulfanyl group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1272).

A present compound (L-1) wherein $R^1$ represents a methylsulfanyl group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1273).

A present compound (L-1) wherein $R^1$ represents a methylsulfinyl group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1274).

A present compound (Z-1) wherein $R^1$ represents a methylsulfinyl group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1275).

A present compound (L-1) wherein $R^1$ represents a methylsulfonyl group, $R^2$ represents a fluorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1275).

A present compound (L-1) wherein $R^1$ represents a methylsulfonyl group, $R^2$ represents a chlorine atom, $R^3$, $R^5$, $R^6$ and $R^7$ represent a hydrogen atom, and $R^4$ represents any one substituent indicated in [Table L1] to [Table L3] (hereinafter, referred to as "Compound class SX1277).

A present compound (Z-1) wherein $R^1$ represents a chlorine atom, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1278).

A present compound (L-1) wherein $R^1$ represents a bromine atom, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1279).

A present compound (L-1) wherein $R^1$ represents a methoxy group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1280).

A present compound (L-1) wherein $R^1$ represents a methoxy group, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1281).

A present compound (L-1) wherein $R^1$ represents a methoxy group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1282).

A present compound (L-1) wherein $R^1$ represents an ethoxy group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1283).

A present compound (L-1) wherein $R^1$ represents an ethoxy group, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1284).

A present compound (L-1) wherein $R^1$ represents an ethoxy group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1285).

A present compound (L-1) wherein $R^1$ represents a propoxy group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1286) .

A present compound (L-1) wherein $R^1$ represents a propoxy group, $R^2$ represents a fluorine atom, $R^5$ represents a fluorine atom, $R^3$, $R^6$ and $R^7$ represent a hydrogen atom, and $R^4$ represents any one substituent indicated in [Table L1) to [Table L3] (hereinafter, referred to as "Compound class SX1287).

A present compound (L-1) wherein $R^1$ represents a propoxy group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1288).

A present compound (L-1) wherein $R^1$ represents an isopropoxy group, $R^2$ represents a hydrogen atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1289).

A present compound (L-1) wherein $R^1$ represents an isopropoxy group, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1290).

A present compound (L-1) wherein $R^1$ represents an isopropoxy group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1291).

A present compound (L-1) wherein $R^1$ represents a methylsulfanyl group, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1292).

A present compound (L-1) wherein $R^1$ represents a methylsulfanyl group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1293).

A present compound (L-1) wherein $R^1$ represents a methylsulfinyl group, $R^2$ represents a fluorine atom, $R^5$ represents a fluorine atom, $R^3$, $R^6$ and $R^7$ represent a hydrogen atom, and $R^4$ represents any one substituent indicated in [Table L1) to [Table L3] (hereinafter, referred to as "Compound class SX1294).

A present compound (L-1) wherein $R^1$ represents a methylsulfinyl group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1295).

A present compound (L-1) wherein $R^1$ represents a methylsulfonyl group, $R^2$ represents a fluorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1296).

A present compound (L-1) wherein $R^1$ represents a methylsulfonyl group, $R^2$ represents a chlorine atom, $R^5$ represents a fluorine atom, $R^3$, $R^6$ and $R^7$ represent a hydrogen atom, and $R^4$ represents any one substituent indicated in [Table L1] to [Table L3] (hereinafter, referred to as "Compound class SX1297).

A present compound (L-1) wherein $R^1$ represents a chlorine atom, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1298) .

A present compound (L-1) wherein $R^1$ represents a bromine atom, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1299) .

A present compound (L-1) wherein $R^1$ represents a methoxy group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1300).

A present compound (L-1) wherein $R^1$ represents a methoxy group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1301).

A present compound (L-1) wherein $R^1$ represents a methoxy group, $R^2$ represents a chlorine atom, $R^5$ represents

a chlorine atom, $R^3$, $R^6$ and $R^7$ represent a hydrogen atom, and $R^4$ represents any one substituent indicated in [Table L1] to [Table L3] (hereinafter, referred to as "Compound class SX1302).

A present compound (L-1) wherein $R^1$ represents an ethoxy group, $R^2$ represents a hydrogen atom, $R^5$ represents a chlorine atom, $R^3$, $R^6$ and $R^7$ represent a hydrogen atom, and $R^4$ represents any one substituent indicated in [Table L1) to [Table L3] (hereinafter, referred to as "Compound class SX1303).

A present compound (Z-1) wherein $R^1$ represents an ethoxy group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1304) .

A present compound (L-1) wherein $R^1$ represents an ethoxy group, $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1305) .

A present compound (L-1) wherein $R^1$ represents a propoxy group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1306).

A present compound (L-1) wherein $R^1$ represents a propoxy group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1307).

A present compound (L-1) wherein $R^1$ represents a propoxy group, $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1308).

A present compound (L-1) wherein $R^1$ represents an isopropoxy group, $R^2$ represents a hydrogen atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1309).

A present compound (L-1) wherein $R^1$ represents an isopropoxy group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1310).

A present compound (L-1) wherein $R^1$ represents an isopropoxy group, $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1311).

A present compound (L-1) wherein $R^1$ represents a methylsulfanyl group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1312).

A present compound (L-1) wherein $R^1$ represents a methylsulfanyl group, $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1313).

A present compound (L-1) wherein $R^1$ represents a methylsulfinyl group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1314).

A present compound (L-1) wherein $R^1$ represents a methylsulfinyl group, $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1315).

A present compound (L-1) wherein $R^1$ represents a methylsulfonyl group, $R^2$ represents a fluorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1316).

A present compound (L-1) wherein $R^1$ represents a methylsulfonyl group, $R^2$ represents a chlorine atom, $R^5$ represents a chlorine atom, $R^3$, $R^6$ and $R^7$ represent a hydrogen atom, and $R^4$ represents any one substituent indicated in [Table L1] to [Table L3] (hereinafter, referred to as "Compound class SX1317).

A present compound (L-1) wherein $R^1$ represents a chlorine atom, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1318).

A present compound (L-1) wherein $R^1$ represents a bromine atom, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1319) .

A present compound (L-1) wherein $R^1$ represents a methoxy group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1320) .

A present compound (L-1) wherein $R^1$ represents a methoxy group, $R^2$ represents a fluorine atom, $R^5$ represents a methyl group, $R^3$, $R^6$ and $R^7$ represent a hydrogen atom, and $R^4$ represents any one substituent indicated in [Table

L1] to [Table L3] (hereinafter, referred to as "Compound class SX1321) .

A present compound (L-1) wherein $R^1$ represents a methoxy group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1322) .

A present compound (L-1) wherein $R^1$ represents an ethoxy group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1323) .

A present compound (L-1) wherein $R^1$ represents an ethoxy group, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1324) .

A present compound (L-1) wherein $R^1$ represents an ethoxy group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1325) .

A present compound (L-1) wherein $R^1$ represents a propoxy group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1326).

A present compound (L-1) wherein $R^1$ represents a propoxy group, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1327).

A present compound (L-1) wherein $R^1$ represents a propoxy group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1328).

A present compound (L-1) wherein $R^1$ represents an isopropoxy group, $R^2$ represents a hydrogen atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1329).

A present compound (L-1) wherein $R^1$ represents an isopropoxy group, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1330).

A present compound (L-1) wherein $R^1$ represents an isopropoxy group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1331).

A present compound (L-1) wherein $R^1$ represents a methylsulfanyl group, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1332).

A present compound (L-1) wherein $R^1$ represents a methylsulfanyl group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1333).

A present compound (L-1) wherein $R^1$ represents a methylsulfinyl group, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1334).

A present compound (L-1) wherein $R^1$ represents a methylsulfinyl group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1335).

A present compound (L-1) wherein $R^1$ represents a methylsulfonyl group, $R^2$ represents a fluorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1336).

A present compound (Z-1) wherein $R^1$ represents a methylsulfonyl group, $R^2$ represents a chlorine atom, $R^5$ represents a methyl group, $R^3$, $R^6$ and $R^7$ represent a hydrogen atom, and $R^4$ represents any one substituent indicated in [Table L1] to [Table L3] (hereinafter, referred to as "Compound class SX1337) .

A present compound (L-1) wherein $R^1$ represents a chlorine atom, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1338).

A present compound (L-1) wherein $R^1$ represents a bromine atom, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1339) .

A present compound (L-1) wherein $R^1$ represents a methoxy group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1340).

A present compound (L-1) wherein $R^1$ represents a methoxy group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1341).

A present compound (L-1) wherein $R^1$ represents a methoxy group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1342).

A present compound (L-1) wherein $R^1$ represents an ethoxy group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1343).

A present compound (L-1) wherein $R^1$ represents an ethoxy group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1344).

A present compound (L-1) wherein $R^1$ represents an ethoxy group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1345).

A present compound (L-1) wherein $R^1$ represents a propoxy group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1346).

A present compound (L-1) wherein $R^1$ represents a propoxy group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1347).

A present compound (L-1) wherein $R^1$ represents a propoxy group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1348) .

A present compound (L-1) wherein $R^1$ represents an isopropoxy group, $R^2$ represents a hydrogen atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1349).

A present compound (L-1) wherein $R^1$ represents an isopropoxy group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1350).

A present compound (L-1) wherein $R^1$ represents an isopropoxy group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1351).

A present compound (L-1) wherein $R^1$ represents a methylsulfanyl group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1352).

A present compound (L-1) wherein $R^1$ represents a methylsulfanyl group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1353).

A present compound (L-1) wherein $R^1$ represents a methylsulfinyl group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1354).

A present compound (L-1) wherein $R^1$ represents a methylsulfinyl group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1355).

A present compound (L-1) wherein $R^1$ represents a methylsulfonyl group, $R^2$ represents a fluorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1356).

A present compound (L-1) wherein $R^1$ represents a methylsulfonyl group, $R^2$ represents a chlorine atom, $R^6$ represents a fluorine atom, $R^3$, $R^5$ and $R^7$ represent a hydrogen atom, and $R^4$ represents any one substituent indicated in [Table L1] to [Table L3] (hereinafter, referred to as "Compound class SX1357).

A present compound (L-1) wherein $R^1$ represents a chlorine atom, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1358).

A present compound (L-1) wherein $R^1$ represents a bromine atom, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1359).

A present compound (L-1) wherein $R^1$ represents a methoxy group, $R^2$ represents a hydrogen atom, $R^6$ rep-

resents a chlorine atom, $R^3$, $R^5$ and $R^7$ represent a hydrogen atom, and $R^4$ represents any one substituent indicated in [Table L1] to [Table L3] (hereinafter, referred to as "Compound class SX1360) .

A present compound (L-1) wherein $R^1$ represents a methoxy group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1361).

A present compound (L-1) wherein $R^1$ represents a methoxy group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1362).

A present compound (L-1) wherein $R^1$ represents an ethoxy group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1363).

A present compound (L-1) wherein $R^1$ represents an ethoxy group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1364).

A present compound (L-1) wherein $R^1$ represents an ethoxy group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1365).

A present compound (L-1) wherein $R^1$ represents a propoxy group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1366).

A present compound (L-1) wherein $R^1$ represents a propoxy group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1367).

A present compound (L-1) wherein $R^1$ represents a propoxy group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1368).

A present compound (L-1) wherein $R^1$ represents an isopropoxy group, $R^2$ represents a hydrogen atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1369).

A present compound (L-1) wherein $R^1$ represents an isopropoxy group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1370).

A present compound (L-1) wherein $R^1$ represents an isopropoxy group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1371).

A present compound (L-1) wherein $R^1$ represents a methylsulfanyl group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1372).

A present compound (L-1) wherein $R^1$ represents a methylsulfanyl group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1373).

A present compound (L-1) wherein $R^1$ represents a methylsulfinyl group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1374).

A present compound (L-1) wherein $R^1$ represents a methylsulfinyl group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1375).

A present compound (L-1) wherein $R^1$ represents a methylsulfonyl group, $R^2$ represents a fluorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1376).

A present compound (L-1) wherein $R^1$ represents a methylsulfonyl group, $R^2$ represents a chlorine atom, $R^6$ represents a chlorine atom, $R^3$, $R^5$ and $R^7$ represent a hydrogen atom, and $R^4$ represents any one substituent indicated in [Table L1] to [Table L3] (hereinafter, referred to as "Compound class SX1377).

A present compound (L-1) wherein $R^1$ represents a chlorine atom, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1378).

A present compound (L-1) wherein $R^1$ represents a bromine atom, $R^2$ represents a fluorine atom, $R^6$ represents a methyl group, $R^3$, $R^5$ and $R^7$ represent a hydrogen atom, and $R^4$ represents any one substituent indicated in [Table

L1] to [Table L3] (hereinafter, referred to as "Compound class SX1379).

A present compound (L-1) wherein $R^1$ represents a methoxy group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1380).

A present compound (L-1) wherein $R^1$ represents a methoxy group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1381).

A present compound (L-1) wherein $R^1$ represents a methoxy group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1382).

A present compound (L-1) wherein $R^1$ represents an ethoxy group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1383).

A present compound (L-1) wherein $R^1$ represents an ethoxy group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1384).

A present compound (L-1) wherein $R^1$ represents an ethoxy group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1385) .

A present compound (L-1) wherein $R^1$ represents a propoxy group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1386) .

A present compound (L-1) wherein $R^1$ represents a propoxy group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1387).

A present compound (L-1) wherein $R^1$ represents a propoxy group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1388).

A present compound (L-1) wherein $R^1$ represents an isopropoxy group, $R^2$ represents a hydrogen atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1389).

A present compound (1-1) wherein $R^1$ represents an isopropoxy group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1390).

A present compound (L-1) wherein $R^1$ represents an isopropoxy group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1391).

A present compound (L-1) wherein $R^1$ represents a methylsulfanyl group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1392).

A present compound (L-1) wherein $R^1$ represents a methylsulfanyl group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1393).

A present compound (L-1) wherein $R^1$ represents a methylsulfinyl group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1394).

A present compound (Z-1) wherein $R^1$ represents a methylsulfinyl group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1395).

A present compound (L-1) wherein $R^1$ represents a methylsulfonyl group, $R^2$ represents a fluorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1396).

A present compound (L-1) wherein $R^1$ represents a methylsulfonyl group, $R^2$ represents a chlorine atom, $R^6$ represents a methyl group, $R^3$, $R^5$ and $R^7$ represent a hydrogen atom, and $R^4$ represents any one substituent indicated in [Table L1] to [Table L3] (hereinafter, referred to as "Compound class SX1397).

Next, the formulation examples of the present compounds (including the compounds of the present invention) are shown below. In the formulation examples, the "parts" represents "part by weight" unless otherwise specified. The present compound S represents the compounds described in the Compound Classes SX1 to SX1397.

Formulation Example 1

Thirty five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

Formulation Example 2

Fifty (50) parts of any one of the present compound S, 3 parts of calcium lignin sulfonate, 2 parts of sodium lauryl sulfate, and 45 parts of silica are well mixed-grinding to obtain a formulation.

Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the mixture is well kneaded and is then granulated with a granulator and dried to obtain a formulation.

Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Formulation Example 6

Zero point one (0.1) parts of any one of the present compound S and 39.9 parts of kerosene are dissolved while mixing, and the mixture is placed in an aerosol container, and 60 parts of liquefied petroleum gas (mixture of propane, butane and isobutane; saturated vapor pressure: 0.47 MPa (25°C)) is filled in the container to obtain a formulation.

Formulation Example 7

Zero point two (0.2) parts of any one of the present compound S, 50 parts of pyrethrum extract dreg powder, 30 parts of Machilus thunbergii powder, and 19.8 parts of wood powder are mixed, and an appropriate amount of water is added thereto, and the mixture is well kneaded, and is extracted with an extruder into a plate-like sheet, and the resulting sheet is made a spiral-like form thereof with a punching machine to obtain a formulation.
Next, an efficacy of the present compound (including the compound of the present invention) on controlling resistant harmful arthropods is shown by Test examples. The following tests were conducted at 25°C.

Test Method 1

Test compounds are made to a formulation according to a similar method to that described in the Formulation example 1, and thereto is added water containing 0.03 v/v% of Shindain (registered trademark) to prepare a diluted solution containing a prescribed concentration of the test compound.
Cucumber (*cucumber sativus*) seedling (on the developmental stage of the second true leaf) is planted in a cup, and approximately 30 resistant cotton aphids (*Aphis gossypii*) (all stages of life) (lineages collected in Miyazaki prefecture in 2012, screened with imidacloprid). After one day, the diluted solutions are sprayed into the seedling at a ratio of 10 mL/seedling. After 5 days, the number of the surviving insects is examined and the controlling value is calculated by the following equation.

$$\text{Controlling value } (\%) = \{1 - (Cb \times Tai)/(Cai \times Tb)\} \times 100$$

wherein the symbols in the equation represent the following descriptions.

Cb: Number of the test insects in untreated group;
Cai: Number of the surviving insects at the time of the examination in untreated group;
Tb: Number of the test insects in treated group;
Tai: Number of the surviving insects at the time of the examination in treated group;

Here the "untreated group" represents a group where a similar treatment procedure to that of treated group except not using the test compound is done.

Test Example 1-1

The test was conducted according to the Test method 1 by making the prescribed concentration 500 ppm and using the below-mentioned present compound and the compound of the present invention as a test compound, and as the result of the test, the below-mentioned present compounds and the compounds of the present invention showed 90% or more as the controlling value.

Present compound Nos:1, 2, 3, 4, 5, 6, 7, 8, 9, 10, 11, 12, 13, 14, 15, 16, 17, 18, 21, 24, 29, 30, 32, 35, 37, 38, 40, 41, 42, 44, 45, 47, 49, 50, 67, 68, 73;
Compound Nos of the present invention: 19, 20, 22, 23, 27, 31, 36, 39, 43, 46, 48, 51, 52, 53, 54, 55, 56, 57, 58, 59, 60, 61, 62, 63, 64, 65, 66, 69, 70, 71, 72, 74, 75, 76, 77, 78

Test Example 1-2

The test is conducted according to the Test method 1 by making the prescribed concentration 200 ppm and using the below-mentioned present compounds (including the Compounds of the present invention) as a test compound to examine an effect on controlling a resistant cotton aphids (*Aphis gossypii*).

Test Method 2

Test compounds are made to a formulation according to a similar method to that described in the Formulation example 5, and thereto is added water containing 0.03 v/v% of Shindain (registered trademark) to prepare a diluted solution containing a prescribed concentration of the test compound.
Rice (Oryza sativa) seedling (on the developmental stage of the second true leaf) is planted in a container, and the diluted solutions are sprayed into the seedling in a ratio of 10 mL/seedling. Thereafter, 20 of 3rd instar larvae of resistant brown planthoppers (*Nilaparvata lugens*) (lineages collected in Kagoshima prefecture in 2018, screened with imidacloprid) are released onto the rice leaves. After 6 days, the number of the surviving insects is examined and the mortality is calculated by the following equation.

Mortality (%) = {1- the number of the surviving insects/20} × 100

Test Example 2-1

The test was conducted according to the Test method 2 by making the prescribed concentration 500 ppm and using the below-mentioned present compound and the compound of the present invention as a test compound, and, as the result of the test, the below-mentioned present compounds and the compounds of the present invention showed 90% or more as the controlling value.

Present compound Nos:1, 2, 3, 4, 5, 6, 7, 8, 9, 10, 11, 12, 13, 14, 15, 16, 17, 18, 21, 24, 29, 30, 32, 35, 37, 38, 40, 41, 42, 44, 45, 47, 49, 50, 67, 68, 73;
Compound Nos of the present invention: 19, 20, 22, 23, 27, 31, 36, 39, 43, 46, 48, 51, 52, 53, 54, 55, 56, 57, 58, 59, 60, 61, 52, 63, 64, 65, 66, 69, 70, 71, 74, 75, 76, 77, 78

Test Example 2-2

The test is conducted according to the Test method 2 by making the prescribed concentration 200 ppm and

using the below-mentioned present compounds (including the Compounds of the present invention) as a test compound to examine an effect on controlling a resistant brown planthoppers (*Nilaparvata lugens*).

Test Method 3

Test compounds are made to a formulation according to a similar method to that described in the Formulation example 5, and thereto is added water containing 0.03 v/v% of Shindain (registered trademark) to prepare a diluted solution containing a prescribed concentration of the test compound.
Rice (Oryza sativa) seedling (on the developmental stage of the second true leaf) is planted in a container, and the diluted solutions are sprayed into the seedling in a ratio of 10 mL/seedling. Thereafter, 20 of 3rd instar larvae of resistant white-backed planthopper (*Sogatella furcifera*) (lineages collected in Kagoshima prefecture in 2018, screened with fipronil) are released onto the rice leaves. After 6 days, the number of the surviving insects is examined and the mortality is calculated by the following equation.

Mortality (%) = {1- the number of the surviving insects/20} $\times$ 100

Test Example 3-1

The test is conducted according to the Test method 2 by making the prescribed concentration 500 ppm and using the below-mentioned present compounds (including the Compounds of the present invention) as a test compound to examine an effect on controlling a resistant white-backed planthopper (*Sogatella furcifera*).

Test Example 3-2

The test is conducted according to the Test method 3 by making the prescribed concentration 200 ppm and using the below-mentioned present compounds (including the Compounds of the present invention) as a test compound to examine an effect on controlling a resistant white-backed planthopper (*Sogatella furcifera*).

Test Method 4

Test compounds are made to a formulation according to a similar method to that described in the Formulation example 5, and thereto is added water containing 0.03 v/v% of Shindain (registered trademark) to prepare a diluted solution containing a prescribed concentration of the test compound.
Rice (Oryza sativa) seedling (on the developmental stage of the second true leaf) is planted in a container, and the diluted solutions are sprayed into the seedling in a ratio of 10 mL/seedling. Thereafter, 20 of 3rd instar larvae of resistant white-backed planthopper (*Sogatella furcifera*) (lineages collected in Kagoshima prefecture in 2018, screened with imidacloprid and fipronil) are released onto the rice leaves. After 6 days, the number of the surviving insects is examined and the mortality is calculated by the following equation.

Mortality (%) = {1- the number of the surviving insects/20} $\times$ 100

Test Example 4-1

The test is conducted according to the Test method 4 by making the prescribed concentration 500 ppm and using the below-mentioned present compounds (including the Compounds of the present invention) as a test compound to examine an effect on controlling a resistant white-backed planthopper (*Sogatella furcifera*).

Test Example 4-2

The test is conducted according to the Test method 4 by making the prescribed concentration 200 ppm and using the below-mentioned present compounds (including the Compounds of the present invention) as a test compound to examine an effect on controlling a resistant white-backed planthopper (*Sogatella furcifera*).

Test Method 5

Test compounds are made to a formulation according to a similar method to that described in the Formulation example 5, and thereto is added water containing 0.03 v/v% of Shindain (registered trademark) to prepare a diluted solution containing a prescribed concentration of the test compound.

Some adults of Resistant tobacco whiteflies (Bemisia tabaci) (Biotype Q or MED lineages) (lineages collected in Kumamoto prefecture in 2005, screened with imidacloprid) are released on tomato (Lycopersicon esculentum) seedling that is planted in a container, and then spawn for about 24 hours. The seedling are stored for 8 days, and the larvae of tobacco whiteflies (*Bemisia tabaci*) are hatched from the laid eggs. The diluted solutions are sprayed into the seedling in a ratio of 10 mL/seedling. After 7 days, the number of the surviving insects is examined, and the controlling value is calculated by the following equation.

$$\text{Controlling value (\%)} = \{1-(Cb \times Tai)/(Cai \times Tb)\} \times 100$$

wherein the symbols in the formula represent the following descriptions.

Cb: Number of the insects shortly before the treatment in untreated group;
Cai: Number of the surviving insects at the time of the investigation in untreated group;
Tb: Number of the insects shortly before the treatment in treated group;
Tai: Number of the surviving insects at the time of the investigation in treated group;

Here the "untreated group" represents a group where a similar treatment procedure to that of treated group except not using the test compound is done.

Test Example 5-1

The test was conducted according to the Test method 5 by making the prescribed concentration 500 ppm and using the below-mentioned present compound and the compound of the present invention as a test compound, and, as the result of the test, the below-mentioned present compounds and the compounds of the present invention showed 90% or more as the controlling value.
Present compound Nos:1, 2, 3, 4, 5, 6, 7, 8, 9, 10, 11, 12, 13, 14, 15, 16, 17, 18, 21, 24, 29, 30, 32, 35, 37, 40, 41;
Compound Nos of the present invention: 19, 20, 22, 23, 27, 31, 36, 46

Test Example 5-2

The test is conducted according to the Test method 5 by making the prescribed concentration 200 ppm and using the below-mentioned present compounds (including the Compounds of the present invention) as a test compound to examine an effect on controlling a resistant tobacco whiteflies (*Bemisia tabaci*).

Test Method 6

Each 1 mg of the present compounds (including the compounds of the present invention) is dissolved into 10 μL of a mixed solution of xylene, DMF, and a surfactant (xylene : DMF : surfactant = 4 : 4 : 1 (v/v ratio)). Thereto is added water containing 0.02% by volume of a spreader to prepare diluted solution A containing a prescribed concentration of the present compound.

Each 1 mg of the present ingredients is dissolved into 10 μL of a mixed solution of xylene, DMF, and a surfactant (xylene : DMF: surfactant = 4 : 4 : 1 (v/v ratio) . Thereto is added water containing 0.02% by volume of a spreader to prepare diluted solution B containing a prescribed concentration of the present ingredient.

The diluted solution A is mixed with the diluted solution B to prepare diluted solution C.

Leaf discs of Cucumber (*cucumber sativus*) cotyledon (length 1.5 cm) are placed in each well of 24-well microplate. Two (2) apterous adults and 8 larvae of cotton aphids (*Aphis gossypii*) per one well are released and the diluted solution C is sprayed at 20 μL per one well. The group is defined as "treated group". A well that is sprayed with 20 μL of water containing 0.02% by volume of a spreader instead of the diluted solution C is defined as "untreated group".

After drying the diluted solution C, the upper microplate is covered with a film sheet. After 5 days, the number of the surviving insects in each well is examined.

The controlling value is calculated by the following equation.

$$\text{Controlling value (\%)} = \{1 - (\text{Tai})/(\text{Cai})\} \times 100$$

wherein the symbols in the equation represent the following descriptions.

Cai: Number of the surviving insects at the time of the examination in untreated group;
Tai: Number of the surviving insects at the time of the examination in treated group.

Specific diluted solutions C, which can confirm their effect according to the Test method 6, are described in the following 1) to 5).
1) The diluted solution C comprises the combination recited in List A wherein a concentration of the present compound (including the compounds of the present invention) is 200 ppm and a concentration of the present ingredient is 2,000 ppm. In List A, Comp X represents any compound selected from the present compounds 1 to 31.
List A:
Comp X + Clothianidin; Comp X + thiamethoxam; Comp X + imidacloprid; Comp X + thiacloprid; Comp X + flupyradifurone; Comp X + sulfoxaflor; Comp X + triflumezopyrim; Comp X + dicloromezotiaz; Comp X + beta-cyfluthrin; Comp X + tefluthrin; Comp X + fipronil; Comp X + chlorantraniliprole; Comp X + cyantraniliprole; Comp X + tetraniliprole; Comp X + thiodicarb; Comp X + carbofuran; Comp X + fluxametamide; Comp X + afoxolaner; Comp X + fluralaner; Comp X + broflanilide; Comp X + abamectin; Comp X + fluopyram; Comp X + fluensulfone; Comp X + fluazaindolizine; Comp X + tioxazafen; Comp X + flupyrimin; Comp X + Mycorrhizal Fungi; Comp X + Bradyrhizobium japonicum TA-11; Comp X + Bacillus firmus; Comp X + Bacillus firmus 1-1582; Comp X + Bacillus amyloliquefaciens; Comp X + Bacillus amyloliquefaciens FZB42; Comp X + Pasteuria nishizawae; Comp X + Pasteuria nishizawae Pn1; Comp X + Pasteuria penetrans; Comp X + tebuconazole; Comp X + prothioconazole; Comp X + metconazole; Comp X + ipconazole; Comp X + triticonazole; Comp X + difenoconazole; Comp X + imazalil; Comp X + triadimenol; Comp X + tetraconazole; Comp X + flutriafol; Comp X + mandestrobin; Comp X + azoxystrobin; Comp X + pyraclostrobin; Comp X + trifloxystrobin; Comp X + fluoxastrobin; Comp X + picoxystrobin; Comp X + fenamidone; Comp X + metalaxyl; Comp X + metalaxyl-M; Comp X + fludioxonil; Comp X + sedaxane; Comp X + penflufen; Comp X + fluxapyroxad; Comp X + benzovindiflupyr; Comp X + boscalid; Comp X + carboxin; Comp X + penthiopyrad; Comp X + flutolanil; Comp X + captan; Comp X + thiram; Comp X + tolclofos-methyl; Comp X + thiabendazole; Comp X + ethaboxam; Comp X + mancozeb; Comp X + picarbutrazox; Comp X + oxathiapiprolin; Comp X + silthiofam; Comp X + inpyrfluxam.
2) The diluted solution C comprises the combination recited in List A wherein a concentration of the present compound (including the compound of the present invention) is 200 ppm, and a concentration of the present ingredient is 200 ppm.
3) The diluted solution C comprises the combination recited in List A wherein a concentration of the present compound (including the compound of the present invention) is 500 ppm, and a concentration of the present ingredient is 50 ppm.
4) The diluted solution C comprises the combination recited in List A wherein a concentration of the present compound (including the compound of the present invention) is 500 ppm, and a concentration of the present ingredient is 5 ppm.
5) The diluted solution C comprises the combination recited in List A wherein a concentration of the present compound (including the compound of the present invention) is 500 ppm, and a concentration of the present ingredient is 0.5 ppm.

Comparative Test Example 1

The test was conducted according to the Test method 1 by making the prescribed concentration 500 ppm and using the compounds represented by the below-mentioned formula (which was described in JP 2002-114783 A) (hereinafter, referred to as "Comparative compound 1") as a test compound, and as the result of the test, the Comparative compound 1 showed 10% as the mortality.

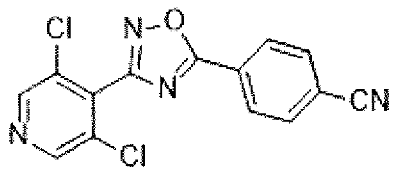

Comparative Compound 1

Comparative Test Example 2

The test was conducted according to the Test method 2 by making the prescribed concentration 500 ppm and using the Comparative compound 1 as a test compound, and as the result of the test, the Comparative compound 1 showed 10% as the mortality.

Comparative Test Example 3

The test was conducted according to the Test method 5 by making the prescribed concentration 500 ppm and using the Comparative compound 1 as a test compound, and as the result of the test, the Comparative compound 1 showed 10% as the mortality.

Industrial Applicability

The present compound (including the compound of the present invention) has excellent control effect on resistant harmful arthropods.

## Claims

1. A method for controlling a resistant harmful arthropod which comprises applying an effective amount of a compound represented by formula (I):

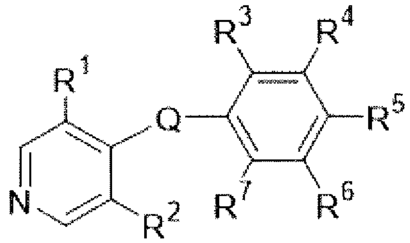

(I)

[wherein

$R^1$ represents a C1-C6 chain hydrocarbon group, a C1-C6 alkoxy group {the C1-C6 chain hydrocarbon group and the C1-C6 alkoxy group may be optionally substituted with one or more substituents selected from Group A}, a C3-C7 cycloalkyl group which may be optionally substituted with one or more substituents selected from Group B, a C3-C7 cycloalkenyl group, a phenyl group, a three- to seven- membered nonaromatic heterocyclic group, a five- or six- membered aromatic heterocyclic group {the C3-C7 cycloalkenyl group, the phenyl group, the three- to seven- membered nonaromatic heterocyclic group, and the five- or six- membered aromatic heterocyclic group may be optionally substituted with one or more substituents selected from Group C}, a nitro group, $NR^8R^9$, $S(O)_mR^{10}$, or a halogen atom,
m is 0, 1 or 2,
$R^2$ represents a C1-C6 chain hydrocarbon group, a C1-C6 alkoxy group {the C1-C6 chain hydrocarbon group and the C1-C6 alkoxy group may be optionally substituted with one or more substituents selected from Group A}, a C3-C7 cycloalkyl group which may be optionally substituted with one or more substituents selected from Group B, a C3-C7 cycloalkenyl group, a phenyl group, a three- to seven- membered nonaromatic heterocyclic group, a five- or six- membered aromatic heterocyclic group {the C3-C7 cycloalkenyl group, the phenyl group, the three- to seven- membered nonaromatic heterocyclic group, and the five- or six- membered aromatic heterocyclic group may be optionally substituted with one or more substituents selected from Group C}, a nitro group, $NR^{8a}R^{9a}$, $S(O)_nR^{10a}$, a halogen atom, or a hydrogen atom,
n is 0, 1 or 2,
$R^3$ and $R^7$ are identical or different from each other and each represents a C1-C6 chain hydrocarbon group which may be optionally substituted with one or more halogen atoms, a halogen atom, or a hydrogen atom,
$R^4$ and $R^6$ are identical or different from each other and each represents a C1-C6 chain hydrocarbon group, a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group, a C3-C6 cycloalkyl group, a phenyl group, a five- or six-membered aromatic heterocyclic group {the C1-C6 chain hydrocarbon group, the C1-C6 alkoxy group, the C3-C6 alkenyloxy group, the C3-C6 alkynyloxy group, the C1-C6 alkylsulfanyl group, the C1-C6 alkylsulfinyl group, the C1-C6 alkylsulfonyl group, the C3-C6 cycloalkyl group, the phenyl group, and the five- or six-membered aromatic heterocyclic group may be optionally substituted with one or more halogen atoms}, a cyano group, a nitro group, a halogen atom, or a hydrogen atom,

$R^5$ represents a C1-C6 chain hydrocarbon group, a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C3-C6 alkenyloxy group, the C3-C6 alkynyloxy group, the C1-C6 alkylsulfanyl group, the C1-C6 alkylsulfinyl group, and the C1-C6 alkylsulfonyl group may be optionally substituted with one or more halogen atoms}, a nitro group, a halogen atom, or a hydrogen atom,

$R^8$ and $R^{8a}$ are identical or different from each other and each represents a C1-C6 chain hydrocarbon group which may be optionally substituted with one or more halogen atoms, or a hydrogen atom,

$R^9$ and $R^{9a}$ are identical or different from each other and each represents a C1-C6 chain hydrocarbon group, a C3-C7 cycloalkyl group, a C3-C7 cycloalkenyl group {the C1-C6 chain hydrocarbon group, the C3-C7 cycloalkyl group and the C3-C7 cycloalkenyl group may be optionally substituted with one or more substituents selected from Group C}, or a hydrogen atom,

$R^{10}$ and $R^{10a}$ are identical or different from each other and each represents a C1-C6 chain hydrocarbon group which may be optionally substituted with one or more halogen atoms,

Q represents a group represented by Q1, a group represented by Q2, a group represented by Q3, or a group represented by Q4 (where # represents a binding site to a pyridine ring, and • represents a binding site to a benzene ring),

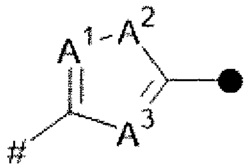
Q1

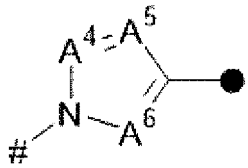
Q2

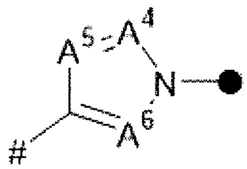
Q3

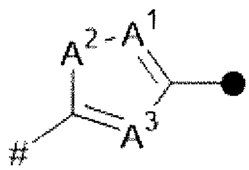
Q4

$A^1$ represents a nitrogen atom, or $CR^{11}$,

$A^2$ represents an oxygen atom, a sulfur atom, or $NR^{12}$,

$A^3$ represents a nitrogen atom, or $CR^{13}$,

$A^4$ represents a nitrogen atom, or $CR^{14}$,

$A^5$ represents a nitrogen atom, or $CR^{15}$,

$A^6$ represents a nitrogen atom, or $CR^{16}$,

$R^{11}$, $R^{13}$, $R^{14}$, $R^{15}$, and $R^{16}$ are identical or different from each other and each represents a C1-C6 chain hydrocarbon group which may be optionally substituted with one or more halogen atoms, a halogen atom, or a hydrogen atom,

$R^{12}$ represents a C1-C6 chain hydrocarbon group which may be optionally substituted with one or more halogen atoms, or a hydrogen atom.

Group A is a group consisting of a C1-C6 alkoxy group, a C3-C6 alkenyloxy group, a C3-C6 alkynyloxy group, a C1-C6 alkylsulfanyl group, a C1-C6 alkylsulfinyl group, a C1-C6 alkylsulfonyl group, a C3-C6 cycloalkyl group {the C1-C6 alkoxy group, the C3-C6 alkenyloxy group, the C3-C6 alkynyloxy group, the C1-C6 alkylsulfanyl group, the C1-C6 alkylsulfinyl group, the C1-C6 alkylsulfonyl group, and the C3-C6 cycloalkyl group may be optionally substituted with one or more halogen atoms}, a cyano group, and a halogen atom.

Group B is a group consisting of a C1-C6 chain hydrocarbon group, a C1-C6 alkoxy group, a C3-C6 alkenyloxy group, a C3-C6 alkynyloxy group {the C1-C6 chain hydrocarbon group, the C1-C6 alkoxy group, the C3-C6 alkenyloxy group, and the C3-C6 alkynyloxy group may be optionally substituted with one or more halogen atoms}, a hydroxy group, a cyano group, a nitro group, and a halogen atom.

Group C is a group consisting of a C1-C6 alkyl group which may be optionally substituted with one or more halogen atoms, a cyano group, and a halogen atom.]

, or its N-oxide or a salt thereof
to the resistant harmful arthropod or a habitat of the resistant harmful arthropod.

**2.** The method according to claim 1 wherein in the compound represented by formula (1), or its N-oxide or a salt thereof,

Q represents a group represented by formula Q1 or a group represented by formula Q4,
$A^1$ and $A^3$ represents a nitrogen atom,
$A^2$ represents an oxygen atom or $NR^{12}$,
$R^1$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_mR^{10}$, or a halogen atom,
$R^2$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_nR^{10a}$, a halogen atom, or a hydrogen atom.

**3.** The method according to claim 1 or claim 2 wherein in the compound represented by formula (I), or its N-oxide or a salt thereof,
Q represents a group represented by formula Q1.

**4.** The method according to claim 1 wherein in the compound represented by formula (I), or its N-oxide or a salt thereof,

Q represents a group represented by formula Q2 or a group represented by formula Q3,
$A^4$ represents $CR^{14}$,
$A^5$ represents a nitrogen atom or $CR^{15}$,
$A^6$ represents a nitrogen atom,
$R^1$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_mR^{10}$, or a halogen atom,
$R^2$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, a nitro group, $S(O)_nR^{10a}$, a halogen atom, or a hydrogen atom.

**5.** The method according to claim 1 or claim 4 wherein in the compound represented by formula (I), or its N-oxide or a salt thereof,
Q represents a group represented by formula Q2.

**6.** A compound represented by formula (11):

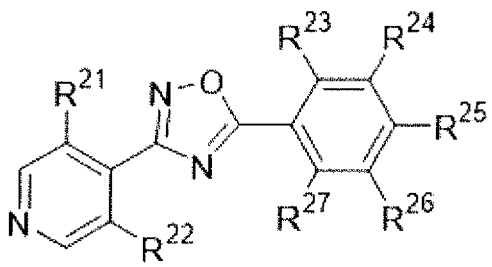

( II )

[wherein

$R^{21}$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, $S(O)_pR^{28}$ or a halogen atom,
$R^{22}$ represents a C1-C6 chain hydrocarbon group, a C1-C6 alkoxy group, a C3-C7 cycloalkyl group {the C1-C6 chain hydrocarbon group, the C1-C6 alkoxy group, and the C3-C7 cycloalkyl group may be optionally substituted with one or more halogen atoms}, $S(O)_qR^{29}$, a halogen atom, or a hydrogen atom,
$R^{23}$, $R^{25}$ and $R^{27}$ are identical or different from each other and each represents a fluorine atom or a hydrogen atom,
$R^{24}$ represents a C1-C6 alkoxy group which is substituted with one or more halogen atoms, or a fluorine atom,
$R^{26}$ represents a halogen atom, or a hydrogen atom,
$R^{28}$ and $R^{29}$ are identical to or different from each other and each represents a C1-C6 chain hydrocarbon group

which may be optionally substituted with one or more halogen atoms, and p and q each independently is 0, 1 or 2] or its N-oxide or a salt thereof.

**7.** The compound according to claim 6 wherein

$R^{21}$ represents a C1-C6 chain hydrocarbon group which may be optionally substituted with one or more halogen atoms, or a halogen atom,
$R^{22}$ represents a C1-C6 chain hydrocarbon group which may be optionally substituted with one or more halogen atoms, a halogen atom, or a hydrogen atom,
$R^{23}$, $R^{25}$ and $R^{27}$ are identical or different from each other and each represents a fluorine atom or a hydrogen atom,
$R^{24}$ represents a C1-C6 alkoxy group which is substituted with one or more halogen atoms, or a fluorine atom, and
$R^{26}$ represents a halogen atom, or a hydrogen atom,

or its N-oxide, or a salt thereof.

**8.** A compound represented by formula (II-a):

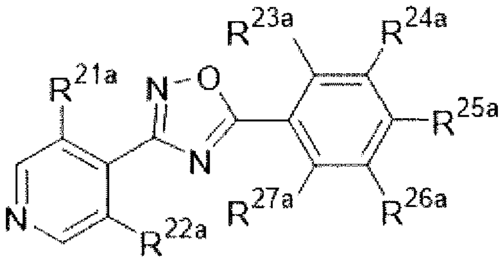

(II-a)

[wherein

$R^{21a}$ represents a C1-C6 alkoxy group, a C3-C7 cycloalkyl group {the C1-C6 alkoxy group, and the C3-C7 cycloalkyl group may be optionally substituted with one or more halogen atoms}, or $S(O)_rR^{28a}$,
$R^{22a}$ represents a C1-C6 alkoxy group, a C3-C7 cycloalkyl group {the C1-C6 alkoxy group, and the C3-C7 cycloalkyl group may be optionally substituted with one or more halogen atoms}, $S(O)_sR^{29a}$ or a halogen atom,
$R^{23a}$, $R^{25a}$ and $R^{27a}$ are identical or different from each other and each represents a halogen atom or a hydrogen atom,
$R^{24a}$ represents a C1-C6 chain hydrocarbon group which may be optionally substituted with one or more halogen atoms, a C1-C6 alkoxy group which is substituted with one or more halogen atoms, or a halogen atom,
$R^{26a}$ represents a C1-C6 chain hydrocarbon group which may be optionally substituted with one or more halogen atoms, a halogen atom, or a hydrogen atom,
$R^{28a}$ and $R^{29a}$ are identical or different from each other and each represents a C1-C6 chain hydrocarbon group which may be optionally substituted with one or more halogen atoms, and
r and s each independently represents 0, 1 or 2]

, its N-oxide or a salt thereof.

**9.** A compound represented by formula (III):

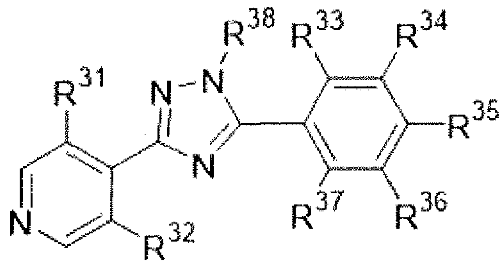

(III)

[wherein

A combination of $R^{31}$, $R^{32}$, $R^{33}$, $R^{34}$, $R^{35}$, $R^{36}$, and $R^{37}$ represents

a combination wherein
$R^{31}$ and $R^{32}$ represent a chlorine atom, and $R^{33}$, $R^{35}$ and $R^{37}$ represent a hydrogen atom,
$R^{34}$ represents a C1-C6 alkoxy group which is substituted with one or more halogen atoms, and
$R^{36}$ represents a halogen atom; or,
a combination wherein
$R^{31}$ represents a C1-C6 chain hydrocarbon group which may be optionally substituted with one or more halogen atoms, or a bromine atom,
$R^{32}$ represents a C1-C6 chain hydrocarbon group which may be optionally substituted with one or more halogen atoms, a bromine atom, or a hydrogen atom,
$R^{33}$, $R^{35}$ and $R^{37}$ are identical or different from each other and each represents a fluorine atom or a hydrogen atom,
$R^{34}$ represents a C1-C6 chain hydrocarbon group which may be optionally substituted with one or more halogen atoms, a C1-C6 alkoxy group which may be optionally substituted with one or more halogen atoms, or a halogen atom, and
$R^{36}$ represents a halogen atom or a hydrogen atom, and

$R^{38}$ represents a C1-C6 chain hydrocarbon group which may be optionally substituted with one or more halogen atoms, or a hydrogen atom],

or its N-oxide, or a salt thereof.

**10.** A compound represented by formula (IV):

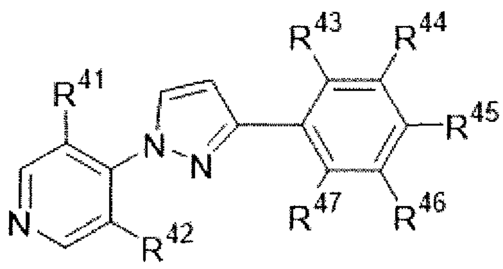

(IV)

[wherein

$R^{41}$ represents a C1-C6 chain hydrocarbon group which may be optionally substituted with one or more halogen atoms, or a halogen atom,
$R^{42}$ represents a C1-C6 chain hydrocarbon group which may be optionally substituted with one or more halogen atoms, a halogen atom, or a hydrogen atom,
$R^{43}$, $R^{45}$ and $R^{47}$ are identical or different from each other and each represents a fluorine atom or a hydrogen atom,
$R^{44}$ represents a C1-C6 chain hydrocarbon group which may be optionally substituted with one or more halogen atoms, a C1-C6 alkoxy group which may be optionally substituted with one or more halogen atoms, a halogen atom, or a hydrogen atom, and
$R^{46}$ represents a halogen atom or a hydrogen atom]

, its N-oxide or a salt thereof.

**11.** A composition comprising the compound according to any one of claims 6 to 10, its N-oxide or a salt thereof, and an inert carrier.

**12.** A composition which comprises one or more ingredients selected from the group consisting of Group (a), Group (b), Group (c) and Group (d), as well as the compound according to any one of claims 6 to 10, or its N-oxide or a salt thereof:

Group (a): a group consisting of an insecticidal ingredient, a miticidal ingredient, and a nematicidal ingredient;
Group (b): a fungicidal ingredient;
Group (c): a plant growth modulating ingredient; and
Group (d): a repellent ingredient.

**13.** A method for controlling a resistant harmful arthropod which comprises applying an effective amount of the compound according to any one of claims 6 to 10, its N-oxide or a salt thereof, or an effective amount of the composition according to claim 12 to the resistant harmful arthropod or a habitat where a resistant harmful arthropod lives.

**14.** The method according to any one of claims 1 to 5 or claim 13 wherein the resistant harmful arthropod is the order of Hemiptera pest.

**15.** The method according to any one of claims 1 to 5 or claim 13 wherein the resistant harmful arthropod is selected from an Aphididae family, a Delphacidae family, or an Aleyrodidae family.

**16.** A seed or vegetative reproduction organ carrying an effective amount of the compound according to any one of claims 6 to 10, its N-oxide or a salt thereof, or an effective amount of the composition according to claim 12.

## INTERNATIONAL SEARCH REPORT

International application No.
**PCT/JP2022/025760**

### A. CLASSIFICATION OF SUBJECT MATTER

***A01N 43/836***(2006.01)i; ***A01M 1/20***(2006.01)i; ***A01P 7/02***(2006.01)i; ***A01P 7/04***(2006.01)i; ***C07D 401/04***(2006.01)i; ***C07D 413/04***(2006.01)i
FI: A01N43/836; A01P7/02; A01P7/04; A01M1/20 A; C07D413/04; C07D401/04

According to International Patent Classification (IPC) or to both national classification and IPC

### B. FIELDS SEARCHED

Minimum documentation searched (classification system followed by classification symbols)

A01N43/836; A01M1/20; A01P7/02; A01P7/04; C07D401/04; C07D413/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | JP 2002-528447 A (DOW AGROSCIENCES LLC) 03 September 2002 (2002-09-03) claims, paragraph [0002], examples | 1-4, 14-15 |
| A | | 5-13, 16 |
| X | WO 2017/094790 A1 (HOKKO CHEMICAL INDUSTRY CO., LTD.) 08 June 2017 (2017-06-08) claims, paragraphs [0003]-[0004], examples | 1, 4, 14-15 |
| A | | 2-3, 5-13, 16 |
| A | JP 2015-525205 A (E.I. DU PONT DE NEMOURS AND COMPANY) 03 September 2015 (2015-09-03) claims, examples | 1-16 |
| A | JP 2002-114783 A (SUMITOMO CHEM CO LTD) 16 April 2002 (2002-04-16) claims, examples | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 September 2022** | **20 September 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/025760**

| Patent document cited in search report | Publication date (day/month/year) | Patent family member(s) | Publication date (day/month/year) |
|---|---|---|---|
| JP 2002-528447 A | 03 September 2002 | US 6413992 B1<br>claims, column 1, examples<br>WO 2000/024735 A1<br>EP 1123287 A1<br>BR 9915534 A | |
| WO 2017/094790 A1 | 08 June 2017 | US 2018/0290986 A1<br>claims, paragraphs [0002]-[0003], examples<br>EP 3385254 A1<br>CN 108368064 A<br>KR 10-2018-0088416 A<br>BR 112018011124 A | |
| JP 2015-525205 A | 03 September 2015 | US 2015/0126364 A1<br>claims, examples<br>WO 2013/173218 A1<br>EP 2850074 A1<br>CN 104302637 A<br>KR 10-2015-0013751 A<br>BR 112014028375 A | |
| JP 2002-114783 A | 16 April 2002 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2021107328 A
- JP 2002114783 A
- WO 2012098416 A1
- US 20180009778 A
- WO 2016121970 A
- WO 2020043866 A
- WO 2012098416 A


### Non-patent literature cited in the description


- FAO Plant Production and Protection Papers. Manual on development and use of FAO and WHO Specifications for pesticides. 2016, 271-276